# EUROPEAN PATENT SPECIFICATION

(11) **EP 4 253 376 B1**
(45) Date of publication and mention of the grant of the patent: **30.07.2025**
(21) Application number: 21897144.8
(22) Date of filing: 26.11.2021
(51) Int. Cl.: C07D 471/04, A61K 31/519, A61P 35/00

(54) **SALT AND CRYSTAL FORM OF NITROGEN-CONTAINING HETEROCYCLIC DERIVATIVE, PREPARATION METHOD THEREFOR AND APPLICATION THEREOF**
SALZ UND KRISTALLFORM EINES STICKSTOFFHALTIGEN HETEROCYCLISCHEN DERIVATS, HERSTELLUNGSVERFAHREN DAFÜR UND ANWENDUNG DAVON
FORME SALINE ET FORME CRISTALLINE D'UN DÉRIVÉ HÉTÉROCYCLIQUE CONTENANT DE L'AZOTE, LEUR PROCÉDÉ DE PRÉPARATION ET LEUR UTILISATION

(30) Priority: 26.11.2020 CN 202011354289; 22.11.2021 CN 202111389216
(43) Date of publication of application: 04.10.2023
(73) Proprietor: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: LIU, Qingxin, Shanghai 201203 (CN); GUO, Linsong, Shanghai 201203 (CN)
(74) Representative: Hoffmann Eitle
(86) International application number: PCT/CN2021/133653
(87) International publication number: WO 2022/111644

(56) References cited:
- WO-A1-2018/217651
- WO-A1-2019/051291
- WO-A1-2019/213516
- WO-A1-2020/233592
- WO-A1-2020/236947
- WO-A1-2020/236948
- WO-A1-2020/239077
- WO-A1-2021/143693
- WO-A1-2021/185233
- WO-A1-2021/236920
- CN-A- 112 390 796
- US-A1- 2019 374 542

## Description

This application claims the priorities of Chinese patent application 2020113542899 filed on November 26, 2020 and Chinese patent application 2021113 892168 filed on November 22, 2021.

### TECHNICAL FIELD

The present disclosure belongs to the field of biomedicine, specifically related to a salt and crystal form of nitrogen-containing heterocyclic derivative, a preparation method therefor and an application thereof.

### BACKGROUND

Rat sarcomas (RAS), encoded by proto-oncogenes HRAS, NRAS, and KRAS, is calssified as 4 proteins, HRAS, NRAS, KRAS4A and KRAS4B, and is a GTP (guanosine triphosphate) binding protein. RAS is located on the inner surface of a cell membrane, upstream of which is receptor tyrosine kinase (RTK), after activation, RAS regulates downstream PI3K, RAF and other signaling pathways, thereby regulating cell growth, survival, migration, differentiation, and other functions.

RAS has two main states in the body: an inactivated state combined with GDP (guanosine diphosphate) and an activated state combined with GTP. Its activity is regulated by two proteins, guanine nucleotide exchange factor (GEF) promotes the release of GDP from the RAS protein, allowing GTP to bind to activate RAS; GTPase activating protein (GAP) activates the GTPase activity of RAS protein, hydrolyzes the GTP bound to RAS protein into GDP, and inactivates the RAS. Under normal circumstances, the RAS protein is in a non-activated state, the conformation changes after mutation, and the RAS is in a continuously activated state, and downstream signaling pathways are also continuously activated, leading to the occurrence of various cancers.

As the first identified oncogene, RAS is the oncogene with the highest mutation rate, accounting for an average of 25% of human cancers. The most common oncegenic mutation in the RAS family is KRAS (85%), while NRAS (12%) and HRAS (3%) are relatively rare. KRAS mutations mainly occur in a series of cancers such as pancreatic cancer (95%), colorectal cancer (52%) and lung cancer (31%), etc. The most common mutation mode of KRAS is point mutation, which mostly occurs in G12, G13 in p-loop (aa 10 to 17) and Q61 in Switch II region (aa 59 to 76), where G12 mutation is the most common (83%). KRAS G12C is one of the most common mutations in non-small cell lung cancer (NSCLC) and colorectal cancer.

Although there are great clinical demands, no drugs that directly target KRAS have been marketed so far, and currently, patients with KRAS mutations in clinical treatment can only be treated with chemotherapy. The difficulty in the development of KRAS inhibitors is mainly due to two factors: first, the structure of RAS protein is smooth, and small molecules are difficult to bind to the protein surface; secondly, the affinity of RAS GTPase for GTP is as high as picomolar (pM) level, and the level of endogenous GTP is high, small molecule drugs are difficult to block the combination of the two. Recent studies have found that after the mutation of Glycine (Gly) at 12-position of KRAS to Cysteine (Cys), the conformation changes and a new pocket is formed for covalent binding of small molecules, which irreversibly locks KRAS G12C in binding to GDP in a non-activated state. Therefore, KRAS G12C inhibitors are expected to be the first drug directly targeting KRAS.

At present, many KRAS G12C inhibitors have entered the clinical research stage, such as AMG 510 developed by Amgen, ARS-3248 developed by Wellspring Biosciences and MTRX849 developed by Mirati, all of which are currently in the clinical Phase I research stage, but none of them have been developed and marketed as KRAS G12C inhibitors yet.

There is no specific target drug for KRAS G12C, and there is a large clinical demand. The KRAS G12C inhibitors with higher selectivity, better activity and better safety have the potential to treat a variety of cancers, and have broad market prospects.

The patent application of Jiangsu Hanson Pharmaceutical Group Co., Ltd. (application No.: PCT/CN2020/093285) disclosed the structure of a series of pyridazine derivative inhibitors. Further KRAS G12C inhibitors have been disclosed, for example, in WO 2019/213516, WO 2018/217651, US 2019/374542 and WO 2019/051291. In the subsequent research and development, in order to make the product easy to process, filter, dry, convenient for storage, long-term stability of the product, and high bioavailability, the present disclosure has carried out a comprehensive study on the salt and crystal form of the above substances, and is committed to obtaining the most suitable crystal form.

### CONTENT OF THE PRESENT INVENTION

The object of the present disclosure is to provide an acid salt of a compound represented by general formula (I): 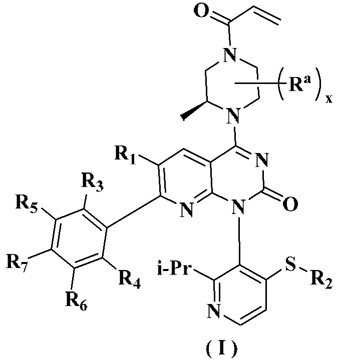 wherein:
R^{a} is each independently selected from hydrogen, deuterium, halogen, amino, hydroxyl, sulfhydryl, cyano, nitro, alkyl, deuterated alkyl, haloalkyl, alkoxy, -SRₐₐ, -C(O)Rₐₐ, -NRₐₐR_{bb}, haloalkoxy or hydroxyalkyl;
R₁ is selected from hydrogen, deuterium, halogen, amino, hydroxyl, cyano, nitro, alkyl, deuterated alkyl, haloalkyl, alkoxy, -SRₐₐ, -C(O)Rₐₐ, -NRₐₐR_{bb}, haloalkoxy or hydroxyalkyl;
R₂ is selected from alkyl;
R₃ is selected from hydrogen, deuterium, halogen, amino, hydroxyl, cyano, nitro, alkyl, deuterated alkyl, haloalkyl, alkoxy, -SRₐₐ, -C(O)Rₐₐ, -NRₐₐR_{bb} or hydroxyalkyl;
R₄ is selected from hydrogen, deuterium, halogen, amino, hydroxyl, cyano, nitro, alkyl, deuterated alkyl, haloalkyl, alkoxy, -SRₐₐ, -C(O)Rₐₐ, -NRₐₐR_{bb} or hydroxyalkyl;
R₅ is selected from hydrogen, deuterium, halogen, amino, hydroxyl, sulfhydryl, cyano, nitro, alkyl, deuterated alkyl, haloalkyl, alkoxy, -SRₐₐ, -C(O)Raa, -NRₐₐR_{bb} or hydroxyalkyl;
R₆ is selected from hydrogen, deuterium, halogen, amino, hydroxyl, sulfhydryl, cyano, nitro, alkyl, deuterated alkyl, haloalkyl, alkoxy, -SRₐₐ, -C(O)Rₐₐ, -NRₐₐR_{bb} or hydroxyalkyl;
R₇ is selected from hydrogen, deuterium, halogen, amino, hydroxyl, sulfhydryl, cyano, nitro, alkyl, deuterated alkyl, haloalkyl, alkoxy, -SRₐₐ, -C(O)Rₐₐ, -NRₐₐR_{bb} or hydroxyalkyl;
Rₐₐ is selected from deuterium, halogen, alkyl, deuterated alkyl or haloalkyl;
R_{bb} is selected from deuterium, halogen, alkyl, deuterated alkyl or haloalkyl; and
x is selected from 0, 1, 2, or 3.

In a preferred embodiment of the present disclosure, in the acid salt of the compound represented by general formula (I), R^{a} is each independently selected from hydrogen, deuterium, halogen, amino, hydroxyl, sulfhydryl, cyano, nitro, C₁₋₆ alkyl, C₁₋₆ deuterated alkyl, C₁₋₆ haloalkyl, C₁₋₆ alkoxy, -SRₐₐ, -C(O)Rₐₐ, -NRₐₐR_{bb}, C₁₋₆ haloalkoxy or C₁₋₆ hydroxyalkyl;
preferably hydrogen, deuterium, halogen, amino, hydroxyl, sulfhydryl, cyano, nitro, C₁₋₃ alkyl, C₁₋₃ deuterated alkyl, C₁₋₃ haloalkyl, C₁₋₃ alkoxy, -SRₐₐ, -C(O)Rₐₐ, -NRₐₐR_{bb}, C₁₋₃ haloalkoxy or C₁₋₃ hydroxyalkyl;
more preferably hydrogen, deuterium, halogen, amino, hydroxyl, sulfhydryl, cyano, nitro, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, propoxy, isopropoxy, methylthio, ethylthio, propylthio, isopropylthio, halomethoxy, haloethoxy, halopropoxy, hydroxymethyl, hydroxyethyl, hydroxypropyl or hydroxyisopropyl;
R₁ is selected from hydrogen, deuterium, halogen, amino, hydroxyl, cyano, nitro, C₁₋₆ alkyl, C₁₋₆ deuterated alkyl, C₁₋₆ haloalkyl, C₁₋₆ alkoxy, -SRₐₐ, -C(O)Rₐₐ, -NRₐₐR_{bb}, C₁₋₆ haloalkoxy or C₁₋₆ hydroxyalkyl;
preferably hydrogen, deuterium, halogen, amino, hydroxyl, cyano, nitro, C₁₋₃ alkyl, C₁₋₃ deuterated alkyl, C₁₋₃ haloalkyl, C₁₋₃ alkoxy, -SRₐₐ, -C(O)Rₐₐ, -NRₐₐR_{bb}, C₁₋₃ haloalkoxy or C₁₋₃ hydroxyalkyl;
more preferably hydrogen, methyl, fluorine, chlorine, amino, hydroxyl or cyano;
R₂ is selected from hydrogen or C₁₋₆ alkyl;
preferably hydrogen or C₁₋₃ alkyl;
more preferably hydrogen, methyl, ethyl, propyl or isopropyl;
R₃ is selected from hydrogen, deuterium, halogen, amino, hydroxyl, cyano, nitro, C₁₋₆ alkyl, C₁₋₆ deuterated alkyl, C₁₋₆ haloalkyl, C₁₋₆ alkoxy, -SRₐₐ, -C(O)Rₐₐ, -NRₐₐR_{bb} or C₁₋₆ hydroxyalkyl;
preferably hydrogen, deuterium, halogen, amino, hydroxyl, cyano, nitro, C₁₋₃ alkyl, C₁₋₃ deuterated alkyl, C₁₋₃ haloalkyl, C₁₋₃ alkoxy, -SRₐₐ or C₁₋₃ hydroxyalkyl;
more preferably hydrogen, deuterium, fluorine, chlorine, bromine, iodine, amino, hydroxyl, cyano, nitro, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, propoxy, isopropoxy, -S(CH₃), hydroxymethyl, hydroxyethyl, hydroxypropyl or hydroxyisopropyl;
R₄ is selected from hydrogen, deuterium, halogen, amino, hydroxyl, cyano, nitro, C₁₋₆ alkyl, C₁₋₆ deuterated alkyl, C₁₋₆ haloalkyl, C₁₋₆ alkoxy, -SRₐₐ, -C(O)Rₐₐ, -NRₐₐR_{bb} or C₁₋₆ hydroxyalkyl;
preferably hydrogen, deuterium, halogen, amino, hydroxyl, cyano, nitro, C₁₋₃ alkyl, C₁₋₃ deuterated alkyl, C₁₋₃ haloalkyl, C₁₋₃ alkoxy, -NRₐₐR_{bb} or C₁₋₃ hydroxyalkyl;
more preferably hydrogen, deuterium, fluorine, chlorine, bromine, iodine, amino, hydroxyl, cyano, nitro, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, propoxy, isopropoxy, -NH(CH₃), -N(CH₃)₂, hydroxymethyl, hydroxyethyl, hydroxypropyl or hydroxyisopropyl;
R₅ is selected from hydrogen, deuterium, halogen, amino, hydroxyl, sulfhydryl, cyano, nitro, C₁₋₆ alkyl, C₁₋₆ deuterated alkyl, C₁₋₆ haloalkyl, C₁₋₆ alkoxy, -SRₐₐ, -C(O)Rₐₐ, -NRₐₐR_{bb} or C₁₋₆ hydroxyalkyl;
preferably hydrogen, deuterium, halogen, amino, hydroxyl, sulfhydryl, cyano, nitro, C₁₋₃ alkyl, C₁₋₃ deuterated alkyl, C₁₋₃ haloalkyl, C₁₋₃ alkoxy or C₁₋₃ hydroxyalkyl;
more preferably hydrogen, deuterium, fluorine, chlorine, bromine, iodine, amino, hydroxyl, sulfhydryl, cyano, nitro, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, propoxy, isopropoxy, hydroxymethyl, hydroxyethyl, hydroxypropyl or hydroxyisopropyl;
R₆ is selected from hydrogen, deuterium, halogen, amino, hydroxyl, sulfhydryl, cyano, nitro, C₁₋₆ alkyl, C₁₋₆ deuterated alkyl, C₁₋₆ haloalkyl, C₁₋₆ alkoxy, -SRₐₐ, -C(O)Rₐₐ, -NRₐₐR_{bb} or C₁₋₆ hydroxyalkyl;
preferably hydrogen, deuterium, halogen, amino, hydroxyl, sulfhydryl, cyano, nitro, C₁₋₃ alkyl, C₁₋₃ deuterated alkyl, C₁₋₃ haloalkyl, C₁₋₃ alkoxy or C₁₋₃ hydroxyalkyl;
more preferably hydrogen, deuterium, fluorine, chlorine, bromine, iodine, amino, hydroxyl, sulfhydryl, cyano, nitro, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, propoxy, isopropoxy, hydroxymethyl, hydroxyethyl, hydroxypropyl or hydroxyisopropyl;
R₇ is selected from hydrogen, deuterium, halogen, amino, hydroxyl, sulfhydryl, cyano, nitro, C₁₋₆ alkyl, C₁₋₆ deuterated alkyl, C₁₋₆ haloalkyl, C₁₋₆ alkoxy, -SRₐₐ, -C(O)Rₐₐ, -NRₐₐR_{bb} or C₁₋₆ hydroxyalkyl;
preferably hydrogen, deuterium, halogen, amino, hydroxyl, sulfhydryl, cyano, nitro, C₁₋₃ alkyl, C₁₋₃ deuterated alkyl, C₁₋₃ haloalkyl, C₁₋₃ alkoxy or C₁₋₃ hydroxyalkyl;
more preferably hydrogen, deuterium, fluorine, chlorine, bromine, iodine, amino, hydroxyl, sulfhydryl, cyano, nitro, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, propoxy, isopropoxy, hydroxymethyl, hydroxyethyl, hydroxypropyl or hydroxyisopropyl;
Rₐₐ is selected from deuterium, halogen, C₁₋₆ alkyl, C₁₋₆ deuterated alkyl or C₁₋₆ haloalkyl;
preferably deuterium, halogen, C₁₋₃ alkyl, C₁₋₃ deuterated alkyl or C₁₋₃ haloalkyl;
more preferably methyl, ethyl, propyl or isopropyl;
R_{bb} is selected from deuterium, halogen, C₁₋₆ alkyl, C₁₋₆ deuterated alkyl or C₁₋₆ haloalkyl;
preferably deuterium, halogen, C₁₋₃ alkyl, C₁₋₃ deuterated alkyl or C₁₋₃ haloalkyl;
more preferably methyl, ethyl, propyl or isopropyl;
x is selected from 0, 1, 2 or 3; preferably, 0, 1, or 2; more preferably, 0 or 1.

In a preferred embodiment of the present disclosure, for the acid salt of the compound, the compound is shown in general formula (II): 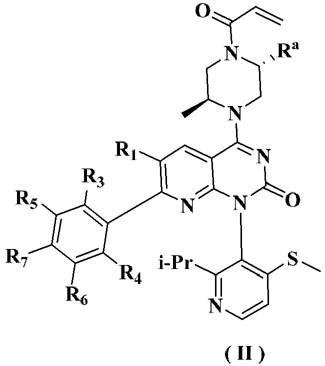 wherein:
R^{a} is selected from hydrogen or methyl;
R₁ is selected from hydrogen, fluorine, chlorine, bromine or methyl;
R₃ is selected from hydrogen, amino, hydroxyl, fluorine, chlorine, methyl, -S(CH₃) or trifluoromethyl;
R₄ is selected from hydrogen, amino, hydroxyl, fluorine, chlorine, -N(CH₃)₂, - NH(CH₃) or fluorine;
R₅ is selected from hydrogen, fluorine, chlorine, bromine, methyl, ethyl, propyl or isopropyl;
R₆ is selected from hydrogen, fluorine, chlorine, bromine, methyl, ethyl, propyl or isopropyl;
R₇ is selected from hydrogen, fluorine, chlorine, bromine or methyl.

In a preferred embodiment of the present disclosure, for the acid salt of the compound, the compound is further shown in general formula (II-A) or (II-B):

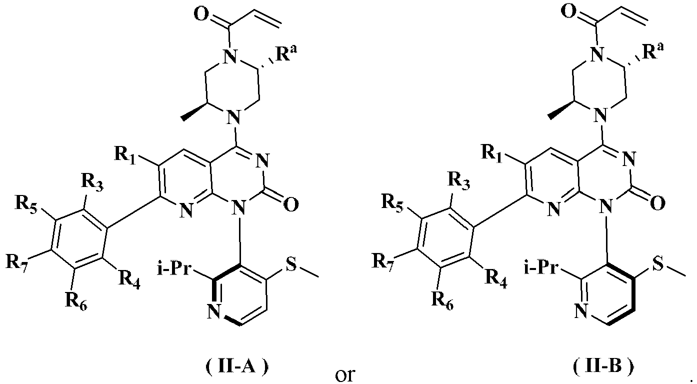

In a preferred embodiment of the present disclosure, for the acid salt of the compound, wherein the compound is selected from: 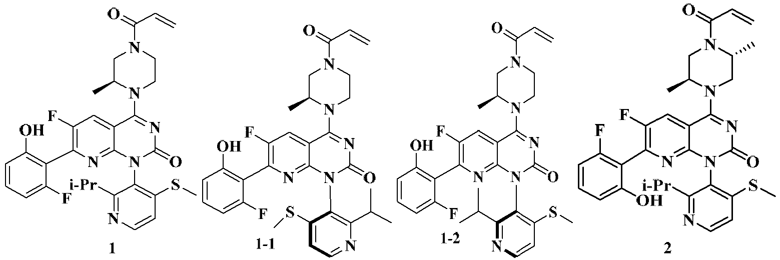 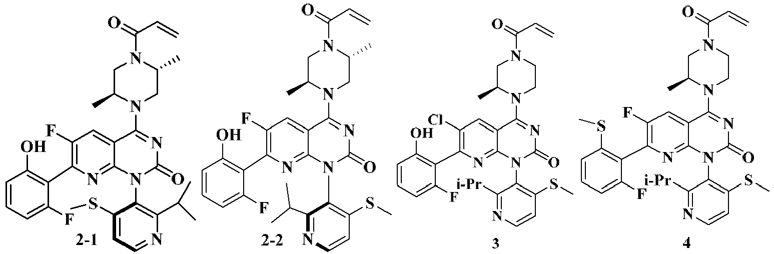 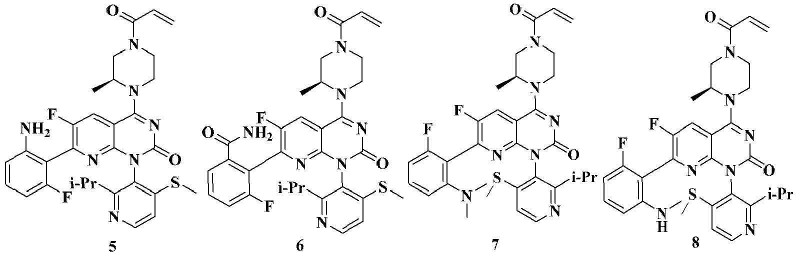 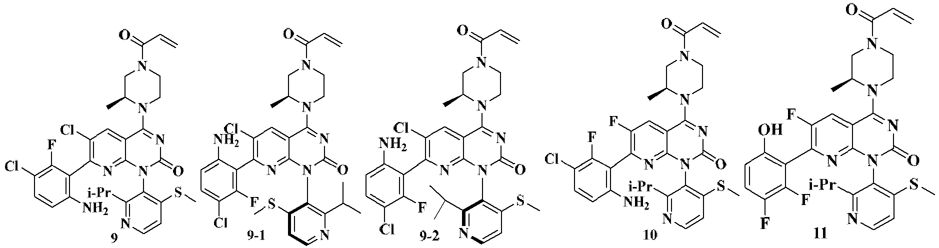 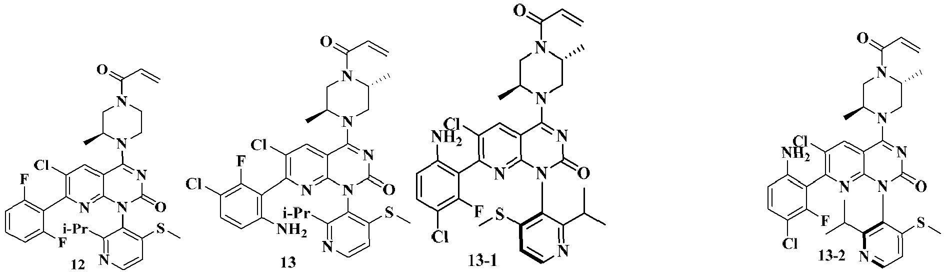 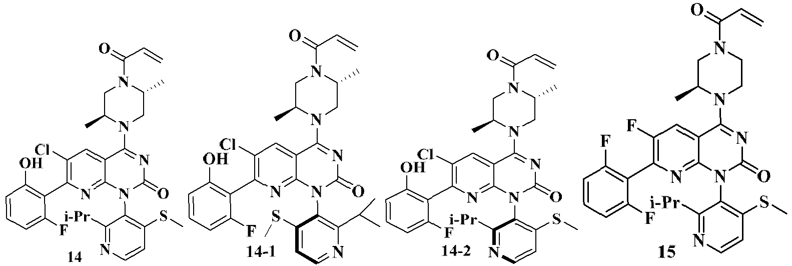 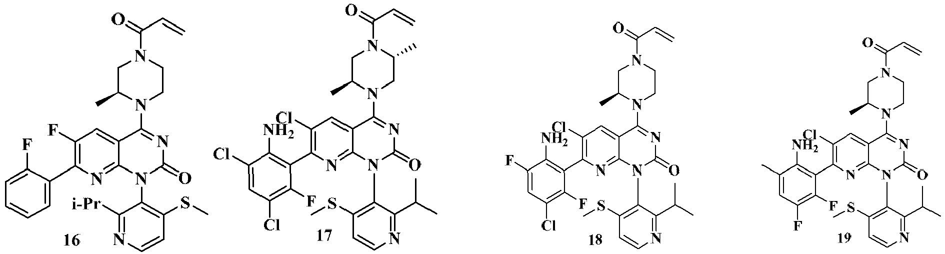 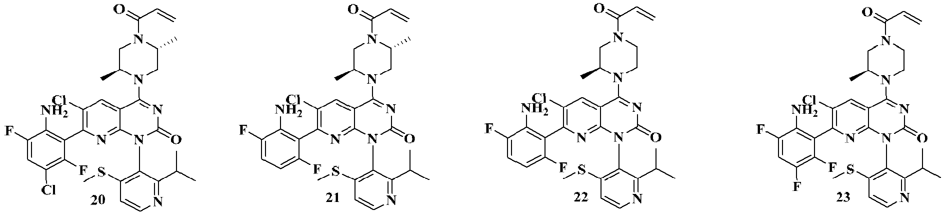 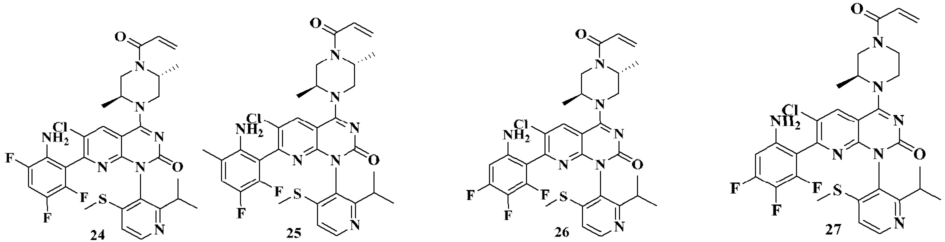 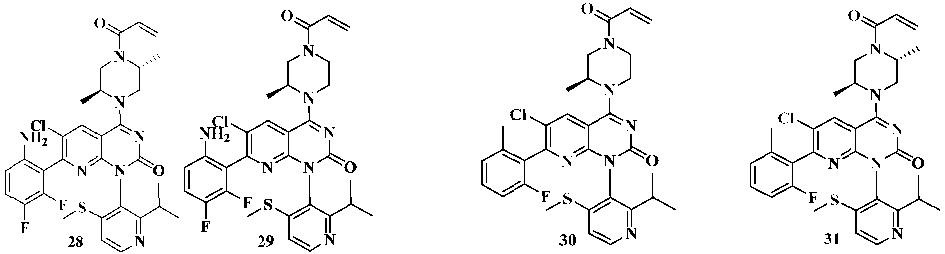 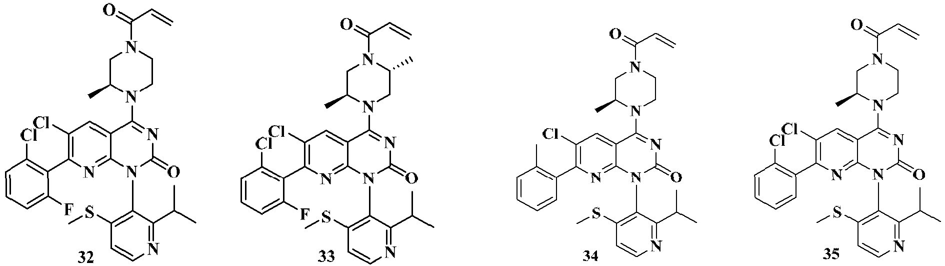 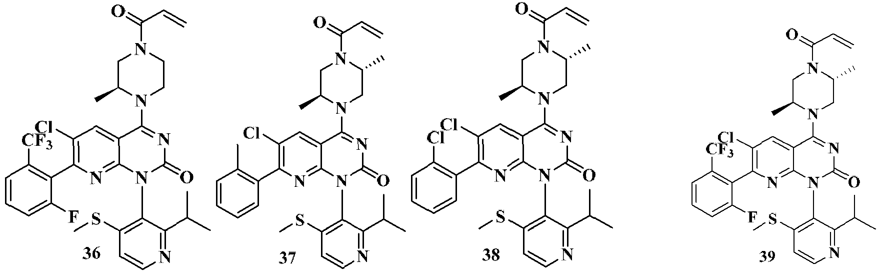 or

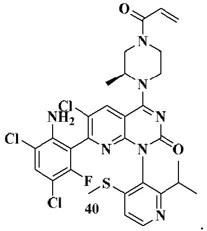

In a more preferred embodiment of the present disclosure, for the acid salt of the compound, wherein the compound is selected from:

| | |
|---|---|
| 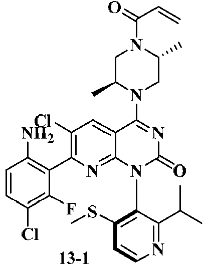 | P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one |
| 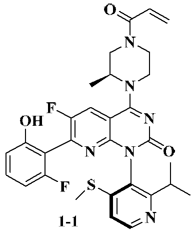 | P-4-((*S*)-4-acryloyl-2-methylpiperazin-1-yl)-6-fluoro-7-(2-fluoro-6-hydroxyphenyl)-1-(2-isopropyl-4-(methylthio)pyridin-3-yl)pyrido[2,3-*d*]pyrimidin-2(1*H*)-one |
| 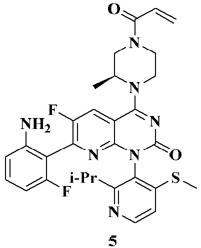 | 4-((*S*)-4-acryloyl-2-methylpiperazin-1-yl)-7-(2-amino-6-fluorophenyl)-6-fluoro-1-(2-isopropyl-4-(methylthio)pyridin-3-yl)pyrido[2,3-*d*]pyrimidin-2(1*H*)-one |
| 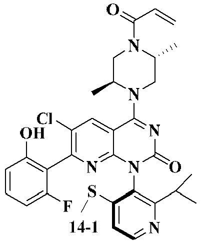 | P-4-((2*S*,5*R*)-4-acryloyl-2,5-dimethylpiperazin-1-yl)-6-chloro-7-(2-fluoro-6-hydroxyphenyl)-1-(2-isopropyl-4-(methylthio)pyridin-3-yl)pyrido[2,3-*d*]pyrimidin-2(1*H*)-one |
| 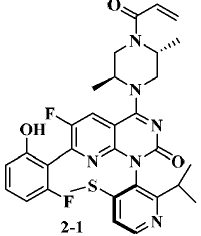 | 4-((2*S*,5*R*)-4-acryloyl-2,5-dimethylpiperazin-1-yl)-6-fluoro-7-(2-fluoro-6-hydroxyphenyl)-1-(2-isopropyl-4-(methylthio)pyridin-3-yl)pyrido[2,3-*d*]pyrimidin-2(1*H*)-one |

Acid in the acid salt is selected from hydroxyethyl sulfonic acid, sulfuric acid, 1,5-naphthalene disulfonic acid, methanesulfonic acid, hydrobromic acid, phosphoric acid, benzenesulfonic acid, oxalic acid, maleate acid, adipic acid, hydrochloric acid, citric acid, malonic acid, L-malic acid, pamoic acid, *p*-toluenesulfonic acid or fumaric acid, preferably hydroxyethyl sulfonic acid or sulfuric acid.

In a further preferred embodiment of the present disclosure, the number of acid is 0.2-3; preferably 0.2, 0.5, 1, 1.5, 2, 2.5 or 3; more preferably 0.5, 1, 2 or 3.

In a further preferred embodiment of the present disclosure, the acid salt is a hydrate or an anhydrate, and when the acid salt is the hydrate, the number of water is 0.2-3; preferably 0.2, 0.5, 1, 1.5, 2, 2.5 or 3; more preferably 0.5, 1, 2 or 3.

In the most preferred embodiment of the present disclosure, an acid salt of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one is provided, wherein the acid in the acid salt is selected from hydroxyethyl sulfonic acid, sulfuric acid, 1,5-naphthalene disulfonic acid, methylsulfonic acid, hydrobromic acid, phosphoric acid, benzenesulfonic acid, oxalic acid, maleate acid, adipic acid, hydrochloric acid, citric acid, malonic acid, L-malic acid, pamoic acid, *p*-toluenesulfonic acid or fumaric acid, wherein structure of the acid salt of the compound is as follows:

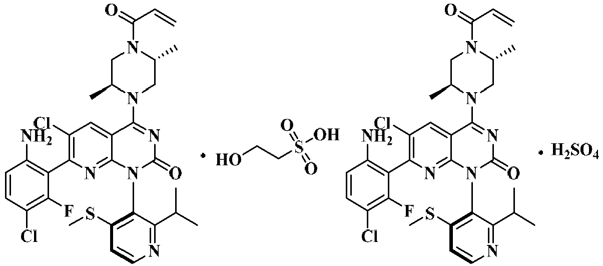

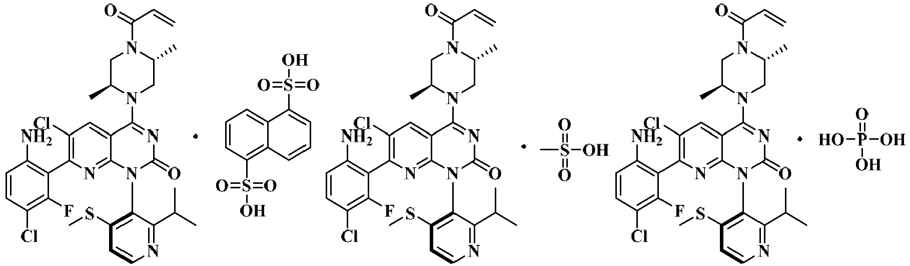

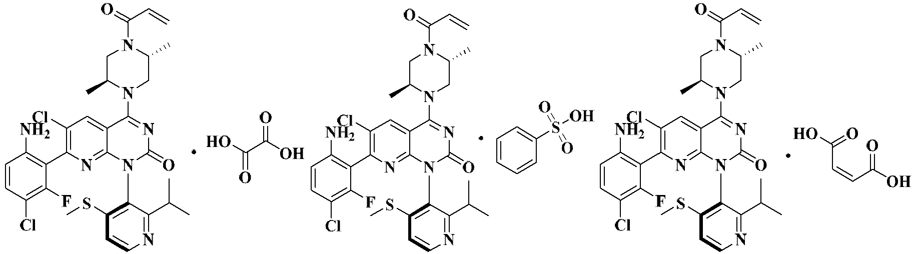

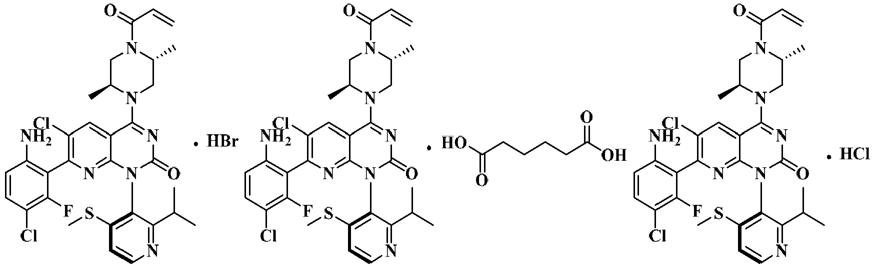

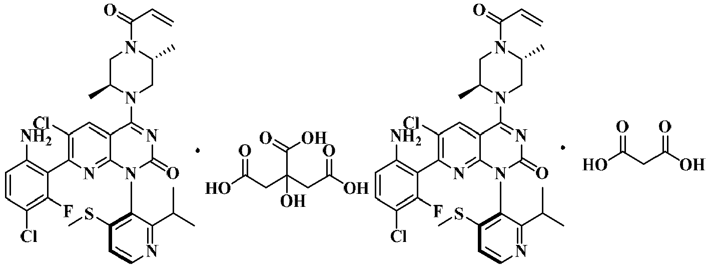

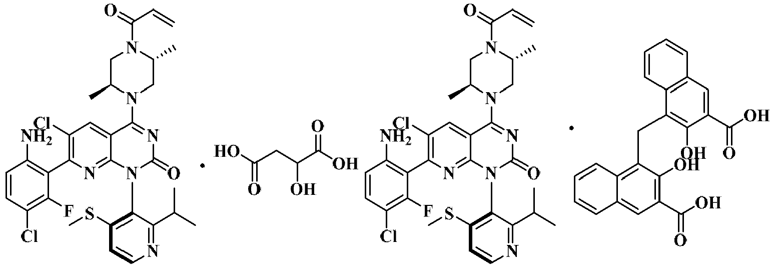

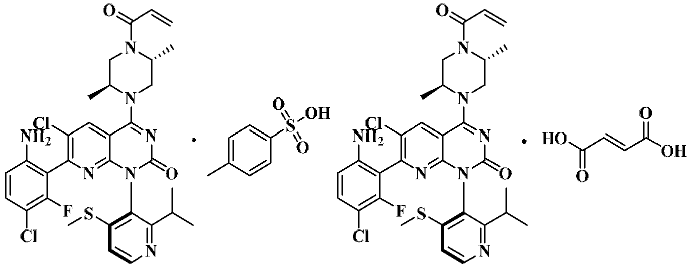

In a preferred embodiment of the present disclosure, the acid salt is in a crystal form; preferably a crystal form of the acid salt of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-d]pyrimidin-2(1*H*)-one;
a crystal form of the acid salt of P-4-((*S*)-4-acryloyl-2-methylpiperazin-1-yl)-6-fluoro-7-(2-fluoro-6-hydroxyphenyl)-1-(2-isopropyl-4-(methylthio)pyridin-3-yl)pyrido[2,3-*d*]pyrimidin-2(1*H*)-one;
a crystal form of the acid salt of P-4-((*S*)-4-acryloyl-2-methylpiperazin-1-yl)-7-(2-amino-6-fluorophenyl)-6-fluoro-1-(2-isopropyl-4-(methylthio)pyridin-3-yl)pyrido[2,3-*d*]pyrimidin-2(1*H*)-one;
a crystal form of the acid salt of P-4-((2*S*,5*R*)-4-acryloyl-2,5-dimethylpiperazin-1-yl)-6-chloro-7-(2-fluoro-6-hydroxyphenyl)-1-(2-isopropyl-4-(methylthio)pyridin-3-yl)pyrido[2,3-*d*]pyrimidin-2(1*H*)-one;
a crystal form of the acid salt of P-4-((2*S*,5*R*)-4-acryloyl-2,5-dimethylpiperazin-1-yl)-6-fluoro-7-(2-fluoro-6-hydroxyphenyl)-1-(2-isopropyl-4-(methylthio)pyridin-3-yl)pyrido[2,3-*d*]pyrimidin-2(1*H*)-one;
more preferably a crystal form of hydroxyethyl sulfonate, a crystal form of sulfate, a crystal form of 1,5-naphthalene disulfonate, a crystal form of methane sulfonate, a crystal form of hydrobromate, a crystal form of phosphate, a crystal form of benzenesulfonate, a crystal form of oxalate, a crystal form of maleate, a crystal form of adipate, a crystal form of hydrochloride, a crystal form of citrate, a crystal form of malonate, a crystal form of L-malate, a crystal form of pamoate, a crystal form of *p*-toluenesulfonate or a crystal form of fumarate.

In a preferred embodiment of the present disclosure, the crystal form of the acid salt of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one is provided.

In a more preferred embodiment of the present disclosure, the acid salt of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one is in a crystal form; preferably the crystal form of hydroxyethyl sulfonate, the crystal form of sulfate, the crystal form of 1,5-naphthalene disulfonate, the crystal form of methanesulfonate, the crystal form of hydrobromate, the crystal form of phosphate, the crystal form of benzenesulfonate, the crystal form of oxalate, the crystal form of maleate, the crystal form of adipate, the crystal form of hydrochloride, the crystal form of citrate, the crystal form of malonate, the crystal form of L-malate, the crystal form of pamoate, the crystal form of *p-*toluenesulfonate or the crystal form of fumarate.

In a preferred embodiment of the present disclosure, the acid salt is in a crystal form; wherein the number of acid is 0.2-3; preferably 0.2, 0.5, 1, 1.5, 2, 2.5 or 3; more preferably 0.5, 1, 2 or 3.

In a preferred embodiment of the present disclosure, crystal forms I-III of hydroxyethyl sulfonate and crystal forms I-IV of sulfate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one are provided:
the crystal form I of hydroxyethyl sulfonate has an X-ray powder diffraction pattern having a diffraction peak at 2θ angle of 21.7±0.2°; or having a diffraction peak at 8.8±0.2°; or having a diffraction peak at 19.3±0.2°; or having a diffraction peak at 27.6±0.2°; or having a diffraction peak at 10.9±0.2°; or having a diffraction peak at 15.4±0.2°; or having a diffraction peak at 16.7±0.2°; or having a diffraction peak at 15.8±0.2°; or having a diffraction peak at 17.5±0.2°; or having a diffraction peak at 23.8±0.2°; or having a diffraction peak at 10.2±0.2°; or having a diffraction peak at 11.8±0.2°; preferably comprising any 2-5, or 3-5, or 3-6, or 3-8, or 5-8, or 6-8 of the above diffraction peaks, more preferably comprising any 6, 7, or 8 of the diffraction peaks;
the crystal form II of hydroxyethyl sulfonate has an X-ray powder diffraction pattern having a diffraction peak at 2θ angle of 21.7±0.2°; or having a diffraction peak at 8.8±0.2°; or having a diffraction peak at 19.3±0.2°; or having a diffraction peak at 27.6±0.2°; or having a diffraction peak at 10.9±0.2°; or having a diffraction peak at 23.8±0.2°; or having a diffraction peak at 16.7±0.2°; or having a diffraction peak at 15.4±0.2°; or having a diffraction peak at 15.8±0.2°; or having a diffraction peak at 10.0±0.2°; preferably comprising any 2-5, or 3-5, or 3-6, or 3-8, or 5-8, or 6-8 of the above diffraction peaks, more preferably comprising any 6, 7, or 8 of the diffraction peaks;
the crystal form III of hydroxyethyl sulfonate has an X-ray powder diffraction pattern having a diffraction peak at 2θ angle of 19.4±0.2°; or having a diffraction peak at 16.9±0.2°; or having a diffraction peak at 26.6±0.2°; or having a diffraction peak at 14.6±0.2°; or having a diffraction peak at 28.0±0.2°; or having a diffraction peak at 25.6±0.2°; or having a diffraction peak at 20.7±0.2°; or having a diffraction peak at 12.8±0.2°; or having a diffraction peak at 19.1±0.2°; or having a diffraction peak at 27.2±0.2°; preferably comprising any 2-5, or 3-5, or 3-6, or 3-8, or 5-8, or 6-8 of the above diffraction peaks, more preferably comprising any 6, 7, or 8 of the diffraction peaks;
the crystal form I of sulfate has an X-ray powder diffraction pattern having a diffraction peak at 2θ angle of 19.0±0.2°; or having a diffraction peak at 19.4±0.2°; or having a diffraction peak at 12.4±0.2°; or having a diffraction peak at 26.2±0.2°; or having a diffraction peak at 17.6±0.2°; or having a diffraction peak at 18.1±0.2°; or having a diffraction peak at 25.3±0.2°; or having a diffraction peak at 8.8±0.2°; or having a diffraction peak at 21.9±0.2°; or having a diffraction peak at 11.5±0.2°; preferably comprising any 2-5, or 3-5, or 3-6, or 3-8, or 5-8, or 6-8 of the above diffraction peaks, more preferably comprising any 6, 7, or 8 of the diffraction peaks;
the crystal form II of sulfate has an X-ray powder diffraction pattern having a diffraction peak at 2θ angle of 15.5±0.2°; or having a diffraction peak at 11.1±0.2°; or having a diffraction peak at 8.9±0.2°; or having a diffraction peak at 19.3±0.2°; or having a diffraction peak at 22.3±0.2°; or having a diffraction peak at 23.6±0.2°; or having a diffraction peak at 17.4±0.2°; or having a diffraction peak at 27.3±0.2°; or having a diffraction peak at 17.0±0.2°; or having a diffraction peak at 27.9±0.2°; preferably comprising any 2-5, or 3-5, or 3-6, or 3-8, or 5-8, or 6-8 of the above diffraction peaks, more preferably comprising any 6, 7, or 8 of the diffraction peaks;
the crystal form III of sulfate has an X-ray powder diffraction pattern having a diffraction peak at 2θ angle of 19.6±0.2°; or having a diffraction peak at 18.0±0.2°; or having a diffraction peak at 18.4±0.2°; or having a diffraction peak at 16.8±0.2°; or having a diffraction peak at 14.3±0.2°; or having a diffraction peak at 11.8±0.2°; or having a diffraction peak at 14.9±0.2°; or having a diffraction peak at 25.7±0.2°; or having a diffraction peak at 15.4±0.2°; or having a diffraction peak at 23.5±0.2°; preferably comprising any 2-5, or 3-5, or 3-6, or 3-8, or 5-8, or 6-8 of the above diffraction peaks, more preferably comprising any 6, 7, or 8 of the diffraction peaks;
the crystal form IV of sulfate has an X-ray powder diffraction pattern having a diffraction peak at 2θ angle of 19.4±0.2°; or having a diffraction peak at 18.9±0.2°; or having a diffraction peak at 15.5±0.2°; or having a diffraction peak at 8.8±0.2°; or having a diffraction peak at 18.1±0.2°; or having a diffraction peak at 24.9±0.2°; or having a diffraction peak at 17.4±0.2°; or having a diffraction peak at 12.3±0.2°; or having a diffraction peak at 26.1±0.2°; or having a diffraction peak at 14.5±0.2°; preferably comprising any 2-5, or 3-5, or 3-6, or 3-8, or 5-8, or 6-8 of the above diffraction peaks, more preferably comprising any 6, 7, or 8 of the diffraction peaks.

In a further preferred embodiment of the present disclosure, crystal forms I-III of hydroxyethyl sulfonate and crystal forms I-IV of sulfate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one are provided:
the X-ray powder diffraction pattern of the crystal form I of hydroxyethyl sulfonate comprises at least one or more diffraction peaks at 2θ angles of 21.7±0.2°, 8.8±0.2°, 19.3±0.2°, preferably comprises two of the above diffraction peaks, more preferably comprises three of the diffraction peaks; optionally, further comprises at least one diffraction peak at 2θ angles of 27.6±0.2°, 10.9±0.2°, 15.4±0.2°, 16.7±0.2°, 15.8±0.2°, 10.2±0.2°, 11.8±0.2°, preferably comprises 2, 3, 4 or 5 of the above diffraction peaks; for example,
21.7±0.2°, 8.8±0.2°;
8.8±0.2°, 27.6±0.2°;
21.7±0.2°, 8.8±0.2°, 10.9±0.2°;
8.8±0.2°, 19.3±0.2°, 15.4±0.2°;
21.7±0.2°, 8.8±0.2°, 27.6±0.2°, 10.9±0.2°;
8.8±0.2°, 19.3±0.2°, 15.4±0.2°, 16.7±0.2°;
15.8±0.2°, 8.8±0.2°, 27.6±0.2°, 10.9±0.2°;
11.7±0.2°, 8.8±0.2°, 27.6±0.2°, 10.9±0.2°;
16.7±0.2°, 8.8±0.2°, 19.3±0.2°, 16.7±0.2°, 10.9±0.2°, 15.4±0.2°;
21.7±0.2°, 8.8±0.2°, 19.3±0.2°, 15.8±0.2°, 10.9±0.2°, 15.4±0.2°;
10.9±0.2°, 8.8±0.2°, 10.2±0.2°, 27.6±0.2°, 10.9±0.2°, 15.8±0.2°;
the X-ray powder diffraction pattern of the crystal form II of hydroxyethyl sulfonate comprises at least one or more diffraction peaks at 2θ angles of 21.7±0.2°, 10.0±0.2° ,8.8±0.2°, preferably comprises two of the above diffraction peaks, more preferably comprises three of the diffraction peaks; optionally, further comprises at least one diffraction peak at 2θ angles of 19.3±0.2°, 27.6±0.2°, 10.9±0.2°, 23.8±0.2°, 16.7±0.2°, preferably comprises 2, 3, 4 or 5 of the above diffraction peaks; for example,
21.7±0.2°, 10.0±0.2°;
10.0±0.2°; 8.8±0.2°;
21.7±0.2°, 10.0±0.2°, 19.3±0.2°;
10.0±0.2°, 8.8±0.2°, 27.6±0.2°;
21.7±0.2°, 10.0±0.2°, 8.8±0.2°, 19.3±0.2°;
10.0±0.2°, 8.8±0.2°, 19.3±0.2°, 27.6±0.2°;
27.6±0.2°, 10.0±0.2°, 8.8±0.2°, 19.3±0.2°, 16.7±0.2°, 10.9±0.2°;
21.7±0.2°, 10.0±0.2°, 8.8±0.2°, 16.7±0.2°, 27.6±0.2°, 10.9±0.2°;
the X-ray powder diffraction pattern of the crystal form III of hydroxyethyl sulfonate comprises at least one or more diffraction peaks at 2θ angles of 19.4±0.2°, 16.9±0.2°, 26.6±0.2°, preferably comprises two of the above diffraction peaks, more preferably comprises three of the diffraction peaks; optionally, further comprises at least one diffraction peak at 2θ angles of 14.6±0.2°, 28.0±0.2°, 25.6±0.2°, 20.7±0.2°, 12.8±0.2°, preferably comprises 2, 3, 4 or 5 of the above diffraction peaks; for example,
19.4±0.2°, 16.9±0.2°, 26.6±0.2°, 14.6±0.2°, 28.0±0.2°, 25.6±0.2°;
19.4±0.2°, 16.9±0.2°, 26.6±0.2°, 12.8±0.2°, 28.0±0.2°, 25.6±0.2°;
the X-ray powder diffraction pattern of the crystal form I of sulfate comprises at least one or more diffraction peaks at 2θ angles of 19.0±0.2°, 19.4±0.2°, 12.4±0.2°, preferably comprises two of the above diffraction peaks, more preferably comprises three of the diffraction peaks; optionally, further comprises at least one diffraction peak at 2θ angles of 26.2±0.2°, 17.6±0.2°, 18.1±0.2°, 25.3±0.2°, 8.8±0.2°, preferably comprises 2, 3, 4 or 5 of the above diffraction peaks; for example,
19.0±0.2°, 19.4±0.2°, 12.4±0.2°, 26.2±0.2°, 17.6±0.2°, 18.1±0.2°;
19.0±0.2°, 19.4±0.2°, 12.4±0.2°, 26.2±0.2°, 17.6±0.2°, 25.3±0.2°;
the X-ray powder diffraction pattern of the crystal form II of sulfate comprises at least one or more diffraction peaks at 2θ angles of 15.5±0.2°, 11.1±0.2°, 8.9±0.2°, preferably comprises two of the above diffraction peaks, more preferably comprises three of the diffraction peaks; optionally, further comprises at least one diffraction peak at 2θ angles of 19.3±0.2°, 22.3±0.2°, 23.6±0.2°, 17.4±0.2°, 27.3±0.2°, preferably comprises 2, 3, 4 or 5 of the above diffraction peaks; for example,
15.5±0.2°, 11.1±0.2°;
11.1±0.2°, 8.9±0.2°;
15.5±0.2°, 11.1±0.2°, 8.9±0.2°;
11.1±0.2°, 8.9±0.2°, 19.3±0.2°;
15.5±0.2°, 11.1±0.2°, 8.9±0.2°, 19.3±0.2°;
15.5±0.2°, 11.1±0.2°, 8.9±0.2°, 19.3±0.2°, 22.3±0.2°, 27.3±0.2°;
8.9±0.2°, 19.3±0.2°, 22.3±0.2°, 23.6±0.2°, 17.4±0.2°, 27.3±0.2°;
the X-ray powder diffraction pattern of the crystal form III of sulfate comprises at least one or more diffraction peaks at 2θ angles of 19.6±0.2°, 18.0±0.2°, 18.4±0.2°, preferably comprises two of the above diffraction peaks, more preferably comprises three of the diffraction peaks; optionally, further comprises at least one diffraction peak at 2θ angles of 16.8±0.2°, 14.3±0.2°, 11.8±0.2°, 14.9±0.2°, 25.7±0.2°, preferably comprises 2, 3, 4 or 5 of the above diffraction peaks; for example,
19.6±0.2°, 18.0±0.2°, 18.4±0.2°, 16.8±0.2°, 14.3±0.2°, 11.8±0.2°;
19.6±0.2°, 18.0±0.2°, 18.4±0.2°, 16.8±0.2°, 14.3±0.2°, 14.9±0.2°;
the X-ray powder diffraction pattern of the crystal form IV of sulfate comprises at least one or more diffraction peaks at 2θ angles of 19.4±0.2°, 18.9±0.2°, 15.5±0.2°, preferably comprises two of the above diffraction peaks, more preferably comprises three of the diffraction peaks; optionally, further comprises at least one diffraction peak at 2θ angles of 8.8±0.2°, 18.1±0.2°, 24.9±0.2°, 17.4±0.2°, 12.3±0.2°, preferably comprises 2, 3, 4 or 5 of the above diffraction peaks; for example,
19.4±0.2°, 18.9±0.2°, 15.5±0.2°, 8.8±0.2°, 18.1±0.2°, 24.9±0.2°;
19.4±0.2°, 18.9±0.2°, 15.5±0.2°, 8.8±0.2°, 12.3±0.2°, 24.9±0.2°.
In a further preferred embodiment of the present disclosure, crystal forms I-III of hydroxyethyl sulfonate and crystal forms I-IV of sulfate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one are provided:
the X-ray powder diffraction pattern of the crystal form I of hydroxyethyl sulfonate optionally also comprises one or more diffraction peaks at 2θ angles of 21.7±0.2°, 8.8±0.2°, 10.2±0.2°, 11.8±0.2°, 13.3±0.2°, 19.3±0.2°, 27.6±0.2°, 10.9±0.2°, 15.4±0.2°, 16.7±0.2°, preferably comprises at least any 2-3, or 4-5, or 6-8 of the above diffraction peaks; further preferably, comprises any 2, 3, 4, 5, 6, 7 or 8 of the diffraction peaks; for example,
8.8±0.2°, 10.2±0.2°, 11.8±0.2°, 13.3±0.2°, 27.6±0.2°, 10.9±0.2°, 15.8±0.2°, 17.5±0.2°;
27.6±0.2°, 10.9±0.2°, 15.4±0.2°, 17.5±0.2°, 15.8±0.2°, 16.7±0.2°, 17.5±0.2°, 23.8±0.2°;
21.7±0.2°, 8.8±0.2°, 19.3±0.2°, 27.6±0.2°, 10.9±0.2°, 17.5±0.2°, 16.7±0.2°, 15.8±0.2°;
the X-ray powder diffraction pattern of the crystal form II of hydroxyethyl sulfonate optionally also comprises one or more diffraction peaks at 2θ angles of 10.0±0.2°, 21.7±0.2°, 8.8±0.2°, 19.3±0.2°, 27.6±0.2°, 10.9+±0.2°, 23.8±0.2°; preferably comprises at least any 2-3, or 4-5, or 6-8 of the above diffraction peaks; further preferably, comprises any 2, 3, 4, 5, 6, 7 or 8 of the diffraction peaks; for example,
21.7±0.2°, 8.8±0.2°, 19.3±0.2°, 27.6±0.2°, 10.9+±0.2°, 23.8±0.2°, 16.7±0.2°, 15.4±0.2°;
8.8±0.2°, 19.3±0.2°, 27.6±0.2°, 10.9+±0.2°, 23.8±0.2°, 15.4±0.2°, 15.8±0.2°, 10.0±0.2°;
the X-ray powder diffraction pattern of the crystal form III of hydroxyethyl sulfonate optionally also comprises one or more diffraction peaks at 2θ angles of 19.4±0.2°, 16.9±0.2°, 26.6±0.2°, 14.6±0.2°, 28.0±0.2°, 25.6±0.2°, 20.7±0.2°; preferably comprises at least any 2-3, or 4-5, or 6-8 of the above diffraction peaks; further preferably, comprises any 2, 3, 4, 5, 6, 7 or 8 of the diffraction peaks; for example,
19.4±0.2°, 16.9±0.2°, 26.6±0.2°, 14.6±0.2°, 28.0±0.2°, 25.6±0.2°, 20.7±0.2°, 27.2±0.2°;
19.4±0.2°, 16.9±0.2°, 26.6±0.2°, 14.6±0.2°, 28.0±0.2°, 27.2±0.2°, 20.7±0.2°, 12.8±0.2°;
the X-ray powder diffraction pattern of the crystal form I of sulfate optionally also comprises one or more diffraction peaks at 2θ angles of 19.0±0.2°, 19.4±0.2°, 12.4±0.2°, 26.2±0.2°, 17.6±0.2°, 18.1±0.2°, 25.3±0.2°; preferably comprises at least any 2-3, or 4-5, or 6-8 of the above diffraction peaks; further preferably, comprises any 2, 3, 4, 5, 6, 7 or 8 of the diffraction peaks; for example,
19.0±0.2°, 19.4±0.2°, 12.4±0.2°, 26.2±0.2°, 17.6±0.2°, 18.1±0.2°, 25.3±0.2°, 8.8±0.2°;
the X-ray powder diffraction pattern of the crystal form II of sulfate optionally also comprises one or more diffraction peaks at 2θ angles of 15.5±0.2°, 11.1±0.2°, 8.9±0.2°, 19.3±0.2°, 22.3±0.2°, 23.6±0.2°, 17.4±0.2°; preferably comprises at least any 2-3, or 4-5, or 6-8 of the above diffraction peaks; further preferably, comprises any 2, 3, 4, 5, 6, 7 or 8 of the diffraction peaks; for example,
15.5±0.2°, 8.9±0.2°, 19.3±0.2°, 22.3±0.2°, 23.6±0.2°, 17.4±0.2°, 27.3±0.2°, 17.0±0.2°;
11.1±0.2°, 8.9±0.2°, 19.3±0.2°, 22.3±0.2°, 17.4±0.2°, 27.3±0.2°, 17.0±0.2°, 27.9±0.2°;
the X-ray powder diffraction pattern of the crystal form III of sulfate optionally also comprises one or more diffraction peaks at 2θ angles of 19.6±0.2°, 18.0±0.2°, 18.4±0.2°, 16.8±0.2°, 14.3±0.2°, 11.8±0.2°, 14.9±0.2°; preferably comprises at least any 2-3, or 4-5, or 6-8 of the above diffraction peaks; further preferably, comprises any 2, 3, 4, 5, 6, 7 or 8 of the diffraction peaks; for example,
19.6±0.2°, 18.0±0.2°, 18.4±0.2°, 16.8±0.2°, 14.3±0.2°, 11.8±0.2°, 14.9±0.2°, 15.4±0.2°;
19.6±0.2°, 18.0±0.2°, 18.4±0.2°, 16.8±0.2°, 14.3±0.2°, 11.8±0.2°, 14.9±0.2°, 23.5±0.2°;
the X-ray powder diffraction pattern of the crystal form IV of sulfate optionally also comprises one or more diffraction peaks at 2θ angles of 19.4±0.2°, 18.9±0.2°, 15.5±0.2°, 8.8±0.2°, 18.1±0.2°, 24.9±0.2°, 17.4±0.2°; preferably comprises at least any 2-3, or 4-5, or 6-8 of the above diffraction peaks; further preferably, comprises any 2, 3, 4, 5, 6, 7 or 8 of the diffraction peaks; for example,
19.4±0.2°, 18.9±0.2°, 15.5±0.2°, 8.8±0.2°, 18.1±0.2°, 24.9±0.2°, 17.4±0.2°, 12.3±0.2°;
19.4±0.2°, 18.9±0.2°, 15.5±0.2°, 8.8±0.2°, 18.1±0.2°, 24.9±0.2°, 17.4±0.2°, 26.1±0.2°.

In a further preferred embodiment of the present disclosure, crystal forms I-III of hydroxyethyl sulfonate and crystal forms I-IV of sulfate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one are provided:
the X-ray powder diffraction pattern of the crystal form I of hydroxyethyl sulfonate comprises one or more diffraction peaks at 2θ angles of 21.7±0.2°, 8.8±0.2°, 10.2±0.2°, 11.8±0.2°, 13.1±0.2°, 19.3±0.2°, 27.6±0.2°, 10.9±0.2°, 13.3±0.2°, 15.4±0.2°, 16.7±0.2°, 15.8±0.2°, 17.5±0.2°, 23.8±0.2°, 14.7±0.2°, 24.3±0.2°, 27.3±0.2°, 23.4±0.2°, 20.6±0.2°, 21.2±0.2°, preferably, comprises any 4, 5, 6, 8 or 10 of the above diffraction peaks; for example,
8.8±0.2°, 19.3±0.2°, 27.6±0.2°, 20.6±0.2°;
19.3±0.2°, 10.9±0.2°, 15.4±0.2°, 16.7±0.2°;
8.8±0.2°, 19.3±0.2°, 27.6±0.2°, 10.9±0.2°, 15.4±0.2°, 20.6±0.2°;
19.3±0.2°, 27.6±0.2°, 10.9±0.2°, 16.7±0.2°, 23.4±0.2°, 20.6±0.2°;
8.8±0.2°, 19.3±0.2°, 27.6±0.2°, 16.7±0.2°, 15.8±0.2°, 17.5±0.2°, 24.3±0.2°, 14.7±0.2°, 27.3±0.2°, 23.4±0.2°;
21.7±0.2°, 8.8±0.2°, 19.3±0.2°, 27.6±0.2°, 10.9±0.2°, 15.4±0.2°, 16.7±0.2°, 15.8±0.2°, 24.3±0.2°, 23.8±0.2°;
8.8±0.2°, 10.2±0.2°, 11.8±0.2°, 13.1±0.2°, 27.6±0.2°, 10.9±0.2°, 13.3±0.2°, 21.2±0.2°, 15.8±0.2°, 17.5±0.2°;
the X-ray powder diffraction pattern of the crystal form II of hydroxyethyl sulfonate comprises one or more diffraction peaks at 2θ angles of 21.7±0.2°, 10.0±0.2°, 8.8±0.2°, 19.3±0.2°, 27.6±0.2°, 10.9±0.2°, 23.8±0.2°, 16.7±0.2°, 15.4±0.2°, 15.8±0.2°, 17.5±0.2°, 14.7±0.2°, 24.4±0.2°, 27.3±0.2°, 29.2±0.2°, preferably, comprises any 4, 5, 6, 8 or 10 of the above diffraction peaks; for example,
10.0±0.2°, 8.8±0.2°, 19.3±0.2°, 29.2±0.2°;
8.8±0.2°, 27.6±0.2°, 27.3±0.2°, 29.2±0.2°;
21.7±0.2°, 10.0±0.2°, 8.8±0.2°, 19.3±0.2°, 27.6±0.2°, 29.2±0.2°;
10.0±0.2°, 8.8±0.2°, 19.3±0.2°, 27.6±0.2°, 27.3±0.2°, 14.7±0.2°;
21.7±0.2°, 8.8±0.2°, 19.3±0.2°, 27.6±0.2°, 10.9±0.2°, 23.8±0.2°, 27.3±0.2°, 17.5±0.2°;
19.3±0.2°, 27.6±0.2°, 10.9±0.2°, 23.8±0.2°, 15.8±0.2°, 16.7±0.2°, 15.4±0.2°, 17.5±0.2°;
10.0±0.2°, 8.8±0.2°, 19.3±0.2°, 27.6±0.2°, 10.9±0.2°, 23.8±0.2°, 16.7±0.2°, 15.4±0.2°, 15.8±0.2°, 17.5±0.2°;
8.8±0.2°, 19.3±0.2°, 27.6±0.2°, 10.9±0.2°, 23.8±0.2°, 16.7±0.2°, 15.4±0.2°, 17.5±0.2°, 27.3±0.2°, 29.2±0.2°;
the X-ray powder diffraction pattern of the crystal form III of hydroxyethyl sulfonate comprises one or more diffraction peaks at 2θ angles of 19.4±0.2°, 16.9±0.2°, 26.6±0.2°, 14.6±0.2°, 28.0±0.2°, 25.6±0.2°, 20.7±0.2°, 12.8±0.2°, 19.1±0.2°, 27.2±0.2°, 24.4±0.2°, 15.3±0.2°, 26.2±0.2°, 30.2±0.2°, 27.4±0.2°, preferably, comprises any 4, 5, 6, 8 or 10 of the above diffraction peaks; for example,
19.4±0.2°, 16.9±0.2°, 26.6±0.2°, 14.6±0.2°;
19.4±0.2°, 16.9±0.2°, 26.6±0.2°, 28.0±0.2°;
19.4±0.2°, 16.9±0.2°, 26.6±0.2°, 14.6±0.2°, 28.0±0.2°, 27.4±0.2°;
19.4±0.2°, 16.9±0.2°, 26.6±0.2°, 14.6±0.2°, 28.0±0.2°, 30.2±0.2°;
19.4±0.2°, 16.9±0.2°, 26.6±0.2°, 14.6±0.2°, 28.0±0.2°, 25.6±0.2°, 20.7±0.2°, 27.4±0.2°;
19.4±0.2°, 16.9±0.2°, 26.6±0.2°, 14.6±0.2°, 28.0±0.2°, 25.6±0.2°, 20.7±0.2°, 30.2±0.2°;
19.4±0.2°, 16.9±0.2°, 26.6±0.2°, 14.6±0.2°, 28.0±0.2°, 25.6±0.2°, 20.7±0.2°, 12.8±0.2°, 19.1±0.2°, 27.2±0.2°;
19.4±0.2°, 16.9±0.2°, 26.6±0.2°, 14.6±0.2°, 28.0±0.2°, 25.6±0.2°, 20.7±0.2°, 12.8±0.2°, 19.1±0.2°, 24.4±0.2°;
the X-ray powder diffraction pattern of the crystal form I of sulfate comprises one or more diffraction peaks at 2θ angles of 19.0±0.2°, 19.4±0.2°, 12.4±0.2°, 26.2±0.2°, 17.6±0.2°, 18.1±0.2°, 25.3±0.2°, 8.8±0.2°, 21.9±0.2°, 11.5±0.2°, preferably, comprises any 4, 5, 6, 8 or 10 of the above diffraction peaks; for example,
19.0±0.2°, 19.4±0.2°, 12.4±0.2°, 26.2±0.2°;
19.0±0.2°, 19.4±0.2°, 12.4±0.2°, 17.6±0.2°;
19.0±0.2°, 19.4±0.2°, 12.4±0.2°, 26.2±0.2°, 17.6±0.2°, 11.5±0.2°;
19.0±0.2°, 19.4±0.2°, 12.4±0.2°, 26.2±0.2°, 17.6±0.2°, 21.9±0.2°;
19.0±0.2°, 19.4±0.2°, 12.4±0.2°, 26.2±0.2°, 17.6±0.2°, 18.1±0.2°, 25.3±0.2°, 8.8±0.2°;
19.0±0.2°, 19.4±0.2°, 12.4±0.2°, 26.2±0.2°, 17.6±0.2°, 18.1±0.2°, 25.3±0.2°, 21.9±0.2°;
19.0±0.2°, 19.4±0.2°, 12.4±0.2°, 26.2±0.2°, 17.6±0.2°, 18.1±0.2°, 25.3±0.2°, 8.8±0.2°, 21.9±0.2°, 11.5±0.2°;
the X-ray powder diffraction pattern of the crystal form II of sulfate comprises one or more diffraction peaks at 2θ angles of 15.5±0.2°, 11.1±0.2°, 8.9±0.2°, 19.3±0.2°, 22.3±0.2°, 23.6±0.2°, 17.4±0.2°, 27.3±0.2°, 17.0±0.2°, 27.9±0.2°, 15.8±0.2°, 24.2±0.2°, 21.8±0.2°, 10.3±0.2°, 20.6±0.2°, preferably, comprises any 4, 5, 6, 8 or 10 of the above diffraction peaks; for example,
11.1±0.2°, 8.9±0.2°, 19.3±0.2°, 21.8±0.2°;
15.5±0.2°, 8.9±0.2°, 22.3±0.2°, 10.3±0.2°;
11.1±0.2°, 8.9±0.2°, 19.3±0.2°, 22.3±0.2°, 20.6±0.2°, 27.9±0.2°;
15.5±0.2°, 11.1±0.2°, 19.3±0.2°, 22.3±0.2°, 21.8±0.2°, 10.3±0.2°;
15.5±0.2°, 11.1±0.2°, 8.9±0.2°, 22.3±0.2°, 23.6±0.2°, 17.4±0.2°, 20.6±0.2°, 27.9±0.2°;
15.5±0.2°, 11.1±0.2°, 8.9±0.2°, 19.3±0.2°, 23.6±0.2°, 17.4±0.2°, 10.3±0.2°, 20.6±0.2°;
11.1±0.2°, 8.9±0.2°, 19.3±0.2°, 22.3±0.2°, 23.6±0.2°, 17.4±0.2°, 27.3±0.2°, 17.0±0.2°, 27.9±0.2°, 20.6±0.2°;
15.5±0.2°, 8.9±0.2°, 19.3±0.2°, 22.3±0.2°, 23.6±0.2°, 17.4±0.2°, 27.3±0.2°, 17.0±0.2°, 15.8±0.2°, 10.3±0.2°;
the X-ray powder diffraction pattern of the crystal form III of sulfate comprises one or more diffraction peaks at 2θ angles of 19.6±0.2°, 18.0±0.2°, 18.4±0.2°, 16.8±0.2°, 14.3±0.2°, 11.8±0.2°, 14.9±0.2°, 25.7±0.2°, 15.4±0.2°, 23.5±0.2°, 18.8±0.2°, 24.7±0.2°, 9.5±0.2°, 8.8±0.2°, 11.1±0.2°, preferably, comprises any 4, 5, 6, 8 or 10 of the above diffraction peaks; for example,
19.6±0.2°, 18.0±0.2°, 18.4±0.2°, 16.8±0.2°;
19.6±0.2°, 18.0±0.2°, 18.4±0.2°, 14.3±0.2°;
19.6±0.2°, 18.0±0.2°, 18.4±0.2°, 16.8±0.2°, 14.3±0.2°, 11.1±0.2°;
19.6±0.2°, 18.0±0.2°, 18.4±0.2°, 16.8±0.2°, 14.3±0.2°, 8.8±0.2°;
19.6±0.2°, 18.0±0.2°, 18.4±0.2°, 16.8±0.2°, 14.3±0.2°, 11.8±0.2°, 14.9±0.2°, 11.1±0.2°;
19.6±0.2°, 18.0±0.2°, 18.4±0.2°, 16.8±0.2°, 14.3±0.2°, 11.8±0.2°, 14.9±0.2°, 8.8±0.2°;
19.6±0.2°, 18.0±0.2°, 18.4±0.2°, 16.8±0.2°, 14.3±0.2°, 11.8±0.2°, 14.9±0.2°, 25.7±0.2°, 15.4±0.2°, 23.5±0.2°;
19.6±0.2°, 18.0±0.2°, 18.4±0.2°, 16.8±0.2°, 14.3±0.2°, 11.8±0.2°, 14.9±0.2°, 25.7±0.2°, 15.4±0.2°, 18.8±0.2°;
the X-ray powder diffraction pattern of the crystal form IV of sulfate comprises one or more diffraction peaks at 2θ angles of 19.4±0.2°, 18.9±0.2°, 15.5±0.2°, 8.8±0.2°, 18.1±0.2°, 24.9±0.2°, 17.4±0.2°, 12.3±0.2°, 26.1±0.2°, 14.5±0.2°, 22.2±0.2°, 24.3±0.2°, 21.7±0.2°, 23.6±0.2°, preferably, comprises any 4, 5, 6, 8 or 10 of the above diffraction peaks; for example,
19.4±0.2°, 18.9±0.2°, 15.5±0.2°, 8.8±0.2°;
19.4±0.2°, 18.9±0.2°, 15.5±0.2°, 18.1±0.2°;
19.4±0.2°, 18.9±0.2°, 15.5±0.2°, 8.8±0.2°, 18.1±0.2°, 23.6±0.2°;
19.4±0.2°, 18.9±0.2°, 15.5±0.2°, 8.8±0.2°, 18.1±0.2°, 21.7±0.2°;
19.4±0.2°, 18.9±0.2°, 15.5±0.2°, 8.8±0.2°, 18.1±0.2°, 24.9±0.2°, 17.4±0.2°, 23.6±0.2°;
19.4±0.2°, 18.9±0.2°, 15.5±0.2°, 8.8±0.2°, 18.1±0.2°, 24.9±0.2°, 17.4±0.2°, 12.3±0.2°, 26.1±0.2°, 14.5±0.2°;
19.4±0.2°, 18.9±0.2°, 15.5±0.2°, 8.8±0.2°, 18.1±0.2°, 24.9±0.2°, 17.4±0.2°, 12.3±0.2°, 26.1±0.2°, 24.3±0.2°.

In a further preferred embodiment of the present disclosure, for the crystal form I of hydroxyethyl sulfonate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one, measured by Cu-Kα radiation, X-ray characteristic diffraction peaks represented by 2θ angles and interplanar spacing d values are shown in Table 1.

**Table 1**

| No. | XRPD diffraction data of the crystal form I of hydroxyethyl sulfonate of compound of embodiment 13-1 | | | |
|---|---|---|---|---|
| | 2θ (±0.2 °) | d value | Peak height | Proportion (I%) |
| 1 | 21.678 | 4.0961 | 667 | 100 |
| 2 | 8.787 | 10.0546 | 554 | 83.1 |
| 3 | 19.266 | 4.6033 | 548 | 82.2 |
| 4 | 27.597 | 3.2296 | 491 | 73.6 |
| 5 | 10.866 | 8.1352 | 443 | 66.4 |
| 6 | 15.355 | 5.7657 | 377 | 56.5 |
| 7 | 16.694 | 5.3063 | 354 | 53.1 |
| 8 | 15.817 | 5.5982 | 330 | 49.5 |
| 9 | 17.487 | 5.0672 | 305 | 45.7 |
| 10 | 23.786 | 3.7377 | 291 | 43.6 |
| 11 | 14.685 | 6.0271 | 253 | 37.9 |
| 12 | 24.312 | 3.658 | 238 | 35.7 |
| 13 | 27.313 | 3.2625 | 213 | 31.9 |
| 14 | 23.424 | 3.7946 | 186 | 27.9 |
| 15 | 20.626 | 4.3027 | 177 | 26.5 |
| 16 | 23.066 | 3.8528 | 176 | 26.4 |
| 17 | 10.343 | 8.5454 | 166 | 24.9 |
| 18 | 29.176 | 3.0583 | 166 | 24.9 |
| 19 | 27.012 | 3.2981 | 154 | 23.1 |
| 20 | 28.167 | 3.1655 | 143 | 21.4 |

The crystal form I of hydroxyethyl sulfonate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one shown in embodiment 13-1 described in the present disclosure has an X-ray powder diffraction pattern basically as shown in FIG. 1; a DSC pattern basically as shown in FIG. 2; and a TGA pattern basically as shown in FIG. 3.

In a further preferred embodiment of the present disclosure, for the crystal form II of hydroxyethyl sulfonate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one shown in embodiment 13-1, measured by Cu-Kα radiation, X-ray characteristic diffraction peaks represented by 2θ angles and interplanar spacing d values are shown in Table 2.

**Table 2**

| No. | XRPD diffraction data of the crystal form II of hydroxyethyl sulfonate of compound of embodiment 13-1 | | | |
|---|---|---|---|---|
| | 2θ (±0.2 °) | d value | Peak height | Proportion (I%) |
| 1 | 21.699 | 4.0922 | 835 | 100 |
| 2 | 8.825 | 10.0123 | 598 | 71.6 |
| 3 | 19.268 | 4.6027 | 581 | 69.6 |
| 4 | 27.618 | 3.2272 | 540 | 64.7 |
| 5 | 10.872 | 8.1307 | 507 | 60.7 |
| 6 | 23.768 | 3.7405 | 456 | 54.6 |
| 7 | 16.732 | 5.294 | 445 | 53.3 |
| 8 | 15.374 | 5.7586 | 381 | 45.6 |
| 9 | 15.839 | 5.5905 | 362 | 43.4 |
| 10 | 17.523 | 5.0569 | 360 | 43.1 |
| 11 | 14.722 | 6.0122 | 323 | 38.7 |
| 12 | 24.353 | 3.6519 | 293 | 35.1 |
| 13 | 27.315 | 3.2623 | 245 | 29.3 |
| 14 | 29.161 | 3.0598 | 218 | 26.1 |
| 15 | 23.426 | 3.7943 | 212 | 25.4 |
| 16 | 23.157 | 3.8378 | 211 | 25.3 |
| 17 | 27.050 | 3.2936 | 208 | 24.9 |
| 18 | 10.364 | 8.5284 | 204 | 24.4 |
| 19 | 20.682 | 4.2912 | 182 | 21.8 |
| 20 | 28.209 | 3.1609 | 173 | 20.7 |
| 21 | 32.016 | 2.7931 | 148 | 17.7 |
| 22 | 9.985 | 8.8515 | 147 | 17.6 |

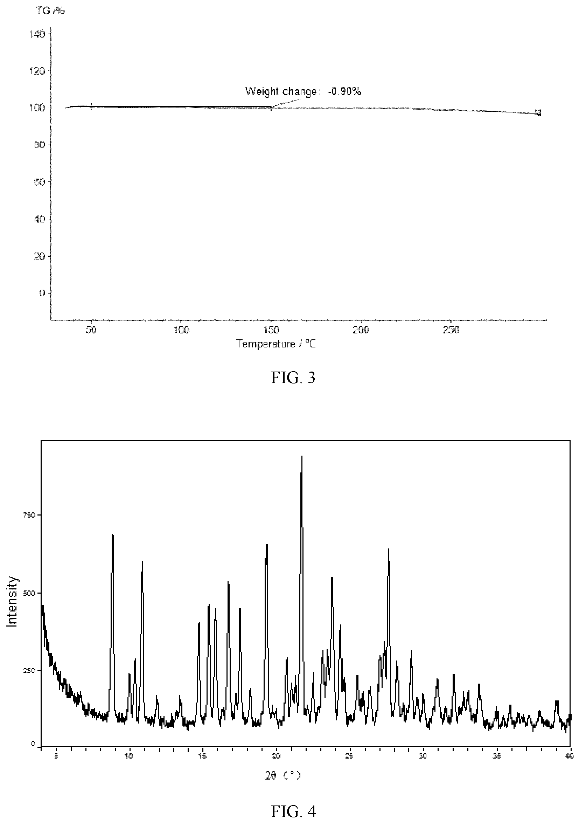

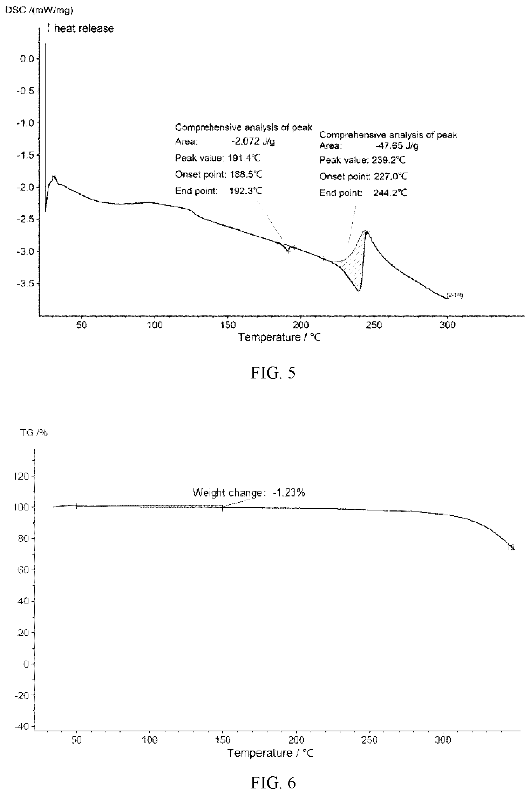

The crystal form II of hydroxyethyl sulfonate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one shown in embodiment 13-1 described in the present disclosure has an X-ray powder diffraction pattern basically as shown in FIG. 4; a DSC pattern basically as shown in FIG. 5; and a TGA pattern basically as shown in FIG. 6.

In a further preferred embodiment of the present disclosure, for the crystal form III of hydroxyethyl sulfonate of compound P-4-((2*S,*5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one shown in embodiment 13-1, measured by Cu-Kα radiation, X-ray characteristic diffraction peaks represented by 2θ angles and interplanar spacing d values are shown in Table 3.

**Table 3**

| No. | XRPD diffraction data of the crystal form III of hydroxyethyl sulfonate of compound of embodiment 13-1 | | | |
|---|---|---|---|---|
| | 2θ (±0.2 °) | d value | Peak height | Proportion (I%) |
| 1 | 19.407 | 4.5701 | 1117 | 100 |
| 2 | 16.854 | 5.256 | 475 | 42.5 |
| 3 | 26.567 | 3.3525 | 412 | 36.9 |
| 4 | 14.624 | 6.0523 | 336 | 30.1 |
| 5 | 28.004 | 3.1835 | 332 | 29.7 |
| 6 | 25.607 | 3.4758 | 284 | 25.4 |
| 7 | 20.667 | 4.2943 | 277 | 24.8 |
| 8 | 12.780 | 6.9213 | 269 | 24.1 |
| 9 | 19.106 | 4.6414 | 215 | 19.2 |
| 10 | 27.153 | 3.2814 | 209 | 18.7 |
| 11 | 24.356 | 3.6515 | 189 | 16.9 |
| 12 | 15.332 | 5.7743 | 185 | 16.6 |
| 13 | 26.198 | 3.3988 | 180 | 16.1 |
| 14 | 30.174 | 2.9594 | 176 | 15.8 |
| 15 | 27.352 | 3.2579 | 139 | 12.4 |
| 16 | 21.537 | 4.1226 | 132 | 11.8 |
| 17 | 11.503 | 7.6866 | 124 | 11.1 |
| 18 | 33.984 | 2.6358 | 124 | 11.1 |
| 19 | 25.005 | 3.5582 | 114 | 10.2 |
| 20 | 10.607 | 8.3332 | 107 | 9.6 |

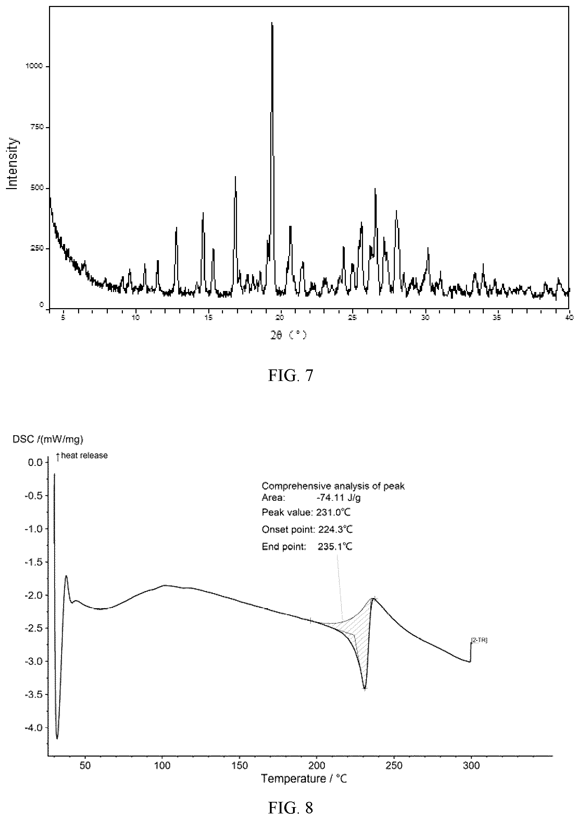

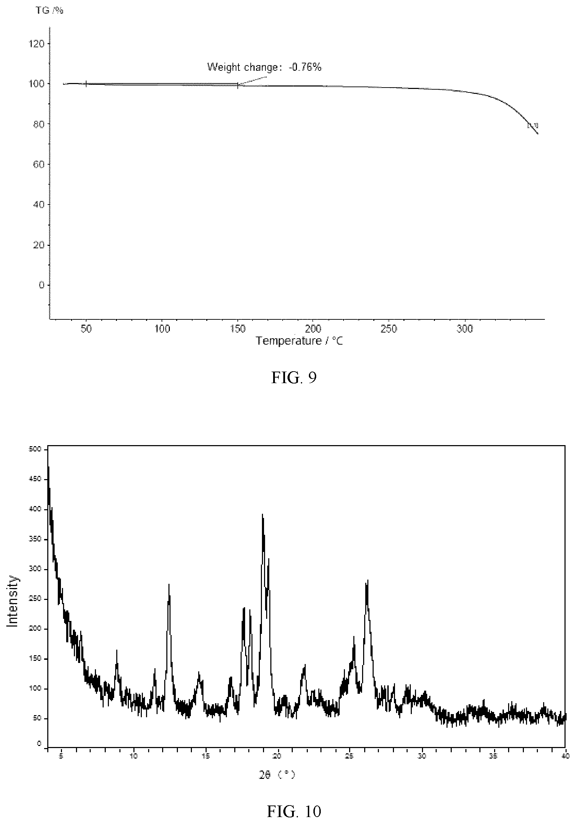

The crystal form III of hydroxyethyl sulfonate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one shown in embodiment 13-1 described in the present disclosure has an X-ray powder diffraction pattern basically as shown in FIG. 7; a DSC pattern basically as shown in FIG. 8; and a TGA pattern basically as shown in FIG. 9.

In a further preferred embodiment of the present disclosure, for the crystal form I of sulfate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one shown in embodiment 13-1, measured by Cu-Kα radiation, X-ray characteristic diffraction peaks represented by 2θ angles and interplanar spacing d values are shown in Table 4.

**Table 4**

| No. | XRPD diffraction data of the crystal form I of sulfate of compound of embodiment 13-1 | | | |
|---|---|---|---|---|
| | 2θ (±0.2 °) | d value | Peak height | Proportion (I%) |
| 1 | 18.962 | 4.6764 | 314 | 100 |
| 2 | 19.366 | 4.5797 | 238 | 75.8 |
| 3 | 12.415 | 7.1235 | 207 | 65.9 |
| 4 | 26.218 | 3.3962 | 190 | 60.5 |
| 5 | 17.643 | 5.0227 | 160 | 51 |
| 6 | 18.071 | 4.9049 | 146 | 46.5 |
| 7 | 25.284 | 3.5195 | 89 | 28.3 |
| 8 | 8.805 | 10.0344 | 81 | 25.8 |
| 9 | 21.859 | 4.0626 | 70 | 22.3 |
| 10 | 11.478 | 7.7032 | 62 | 19.7 |

The crystal form I of sulfate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one shown in embodiment 13-1 described in the present disclosure has an X-ray powder diffraction pattern basically as shown in FIG. 10.

In a further preferred embodiment of the present disclosure, for the crystal form II of sulfate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one shown in embodiment 13-1, measured by Cu-Kα radiation, X-ray characteristic diffraction peaks represented by 2θ angles and interplanar spacing d values are shown in Table 5.

**Table 5**

| No. | XRPD diffraction data of the crystal form II of sulfate of compound of embodiment 13-1 | | | |
|---|---|---|---|---|
| | 2θ (±0.2 °) | d value | Peak height | Proportion (I%) |
| 1 | 15.457 | 5.7279 | 620 | 100 |
| 2 | 11.133 | 7.9408 | 593 | 95.6 |
| 3 | 8.864 | 9.968 | 582 | 93.9 |
| 4 | 19.327 | 4.5888 | 562 | 90.6 |
| 5 | 22.265 | 3.9895 | 335 | 54 |
| 6 | 23.625 | 3.7628 | 300 | 48.4 |
| 7 | 17.420 | 5.0865 | 260 | 41.9 |
| 8 | 27.252 | 3.2697 | 257 | 41.5 |
| 9 | 16.952 | 5.2259 | 249 | 40.2 |
| 10 | 27.862 | 3.1994 | 249 | 40.2 |
| 11 | 15.818 | 5.5981 | 245 | 39.5 |
| 12 | 24.237 | 3.6691 | 238 | 38.4 |
| 13 | 21.760 | 4.0808 | 211 | 34 |
| 14 | 10.324 | 8.5617 | 194 | 31.3 |
| 15 | 20.624 | 4.303 | 191 | 30.8 |
| 16 | 15.009 | 5.8978 | 185 | 29.8 |
| 17 | 24.963 | 3.564 | 182 | 29.4 |
| 18 | 22.041 | 4.0296 | 160 | 25.8 |
| 19 | 34.611 | 2.5895 | 145 | 23.4 |
| 20 | 22.955 | 3.8711 | 143 | 23.1 |

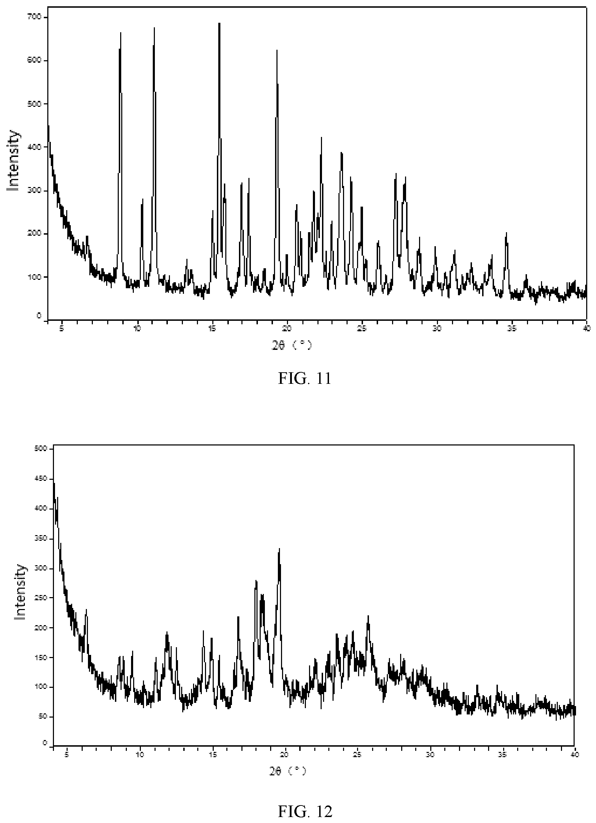

The crystal form II of sulfate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one shown in embodiment 13-1 described in the present disclosure has an X-ray powder diffraction pattern basically as shown in FIG. 11.

In a further preferred embodiment of the present disclosure, for the crystal form III of sulfate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one shown in embodiment 13-1, measured by Cu-Kα radiation, X-ray characteristic diffraction peaks represented by 2θ angles and interplanar spacing d values are shown in Table 6.

**Table 6**

| No. | XRPD diffraction data of the crystal form III of sulfate of compound of embodiment 13-1 | | | |
|---|---|---|---|---|
| | 2θ (±0.2 °) | d value | Peak height | Proportion (I%) |
| 1 | 19.589 | 4.528 | 226 | 100 |
| 2 | 17.950 | 4.9376 | 175 | 77.4 |
| 3 | 18.375 | 4.8244 | 148 | 65.5 |
| 4 | 16.754 | 5.2872 | 138 | 61.1 |
| 5 | 14.342 | 6.1706 | 119 | 52.7 |
| 6 | 11.845 | 7.4653 | 106 | 46.9 |
| 7 | 14.904 | 5.9393 | 103 | 45.6 |
| 8 | 25.713 | 3.4618 | 98 | 43.4 |
| 9 | 15.449 | 5.7308 | 80 | 35.4 |
| 10 | 23.546 | 3.7752 | 80 | 35.4 |
| 11 | 18.776 | 4.7221 | 76 | 33.6 |
| 12 | 24.653 | 3.6082 | 71 | 31.4 |
| 13 | 9.453 | 9.3479 | 69 | 30.5 |
| 14 | 8.827 | 10.0092 | 64 | 28.3 |
| 15 | 11.076 | 7.9818 | 64 | 28.3 |

The crystal form III of sulfate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one shown in embodiment 13-1 described in the present disclosure has an X-ray powder diffraction pattern basically as shown in FIG. 12.

In a further preferred embodiment of the present disclosure, for the crystal form IV of sulfate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-d]pyrimidin-2(1*H*)-one shown in embodiment 13-1, measured by Cu-Kα radiation, the X-ray characteristic diffraction peaks represented by 2θ angles and interplanar spacing d values are shown in Table 7.

**Table 7**

| No. | XRPD diffraction data of the crystal form IV of sulfate of compound of embodiment 13-1 | | | |
|---|---|---|---|---|
| | 2θ (±0.2 °) | d value | Peak height | Proportion (I%) |
| 1 | 19.388 | 4.5745 | 273 | 100 |
| 2 | 18.898 | 4.6919 | 189 | 69.2 |
| 3 | 15.473 | 5.722 | 124 | 45.4 |
| 4 | 8.848 | 9.9859 | 109 | 39.9 |
| 5 | 18.070 | 4.9049 | 102 | 37.4 |
| 6 | 24.885 | 3.5751 | 95 | 34.8 |
| 7 | 17.443 | 5.08 | 92 | 33.7 |
| 8 | 12.256 | 7.2159 | 90 | 33 |
| 9 | 26.097 | 3.4117 | 87 | 31.9 |
| 10 | 14.479 | 6.1124 | 82 | 30 |
| 11 | 22.207 | 3.9997 | 81 | 29.7 |
| 12 | 24.334 | 3.6548 | 79 | 28.9 |
| 13 | 21.678 | 4.0962 | 77 | 28.2 |
| 14 | 23.600 | 3.7668 | 77 | 28.2 |

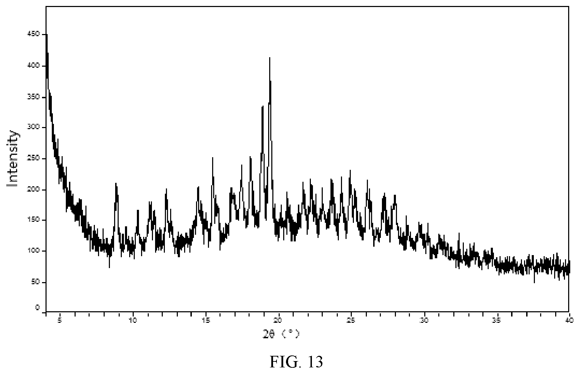

The crystal form IV of sulfate of compound P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one shown in embodiment 13-1 described in the present disclosure has an X-ray powder diffraction pattern basically as shown in FIG. 13.

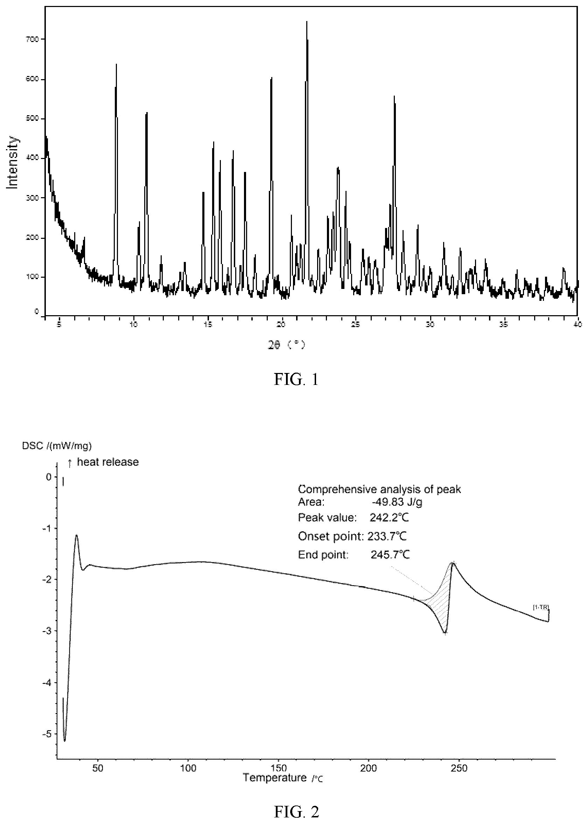

In a further preferred embodiment of the present disclosure, positions of diffraction peaks with relative peak intensity of top ten in the X-ray powder diffraction pattern of the crystal form I of hydroxyethyl sulfonate and diffraction peaks at the corresponding positions in FIG. 1 have a 2θ error of ±0.2° to ±0.5°, preferably ±0.2° to ±0.3°, most preferably ±0.2°;
positions of diffraction peaks with relative peak intensity of top ten in the X-ray powder diffraction pattern of the crystal form II of hydroxyethyl sulfonate and diffraction peaks at the corresponding positions in FIG. 4 have a 2θ error of ±0.2° to ±0.5°, preferably ±0.2° to ±0.3°, most preferably ±0.2°;
positions of diffraction peaks with relative peak intensity of top ten in the X-ray powder diffraction pattern of the crystal form III of hydroxyethyl sulfonate and diffraction peaks at the corresponding positions in FIG. 7 have a 2θ error of ±0.2° to ±0.5°, preferably ±0.2° to ±0.3°, most preferably ±0.2°;
positions of diffraction peaks with relative peak intensity of top ten in the X-ray powder diffraction pattern of the crystal form I of sulfate and diffraction peaks at the corresponding positions in FIG. 10 have a 2θ error of ±0.2° to ±0.5°, preferably ±0.2° to ±0.3°, most preferably ±0.2°;
positions of diffraction peaks with relative peak intensity of top ten in the X-ray powder diffraction pattern of the crystal form II of sulfate and diffraction peaks at the corresponding positions in FIG. 11 have a 2θ error of ±0.2° to ±0.5°, preferably ±0.2° to ±0.3°, most preferably ±0.2°;
positions of diffraction peaks with relative peak intensity of top ten in the X-ray powder diffraction pattern of the crystal form III of sulfate and diffraction peaks at the corresponding positions in FIG. 12 have a 2θ error of ±0.2° to ±0.5°, preferably ±0.2° to ±0.3°, most preferably ±0.2°;
positions of diffraction peaks with relative peak intensity of top ten in the X-ray powder diffraction pattern of the crystal form IV of sulfate and diffraction peaks at the corresponding positions in FIG. 13 have a 2θ error of ±0.2° to ±0.5°, preferably ±0.2° to ±0.3°, most preferably ±0.2°.

In a further preferred embodiment of the present disclosure, for the acid salt of the compound, the crystal form of the acid salt is a hydrate or an anhydrate, when the crystal form of the acid salt is the hydrate, the number of water is 0.2 to 3, preferably 0.2, 0.5, 1, 1.5, 2, 2.5 or 3, more preferably 0.5, 1, 2 or 3; further, the water in the hydrate is pipeline water, or crystal water, or a combination of both.

In a further preferred embodiment of the present disclosure, a method for preparing an acid salt comprises the following steps:
1) weighing an appropriate amount of a free base, and adding a solvent to dissolve;
2) adding an appropriate amount of acid and stirring; wherein an amount of the acid is preferably 1.2 equivalents;
3) centrifuging, rapidly, or standing to obtain a salt of the compound;

the solvent is an organic solvent, preferably at least one of ethanol, 2-methyltetrahydrofuran, toluene, isopropyl acetate, *tert*-butanol, *n*-butanol, tetrahydrofuran, acetone, 2-butanone, ethyl acetate or 1,4-dioxane;
the acid is selected from hydrochloric acid, sulfuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, ethanesulfonic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, capric acid, caproic acid, octanoic acid, cinnamic acid, citric acid, cyclamic acid, camphor sulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecyl sulfuric acid, dibenzoyl tartaric acid, 1,2-ethanedisulfonic acid, ethanesulfonic acid, formic acid, fumaric acid, galactonic acid, gentisic acid, glutaric acid, 2-ketoglutaric acid, glycolic acid, hippuric acid, hydroxyethyl sulfonic acid, lactobionic acid, ascorbic acid, aspartic acid, lauric acid, camphoric acid, maleate acid, malonic acid, methanesulfonic acid, 1,5-naphthalene disulfonic acid, naphthalene-2-sulfonic acid, nicotinic acid, oleic acid, orotic acid, oxalic acid, palmitic acid, pamoic acid, propionic acid, salicylic acid, 4-aminosalicylic acid, sebacic acid, stearic acid, succinic acid, thiocyanic acid, undecylenic acid, trifluoroacetic acid, benzenesulfonic acid, *p-*toluenesulfonic acid or L-malic acid; preferably hydrochloric acid, phosphoric acid, ethanesulfonic acid, benzenesulfonic acid, methanesulfonic acid, fumaric acid, hydroxyethyl sulfonic acid, oxalic acid or hydrobromic acid.

In a further preferred embodiment of the present disclosure, a method for preparing the acid salt of the compound and a crystal form thereof comprises the following steps:
1) weighing an appropriate amount of a free base, and adding a reaction solvent to dissolve;
2) adding an appropriate amount of acid and stirring; wherein an amount of the acid is preferably 1.2 equivalents;
3) centrifuging and drying to obtain a crystal form of the acid salt of the compound;

the reaction solvent is an organic solvent, preferably at least one of ethanol, 2-methyltetrahydrofuran, *n*-heptane, methyl *tert*-butyl ether, toluene, isopropyl acetate, *tert-*butanol, *n*-butanol, tetrahydrofuran, acetone, 2-butanone, ethyl acetate or 1,4-dioxane;
the acid is selected from hydrochloric acid, sulfuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, ethanesulfonic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, capric acid, caproic acid, octanoic acid, cinnamic acid, citric acid, cyclamic acid, camphor sulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecyl sulfuric acid, dibenzoyl tartaric acid, 1,2-ethanedisulfonic acid, ethanesulfonic acid, formic acid, fumaric acid, galactonic acid, gentisic acid, glutaric acid, 2-ketoglutaric acid, glycolic acid, hippuric acid, hydroxyethyl sulfonic acid, lactobionic acid, ascorbic acid, aspartic acid, lauric acid, camphoric acid, maleate acid, malonic acid, methanesulfonic acid, 1,5-naphthalene disulfonic acid, naphthalene-2-sulfonic acid, nicotinic acid, oleic acid, orotic acid, oxalic acid, palmitic acid, pamoic acid, propionic acid, salicylic acid, 4-aminosalicylic acid, sebacic acid, stearic acid, succinic acid, thiocyanic acid, undecylenic acid, trifluoroacetic acid, benzenesulfonic acid, *p-*toluenesulfonic acid or L-malic acid; preferably hydrochloric acid, phosphoric acid, ethanesulfonic acid, benzenesulfonic acid, methanesulfonic acid, fumaric acid, hydroxyethyl sulfonic acid, oxalic acid or hydrobromic acid.

In a further preferred embodiment of the present disclosure, a method for preparing a crystal form of the acid salt of the compound comprises the following steps:
1) weighing an appropriate amount of a salt of the compound, and adding an organic solvent for suspension;
2) stirring, centrifuging and drying to obtain the crystal form of the acid salt of the compound;
   the organic solvent is selected from at least one of ethanol, 2-methyltetrahydrofuran, *n*-heptane, methyl *tert*-butyl ether, toluene, isopropyl acetate, *tert*-butanol, *n*-butanol, tetrahydrofuran, acetone, 2-butanone, ethyl acetate or 1,4-dioxane.

In a further preferred embodiment of the present disclosure, a method for preparing the acid salt of the compound or the crystal form thereof comprises the following steps:
1) weighing an appropriate amount of a free base, and adding a reaction solvent to dissolve;
2) adding an appropriate amount of acid and an organic solvent, stirring and dissolving to clear;
3) adding, optionally, a seed crystal;
4) cooling, filtering to precipitate a solid, and washing with a solvent, drying.

The reaction solvent used in step 1) is an organic solvent, preferably at least one of ethanol, propanol, isopropanol, 2-methyltetrahydrofuran, n-heptane, methyl *tert*-butyl ether, toluene, isopropyl acetate, *tert*-butanol, *n*-butanol, tetrahydrofuran, acetone, 2-butanone, ethyl acetate or 1,4-dioxane;
the acid in step 2) is selected from hydrochloric acid, sulfuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, ethanesulfonic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, capric acid, caproic acid, octanoic acid, cinnamic acid, citric acid, cyclamic acid, camphor sulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecyl sulfuric acid, dibenzoyl tartaric acid, 1,2-ethanedisulfonic acid, ethanesulfonic acid, formic acid, fumaric acid, galactonic acid, gentisic acid, glutaric acid, 2-ketoglutaric acid, glycolic acid, hippuric acid, hydroxyethyl sulfonic acid, lactobionic acid, ascorbic acid, aspartic acid, lauric acid, camphoric acid, maleate acid, malonic acid, methanesulfonic acid, 1,5-naphthalene disulfonic acid, naphthalene-2-sulfonic acid, nicotinic acid, oleic acid, orotic acid, oxalic acid, palmitic acid, pamoic acid, propionic acid, salicylic acid, 4-aminosalicylic acid, sebacic acid, stearic acid, succinic acid, thiocyanic acid, undecylenic acid, trifluoroacetic acid, benzenesulfonic acid, *p*-toluenesulfonic acid or L-malic acid; preferably hydrochloric acid, phosphoric acid, ethanesulfonic acid, benzenesulfonic acid, methanesulfonic acid, fumaric acid, hydroxyethyl sulfonic acid, oxalic acid or hydrobromic acid.

The organic solvent in step 2) is selected from one or more of alcohol, ether, ketone or ester solvents, preferably at least one of ethanol, propanol, isopropanol, 2-methyltetrahydrofuran, *n*-heptane, methyl-*tert*-butyl ether, toluene, isopropyl acetate, *tert-*butanol, n-butanol, tetrahydrofuran, acetone, 2-butanone, ethyl acetate or 1,4-dioxane;
the solvent in step 3) is selected from one or more of alcohol, ether, ketone or ester solvents, preferably at least one of ethanol, propanol, isopropanol, 2-methyltetrahydrofuran, *n-*heptane, methyl *tert*-butyl ether, toluene, isopropyl acetate, *tert*-butanol, *n*-butanol, tetrahydrofuran, acetone, 2-butanone, ethyl acetate or 1,4-dioxane.

The present disclosure also provides a preferred embodiment, and relates to a pharmaceutical composition comprising a therapeutically effective amount of the acid salt of the compound represented by general formula (I) or the crystal form thereof, and one or more pharmaceutically acceptable carriers, diluents or excipients.

The present disclosure further relates to a use of any one of the acid salts of the compound represented by general formula (I) or the crystal forms thereof, or the pharmaceutical composition in the manufacture of a medicament of a KRAS inhibitor; preferably a use in the manufacture of a medicament of a KRAS G12C mutation inhibitor.

In some embodiments, the pharmaceutically acceptable salt of the compound and the crystal form thereof, or the composition of the present disclosure is used for treating Noonan syndrome, leopard syndrome, leukemia, neuroblastoma, melanoma, esophagus cancer, head and neck tumor, breast cancer, lung cancer and colon cancer; preferably non-small cell lung cancer, colon cancer, esophagus cancer, and head and neck tumor.

### Detailed description of the invention

Unless otherwise stated, the terms used in the description and claims have the following meanings.

The term "alkyl" refers to a saturated aliphatic hydrocarbon group, which is a straight or branched chain group containing 1 to 20 carbon atoms, preferably alkyl containing 1 to 8 carbon atoms, more preferably alkyl containing 1 to 6 carbon atoms, most preferably alkyl containing 1 to 3 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers. More preferably lower alkyl containing 1 to 6 carbon atoms, non-limiting examples include methyl, ethyl, *n*-propyl, isopropyl, *n*-butyl, isobutyl, *tert*-butyl, sec-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl may be substituted or unsubstituted, when substituted, the substituents may be substituted at any available attachment point, the substituents are preferably one or more of the following groups, which are independently selected from alkyl, alkenyl, alkynyl, alkoxyl, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate, preferably alkyl substituted by methyl, ethyl, isopropyl, tert-butyl, haloalkyl, deuterated alkyl, alkoxy-substituted alkyl and hydroxyl-substituted alkyl.

The term "alkylene" refers to that one hydrogen atom of alkyl is further substituted, for example: "methylene" refers to -CH₂-, "ethylene" refers to -(CH₂)₂-, and "propylene" refers to -(CH₂)₃-, "butylene" refers to -(CH₂)₄-, etc. The term "alkenyl" refers to alkyl as defined above containing at least two carbon atoms and at least one carbon-carbon double bond, such as vinyl, 1-propenyl, 2-propenyl, 1-, 2-, or 3 -butenyl etc. The alkenyl may be substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio.

The term "cycloalkyl" refers to a saturated or partially unsaturated monocyclic or polycyclic cyclic hydrocarbon substituent, the cycloalkyl ring contains 3 to 20 carbon atoms, preferably 3 to 12 carbon atoms, more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctanyl, etc.; polycylic cycloalkyl includes spiro, fused and bridged cycloalkyl, preferably cyclopropyl, cyclobutyl, cyclohexyl, cyclopentyl and cycloheptyl.

The term "spirocycloalkyl" refers to polycyclyl that shares one carbon atom (called a spiro atom) between 5- to 20-membered monocyclic rings, which may contain one or more double bonds, but none of the rings has a fully conjugated π-electron system. Preferably 6-14-membered, more preferably 7-10-membered. According to the number of shared spiro atoms between the rings, the spirocycloalkyl is classified into monospirocycloalkyl, bispirocycloalkyl or polyspirocycloalkyl, preferably monospirocycloalkyl and bispirocycloalkyl. More preferably, 3-membered/6-membered, 3-membered/5-membered, 4-membered/4-membered, 4-membered/5-membered, 4-membered/6-membered, 5-membered/5-membered, or 5-membered/6-membered monospirocycloalkyl. Non-limiting examples of spirocycloalkyl include: 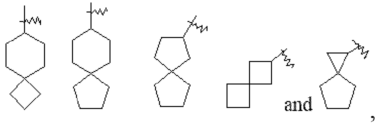 etc.;
also include spirocycloalkyl in which monospirocycloalkyl and heterocycloalkyl share a spiro atom, and non-limiting examples include: 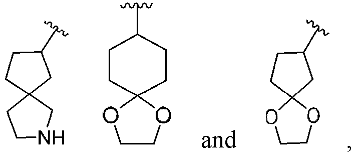 etc.

The term "fused cycloalkyl" refers to a 5-20-membered all-carbon polycyclic group in which each ring in the system shares an adjacent pair of carbon atoms with other rings in the system, wherein one or more of the rings may comprise one or multiple double bonds, but none of the rings has a fully conjugated π-electron system. Preferably 6-14-membered, more preferably 7-10-membered. According to the number of constituent rings, it can be classified into bicyclic, tricyclic, tetracyclic or polycyclic fused cycloalkyl, preferably bicyclic or tricyclic, and more preferably 5-membered/5-membered or 5-membered/6-membered bicyclic alkyl. Non-limiting examples of fused cycloalkyls include: 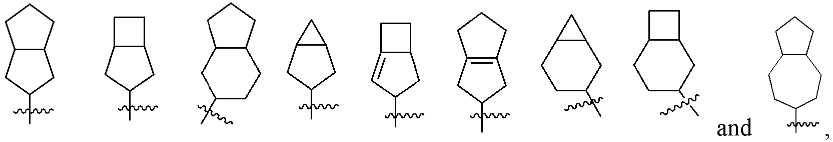 etc.

The term "bridged cycloalkyl" refers to 5 to 20-membered all-carbon polycyclic group, in which any two rings share two carbon atoms that are not directly connected, it may contain one or more double bonds, but none of the rings has a fully conjugated π-electron system. Preferably 6-14-membered, more preferably 7-10-membered. According to the number of constituent rings, it can be classified into bicyclic, tricyclic, tetracyclic or polycyclic bridged cycloalkyl, preferably bicyclic, tricyclic, or tetracyclic, and more preferably bicyclic or tricyclic. Non-limiting examples of bridged cycloalkyl include:

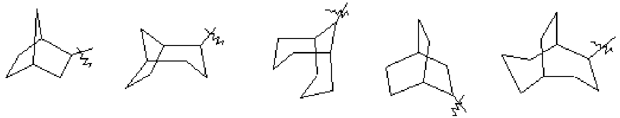

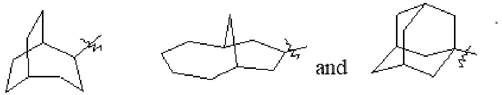

The cycloalkyl ring may be fused to an aryl, heteroaryl or heterocycloalkyl ring, wherein the ring connected to the parent structure is cycloalkyl, non-limiting examples include indanyl, tetrahydronaphthyl, benzocycloheptanyl, etc. The cycloalkyl may be substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboylate.

The term "heterocyclyl" refers to saturated or partially unsaturated monocyclic or polycyclic hydrocarbon substituent containing 3 to 20 ring atoms, wherein one or more of the ring atoms are heteroatoms selected from nitrogen, oxygen or S(O)ₘ (wherein m is an integer of 0 to 2), but not including the ring part of -O-O-, -O-S- or -S-S-, and the remaining ring atoms are carbon. It preferably contains 3 to 12 ring atoms, wherein 1 to 4 ring atoms are heteroatoms; more preferably contains 3 to 8 ring atoms; most preferably contains 3 to 8 ring atoms; further preferably 3-8-membered heterocyclyl containing 1 to 3 nitrogen atoms, optionally substituted by 1 to 2 oxygen atoms, sulfur atoms or oxo, including nitrogen-containing monocyclic heterocyclyl, nitrogen-containing spiro heterocyclyl or nitrogen-containing fused heterocyclyl.

Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, azepyl, 1,4-diazepanyl, pyranyl, etc., preferably pyrrolidinyl, morpholinyl, piperidinyl, azepanyl, 1,4-diazepanyl and piperazinyl. Polycyclic heterocyclyl include spiro-, fused- and bridged heterocyclyl; the spiro-, fused- and bridged heterocyclyl are optionally connected to other groups through a single bond, or to connect to other cycloalkyl, heterocyclyl, aryl and heteroaryl through any two or more of ring atoms.

The term "spiroheterocyclyl" refers to polycyclic heterocyclyl sharing one atom (called a spiro atom) between 5-20-membered monocyclic ring, wherein one or more ring atoms are selected from nitrogen, oxygen or S(O)ₘ (wherein m is an integer of 0 to 2) heteroatoms, and the remaining ring atoms are carbon. It may contain one or more double bonds, but none of the rings has fully conjugated π-electron system. Preferably 6-14-membered, more preferably 7-10-membered. According to the number of spiro atoms shared between the rings, the spiro heterocyclyl is classified into monospiroheterocyclyl, dispiroheterocyclyl or polyspiroheterocyclyl, preferably monospiroheterocyclyl and dispiroheterocyclyl. More preferably, 3-membered/5-membered, 3-membered/6-membered, 4-membered/4-membered, 4-membered/5-membered, 4-membered/6-membered, 5-membered/5-membered, or 5-membered/6-membered monospiroheterocyclyl. Non-limiting examples of spiroheterocyclyl include: 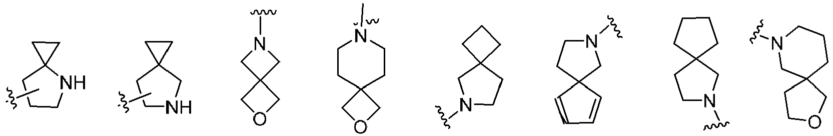 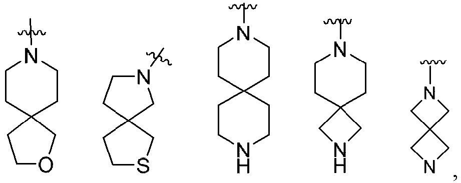 etc.

The term "fused heterocyclyl" refers to a 5-20-membered polycyclic heterocylic group in which each ring in the system shares an adjacent pair of atoms with other rings in the system, one or more of the rings may comprise one or multiple double bonds, but none of the rings has a fully conjugated π-electron system, wherein one or more of the ring atoms are heteroatoms selected from nitrogen, oxygen or S(O)ₘ (wherein m is an integer of 0 to 2), the rest of the ring atoms are carbon. Preferably 6-14-membered, more preferably 7-10-membered. According to the number of constituent rings, it can be classified into bicyclic, tricyclic, tetracyclic or polycyclic fused heterocyclyl, preferably bicyclic or tricyclic, and more preferably 5-membered/5-membered or 5-membered/6-membered bicyclic fused heterocylyl. Non-limiting examples of fused heterocylyl include: 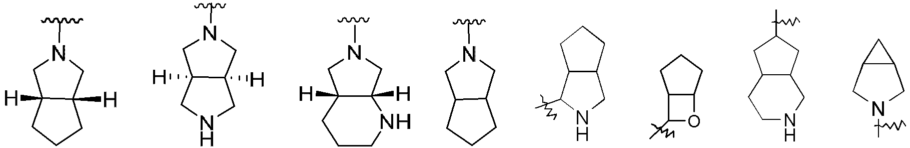 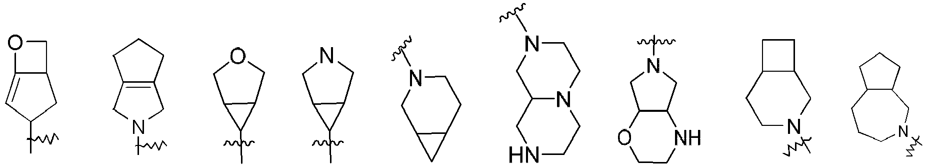 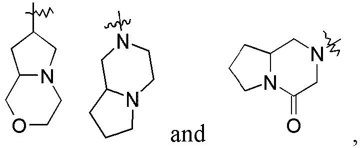 etc.

The term "bridged heterocyclyl" refers to polycyclic heterocylic group in which any two rings share two atoms that are not directly connected, it may contain one or multiple double bonds, but none of the rings has a fully conjugated π-electron system, wherein one or more of the ring atoms are heteroatoms selected from nitrogen, oxygen or S(O)ₘ (wherein m is an integer of 0 to 2), the rest of the ring atoms are carbon. Preferably 6-14-membered, more preferably 7-10-membered. According to the number of constituent rings, it can be classified into bicyclic, tricyclic, tetracyclic or polycyclic bridged heterocyclyl, preferably bicyclic, tricyclic, or tetracyclic, and more preferably bicyclic or tricyclic. Non-limiting examples of bridged heterocylyl include: 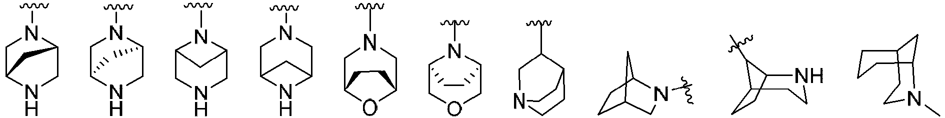 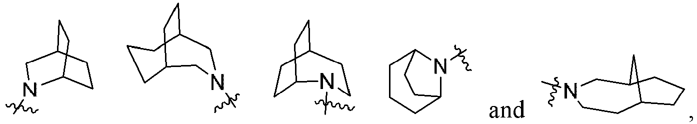 etc.

The heterocyclic ring may be fused to an aryl, heteroaryl or cycloalkyl ring, wherein the ring connected to the parent structure is heterocyclyl, and non-limiting examples of heterocyclyl include: 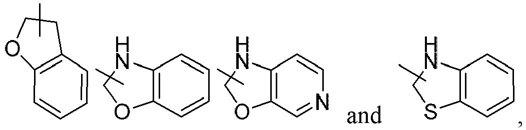 etc.

The heterocyclyl may be substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboylate.

The term "aryl" refers to a 6-14-membered all-carbon monocyclic or fused polycyclic (that is, rings sharing adjacent pairs of carbon atoms) with conjugated π-electron system, preferably 6-12-membered, such as phenyl and naphthyl. More preferably phenyl. The aryl ring may be fused on a heteroaryl, heterocyclyl or cycloalkyl ring, including benzo 5-10-membered heteroaryl, benzo 3-8-membered cycloalkyl and benzo 3-8-membered heteroalkyl, preferably benzo 5-6-membered heteroaryl, benzo 3-6-membered cycloalkyl and benzo 3-6-membered heteroalkyl, wherein the heterocyclyl is heterocyclyl containing 1 to 3 nitrogen atoms, oxygen atoms and sulfur atoms; or a 3-membered nitrogen-containing fused ring containing a benzene ring.

Herein, the ring connected to the parent structure is an aryl ring, and non-limiting examples of aryl include: 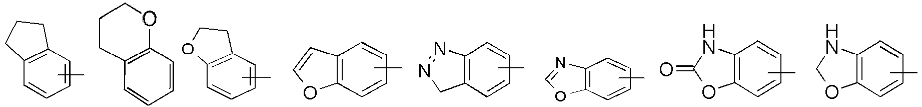 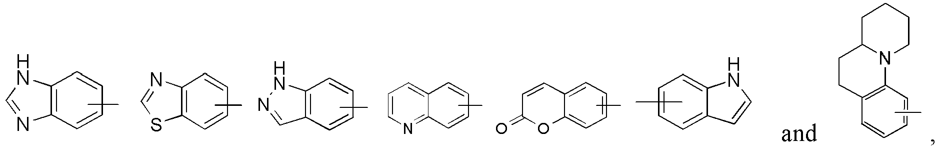 etc.

The aryl may be substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalky, heterocycloalky, aryl, heteroaryl, cycloalkoxyl, heterocycloalkoxyl, cycloalkylthio, heterocycloalkylthio, carboxyl or carboylate.

The term "heteroaryl" refers to a heteroaromatic system containing 1 to 4 heteroatoms and 5 to 14 ring atoms, wherein the heteroatoms are selected from oxygen, sulfur, and nitrogen. The heteroaryl is preferably 5-12-membered, more preferably 5- or 6-membered, such as imidazole, furanyl, thiophenyl, thiazolyl, pyrazolyl, oxazolyl, pyrrolyl, triazolyl, tetrazolyl, pyridyl, pyrimidinyl, thiadiazole, pyrazinyl, etc., preferably triazolyl, thiophenyl, imidazolyl, pyrazolyl, oxazolyl, pyrimidinyl or thiazolyl; more preferably pyrazolyl, pyrrolyl and oxazolyl. The heteroaryl ring may be fused to an aryl, heteroaryl or cycloalkyl ring, wherein the ring connected to the parent structure is the heteroaryl ring, and non-limiting examples of heteroaryl include: 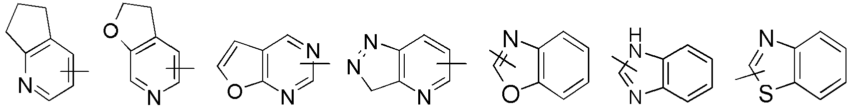 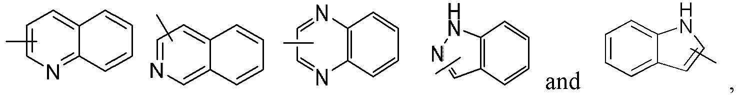 etc.

The heteroaryl may be optionally substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalky, heterocycloalky, aryl, heteroaryl, cycloalkoxyl, heterocycloalkoxyl, cycloalkylthio, heterocycloalkylthio, carboxyl or carboylate.

The term "alkoxy" refers to -O-(alkyl) and -O-(unsubstituted cycloalkyl), wherein the definition of alkyl is as described above, preferably alkyl containing 1 to 8 carbon atoms, more preferably alkyl containing 1 to 6 carbon atoms, most preferably alkyl containing 1 to 3 carbon atoms. Non-limiting examples of alkoxy include: methoxy, ethoxy, propoxy, butoxy, cyclopropoxy, cyclobutoxy, cyclopentyloxy, cyclohexyloxy. The alkoxy may be optionally substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

The term "alkylthio" refers to -S-(alkyl) and -S-(unsubstituted cycloalkyl), wherein the definition of alkyl is as described above. Preferably alkyl containing 1 to 8 carbon atoms, more preferably alkyl containing 1 to 6 carbon atoms, most preferably alkyl containing 1 to 3 carbon atoms. Non-limiting examples of alkylthio include: methylthio, ethylthio, propylthio, butylthio, cyclopropylthio, cyclobutylthio, cyclopentylthio, cyclohexylthio. The alkylthio may be optionally substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

"Alkylthio-alkyl" refers to alkylthio attached to alkyl, wherein the alkyl and the alkylthio are as defined above.

"Alkylaminocarbonyl" refers to (alkyl)-N-C(O)-, wherein the alkyl is as defined above.

"Haloalkyl" refers to alkyl substituted by one or more halogens, wherein the alkyl is as defined above.

"Haloalkoxy" refers to alkoxy substituted by one or more halogens, wherein the alkoxy is as defined above.

"Haloalkoxy" refers to alkylthio substituted by one or more halogens, wherein the alkylthio is as defined above.

"Hydroxyalkyl" refers to alkyl substituted by one or more hydroxyl, wherein the alkyl is as defined above.

"Alkenyl" refers to chain alkenyl, also known as alkylene, preferably alkyl containing 2 to 8 carbon atoms, more preferably alkyl containing 2 to 6 carbon atoms, most preferably alkyl containing 2 to 3 carbon atoms. Herein, the alkenyl may be further substituted with other related groups, such as: alkyl, alkenyl, alkynyl, alkoxy, alkylthio, alkylamino, halogen, sulfhydryl, hydroxyl, nitro, cyano, cycloalky, heterocycloalkyl, aryl, heteroaryl, cycloalkoxy, heterocycloalkoxy, cycloalkylthio, heterocycloalkylthio, carboxyl or carboxylate.

"Alknyl" refers to (CH=C-), preferably alkyl containing 2 to 8 carbon atoms, more preferably alkyl containing 2 to 6 carbon atoms, most preferably alkyl containing 2 to 3 carbon atoms. Herein, the alknyl may be further substituted by other related groups, for example: alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

The term "alkenylcarbonyl" refers to -C(O)-(alkenyl), wherein the alkenyl is as defined above. Non-limiting examples of alkenylcarbonyl include: vinylcarbonyl, propenylcarbonyl, butenylcarbonyl. The alkenylcarbonyl may be optionally substituted or unsubstituted, when substituted, the substituents are preferably one or more of the following groups, which are independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

"Hydroxyl" refers to an -OH group.

"Halogen" refers to fluorine, chlorine, bromine or iodine.

"Amino" refers to -NH₂.

"Cyano" refers to -CN.

"Nitro" refers to -NO₂.

"Carbonyl" refers to -C(O)-.

"Carboxyl" refers to -C(O)OH.

"THF" refers to tetrahydrofuran.

"EtOAc" refers to ethyl acetate.

"MeOH" refers to methanol.

"DMF" refers to *N,N*-dimethylformamide.

"DIPEA" refers to diisopropylethylamine.

"TFA" refers to trifluoroacetic acid.

"MeCN" refers to acetonitrile.

"DMA" refers to *N,N*-dimethylacetamide.

"Et₂O" refers to diethyl ether.

"DCE" refers to 1,2 dichloroethane.

"DIPEA" refers to *N,N-*diisopropylethylamine.

"NBS" refers to *N*-bromosuccinimide.

"NIS" refers to *N*-iodosuccinimide.

"Cbz-Cl" refers to benzyl chloroformate.

"Pd₂(dba)₃" refers to tris(dibenzylideneacetone)dipalladium.

"Dppf" refers to 1,1'-bis(diphenylphosphino)ferrocene.

"HATU" refers to 2-(7-azabenzotriazol-1-yl)-*N,N,N*',*N*'-tetramethyluronium hexafluorophosphate.

"KHMDS" refers to potassium hexamethyldisilazide.

"LiHMDS" refers to lithium bistrimethylsilylamide.

"MeLi" refers to methyl lithium.

"N-BuLi" refers to *n*-butyl lithium.

"NaBH(OAc)₃" refers to sodium triacetoxyborohydride.

"X is selected from A, B, or C", "X is selected from A, B and C", "X is A, B or C", "X is A, B and C" and other terms all express the same meaning, which means that X can be any one or more of A, B, and C.

The hydrogen atom described in the present disclosure may be replaced by its isotope deuterium, and any hydrogen atom in the compounds according to the embodiments of the present disclosure may also be replaced by a deuterium atom.

"Optional" or "optionally" refers to that the event or environment described later may, but not necessarily, occur, and the description includes occasions where the event or environment occurs or does not occur. For example, "heterocyclic group optionally substituted by alkyl" refers to that alkyl may, but not necessarily, be present, and the description includes the case where the heterocyclic group is substituted by alkyl and the case where the heterocyclic group is not substituted by alkyl.

"Substituted" refers to one or more hydrogen atoms in the group, preferably up to 5, more preferably 1 to 3 hydrogen atoms, independently substituted by a corresponding number of substituents. It goes without saying that the substituents are only in their possible chemical positions, and those skilled in the art can determine possible or impossible substitutions (by experiment or theory) without too much effort. For example, amino or hydroxyl having free hydrogen may be unstable when combined with a carbon atom having an unsaturated (e.g., olefinic) bond.

"Pharmaceutical composition" refers to a mixture containing one or more of the compounds described herein or the physiologically/pharmaceutically acceptable salt or prodrug thereof and other chemical components, and the other component is, for example, physiological/pharmaceutically acceptable carrier and excipient. The purpose of the pharmaceutical composition is to promote the administration to an organism, facilitate the absorption of an active ingredient and then exert the biological activity.

"Pharmaceutically acceptable salt" refers to the salt of the compound of the present disclosure, which is safe and effective when used in mammals, and has due biological activity.

### BRIEF DESCRIPTION OF THE DRAWINGS

FIG. 1 is an XRPD pattern of a crystal form I of hydroxyethyl sulfonate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one.
FIG. 2 is a DSC pattern of the crystal form I of hydroxyethyl sulfonate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one.
FIG. 3 is a TGA pattern of the crystal form I of hydroxyethyl sulfonate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one.
FIG. 4 is an XRPD pattern of a crystal form II of hydroxyethyl sulfonate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one.
FIG. 5 is a DSC pattern of the crystal form II of hydroxyethyl sulfonate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one.
FIG. 6 is a TGA pattern of the crystal form II of hydroxyethyl sulfonate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one.
FIG. 7 is an XRPD pattern of a crystal form III of hydroxyethyl sulfonate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one.
FIG. 8 is a DSC pattern of the crystal form III of hydroxyethyl sulfonate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one.
FIG. 9 is a TGA pattern of the crystal form III of hydroxyethyl sulfonate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one.
FIG. 10 is an XRPD pattern of a crystal form I of sulfate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one.
FIG. 11 is an XRPD pattern of a crystal form II of sulfate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one.
FIG. 12 is an XRPD pattern of a crystal form III of sulfate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1H)-one.
FIG. 13 is an XRPD pattern of a crystal form IV of sulfate of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one.

### DETAILED DESCRIPTION OF THE PREFERRED EMBODIMENT

The following embodiments will further describe the present disclosure, but these embodiments do not limit the scope of the present disclosure.

### 1. Preparation of compounds

### Embodiment

The structures of the compounds of the present disclosure were determined by nuclear magnetic resonance (NMR) or/and liquid chromatography-mass spectrometry (LC-MS). NMR chemical shift (δ) was given in units of parts per million (ppm). NMR was determined using a Bruker AVANCE-400 NMR instrument with deuterated dimethyl sulfoxide (DMSO-*d₆*)*,* deuterated methanol (CD₃OD) and deuterated chloroform (CDCl₃) as solvents and tetramethylsilane (TMS) as internal standard.

Liquid chromatography-mass spectrometry LC-MS was determined with an Agilent 1200 Infinity Series mass spectrometer. HPLC determinations were performed using an Agilent 1200DAD high pressure liquid chromatograph (Sunfire C18 150 × 4. 6 mm column) and a Waters 2695-2996 high pressure liquid chromatograph (Gimini C18 150 × 4. 6 mm column).

Yantai Huanghai HSGF254 or Qingdao GF254 silica gel plate was used as a thin-layer chromatography silica gel plate, the specification of TLC was 0.15 mm to 0.20 mm, and the specification of thin-layer chromatography separation and purification products was 0.4 mm to 0.5 mm. Generally, Yantai Huanghai silica gel 200 to 300 mesh silica gel was used as a carrier for column chromatography.

The starting materials in the embodiments of the present disclosure are known and commercially available, or can be synthesized by using or following methods known in the art.

Unless otherwise specified, all reactions of the present disclosure were carried out under continuous magnetic stirring under dry nitrogen or argon atmosphere, the solvent was a dry solvent, and the unit of the reaction temperature was degrees Celsius.

### Embodiment 1

### 4-((S)-4-Acryloyl-2-methylpiperazin-1-yl)-6-fluoro-7-(2-fluoro-6-hydroxyphenyl)-1-(2-isopropyl-4-(methylthio)pyridin-3-yl)pyrido[2,3-d]pyrimidin-2(1H)-one

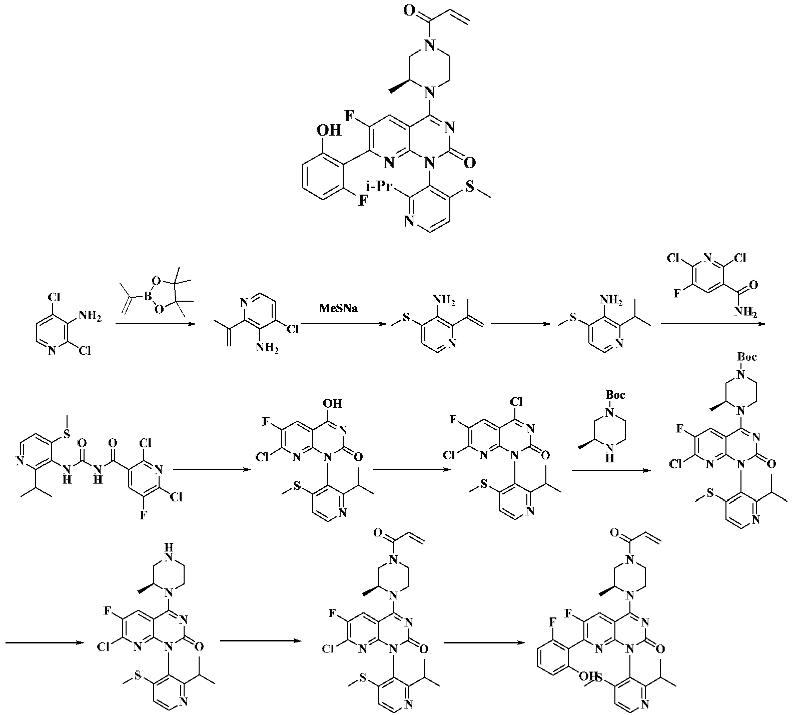

### Step 1: Preparation of 4-chloro-2-(prop-1-en-2-yl)pyridin-3-amine

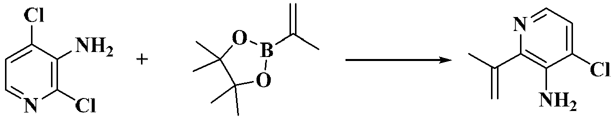

2,4-Dichloropyridin-3-amine (4.5 g, 27.78 mmol), 4,4,5,5-tretramethyl-2-(prop-1-en-2-yl)-1,3,2-dioxaborolane (5.13 g, 30.56 mmol), potassium carbonate (11.5 g, 83.34 mmol), Pd(PPh₃)₄ were added to dioxane (120 mL), and the reaction mixture was uniformly mixed and then stirred overnight in an oil bath at 100°C. The mixture was concentrated under reduced pressure, and the resulting crude product was purified by rapid silica gel column chromatography to obtain the target compound 4-chloro-2-(prop-1-en-2-yl)pyridin-3-amine as a colorless oily liquid (4.5 g, yield: 96%).

MS m/z (ESI): 169.1 [M+H]⁺.

### Step 2: Preparation of 4-(methylthio)-2-(prop-1-en-2-yl)pyridin-3-amine

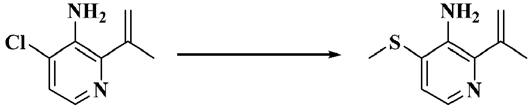

4-Chloro-2-(prop-1-en-2-yl)pyridin-3-amine (2 g, 11.9 mmol) and sodium thiomethoxide (10 mL, 20% aqueous solution) were added to dioxane (3 mL). The reaction mixture was uniformly mixed, then reacted at 100°C for 2 days, cooled to room temperature, and concentrated under reduced pressure, and the resulting crude product was purified by rapid silica gel column chromatography to obtain the compound 4-(methylthio)-2-(prop-1-en-2-yl)pyridin-3-amine as a pale yellow liquid (1.7 g, yield: 79%).

MS m/z (ESI): 181.2 [M+H]⁺.

### Step 3: Preparation of 2-isopropyl-4-(methylthio)pyridin-3-amine

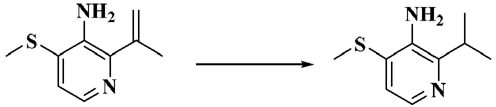

Methanol (50 mL) was added to 4-(methylthio)-2-(prop-1-en-2-yl)pyridin-3-amine (2 g, 11.11 mmol) and Pd/C (4 g), the reaction mixture was uniformly mixed, then reacted overnight at room temperature and concentrated under reduced pressure. The resulting crude product was added to a mixed solution of methanol (5 mL), *N,N*-diisopropylethylamine (0.5 mL) and acrylonitrile (1 mL), and the reaction was carried out at room temperature for 2 hours. The mixture was concentrated under reduced pressure and purified by rapid silica gel column chromatography to obtain the compound 2-isopropyl-4-(methylthio)pyridin-3-amine as a colorless liquid (500 mg, yield: 25%).

MS m/z (ESI): 183.2 [M+H]⁺.

### Step 4: Preparation of 2,6-dichloro-5-fluoro-N-((2-isopropyl-4-(methylthio)pyridin-3-yl)carbamoyl)nicotinamide

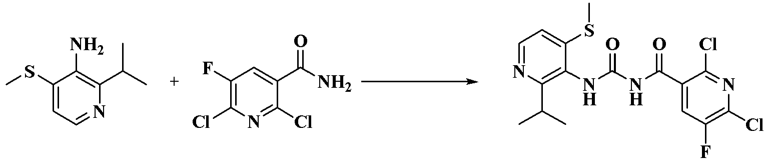

THF (10 mL) was added to 2,6-dichloro-5-fluoronicotinamide (500 mg, 2.44 mmol) and oxalyl chloride (1.32 mL, 2.54 mmol), and the reaction mixture was uniformly mixed and then the reaction was carried out at 60°C for 3 hours, the reaction temperature was reduced to room temperature, and triethylamine (680 mg, 6.6 mmol) and 2-isopropyl-4-(methylthio)pyridin-3-amine (400 mg, 2.2 mmol) were added thereto, and the reaction was carried out at room temperature for 1 hour. The mixture was concentrated under reduced pressure, and the resulting crude product was purified by rapid silica gel column chromatography to obtain the compound 2,6-dichloro-5-fluoro-N-((2-isopropyl-4-(methylthio)pyridin-3-yl)carbamoyl)nicotinamide as a white solid (800 mg, yield: 87%).

MS m/z (ESI): 417.1 [M+H]⁺.

### Step 5: Preparation of 7-chloro-6-fluoro-4-hydroxy-1-(2-isopropyl-4-(methylthio)pyridin-3-yl)pyrido[2,3-d]pyrimidin-2(1H)-one

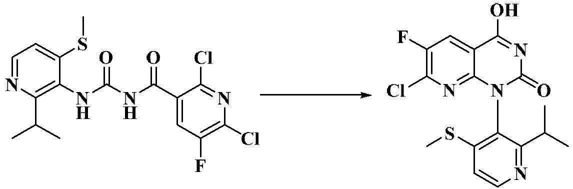

2,6-Dichloro-5-fluoro-*N*-((2-isopropyl-4-(methylthio)pyridin-3-yl)carbamoyl)nicotinamide (800 mg, 1.92 mmol) was added to THF (20 mL), and after the reaction mixture was uniformly mixed, KHMDS (4.8 mL, 4.8 mmol) was slowly added thereto at 0°C, and the reaction was carried out at room temperature for 1 hour. The mixture was concentrated under reduced pressure, and the resulting crude product was purified by rapid silica gel column chromatography to obtain the compound 7-chloro-6-fluoro-4-hydroxy-1-(2-isopropyl-4-(methylthio)pyridin-3-yl)pyrido[2,3-d]pyrimidin-2(1*H*)-one as a white solid (600 mg, yield: 82%).

MS m/z (ESI): 381.1 [M+H]⁺.

### Step 6: Preparation of tert-butyl (S)-4-(7-chloro-6-fluoro-1-(2-isopropyl-4-(methylthio)pyridin-3-yl)-2-carbonyl-1,2-d]hydropyrido[2,3-d]pyrimidin-4-yl)-3-methylpiperazine-1-carboxylate

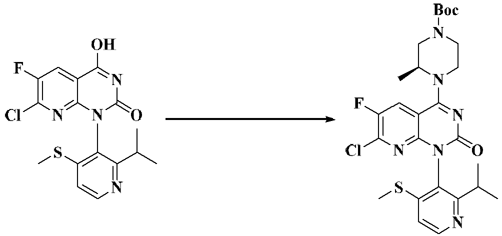

7-Chloro-6-fluoro-4-hydroxy-1-(2-isopropyl-4-(methylthio)pyridin-3-yl)pyrido[2,3-d]pyrimidin-2(1*H*)-one (300 mg, 0.79 mmol), phosphorus oxychloride (600 mg, 3.95 mmol), DIPEA (1 g, 7.9 mmol) were added to THF (40 mL), the reaction mixture was uniformly mixed, and then the reaction was carried out at 80°C for 1 hour, the reaction temperature was reduced to room temperature, and *tert*-butyl (S)-3-methylpiperazine-1-carboxylate (240 mg, 1.19 mmol) was slowly added to the reaction solution, then the reaction was carried out at room temperature for 1 hour. The mixture was concentrated under reduced pressure, and the resulting crude product was purified by rapid silica gel column chromatography to obtain compound *tert*-butyl (S)-4-(7-chloro-6-fluoro-1-(2-isopropyl-4-(methylthio)pyridin-3-yl)-2-carbonyl-1,2-dihydropyrido[2,3-d]pyrimidin-4-yl)-3-methylpiperazine-1-carboxylate as a white solid (400 mg, yield: 90%).

MS m/z (ESI): 563.1 [M+H]⁺.

### Step 7: Preparation of (S)-4-(4-acryloyl-2-methylpiperazin-1-yl)-7-chloro-6-fluoro-1-(2-isopropyl-4-(methylthio)pyridin-3-yl)pyrido[2,3-d]pyrimidin-2(1H)-one

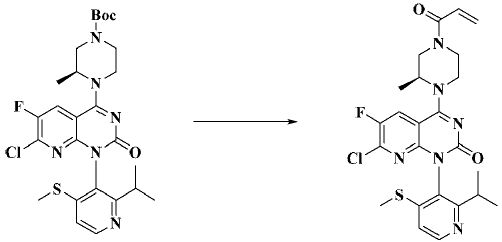

*tert*-Butyl (*S*)-4-(7-chloro-6-fluoro-1-(2-isopropyl-4-(methylthio)pyridin-3-yl)-2-carbonyl-1,2-dihydropyrido[2,3-*d*]pyrimidin-4-yl)-3-methylpiperazine-1-carboxylate (400 mg, 0.71 mmol), TFA (2 mL) were added to CH₂Cl₂ (30 mL), the reaction mixture was uniformly mixed, and then the reaction was carried out at room temperature for 1 hour, then the mixture was concentrated under reduced pressure. CH₂Cl₂ (20 mL) and DIPEA (0.3 mL) were added to the resulting crude product, the reaction temperature was reduced to 0°C, acryloyl chloride (0.1 mL) was slowly added to the reaction solution, and the reaction was carried out at room temperature for 1 hour, the mixture was then concentrated under reduced pressure. The resulting crude product was purified by rapid silica gel column chromatography to obtain the compound (*S*)-4-(4-acryloyl-2-methylpiperazin-1-yl)-7-chloro-6-fluoro-1-(2-isopropyl-4-(methylthio)pyridin-3-yl)pyrido[2,3-d]pyrimidin-2(1*H*)-one as a yellow solid (200 mg, yield: 55%).

MS m/z (ESI): 517.1 [M+H]⁺.

### Step 8: Preparation of 4-((S)-4-acryloyl-2-methylpiperazin-1-yl)-6-fluoro-7-(2-fluoro-6-hydroxyphenyl)-1-(2-isopropyl-4-(methylthio)pyridin-3-yl)pyrido[2,3-d]pyrimidin-2(1H)-one

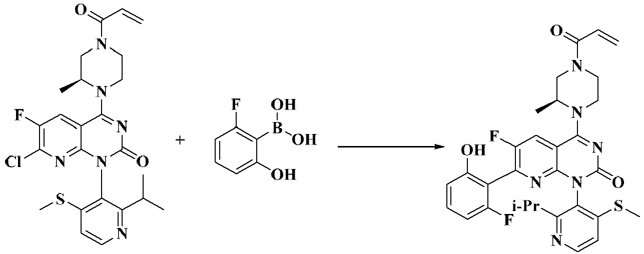

(*S*)-4-(4-Acryloyl-2-methylpiperazin-1-yl)-7-chloro-6-fluoro-1-(2-isopropyl-4-(methylthio)pyridin-3-yl)pyrido[2,3-*d*]pyrimidin-2(1*H*)-one (50 mg, 0.1 mmol), (2-fluoro-6-hydroxyphenyl) boric acid (30 mg, 0.2 mmol), Pd(dppf)Cl₂ (16 mg, 0.02 mmol) and cesium carbonate (100 mg, 0.3 mmol) were added to dixoane (1.5 mL), the reaction mixture was uniformly mixed, and then the reaction was carried out at 100°C under microwave irradiation for 1 hour, then the mixture was concentrated under reduced pressure. CH₂Cl₂ (20 mL) and DIPEA (0.3 mL) were added into the resulting crude product, and the reaction temperature was reduced to 0°C, acryloyl chloride (0.1 mL) was slowly added to the reaction solution, and the reaction was carried out at room temperature for 1 hour, then the mixture was concentrated under reduced pressure. The resulting crude product was purified by Pre-HPLC to obtain the compound 4-((*S*)-4-acryloyl-2-methylpiperazin-1-yl)-6-fluoro-7-(2-fluoro-6-hydroxyphenyl)-1-(2-isopropyl-4-(methylthio)pyridin-3-yl)pyrido[2,3-*d*]pyrimidin-2(1*H*)-one as a white solid (14 mg, yield: 24%).

MS m/z (ESI):593.1 [M+H]⁺.

¹H NMR (400 MHz, MeOD-*d₄*) δ 8.40 (d, *J*=5.6 Hz, 1H), 8.22-8.27 (m, 1 H),7.21-7.27 (m, 2H), 6.79-6.88(m, 1H), 6.58-6.66(m, 2H), 6.28-6.34 (m, 1H), 5.84(d, *J*=12.0 Hz, 1H), 5.06 (s, 1 H), 4.43-4.59 (m, 2H), 4.07-4.23 (s, 1H), 3.57-3.85 (m, 2 H), 3.20-3.48(m, 1H), 2.79-2.85(m, 1H), 2.41(s, 3H), 1.47(d, *J*=4.8 Hz, 3H), 1.20 (d, *J*=6.4 Hz, 3H), 1.06 (d, *J*=6.8 Hz, 3H).

### Embodiment 1-1 and embodiment 1-2

### (P-4-((S)-4-acryloyl-2-methylpiperazin-1-yl)-6-fluoro-7-(2-fluoro-6-hydroxyphenyl)-1-(2-isopropyl-4-(methylthio)pyridin-3-yl)pyrido [2,3-d] pyrimidin-2(1H)-one) and (M-4-((S)-4-acryloyl-2-methylpiperazin-1-yl)-6-fluoro-7-(2-fluoro-6-hydroxyphenyl)-1-(2-isopropyl-4-(methylthio)pyridin-3-yl)pyrido [2,3-d]pyrimidin-2(1H)-one)

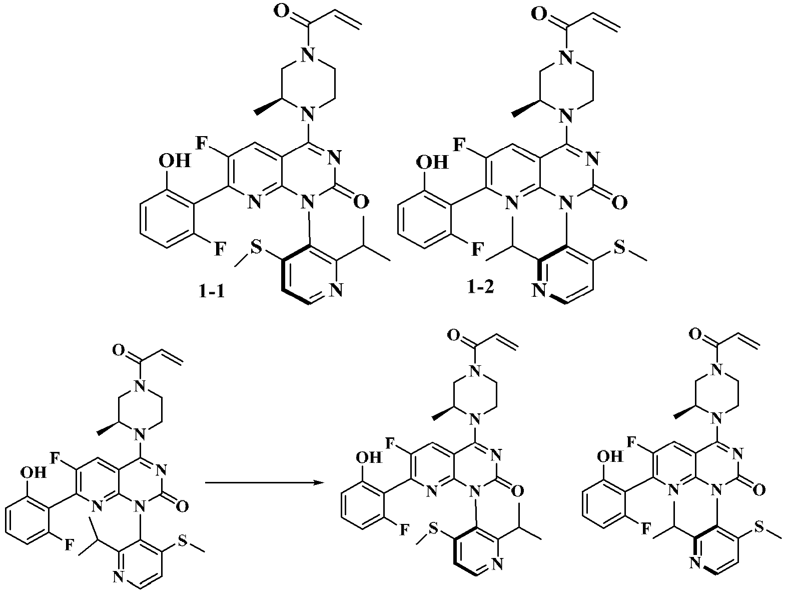

Embodiment 1 was resolved by SFC to obtain two axial chiral isomers, embodiment 1-1 and embodiment 1-2, SFC: chiral preparation conditions:

| | |
|---|---|
| Instrument | SFC-150 (Thar, Waters) |
| Column type | IC 20*250 mm, 10 µm (Daicel) |
| Column pressure | 100 bar |
| Mobile phase | CO₂/ Methanol (0.2% Methanol Ammonia) = 50/50 |
| Flow rate | 120 g/min |
| Detection wavelength | UV 214 nm |
| Column temperature | 35°C |

### Embodiment 1-1:

t*_{R}*=1.92 min
MS m/z (ESI):593.1 [M+H]⁺.

¹H NMR (400 MHz, MeOD-*d₄*) δ 8.40 (d, *J*=5.6 Hz, 1H), 8.22-8.27 (m, 1 H),7.21-7.27 (m, 2H), 6.79-6.88(m, 1H), 6.58-6.66(m, 2H), 6.28-6.34 (m, 1H), 5.84(d, *J*=12.0 Hz, 1H), 5.06 (s, 1 H), 4.43-4.59 (m, 2H), 4.07-4.23 (s, 1H), 3.57-3.85 (m, 2 H), 3.20-3.48(m, 1H), 2.79-2.85(m, 1H), 2.41(s, 3H), 1.47(d, *J*=4.8 Hz, 3H), 1.20 (d, *J*=6.4 Hz, 3H), 1.06 (d, *J*=6.8 Hz, 3H).

### Embodiment 1-2:

t*_{R}*=2.43 min
MS m/z (ESI):593.1 [M+H]⁺.

¹HNMR(400 MHz, MeOD-*d₄*) δ 8.40(d, *J*=5.6Hz, 1H), 8.25(t, *J*=10.8Hz, 1H), 7.21-7.27(m, 2H), 6.79-6.90(m, 1H), 6.58-6.66(m, 2H), 6.28-6.34(m, 1H), 5.83(dd, *J*=10.8Hz, 2.0Hz, 1H), 5.05-5.10(m, 1H), 4.41-4.57(m, 2H), 4.07-4.21(m, 1H), 3.61-3.87(m, 2H), 3.24-3.36(m, 1H), 2.77-2.83(m, 1 H), 2.41(s, 3H), 1.46-1.49 (m, 3H), 1.19(d, *J*=6.8Hz, 3H), 1.06(d, *J*=6.8Hz, 3H).

### Embodiment 2

### Preparation of 4-((2S,5R)-4-acryloyl-2,5-dimethylpiperazin-1-yl)-6-fluoro-7-(2-fluoro-6-hydroxyphenyl)-1-(2-isopropyl-4-(methylthio)pyridin-3-yl)pyrido[2,3-d]pyrimidin-2(1H)-one

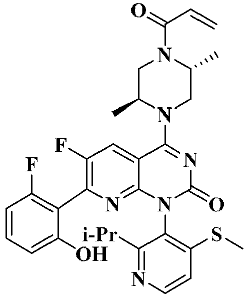

### Step 1: Preparation of 4,7-dichloro-6-fluoro-1-(2-isopropyl-4-(methylthio)pyridin-3-yl)pyrido[2,3-d]pyrimidin-2(1H)-one

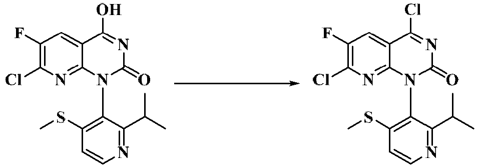

*N,N-*Diisopropylethylamine (407 mg, 3.16 mmol) was added to a solution of 7-chloro-6-fluoro-4-hydroxy-1-(2-isopropyl-4-(methylthio)pyridin-3-yl)pyrido[2,3-*d*]pyrimidin-2(1*H*)-one (200 mg, 0.526 mmol) in acetonitrile (10 mL) at room temperature, then phosphorus oxychloride (242 mg, 1.58 mmol) was added thereto and the mixture was stirred at 80°C for 1 hour. The mixture was cooled to room temperature and directly used in the next reaction.

### Step 2: Preparation of tert-butyl (2R,5S)-4-(7-chloro-6-fluoro-1-(2-isopropyl-4-(methylthio)pyridin-3-yl)-2-carbonyl-1,2-dihydropyrido[2,3-d]pyrimidin-4-yl)-2,5-dimethylpiperazine-1-carboxylate

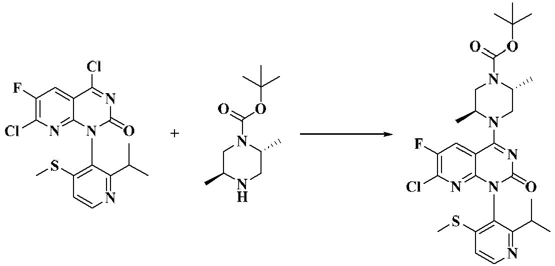

*N,N-Diisopropylethylamine* (678 mg, 5.26 mmol) and *tert*-butyl (2*R*,5*S*)-2,5-dimethylpiperazine-1-carboxylate (224 mg, 1.005 mmol) were added to the reaction mixture of the previous step and stirred for 1 hour at room temperature after the addition. Water (60 mL) was added thereto and the mixture was extracted with ethyl acetate (40 mL × 3), the organic phase was washed with ammonium chloride aqueous solution (40 mL) and then washed with sodium chloride aqueous solution (30 mL), concentrated and then subjected to column chromatography [eluent: dichloromethane to methanol/dichloromethane from 0% to 2.2%] to obtain *tert*-butyl (2*R*,5*S*)-4-(7-chloro-6-fluoro-1-(2-isopropyl-4-(methylthio)pyridin-3-yl)-2-carbonyl-1,2-dihydropyrido[2,3-*d*]pyrimidin-4-yl)-2,5-dimethylpiperazine-1-carboxylate (200 mg, two-step yield: 66%) as a yellow solid.

MS m/z (ESI): 577.2 [M+H]⁺, 579.2 [M+H+2]⁺.

### Step 3: Preparation of 7-chloro-4-((2S,5R)-2,5-dimethylpiperazin-1-yl)-6-fluoro-1-(2-isopropyl-4-(methylthio)pyridin-3-yl)pyrido[2,3-d]pyrimidin-2(1H)-one trifluoroacetate

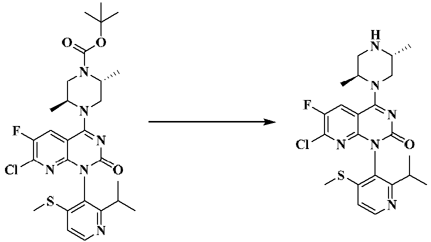

Trifluoroacetic acid (1.2 mL) was added to a solution of *tert*-butyl (2*R*,5*S*)-4-(7-chloro-6-fluoro-1-(2-isopropyl-4-(methylthio)pyridin-3-yl)-2-carbonyl-1,2-dihydropyrido[2,3-*d*]pyrimidin-4-yl)-2,5-dimethylpiperazine-1-carboxylate (200 mg, 0.347 mmol) in dichloromethane (6 mL), and the mixture was stirred at room temperature for 1.5 hours after the addition. Then the reaction mixture was concentrated at low temperature to obtain 7-chloro-4-((2*S*,5*R*)-2,5-dimethylpiperazin-1-yl)-6-fluoro-1-(2-isopropyl-4-(methylthio)pyridin-3-yl)pyrido[2,3-*d*]pyrimidin-2(1*H*)-one trifluoroacetate (200 mg) as a red oil, which was used rapidly in the next reaction.

MS m/z (ESI): 477.2 [M+H]⁺, 479.2 [M+H+2]⁺.

### Step 4: Preparation of 4-((2S,5R)-4-acryloyl-2,5-dimethylpiperazin-1-yl)-7-chloro-6-fluoro-1-(2-isopropyl-4-(methylthio)pyridin-3-yl)pyrido[2,3-d]pyrimidin-2(1H)-one

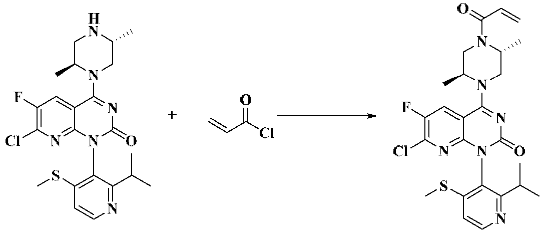

*N,N*-Diisopropylethylamine (447 mg, 3.47 mmol) was added to a solution of 7-chloro-4-((2*S*,5*R*)-2,5-dimethylpiperazin-1-yl)-6-fluoro-1-(2-isopropyl-4-(methylthio)pyridin-3-yl)pyrido[2,3-*d*]pyrimidin-2(1H)-one trifluoroacetate (200 mg, 0.347 mmol) in dichloromethane (15 mL), then acryloyl chloride (63 mg, 0.694 mmol) was added dropwise thereto at 0°C, and the mixture was stirred for 1 hour after the addition. The reaction mixture was quenched with ammonium chloride aqueous solution (30 mL), extracted with dichloromethane (30 mL×3), the dichloromethane phase was washed with saturated NaCl aqueous solution (20 mL), dried over anhydrous sodium sulfate, concentrated and then subjected to column chromatography [eluent: dichloromethane to methanol/dichloromethane from 0% to 2.5%] to obtain 4-((2*S*,5*R*)-4-acryloyl-2,5-dimethylpiperazin-1-yl)-7-chloro-6-fluoro-1-(2-isopropyl-4-(methylthio)pyridin-3-yl)pyrido[2,3-*d*]pyrimidin-2(1*H*)-one (130 mg, two-step yield: 71%) as a yellow solid.

MS m/z (ESI): 530.2 [M+H]⁺, 532.2 [M+H+2]⁺.

### Step 5: Preparation of 4-((2S,5R)-4-acryloyl-2,5-dimethylpiperazin-1-yl)-6-fluoro-7-(2-fluoro-6-hydroxyphenyl)-1-(2-isopropyl-4-(methylthio)pyridin-3-yl)pyrido[2,3-d]pyrimidin-2(1H)-one

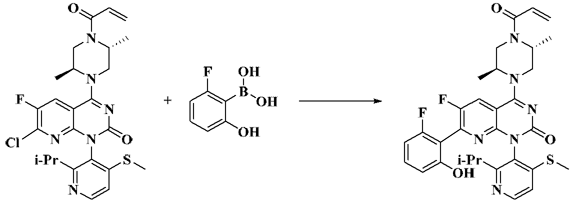

4-((2*S*,5*R*)-4-Acryloyl-2,5-dimethylpiperazin-1-yl)-7-chloro-6-fluoro-1-(2-isopropyl-4-(methylthio)pyridin-3-yl)pyrido[2,3-*d*]pyrimidin-2(1*H*)-one (130 mg, 0.246 mmol), (2-fluoro-6-hydroxyphenyl) boric acid (77 mg, 0.491 mmol), [1,1'-bis(diphenylphosphino)ferrocene]palladium(II)dichloride dichloromethane complex (40 mg, 0.0491 mmol) and cesium carbonate (240 mg, 0.738 mmol) were added to dioxane (4 mL) and water (1 mL), the mixture was replaced with nitrogen, and stirred at 100°C under microwave irradiation for 1 hour. The reaction mixture was concentrated, then subjected to column chromatography [eluent: dichloromethane to methanol/dichloromethane from 0% to 2.5%] to obtain 4-((2*S*,5*R*)-4-acryloyl-2,5-dimethylpiperazin-1-yl)-6-fluoro-7-(2-fluoro-6-hydroxyphenyl)-1-(2-isopropyl-4-(methylthio)pyridin-3-yl)pyrido[2,3-*d*]pyrimidin-2(1*H*)-one (90 mg, yield: 60%) as a yellow solid.

MS m/z (ESI): 606.2 [M+H]⁺.

¹H NMR (400 MHz, MeOD-*d₄*) δ 8.40 (d, *J* = 8 Hz, 1H), 8.29-8.18 (m, 1H), 7.30-7.18 (m, 2H), 6.93-6.73 (m, 1H), 6.70-6.56 (m, 2H), 6.36-6.20 (m, 1H), 5.89-5.75 (m, 1H), 5.15-4.98 (m, 1H), 4.63-4.22 (m, 2H), 4.11-3.82 (m, 2H), 3.68-3.40 (m, 1H), 2.88-2.65 (m, 1H), 2.40 (d, *J* = 4 Hz, 3H), 1.53 - 1.43 (m, 3H), 1.36 (t, *J* = 8 Hz, 1H), 1.28 (t, *J* = 8 Hz, 2H), 1.23 - 1.16 (m, 3H), 1.10 - 1.01 (m, 3H).

### Embodiment 2-1 and embodiment 2-2

### (P-4-((2S,5R)-4-acryloyl-2,5-dimethylpiperazin-1-yl)-6-fluoro-7-(2-fluoro-6-hydroxyphenyl)-1-(2-isopropyl-4-(methylthio)pyridin-3-yl)pyrido [2,3-d]pyrimidin-2(1H)-one) and (M-4-((2S,5R)-4-acryloyl-2,5-dimethylpiperazin-1-yl)-6-fluoro-7-(2-fluoro-6-hydroxyphenyl)-1-(2-isopropyl-4-(methylthio)pyridin-3-yl)pyrido [2,3-d]pyrimidin-2(1H)-one)

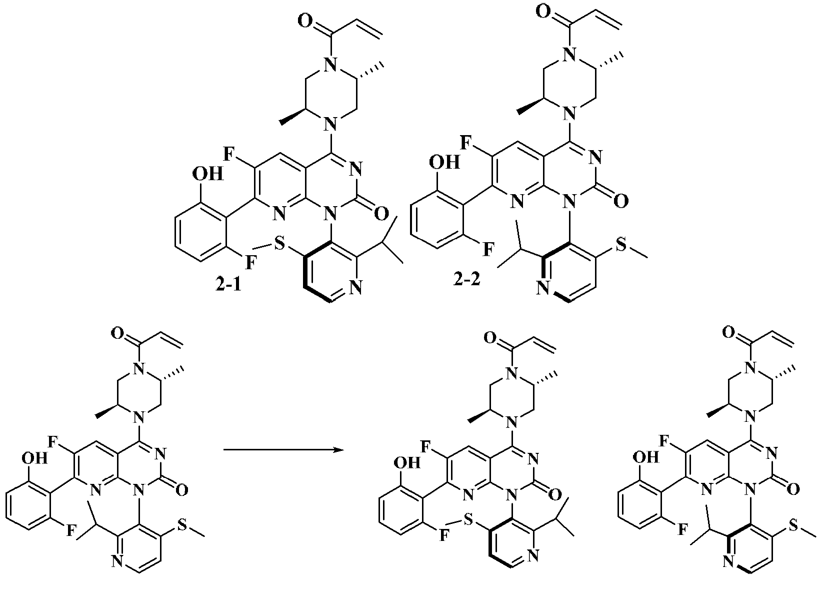

Embodiment 2 was resolved by SFC to obtain two axial chiral isomers, embodiment 2-1 and embodiment 2-2, SFC: chiral preparation conditions:

| | |
|---|---|
| Instrument | SFC-150 (Thar, Waters) |
| Column type | IC 20*250mm, 10 µm (Daicel) |
| Column pressure | 100 bar |
| Mobile phase | CO₂/ Methanol (0.2% Methanol Ammonia) = |
| Flow rate | 100 g/min |
| Detection | UV 214 nm |
| Column temperature | 35°C |

### Embodiment 2-1:

t*_{R}*=1.99 min
MS m/z (ESI): 606.2 [M+H]⁺.

¹H NMR (400 MHz, MeOD-*d₄*) δ 8.40 (d, *J* = 8 Hz, 1H), 8.29-8.18 (m, 1H), 7.30-7.18 (m, 2H), 6.93-6.73 (m, 1H), 6.70-6.56 (m, 2H), 6.36-6.20 (m, 1H), 5.89-5.75 (m, 1H), 5.15-4.98 (m, 1H), 4.63-4.22 (m, 2H), 4.11-3.82 (m, 2H), 3.68-3.40 (m, 1H), 2.88-2.65 (m, 1H), 2.40 (d, *J* = 4 Hz, 3H), 1.53 - 1.43 (m, 3H), 1.36 (t, *J* = 8 Hz, 1H), 1.28 (t, *J* = 8 Hz, 2H), 1.23 - 1.16 (m, 3H), 1.10 - 1.01 (m, 3H).

### Embodiment 2-2:

t*_{R}*=2.87 min
MS m/z (ESI): 606.2 [M+H]⁺.

¹H NMR (400 MHz, MeOD-*d₄*) δ 8.40 (d, *J* = 8 Hz, 1H), 8.27-8.18 (m, 1H), 7.30 - 7.19 (m, 2H), 6.94 - 6.74 (m, 1H), 6.70 - 6.56 (m, 2H), 6.36 - 6.20 (d, *J* = 16 Hz, 1H), 5.90 - 5.75 (m, 1H), 5.14 - 4.98 (m, 1H), 4.63 - 4.22 (m, 2H), 4.12 - 3.82 (m, 2H), 3.68 - 3.41 (m, 1H), 2.87 - 2.65 (m, 1H), 2.40 (d, *J* = 4 Hz, 3H), 1.53 - 1.42 (m, 3H), 1.36 (t, *J=* 8 Hz, 1H), 1.28 (t, *J* = 8 Hz, 2H), 1.23 - 1.16 (d, *J* = 4 Hz, 3H), 1.10 - 1.01 (d, *J* = 4 Hz, 3H).

### Embodiment 3

### 4-((S)-4-Acryloyl-2-methylpiperazin-1-yl)-6-chloro-7-(2-fluoro-6-hydroxyphenyl)-1-(2-isopropyl-4-(methylthio)pyridin-3-yl)pyrido[2,3-d]pyrimidin-2(1H)-one

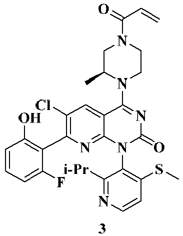

4-((*S*)-4-Acryloyl-2-methylpiperazin-1-yl)-6-chloro-7-(2-fluoro-6-hydroxyphenyl)-1-(2-isopropyl-4-(methylthio)pyridin-3-yl)pyrido[2,3-*d*]pyrimidin-2(1*H*)-one was prepared with reference to embodiment 1.

MS m/z (ESI):609.1 [M+H]⁺.

### Embodiment 4

### 4-((S)-4-Acryloyl-2-methylpiperazin-1-yl)-6-fluoro-7-(2-fluoro-6-(methylthio)phenyl)-1-(2-isopropyl-4-(methylthio)pyridin-3-yl)pyrido[2,3-d]pyrimidin-2(1H)-one

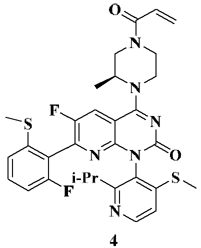

4-((*S*)-4-Acryloyl-2-methylpiperazin-1-yl)-6-fluoro-7-(2-fluoro-6-(methylthio)phenyl)-1-(2-isopropyl-4-(methylthio)pyridin-3-yl)pyrido[2,3-*d*]pyrimidin-2(1*H*)-one was prepared with reference to embodiment 1.

MS m/z (ESI):622.8 [M+H]⁺.

### Embodiment 5

### Preparation of 4-((S)-4-acryloyl-2-methylpiperazin-1-yl)-7-(2-amino-6-fluorophenyl)-6-fluoro-1-(2-isopropyl-4-(methylthio)pyridin-3-yl)pyrido[2,3-d]pyrimidin-2(1H)-one

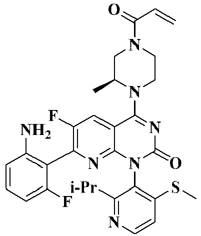

### Step 1: Preparation of N-(4-chloro-3-fluorophenyl)-2,2,2-trifluoroacetamide

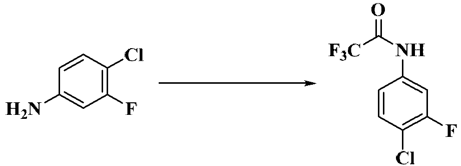

4-Chloro-3-fluoroaniline (1.45 g, 0.01 mol) was dissolved in THF (150 mL), Na₂CO₃ (3.18 g, 0.03 mol) was added thereto, the mixture was cooled to 0°C under nitrogen atmosphere, trifluoroacetic anhydride (4.2 mL, 0.03 mol) was added dropwise thereto, and the mixture was then stirred at room temperature for 10 hours after the addition. The reaction mixture was added to water (150 mL). The mixture was then extracted three times with ethyl acetate (100 mL). The organic phases were combined, dried over anhydrous sodium sulfate, concentrated to obtain a crude product, and the crude product was purified by column chromatography (PE/EA=5:1) to obtain a white solid target product *N*-(4-chloro-3-fluorophenyl)-2,2,2-trifluoroacetamide (2.3 g, yield: 95%).
¹H NMR (400 MHz, MeOD-*d₄*) δ 7.70 (dd, *J* = 11.1, 2.0 Hz, 1H), 7.49 - 7.40 (m, 2H);
¹⁹F NMR (376 MHz, MeOD-*d₄*) δ -77.17 (s);
MS m/z (ESI): 242.1 [M+H]⁺.

### Step 2: Preparation of (6-amino-3-chloro-2-fluorophenyl) boric acid

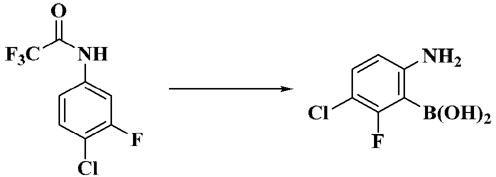

*N*-(4-Chloro-3-fluorophenyl)-2,2,2-trifluoroacetamide (2.3 g, 9.5 mmol) was dissolved in THF (40 mL), the mixture was cooled to -78°C under nitrogen atmosphere, and *n-*BuLi (7.9 mL, 19.0 mmol, 2.4 M) was added dropwise thereto, then the mixture was stirred at -50°C for 50 minutes after the addition. The reaction mixture was cooled to -78°C, triisopropyl borate (2.3 g, 9.5 mmol) (4.8 mL, 20.9 mmol) was added dropwise thereto, the mixture was stirred at the same temperature for 20 minutes after the addition, the dry ice bath was removed, and the mixture was stirred at room temperature for 2 hours. Then, the reaction mixture was cooled to 0°C, dilute hydrochloric acid (19 mL, 1M) was added dropwise thereto, the temperature was raised to 40°C, and the mixture was stirred for 1 hour. The mixture was then extracted three times with ethyl acetate (100 mL). The organic phases were combined, dried over anhydrous sodium sulfate, concentrated to obtain a crude product, and the crude product was purified by column chromatography (PE/EA=4:1) to obtain a gray solid target product (6-amino-3-chloro-2-fluorophenyl) boric acid (1.1 g, yield: 56%).

MS m/z (ESI): 190.0 [M+H]⁺.

### Step 3: Preparation of (2-amino-6-fluorophenyl) boric acid

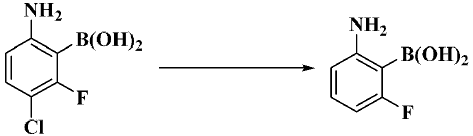

(6-Amino-3-chloro-2-fluorophenyl) boric acid (100 mg, 0.53 mmol) was dissolved in MeOH (20mL), Pd/C (20 mg) was added thereto, the mixture was replaced with hydrogen for three times, then stirred and reacted for 2 hours at 15 psi, and the complete reaction was detected by TLC (PE/EA 1:1). The mixture was filtered, and the filtrate was concentrated to obtain a yellow solid target product (2-amino-6-fluorophenyl) boric acid (80 mg, yield: 97%), which was used directly in the next reaction without purification.

MS m/z (ESI): 156.0 [M+H]⁺.

### Step 4: Preparation of 4-((S)-4-acryloyl-2-methylpiperazin-1-yl)-7-(2-amino-6-fluorophenyl)-6-fluoro-1-(2-isopropyl-4-(methylthio)pyridin-3-yl)pyrido[2,3-d]pyrimidin-2(1H)-one

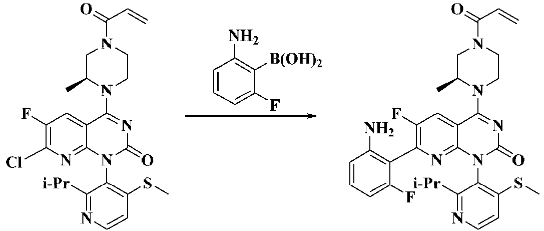

(*S*)-4-(4-Acryloyl-2-methylpiperazin-1-yl)-7-chloro-6-fluoro-1-(2-isopropyl-4-(methylthio)pyridin-3-yl)pyrido[2,3-*d*]pyrimidin-2(1*H*)-one (26 mg, 0.05 mmol), (6-amino-3-chloro-2-fluorophenyl) boric acid (23.2 mg, 0.15 mmol) and cesium carbonate (48.87 mg, 0.15 mmol) were dissolved in dioxane/H₂O (1.5 mL/0.3 mL). The mixture was replaced with nitrogen for 1 minute, and the reaction was carried out at 100°C under microwave irradiation for 1 hour. When the reaction was completed, the reaction mixture was concentrated, purified by column chromatography (CH₂Cl₂/MeOH=20:1) to obtain a crude product, and then the crude product was purified by preparative HPLC to obtain a yellow solid target product 4-((*S*)-4-acryloyl-2-methylpiperazin-1-yl)-7-(2-amino-6-fluorophenyl)-6-fluoro-1-(2-isopropyl-4-(methylthio)pyridin-3-yl)pyrido[2,3-d]pyrimidin-2(1*H*)-one (7.0 mg, yield: 24%).

¹H NMR (400 MHz, MeOD-*d₄*) δ 8.46 (d, *J* = 5.4 Hz, 1H), 8.25 (dd, *J* = 21.2, 12.0 Hz, 1H), 7.27 (d, *J =* 5.5 Hz, 1H), 7.11 (dd, *J* = 14.7, 8.2 Hz, 1H), 6.84 (d, *J* = 14.2 Hz, 1H), 6.49 (d, *J* = 8.3 Hz, 1H), 6.41 - 6.27 (m, 2H), 5.83 (dd, *J =* 10.6, 1.6 Hz, 1H), 4.48 (dd, *J =* 52.4, 11.6 Hz, 2H), 4.30 - 3.83 (m, 2H), 3.74 (d, *J* = 9.7 Hz, 2H), 3.22 (s, 1H), 2.98 - 2.80 (m, 1H), 2.43 (d, *J* = 0.7 Hz, 3H), 1.56 - 1.40 (m, 3H), 1.22 (d, *J* = 6.6 Hz, 3H), 1.01 (d, *J* = 6.6 Hz, 3H).

¹⁹F NMR (376 MHz, MeOD-*d₄*) δ -114.58 - -114.95 (m), -114.95 - -115.34 (m), - 125.12 - -126.48 (m).

MS m/z (ESI): 592.2 [M+H]⁺.

### Embodiment 6

### 2-(4-((S)-4-Acryloyl-2-methylpiperazin-1-yl)-6-fluoro-1-(2-isopropyl-4-(methylthio)pyridin-3-yl)-2-oxo-1,2-dihydropyrido[2,3-d]pyrimidin-7-yl)-3-fluorobenzamide

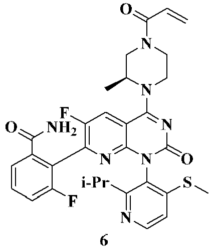

2-(4-((*S*)-4-Acryloyl-2-methylpiperazin-1-yl)-6-fluoro-1-(2-isopropyl-4-(methylthio)pyridin-3-yl)-2-oxo-1,2-dihydropyrido[2,3-d]pyrimidin-7-yl)-3-fluorobenzamide was prepared with reference to embodiment 1.

MS m/z (ESI): 619.7 [M+H]⁺.

### Embodiment 7

### 4-((S)-4-Acryloyl-2-methylpiperazin-1-yl)-7-(2-(dimethylamino)-6-fluorophenyl)-6-fluoro-1-(2-isopropyl-4-(methylthio)pyridin-3-yl)pyrido[2,3-d]pyrimidin-2(1H)-one

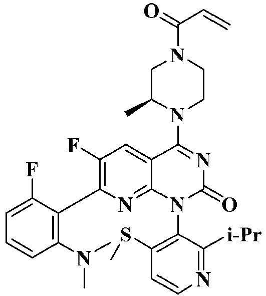

4-((*S*)-4-Acryloyl-2-methylpiperazin-1-yl)-7-(2-(dimethylamino)-6-fluorophenyl)-6-fluoro-1-(2-isopropyl-4-(methylthio)pyridin-3-yl)pyrido[2,3-*d*]pyrimidin-2(1H)-one was prepared with reference to embodiment 1.

MS m/z (ESI): 619.7 [M+H]⁺.

### Embodiment 8

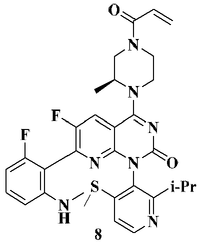

4-((*S*)-4-Acryloyl-2-methylpiperazin-1-yl)-6-fluoro-7-(2-fluoro-6-(methylamino)phenyl)-1-(2-isopropyl-4-(methylthio)pyridin-3-yl)pyridinyl[2,3-*d*]pyrimidin-2(1H)-one was prepared with reference to embodiment 1.

MS m/z (ESI): 605.7 [M+H]⁺.

### Embodiment 9

### Preparation of 4-((S)-4-acryloyl-2-methylpiperazin-1-yl)-7-(6-amino-3-chloro-2-fluorophenyl)-6-chloro-1-(2-isopropyl-4-(methylthio)pyridin-3-yl)pyrido[2,3-d]pyrimidin-2(1H)-one

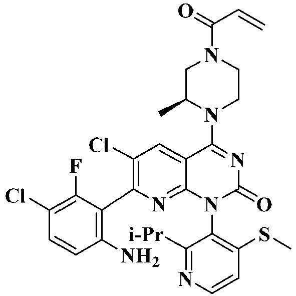

(*S*)-4-(4-Acryloyl-2-methylpiperazin-1-yl)-7-chloro-6-chloro-1-(2-isopropyl-4-(methylthio)pyridin-3-yl)pyrido[2,3-d]pyrimidin-2(1*H*)-one (26.7 mg, 0.05 mmol), (6-amino-3-chloro-2-fluorophenyl) boric acid (28.4 mg, 0.15 mmol) and potassium acetate (15.0 mg, 0.15 mmol) were dissolved in dioxane/H₂O (1.5 mL/0.3 mL). The mixture was replaced with nitrogen for 1 minute, and the reaction was carried out at 100°C under microwave irradiation for 1 hour. When the reaction was completed, the reaction mixture was concentrated, purified by column chromatography (CH₂Cl₂/MeOH=20:1) to obtain a crude product, and the crude product was then purified by preparative HPLC to obtain a yellow solid target product 4-((*S*)-4-acryloyl-2-methylpiperazin-1-yl)-7-(2-amino-6-fluorophenyl)-6-fluoro-1-(2-isopropyl-4-(methylthio)pyridin-3-yl)pyrido[2,3-*d*]pyrimidin-2(1*H*)-one (3.7 mg, yield: 14%).

MS m/z (ESI): 642.1 [M+H]⁺.

¹H NMR (400 MHz, MeOD-*d₄*) δ 8.56 - 8.39 (m, 2H), 7.24 (t, *J =* 5.3 Hz, 1H), 7.15 (dd, *J* = 15.4, 6.9 Hz, 1H), 6.84 (d, *J* = 9.9 Hz, 1H), 6.53 - 6.46 (m, 1H), 6.32 (d, *J* = 15.9 Hz, 1H), 5.84 (d, *J* = 12.2 Hz, 1H), 4.68 - 4.36 (m, 3H), 4.10 (dd, *J* = 45.7, 31.6 Hz, 2H), 3.76 (s, 1H), 2.94 (s, 2H), 2.42 (d, *J* = 6.2 Hz, 3H), 1.57 - 1.43 (m, 3H), 1.22 (d, *J* = 6.7 Hz, 3H), 1.06 (dd, *J =* 42.4, 6.7 Hz, 3H). ¹⁹F NMR (376 MHz, MeOD) δ -117.04 - -117.24 (m), -117.24 - -117.51 (m).

**Embodiment 9-1 and embodiment 9-2**

### (P-4-((S)-4-acryloyl-2-methylpiperazin-1-yl)-7-(2-amino-6-fluorophenyl)-6-fluoro-1-(2-isopropyl-4-(methylthio)pyridin-3-yl)pyrido[2,3-d]pyrimidin-2(1H)-one) and (M-4-((S)-4-acryloyl-2-methylpiperazin-1-yl)-7-(2-amino-6-fluorophenyl)-6-fluoro-1-(2-isopropyl-4-(methylthio)pyridin-3-yl)pyrido[2,3-d]pyrimidin-2(1H)-one)

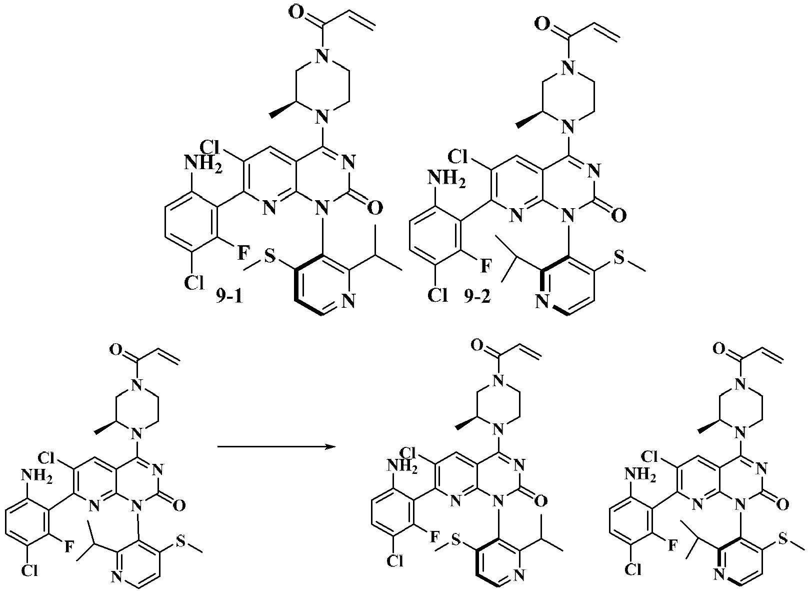

Embodiment 9 was resolved by SFC to obtain two axial chiral isomers, embodiment 9-1 and embodiment 9-2, SFC: chiral preparation conditions:

| | |
|---|---|
| Instrument | SFC-80 (Thar, Waters) |
| Column type | IC 20*250 mm, 10 µm (Daicel) |
| Column pressure | 100 bar |
| Mobile phase | CO₂/ Methanol (0.2% Methanol Ammonia) |
| Flow rate | 80 g/min |
| Detection wavelength | UV 214 nm |
| Column temperature | 35°C |

### Embodiment 9-1:

t*_{R}*=1.74 min
MS m/z (ESI): 642.1 [M+H]⁺.

¹H NMR (400 MHz, MeOD-*d₄*) δ 8.56 - 8.39 (m, 2H), 7.24 (t, *J =* 5.3 Hz, 1H), 7.15 (dd, *J* = 15.4, 6.9 Hz, 1H), 6.84 (d, *J* = 9.9 Hz, 1H), 6.53 - 6.46 (m, 1H), 6.32 (d, *J* = 15.9 Hz, 1H), 5.84 (d, *J =* 12.2 Hz, 1H), 4.68 - 4.36 (m, 3H), 4.10 (dd, *J =* 45.7, 31.6 Hz, 2H), 3.76 (s, 1H), 2.94 (s, 2H), 2.42 (d, *J* = 6.2 Hz, 3H), 1.57 - 1.43 (m, 3H), 1.22 (d, *J* = 6.7 Hz, 3H), 1.06 (dd, *J* = 42.4, 6.7 Hz, 3H).

¹⁹F NMR (376 MHz, MeOD-*d₄*) δ -117.04 - -117.24 (m), -117.24 - -117.51 (m).

### Embodiment 9-2:

t*_{R}*=2.49 min
MS m/z (ESI): 642.1 [M+H]⁺.

¹H NMR (400 MHz, MeOD-*d₄*) δ 8.56 - 8.39 (m, 2H), 7.27 - 7.10 (m, 2H), 6.84 (dd, J = 28.3, 17.7 Hz, 1H), 6.50 (d, J = 8.8 Hz, 1H), 6.32 (d, J = 16.9 Hz, 1H), 5.83 (d, J = 11.7 Hz, 1H), 4.63 - 4.41 (m, 2H), 4.23 - 4.02 (m, 1H), 3.79 - 3.57 (m, 2H), 3.36 (s, 2H), 2.99 - 2.86 (m, 1H), 2.41 (d, J = 7.6 Hz, 3H),1.51 (d, J = 25.9 Hz, 3H), 1.21 (d, J = 6.6 Hz, 3H), 1.05 (dd, J = 44.8, 6.7 Hz, 3H).

### Embodiment 10

### Preparation of 4-((S)-4-acryloyl-2-methylpiperazin-1-yl)-7-(6-amino-3-chloro-2-fluorophenyl)-6-fluoro-1-(2-isopropyl-4-(methylthio)pyridin-3-yl)pyrido[2,3-d]pyrimidin-2(1H)-one

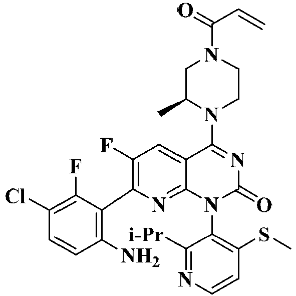

(*S*)-4-(4-Acryloyl-2-methylpiperazin-1-yl)-7-chloro-6-fluoro-1-(2-isopropyl-4-(methylthio)pyridin-3-yl)pyrido[2,3-*d*]pyrimidin-2(1*H*)-one (26 mg, 0.05 mmol), (6-amino-3-chloro-2-fluorophenyl) boric acid (28.4 mg, 0.15 mmol) and cesium carbonate (48.8 mg, 0.15 mmol) were dissolved in dioxane/H₂O (1.5 mL/0.3 mL). The mixture was replaced with nitrogen for 1 minute, and the reaction was carried out at 100°C under microwave irradiation for 1 hour. When the reaction was completed, the reaction mixture was evaporated to dryness by rotary evaporation, purified by column chromatography (CH₂Cl₂/MeOH=20:1) to obtain a crude product, and then the crude product was purified by preparative HPLC to obtain a yellow solid target product 4-((,S)-4-acryloyl-2-methylpiperazin-1-yl)-7-(2-amino-6-fluorophenyl)-6-fluoro-1-(2-isopropyl-4-(methylthio)pyridin-3-yl)pyrido[2,3-*d*]pyrimidin-2(1*H*)-one (4.4 mg, yield: 14%).

¹H NMR (400 MHz, MeOD-*d₄*) δ 8.47 (d, *J* = 5.4 Hz, 1H), 8.38 - 8.24 (m, 1H), 7.27 (d, *J* = 5.4 Hz, 1H), 7.17 (t, *J* = 8.6 Hz, 1H), 6.85 (d, *J* = 14.9 Hz, 1H), 6.49 (d, *J* = 8.9 Hz, 1H), 6.32 (d, *J =* 16.3 Hz, 1H), 5.84 (d, *J* = 10.5 Hz, 1H), 4.57 (d, *J =* 23.5 Hz, 2H), 4.42 (s, 1H), 4.24 - 3.89 (m, 2H), 3.73 (dd, *J* = 14.4, 7.9 Hz, 1H), 2.92 (s, 1H), 2.43 (s, 3H), 1.54 - 1.40 (m, 3H), 1.22 (d, *J* = 6.7 Hz, 3H), 1.01 (d, *J* = 6.6 Hz, 3H).

¹⁹F NMR (376 MHz, MeOD-*d₄*) δ -116.46 - -116.73 (m), -116.87 (dd, *J* = 39.0, 8.4 Hz), -126.18 (dd, *J* = 24.9, 15.2 Hz).

MS m/z (ESI): 626.1 [M+H]⁺.

### Embodiment 11

### Preparation of 4-((S)-4-acryloyl-2-methylpiperazin-1-yl)-7-(2,3-dilfuoro-6-hydroxyphenyl)-6-fluoro-1-(2-isopropyl-4-(methylthio)pyridin-3-yl)pyrido[2,3-d]pyrimidin-2(1H)-one

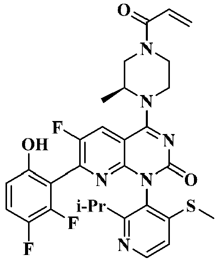

4-((*S*)-4-Acryloyl-2-methylpiperazin-1-yl)-7-(2,3-dilfuoro-6-hydroxyphenyl)-6-fluoro-1-(2-isopropyl-4-(methylthio)pyridin-3-yl)pyrido[2,3-*d*]pyrimidin-2(1*H*)-one was prepared with reference to embodiment 2.

MS m/z (ESI):611.1 [M+H]⁺.

¹H NMR (400 MHz, MeOD-*d₄*) δ 8.41 (d, *J =* 5.6 Hz, 1H), 8.32 - 8.25 (m, 1H), 7.25 (d, *J* = 5.6 Hz, 1H), 7.20 - 7.13 (m, 1H), 6.92 - 6.82 (m, 1H), 6.62 - 6.58 (m, 1H), 6.34 - 6.28 (m, 1H), 5.83 (d, *J* = 10.4 Hz, 1H), 5.14 - 5.04 (m, 1H), 4.64 - 4.42 (m, 2H), 4.25 - 4.07 (m, 1H), 3.89 - 3.61 (m, 3H), 2.88 - 2.77 (m, 1H), 2.42 (s, 3H), 1.52 - 1.46 (m, 3H), 1.20 (d, *J* = 6.4 Hz, 3H), 1.05 (d, *J* = 6.4 Hz, 3H).

### Embodiment 12

### Preparation of (S)-4-(4-acryloyl-2-methylpiperazin-1-yl)-7-(2,6-dilfuorophenyl)-6-chloro-1-(2-isopropyl-4-(methylthio)pyridin-3-yl)pyrido[2,3-d]pyrimidin-2(1H)-one

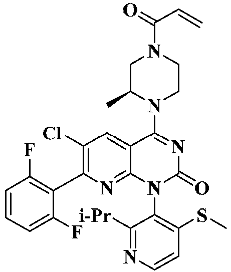

(*S*)-4-(4-Acryloyl-2-methylpiperazin-1-yl)-7-(2,6-dilfuorophenyl)-6-chloro-1-(2-isopropyl-4-(methylthio)pyridin-3-yl)pyrido[2,3-*d*]pyrimidin-2(1*H*)-one was prepared with reference to embodiment 2.

MS m/z (ESI):611.1 [M+H]⁺.

### Embodiment 13

### Preparation of 4-((2S,5R)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-d]pyrimidin-2(1H)-one

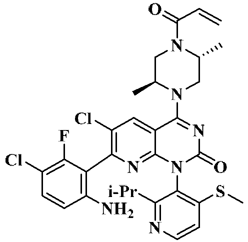

### Step 1: Preparation of 2,5,6-trichloro-N-(2-isopropyl-4-(methylthio)pyridin-3-yl)carbamoyl)nicotinamide

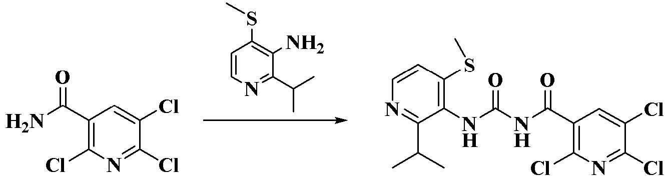

Under N₂ atmosphere, 2,5,6-trichloronicotinamide (6.2 g, 27.7 mmol) was dissolved in THF (60 mL), oxalyl chloride (15.2 mL, 31.5 mmol) (2 M/L dichloromethane solution) was added dropwise thereto at -78°C, and the mixture was stirred at -78°C for 10 minutes, then the mixture was stirred at 60°C for 3 hours, then the reaction mixture was cooled to 0°C, triethylamine (18 mL, 111 mmol) was added dropwise thereto. A solution of 2-isopropyl-4-(methylthio)pyridin-3-amine (5 g, 27.7 mmol) in THF was added dropwise thereto, and the mixture was stirred at room temperature for 2 hours. The reaction mixture was quenched with brine, extracted with water and ethyl acetate (3*100 mL), the organic phases were combined, dried over anhydrous sodium sulfate, filtered and concentrated to obtain a crude product, then the crude product was purified by column chromatography (DCM/MeOH = 100:1 to 70:1) to obtain the target product 2,5,6-trichloro-N-((2-isopropyl-4-(methylthio)pyridin-3-yl)carbamoyl)nicotinamide (8.6 g, yield: 72%).

MS m/z (ESI):433.1 [M+H]⁺, 435.1 [M+H+2]⁺.

### Step 2: Preparation of 6,7-dichloro-1-(2-isopropyl-4-(methylthio)pyridin-3-yl)pyrido[2,3-d]pyrimidin-2,4(1H,3H)-dione

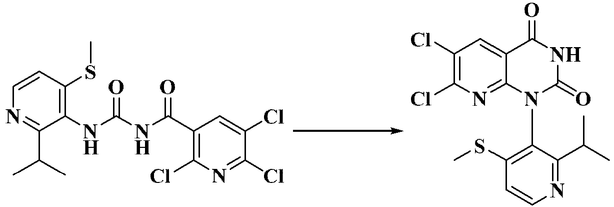

2,5,6-Trichloro-*N*-((2-isopropyl-4-(methylthio)pyridin-3-yl)carbamoyl)nicotinamide (10.4 g, 24.1 mmol) was dissolved in anhydrous THF (80 mL), cooled to 0°C under nitrogen atmosphere, KHMDS (48 mL, 48.2 mmol) was added dropwise thereto, and the mixture was stirred for 0.5 hours. The reaction mixture was then quenched with saturated ammonium chloride aqueous solution, then extracted with water and ethyl acetate (3* 100 mL). The organic phases were combined, dried over anhydrous sodium sulfate, filtered, concentrated to obtain a crude product, and the crude product was slurried with ethyl acetate and purified to obtain the target product 6,7-dichloro-1-(2-isopropyl-4-(methylthio)pyridin-3-yl)pyrido[2,3-*d*]pyrimidin-2,4(1*H*,3*H*)-dione (8 g, yield: 84%).

MS m/z (ESI): 397.1 [M+H]⁺, 399.1 [M+H+2]⁺.

### Step 3: Preparation of 4,6,7-trichloro-1-(2-isopropyl-4-(methylthio)pyridin-3-yl)pyrido[2,3-d]pyrimidin-2(1H)-one

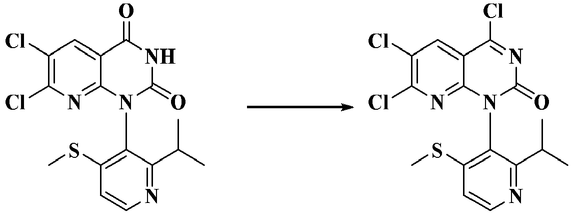

6,7-Dichloro-1-(2-isopropyl-4-(methylthio)pyridin-3-yl)pyrido[2,3-*d*]pyrimidin-2,4(1*H*,3*H*)-dione (5.2 g, 13.1 mmol) was dissolved in ACN (50 mL); DIEA (23 mL, 66 mmol) and POCl₃ (3 mL, 19.7 mmol) were added thereto, and the mixture was stirred at 80°C for 0.5 hours. The resulting product was directly used in the next reaction.

MS m/z (ESI): 415.1 [M+H]⁺, 417.1 [M+H+2]⁺.

### Step 4: Preparation of tert-butyl (2R,5S)-4-(6,7-dichloro-1-(2-isopropyl-4-(methylthio)pyridin-3-yl)-2-carbonyl-1,2-dihydropyrido[2,3-d]pyrimidin-4-yl)-2,5-dimethylpiperazine-1-carboxylate

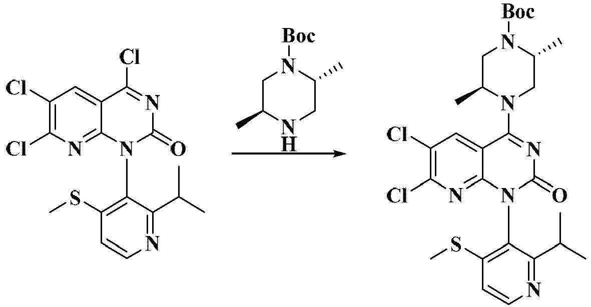

DIEA(23 mL, 66 mmol) and *tert*-butyl (2*R*,5*S*)-2,5-dimethylpiperazine-1-carboxylate (6.2 g, 26.2 mmol) were added to a solution of 4,6,7-trichloro-1-(2-isopropyl-4-(methylthio)pyridin-3-yl)pyrido[2,3-d]pyrimidin-2(1*H*)-one in acetonitrile (50 mL), and the mixture was stirred at room temperature for 1 hour. The reaction mixture was then quenched with water, then extracted with water and ethyl acetate (3* 100 mL). The organic phases were combined, dried over anhydrous sodium sulfate, filtered, concentrated to obtain a crude product, and the crude product was purified by column chromatography (CH₂Cl₂/MeOH=30:1) to obtain the target product *tert*-butyl (2*R*,5*S*)-4-(6,7-dichloro-1-(2-isopropyl-4-(methylthio)pyridin-3-yl)-2-carbonyl-1,2-dihydropyrido[2,3-*d*]pyrimidin-4-yl)-2,5-dimethylpiperazine-1-carboxylate (6.1 g, yield: 77%).

MS m/z (ESI): 593.1 [M+H]⁺, 595.1 [M+H+2]⁺.

### Step 5: Preparation of 6,7-dichloro-4-((2S,5R)-2,5-dimethylpiperazin-1-yl)-1-(2-isopropyl-4-(methylthio)pyridin-3-yl)pyrido[2,3-d]pyrimidin-2(1H)-one

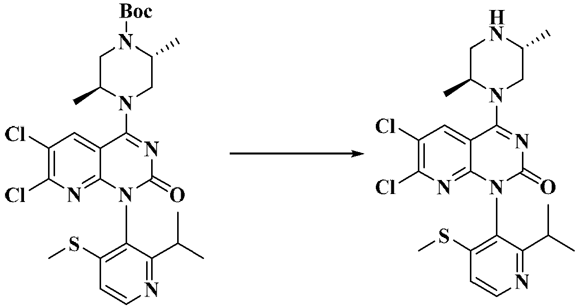

*tert*-Butyl (2*R*,5*S*)-4-(6,7-dichloro-1-(2-isopropyl-4-(methylthio)pyridin-3-yl)-2-carbonyl-1,2-dihydropyrido[2,3-*d*]pyrimidin-4-yl)-2,5-dimethylpiperazine-1-carboxylate(6.1 g, 10.3 mmol) was dissolved in dichloromethane (20 mL), TFA (20 mL) was added thereto, and the mixture was stirred at room temperature for 1 hour. The mixture was concentrated to obtain the crude target product 6,7-dichloro-4-((2*S*,5*R*)-2,5-dimethylpiperazin-1-yl)-1-(2-isopropyl-4-(methylthio)pyridin-3-yl)pyrido[2,3-*d*]pyrimidin-2(1*H*)-one (6.1 g, yield: 100%).

MS m/z (ESI): 493.1 [M+H]⁺, 495.1 [M+H+2]⁺.

### Step 6: Preparation of 4-((2S,5R)-4-acryloyl-2,5-dimethylpiperazin-1-yl)-6,7-dichloro-1-(2-isopropyl-4-(methylthio)pyridin-3-yl)pyrido[2,3-d]pyrimidin-2(1H)-one

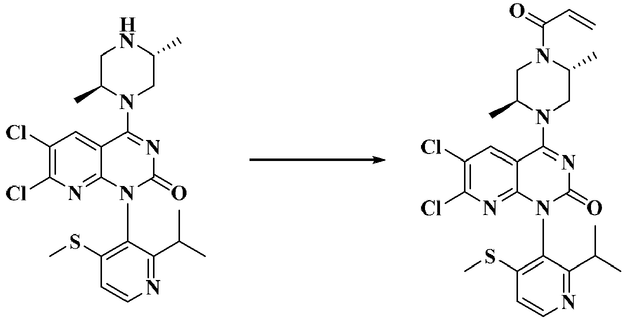

6,7-Dichloro-4-((2*S*,5*R*)-2,5-dimethylpiperazin-1-yl)-1-(2-isopropyl-4-(methylthio)pyridin-3-yl)pyrido[2,3-*d*]pyrimidin-2(1*H*)-one (6 g, 12.2 mmol) was dissolved in dichloromethane (30 mL); DIEA (30 mL, 131 mmol) and acryloyl chloride (1.08 mL, 13.13 mmol) were added thereto, and the mixture was stirred at room temperature for 1 hour. The reaction mixture was then quenched with water, then extracted with water and ethyl acetate (3* 100 mL). The organic phases were combined, dried over anhydrous sodium sulfate, filtered, concentrated to obtain a crude product, and the crude product was purified by column chromatography (CH₂Cl₂/MeOH=20:1) to obtain the target product 4-((2*S*,5*R*)-4-acryloyl-2,5-dimethylpiperazin-1-yl)-6,7-dichloro-1-(2-isopropyl-4-(methylthio)pyridin-3-yl)pyrido[2,3-*d*]pyrimidin-2(1*H*)-one (1.6 g, yield: 22%).

MS m/z (ESI): 547.1 [M+H]⁺, 549.1 [M+H+2]⁺.

### Step 7: Preparation of 4-((2S,5R)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-d]pyrimidin-2(1H)-one

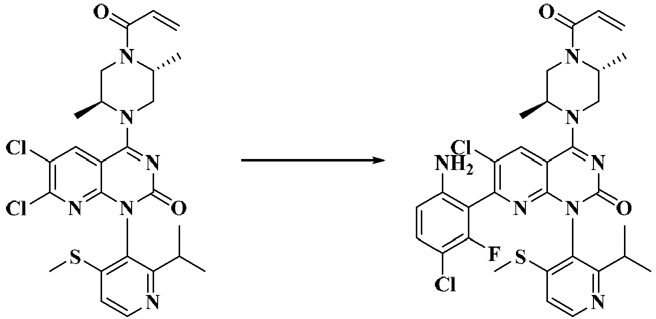

Under N₂ atmosphere, 4-((2*S*,5*R*)-4-acryloyl-2,5-dimethylpiperazin-1-yl)-6,7-dichloro-1-(2-isopropyl-4-(methylthio)pyridin-3-yl)pyrido[2,3-*d*]pyrimidin-2(1*H*)-one (700 mg, 1.3 mmol), (6-amino-3-chloro-2-fluorophenyl) boric acid (380 mg, 2.6 mmol) was dissolved in a mixture of 1, 4-dioxane and water (6 mL:0.3 mL); and Pd(dppf)Cl₂.DCM (100 mg, 0.1 mmol) and KOAc (400 mg, 4 mmol) were added thereto, and the reaction was carried out at 100 °C for 1 hour under microwave irradiation. The reaction mixture was then quenched with water, then extracted with water and ethyl acetate (3* 50 mL). The organic phases were combined, dried over anhydrous sodium sulfate, filtered, concentrated to obtain a crude product, and the crude product was purified by column chromatography (CH₂Cl₂/MeOH= 200:1 to 80:1) to obtain the target product 4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one (400 mg, yield: 48%).

MS m/z (ESI): 656.1 [M+H]⁺, 658.1 [M+H+2]⁺.

¹H NMR (400 MHz, Methanol-*d*₄) δ 8.47 - 8.34 (m, 2H), 7.24-7.20 (m, 1H), 7.10-7.14 (m, 1H), 6.79-6.68 (m, 1H), 6.42 - 6.40 (d, *J* = 8.0 Hz, 1H), 6.24 - 6.17 (m, 1H), 5.75-5.71 (m, 1H), 5.01 -4.94 (m, 2H), 4.46-4.40 (m, 1H), 4.26-4.17 (m, 1H), 4.03-3.99 (m, 1H), 3.84-3.79 (m, 1H), 2.86-2.77 (m, 1H), 2.36 (s, 3H), 1.26-1.19 (m, 9H), 1.14-1.11 (m, 3H).

### Embodiment 13-1 and Embodiment 13-2

### (P-4-((2S,5R)-4-Acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-d]pyrimidin-2(1H)-one) and (M-4-((2S,5R)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-d]pyrimidin-2(1H)-one)

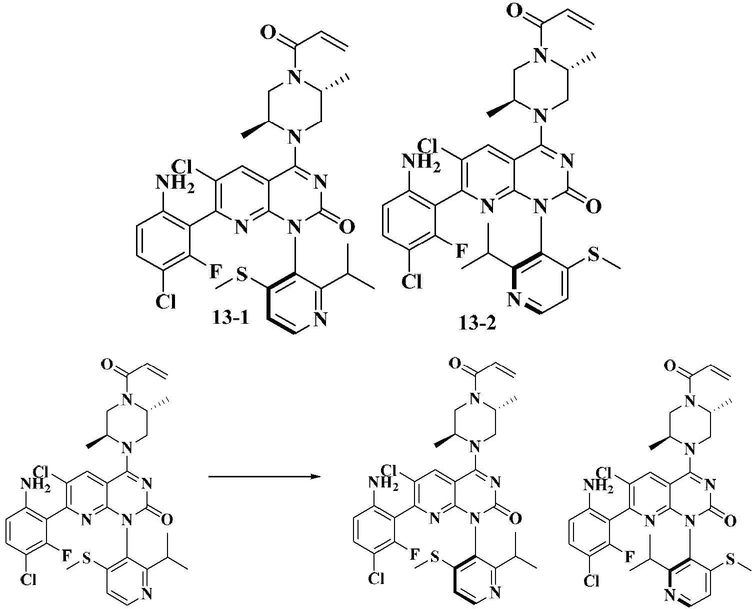

Embodiment 13 was resolved by SFC to obtain two axial chiral isomers, embodiment 13-1 and embodiment 13-2, SFC: chiral preparation conditions:

| | |
|---|---|
| Instrument | SFC-150 (Thar, Waters) |
| Column type | IC 20*250 mm, 10 µm (Daicel) |
| Column pressure | 100 bar |
| Mobile phase | CO₂/ Methanol (0.2% Methanol Ammonia) |
| Flow rate | 120 g/min |
| Detection wavelength | UV 214 nm |
| Column temperature | 35°C |

### Embodiment 13-1:

t*_{R}*=1.74 min
MS m/z (ESI): 656.1 [M+H]⁺, 658.1 [M+H+2]⁺.

¹H NMR (400 MHz, MeOD-*d₄*) δ 8.47 - 8.34 (m, 2H), 7.24-7.20 (m, 1H), 7.10-7.14 (m, 1H), 6.79-6.68 (m, 1H), 6.42 - 6.40 (d, *J* = 8.0 Hz, 1H), 6.24 - 6.17 (m, 1H), 5.75-5.71 (m, 1H), 5.01 -4.94 (m, 2H), 4.46-4.40 (m, 1H), 4.26-4.17 (m, 1H), 4.03-3.99 (m, 1H), 3.84-3.79 (m, 1H), 2.86-2.77 (m, 1H), 2.36 (s, 3H), 1.26-1.19 (m, 9H), 1.14-1.11 (m, 3H).

### Embodiment 13-2:

t*_{R}*=2.49 min
MS m/z (ESI): 656.1 [M+H]⁺, 658.1 [M+H+2]⁺.

¹H NMR (400 MHz, DMSO-*d*₆) δ 8.55 - 8.38 (m, 2H), 7.25-7.20 (m, 1H), 7.18-7.11 (m, 1H), 6.88-6.76 (m, 1H), 6.51- 6.47 (d, *J* = 8.0 Hz, 1H), 6.33 - 6.27 (m, 1H), 5.84-5.80 (m, 1H), 5.12 -5.10 (m, 2H), 4.46-4.23 (m, 2H), 4.15-3.89 (m, 2H), 3.64-3.50 (m, 1H), 2.89-2.82 (m, 1H), 2.43 (s, 3H), 1.51-0.99 (m, 12H).

### Embodiment 14

### Preparation of 4-((2S,5R)-4-acryloyl-2,5-dimethylpiperazin-1-yl)-6-chloro-7-(2-fluoro-6-hydroxyphenyl)-1-(2-isopropyl-4-(methylthio)pyridin-3-yl)pyrido[2,3-d]pyrimidin-2(1H)-one

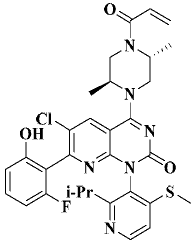

4-((2*S*,5*R*)-4-Acryloyl-2,5-dimethylpiperazin-1-yl)- 6-chloro-7-(2-fluoro-6-hydroxyphenyl)-1-(2-isopropyl-4-(methylthio)pyridin-3-yl)pyrido[2,3-*d*]pyrimidin-2(1*H*)-one was prepared with reference to embodiment 13.

MS m/z (ESI): 623.1 [M+H]⁺, 625.1 [M+H+2]⁺.

¹H NMR (400 MHz, Methanol-*d*₄) δ 8.47 - 8.34 (m, 2H), 7.21-7.20 (m, 2H), 6.89-6.77 (m, 1H), 6.64 - 6.55 (m, 2H), 6.32 - 6.26 (m, 1H), 5.84-5.80 (m, 1H), 5.08 -5.03 (m, 2H), 4.56-4.49 (m, 1H), 4.34-4.26 (m, 1H), 4.13-4.04 (m, 1H), 3.92-3.88 (m, 1H), 2.79-2.72 (m, 1H), 2.40 (s, 3H), 1.55 - 1.43 (m, 3H), 1.35-1.27 (m, 3H), 1.20-1.17 (m, 3H), 1.08-1.05 (t, *J* = 8.0 Hz, 3H).

### Embodiment 14-1 and embodiment 14-2

### (P-4-((2S,5R)-4-Acryloyl-2,5-dimethylpiperazin-1-yl)-6-chloro-7-(2-fluoro-6-hydroxyphenyl)-1-(2-isopropyl-4-(methylthio)pyridin-3-yl)pyrido [2,3-d] pyrimidin-2(1H)-one) and (M-4-((2S,5R)-4-acryloyl-2,5-dimethylpiperazin-1-yl)-6-chloro-7-(2-fluoro-6-hydroxyphenyl)-1-(2-isopropyl-4-(methylthio)pyridin-3-yl)pyrido[2,3-d]pyrimidin-2(1H)-one)

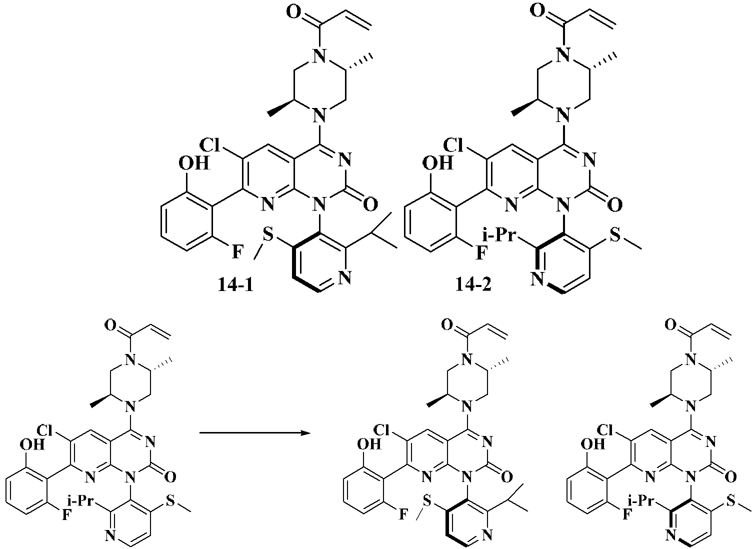

Embodiment 14 was resolved by SFC to obtain two axial chiral isomers, embodiment 14-1 and embodiment 14-2, SFC: chiral preparation conditions:

| | |
|---|---|
| Instrument | SFC-150 (Thar, Waters) |
| Column type | IC 20*250 mm, 10 µm (Daicel) |
| Column pressure | 100 bar |
| Mobile phase | CO₂/ Methanol (0.2% Methanol |
| Flow rate | 120 g/min |
| Detection wavelength | UV 214 nm |
| Column temperature | 35°C |

### Embodiment 14-1:

t*_{R}*=2.46 min
MS m/z (ESI): 623.1 [M+H]⁺, 625.1 [M+H+2]⁺.

¹H NMR (400 MHz, Methanol-*d*₄) δ 8.47 - 8.34 (m, 2H), 7.21-7.20 (m, 2H), 6.89-6.77 (m, 1H), 6.64 - 6.55 (m, 2H), 6.32 - 6.26 (m, 1H), 5.84-5.80 (m, 1H), 5.08 -5.03 (m, 2H), 4.56-4.49 (m, 1H), 4.34-4.26 (m, 1H), 4.13-4.04 (m, 1H), 3.92-3.88 (m, 1H), 2.79-2.72 (m, 1H), 2.40 (s, 3H), 1.55 - 1.43 (m, 3H), 1.35-1.27 (m, 3H), 1.20-1.17 (m, 3H), 1.08-1.05 (t, *J=* 8.0 Hz, 3H).

### Embodiment 14-2:

t*_{R}*=3.08 min
MS m/z (ESI): 623.1 [M+H]⁺, 625.1 [M+H+2]⁺.

¹H NMR (400 MHz, Methanol-*d*₄) δ 8.48 - 8.34 (m, 2H), 7.23-7.21 (m, 2H), 6.90-6.78 (m, 1H), 6.66 - 6.58 (m, 2H), 6.33 - 6.28 (m, 1H), 5.85-5.82 (m, 1H), 5.10 -5.06 (m, 2H), 4.58-4.50 (m, 1H), 4.34-4.27 (m, 1H), 4.13-4.06 (m, 1H), 3.93-3.88 (m, 1H), 2.79-2.71 (m, 1H), 2.41 (s, 3H), 1.56 - 1.46 (m, 3H), 1.37-1.29 (m, 3H), 1.21-1.18 (m, 3H), 1.07-1.05 (t, *J =* 8.0 Hz, 3H).

### Embodiment 15

### Preparation of (S)-4-(4-acryloyl-2-methylpiperazin-1-yl)-7-(2,6-difluorophenyl)-6-fluoro-1-(2-isopropyl-4-(methylthio)pyridin-3-yl)pyrido[2,3-d]pyrimidin-2(1H)-one

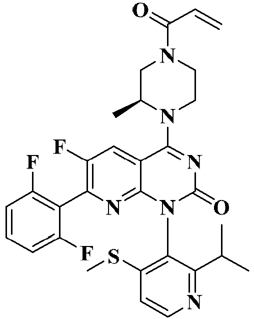

(*S*)-4-(4-Acryloyl-2-methylpiperazin-1-yl)-7-(2,6-difluorophenyl)-6-fluoro-1-(2-isopropyl-4-(methylthio)pyridin-3-yl)pyrido[2,3-*d*]pyrimidin-2(1*H*)-one was prepared with reference to embodiment 2.

MS m/z (ESI):595.1 [M+H]⁺.

¹H NMR (400 MHz, Methanol-*d*₄) δ 8.40 - 8.32 (m, 2H), 7.51 (t, *J* = 7.6 Hz, 1H), 7.22 (d, *J =* 5.4 Hz, 1H), 7.05 (t, *J =* 8.4 Hz, 2H), 6.86 - 6.79 (m, 1H), 6.37 - 6.26 (m, 1H), 5.84 (d, *J* = 10.6 Hz, 1H), 5.08 (m, 2H), 4.56-4.46 (m, 2H), 4.21-4.08 (m, 1H), 3.85-3.62 (m, 2H), 2.86-2.82 (m, 1H), 2.40 (s, 3H), 1.47 (d, *J* = 6.6 Hz, 3H), 1.21 - 1.19 (d, *J =* 6.8 Hz, 3H), 1.04 (d, *J* = 6.8 Hz, 3H).

### Embodiment 16

### Preparation of (S)-4-(4-acryloyl-2-methylpiperazin-1-yl)-6-fluoro-7-(2-fluorophenyl)-1-(2-isopropyl-4-(methylthio)pyridin-3-yl)pyrido[2,3-d]pyrimidin-2(1H)-one

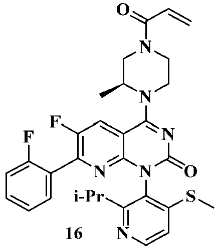

(*S*)-4-(4-Acryloyl-2-methylpiperazin-1-yl)-6-fluoro-7-(2-fluorophenyl)-1-(2-isopropyl-4-(methylthio)pyridin-3-yl)pyrido[2,3-*d*]pyrimidin-2(1*H*)-one was prepared with reference to embodiment 2.

MS m/z (ESI): 576.7 [M+H]⁺.

### Embodiment 17

### Preparation of 4-((2S,5R)-4-acryloyl-2,5-dimethylpiperazin-1-yl)-7-(2-amino-3,5-dichloro-6-fluorophenyl)-6-chloro-1-(2-isopropyl-4-(methylthio)pyridin-3-yl)pyrido[2,3-d]pyrimidin-2(1H)-one

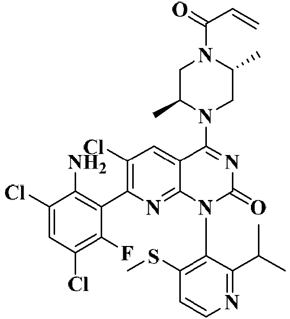

4-((2*S*,5*R*)-4-Acryloyl-2,5-dimethylpiperazin-1-yl)-7-(2-amino-3,5-dichloro-6-fluorophenyl)-6-chloro-1-(2-isopropyl-4-(methylthio)pyridin-3-yl)pyrido[2,3-*d*]pyrimidin-2(1H)-one was prepared with reference to embodiment 13.

MS m/z (ESI): 690.1 [M+H]⁺, 692.1 [M+H+2]⁺.

¹H NMR (400 MHz, Methanol-*d*₄) δ 8.46 - 8.34 (m, 2H), 7.25-7.21 (m, 1H), 7.11-7.14 (m, 1H), 6.44 - 6.42 (d, *J* = 8.0 Hz, 1H), 6.23 - 6.16 (m, 1H), 5.73-5.70 (m, 1H), 5.03 - 4.97 (m, 2H), 4.47-4.42 (m, 1H), 4.25-4.16 (m, 1H), 4.06-4.02 (m, 1H), 3.86-3.83 (m, 1H), 2.84-2.79 (m, 1H), 2.34 (s, 3H), 1.27-1.19 (m, 9H), 1.16-1.14 (m, 3H).

### Embodiment 18

### Preparation of 4-((S)-4-acryloyl-2-methylpiperazin-1-yl)-7-(2-amino-5-chloro-3,6-difluorophenyl)-6-chloro-1-(2-isopropyl-4-(methylthio)pyridin-3-yl)pyrido[2,3-d]pyrimidin-2(1H)-one

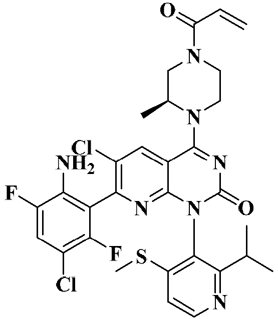

4-((S)-4-Acryloyl-2-methylpiperazin-1-yl)-7-(2-amino-5-chloro-3,6-difluorophenyl)-6-chloro-1-(2-isopropyl-4-(methylthio)pyridin-3-yl)pyrido[2,3-d]pyrimidin-2(1*H*)-one was prepared with reference to embodiment 13.

MS m/z (ESI): 660.1 [M+H]⁺, 662.1 [M+H+2]⁺.

¹H NMR (400 MHz, Methanol-*d*₄) δ 8.58 - 8.38 (m, 2H), 7.53 - 7.36 (m, 1H), 7.23 - 7.15 (m, 1H), 6.97 - 6.79 (m, 1H), 6.22 (d, *J =* 16 Hz, 1H), 5.77 (d, *J =* 8 Hz, 1H), 5.45 - 5.40 (m, 2H), 5.07 - 4.82 (m, 1H), 4.50 - 3.98 (m, 3H), 3.92 - 3.49 (m, 2H), 3.17 - 3.02 (m, 1H), 2.93 - 2.63 (m, 1H), 2.44 - 2.26 (m, 3H), 1.43 - 1.27 (m, 3H), 1.08 (d, *J =* 4 Hz, 3H), 1.04 - 0.86 (m, 3H).

### Embodiment 19

### Preparation of 4-((S)-4-acryloyl-2-methylpiperazin-1-yl)-7-(2-amino-5,6-difluoro-3-methylphenyl)-6-chloro-1-(2-isopropyl-4-(methylthio)pyridin-3-yl)pyrido[2,3-d]pyrimidin-2(1H)-one

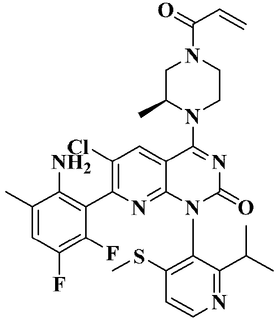

4-((*S*)-4-Acryloyl-2-methylpiperazin-1-yl)-7-(2-amino-5,6-difluoro-3-methylphenyl)-6-chloro-1-(2-isopropyl-4-(methylthio)pyridin-3-yl)pyrido[2,3-d]pyrimidin-2(1*H*)-one was prepared with reference to embodiment 13.

MS m/z (ESI): 640.1 [M+H]⁺, 642.1 [M+H+2]⁺.

¹H NMR (400 MHz, Methanol-*d*₄) δ 8.57 - 8.35 (m, 3H), 7.25 - 7.04 (m, 2H), 6.96 - 6.79 (m, 1H), 6.29 - 6.14 (m, 1H), 5.77 (d, *J =* 12 Hz, 1H), 5.09 - 4.82 (m, 1H), 4.76 - 4.58 (m, 2H), 4.48 - 3.98 (m, 3H), 3.94 - 3.59 (m, 2H), 2.93 - 2.69 (m, 1H), 2.44 - 2.29 (m, 3H), 2.10 - 1.95 (m, 3H), 1.42 - 1.26 (m, 3H), 1.08 (d, *J =* 4 Hz, 3H), 1.05 - 0.87 (m, 3H).

### Embodiment 20

### Preparation of 4-((2S,5R)-4-acryloyl-2,5-dimethylpiperazin-1-yl)-7-(2-amino-5-chloro-3,6-difluorophenyl)-6-chloro-1-(2-isopropyl-4-(methylthio)pyridin-3-yl)pyrido[2,3-d]pyrimidin-2(1H)-one

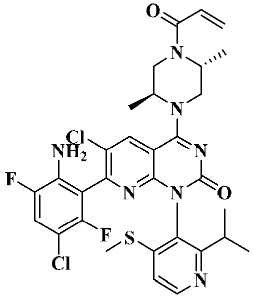

4-((2*S*,5*R*)-4-Acryloyl-2,5-dimethylpiperazin-1-yl)-7-(2-amino-5-chloro-3,6-difluorophenyl)-6-chloro-1-(2-isopropyl-4-(methylthio)pyridin-3-yl)pyrido[2,3-d]pyrimidin-2(1H)-one was prepared with reference to embodiment 13.

MS m/z (ESI): 674.1 [M+H]⁺, 676.1 [M+H+2]⁺.

¹H NMR (400 MHz, Methanol-*d*₄) δ 8.61 - 8.39 (m, 2H), 7.56 - 7.35 (m, 1H), 7.27 - 7.14 (m, 1H), 6.96 - 6.75 (m, 1H), 6.20 (d, *J =* 16 Hz, 1H), 5.82 - 5.71 (m, 1H), 5.53 - 5.38 (m, 2H), 4.95 - 4.69 (m, 1H), 4.57 - 4.30 (m, 1H), 4.24 - 4.00 (m, 2H), 3.98 - 3.79 (m, 2H), 2.95 - 2.60 (m, 1H), 2.44 - 2.25 (m, 3H), 1.40 - 1.13 (m, 6H), 1.10 - 0.87 (m, 6H).

### Embodiment 21

### 4-((2S,5R)-4-Acryloyl-2,5-dimethylpiperazin-1-yl)-7-(2-amino-3,6-difluorophenyl)-6-chloro-1-(2-isopropyl-4-(methylthio)pyridin-3-yl)pyrido[2,3-d]pyrimidin-2(1H)-one

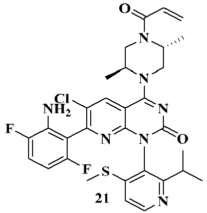

4-((2*S*,5*R*)-4-Acryloyl-2,5-dimethylpiperazin-1-yl)-7-(2-amino-3,6-difluorophenyl)-6-chloro-1-(2-isopropyl-4-(methylthio)pyridin-3-yl)pyrido[2,3-*d*]pyrimidin-2(1*H*)-one was prepared with reference to embodiment 13.

MS m/z (ESI): 674.1 [M+H]⁺.

### Embodiment 22

### Preparation of 4-((S)-4-acryloyl-2-methylpiperazin-1-yl)-7-(2-amino-3,6-difluorophenyl)-6-chloro-1-(2-isopropyl-4-(methylthio)pyridin-3-yl)pyrido[2,3-d]pyrimidin-2(1H)-one

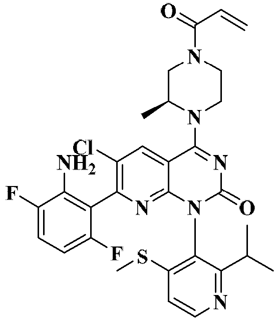

4-((*S*)-4-Acryloyl-2-methylpiperazin-1-yl)-7-(2-amino-3,6-difluorophenyl)-6-chloro-1-(2-isopropyl-4-(methylthio)pyridin-3-yl)pyrido[2,3-d]pyrimidin-2(1H)-one was prepared with reference to embodiment 13.

MS m/z (ESI): 626.1 [M+H]⁺, 628.1 [M+H+2]⁺.

¹H NMR (400 MHz, Methanol-*d*₄) δ 8.58 - 8.34 (m, 2H), 7.26 - 6.99 (m, 2H), 6.95 - 6.77 (m, 1H), 6.47 - 6.27 (m, 1H), 6.26 - 6.13 (m, 1H), 5.77 (d, *J* = 16 Hz, 1H), 5.22 (s, 2H), 5.09 - 4.80 (m, 1H), 4.50 - 3.99 (m, 3H), 3.95 - 3.53 (m, 2H), 3.20 - 2.98 (m, 1H), 2.94 - 2.65 (m, 1H), 2.42 - 2.24 (m, 3H), 1.43 - 1.25 (m, 3H), 1.09 (d, *J* = 4 Hz, 3H), 1.04 - 0.82 (m, 3H).

### Embodiment 23

### Preparation of 4-((S)-4-acryloyl-2-methylpiperazin-1-yl)-7-(2-amino-3,5,6-trifluorophenyl)-6-chloro-1-(2-isopropyl-4-(methylthio)pyridin-3-yl)pyrido[2,3-d]pyrimidin-2(1H)-one

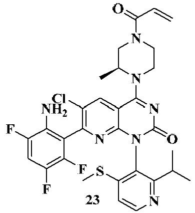

4-((*S*)-4-Acryloyl-2-methylpiperazin-1-yl)-7-(2-amino-3,5,6-trifluorophenyl)-6-chloro-1-(2-isopropyl-4-(methylthio)pyridin-3-yl)pyrido[2,3-d]pyrimidin-2(1H)-one was prepared with reference to embodiment 13.

MS m/z (ESI): 644.1 [M+H]+.

### Embodiment 24

### Preparation of 4-((2S,5R)-4-acryloyl-2,5-dimethylpiperazin-1-yl)-7-(2-amino-3,5,6-trifluorophenyl)-6-chloro-1-(2-isopropyl-4-(methylthio)pyridin-3-yl)pyrido[2,3-d]pyrimidin-2(1H)-one

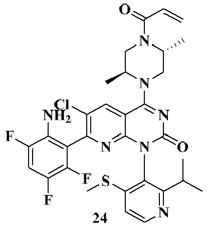

4-((2*S*,5*R*)-4-Acryloyl-2,5-dimethylpiperazin-1-yl)-7-(2-amino-3,5,6-trifluorophenyl)-6-chloro-1-(2-isopropyl-4-(methylthio)pyridin-3-yl)pyrido[2,3-*d*]pyrimidin-2(1*H*)-one was prepared with reference to embodiment 13.

MS m/z (ESI): 658.1 [M+H]+.

### Embodiment 25

### Preparation of 4-((2S,5R)-4-acryloyl-2,5-dimethylpiperazin-1-yl)-7-(2-amino-5,6-difluoro-3-methylphenyl)-6-chloro-1-(2-isopropyl-4-(methylthio)pyridin-3-yl)pyrido[2,3-d]pyrimidin-2(1H)-one

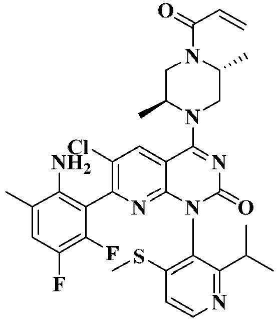

4-((2*S*,5*R*)-4-Acryloyl-2,5-dimethylpiperazin-1-yl)-7-(2-amino-5,6-difluoro-3-methylphenyl)-6-chloro-1-(2-isopropyl-4-(methylthio)pyridin-3-yl)pyrido[2,3-*d*]pyrimidin-2(1H)-one was prepared with reference to embodiment 13.

MS m/z (ESI): 654.1 [M+H]⁺, 656.1 [M+H+2]⁺.

¹H NMR (400 MHz, Methanol-*d*₄) δ 8.58 - 8.31 (m, 2H), 7.25 - 7.03 (m, 2H), 6.94 - 6.73 (m, 1H), 6.19 (d, *J =* 16 Hz, 1H), 5.81 - 5.69 (m, 1H), 4.96 - 4.59 (m, 3H), 4.55 - 4.38 (m, 1H), 4.29 - 3.96 (m, 2H), 3.93 - 3.72 (m, 2H), 3.00 - 2.60 (m, 1H), 2.45 - 2.25 (m, 3H), 2.07 - 1.94 (m, 3H), 1.43 - 1.13 (m, 6H), 1.12 - 0.82 (m, 6H).

### Embodiment 26

### Preparation of 4-((2S,5R)-4-acryloyl-2,5-dimethylpiperazin-1-yl)-7-(6-amino-2,3,4-trifluorophenyl)-6-chloro-1-(2-isopropyl-4-(methylthio)pyridin-3-yl)pyrido[2,3-d]pyrimidin-2(1H)-one

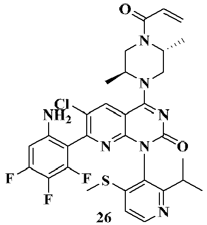

4-((2*S*,5*R*)-4-Acryloyl-2,5-dimethylpiperazin-1-yl)-7-(6-amino-2,3,4-trifluorophenyl)-6-chloro-1-(2-isopropyl-4-(methylthio)pyridin-3-yl)pyrido[2,3-*d*]pyrimidin-2(1H)-one was prepared with reference to embodiment 13.

MS m/z (ESI): 658.1 [M+H]+,

### Embodiment 27

### Preparation of 4-((S)-4-acryloyl-2-methylpiperazin-1-yl)-7-(6-amino-2,3,4-trifluorophenyl)-6-chloro-1-(2-isopropyl-4-(methylthio)pyridin-3-yl)pyrido[2,3-d]pyrimidin-2(1H)-one

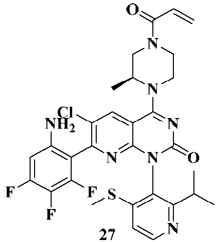

4-((*S*)-4-Acryloyl-2-methylpiperazin-1-yl)-7-(6-amino-2,3,4-trifluorophenyl)-6-chloro-1-(2-isopropyl-4-(methylthio)pyridin-3-yl)pyrido[2,3-*d*]pyrimidin-2(1*H*)-onewas prepared with reference to embodiment 13.

MS m/z (ESI): 644.1 [M+H]+,

### Embodiment 28

### Preparation of 4-((2S,5R)-4-acryloyl-2,5-dimethylpiperazin-1-yl)-7-(6-amino-2,3-difluorophenyl)-6-chloro-1-(2-isopropyl-4-(methylthio)pyridin-3-yl)pyrido[2,3-d]pyrimidin-2(1H)-one

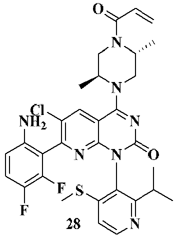

4-((2*S*,5*R*)-4-Acryloyl-2,5-dimethylpiperazin-1-yl)-7-(6-amino-2,3-difluorophenyl)-6-chloro-1-(2-isopropyl-4-(methylthio)pyridin-3-yl)pyrido[2,3-*d*]pyrimidin-2(1*H*)-one was prepared with reference to embodiment 13.

MS m/z (ESI): 640.2 [M+H]+,

### Embodiment 29

### Preparation of 4-((S)-4-acryloyl-2-methylpiperazin-1-yl)-7-(6-amino-2,3-difluorophenyl)-6-chloro-1-(2-isopropyl-4-(methylthio)pyridin-3-yl)pyrido[2,3-d]pyrimidin-2(1H)-one

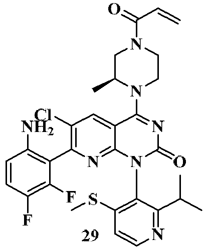

4-((*S*)-4-Acryloyl-2-methylpiperazin-1-yl)-7-(6-amino-2,3-difluorophenyl)-6-chloro-1-(2-isopropyl-4-(methylthio)pyridin-3-yl)pyrido[2,3-*d*]pyrimidin-2(1H)-one was prepared with reference to embodiment 13.

MS m/z (ESI): 626.1 [M+H]+,

### Embodiment 30

### Preparation of 4-((S)-4-acryloyl-2-methylpiperazin-1-yl)-6-chloro-7-(2-fluoro-6-methylphenyl)-1-(2-isopropyl-4-(methylthio)pyridin-3-yl)pyrido[2,3-d]pyrimidin-2(1H)-one

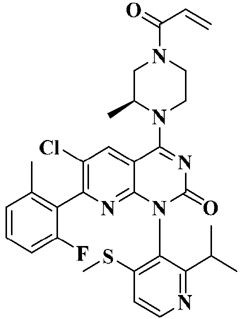

4-((*S*)-4-Acryloyl-2-methylpiperazin-1-yl)-6-chloro-7-(2-fluoro-6-methylphenyl)-1-(2-isopropyl-4-(methylthio)pyridin-3-yl)pyrido[2,3-*d*]pyrimidin-2(1*H*)-one was prepared with reference to embodiment 13.

MS m/z (ESI): 607.1 [M+H]⁺, 609.1 [M+H+2]⁺.

¹H NMR (400 MHz, Methanol-*d*₄) δ 8.57 - 8.34 (m, 2H), 7.43 - 7.31 (m, 1H), 7.18 (d, *J=* 4 Hz, 1H), 7.15 - 7.01 (m, 2H), 6.95 - 6.78 (m, 1H), 6.28 - 6.14 (m, 1H), 5.77 (d, *J =* 12 Hz, 1H), 5.07 - 4.86 (m, 1H), 4.45 - 4.25 (m, 2H), 4.22 - 3.98 (m, 1H), 3.93 - 3.58 (m, 2H), 3.21 - 3.02 (m, 1H), 2.87 - 2.69 (m, 1H), 2.40 - 2.27 (m, 3H), 1.98 - 1.85 (m, 3H), 1.41 - 1.28 (m, 3H), 1.08 (d, *J* = 8 Hz, 3H), 1.02 - 0.79 (m, 3H).

### Embodiment 31

### Preparation of 4-((2S,5R)-4-acryloyl-2-methylpiperazin-1-yl)-6-chloro-7-(2-fluoro-6-methylphenyl)-1-(2-isopropyl-4-(methylthio)pyridin-3-yl)pyrido[2,3-d]pyrimidin-2(1H)-one

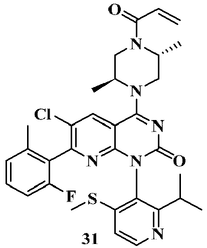

4-((2*S*,5*R*)-4-Acryloyl-2-methylpiperazin-1-yl)-6-chloro-7-(2-fluoro-6-methylphenyl)-1-(2-isopropyl-4-(methylthio)pyridin-3-yl)pyrido[2,3-d]pyrimidin-2(1H)-one was prepared with reference to embodiment 13.

MS m/z (ESI): 621.2 [M+H]+,

### Embodiment 32

### Preparation of 4-((S)-4-acryloyl-2-methylpiperazin-1-yl)-6-chloro-7-(2-chloro-6-fluorophenyl)-1-(2-isopropyl-4-(methylthio)pyridin-3-yl)pyrido[2,3-d]pyrimidin-2(1H)-one

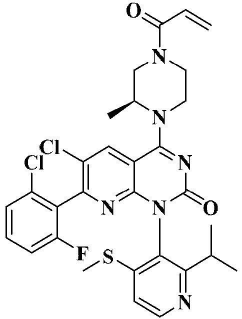

4-((*S*)-4-Acryloyl-2-methylpiperazin-1-yl)-6-chloro-7-(2-chloro-6-fluorophenyl)-1-(2-isopropyl-4-(methylthio)pyridin-3-yl)pyrido[2,3-*d*]pyrimidin-2(1*H*)-one was prepared with reference to embodiment 13.

MS m/z (ESI): 627.1 [M+H]⁺, 629.1 [M+H+2]⁺.

¹H NMR (400 MHz, Methanol-*d*₄) δ 8.56- 8.30 (m, 2H), 7.58-7.36 (m, 3H), 7.19 (s, 1H), 6.87 (s, 1H), 6.24-6.19 (d, *J* = 20.0 Hz, 1H), 5.79-5.76 (d, *J =* 12.0 Hz, 1H), 4.97 (s, 1H), 4.32-4.04 (m, 3H), 3.80-3.49 (m, 3H), 2.72 (s, 1H), 2.35 (s, 3H), 1.34-0.91 (m, 9H).

### Embodiment 33

### Preparation of 4-((2S,5R)-4-acryloyl-2,5-dimethylpiperazin-1-yl)-6-chloro-7-(2-chloro-6-fluorophenyl)-1-(2-isopropyl-4-(methylthio)pyridin-3-yl)pyrido[2,3-d]pyrimidin-2(1H)-one

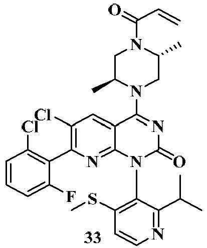

4-((2*S,*5*R*)-4-Acryloyl-2,5-dimethylpiperazin-1-yl)-6-chloro-7-(2-chloro-6-fluorophenyl)-1-(2-isopropyl-4-(methylthio)pyridin-3-yl)pyrido[2,3-d]pyrimidin-2(1H)-one was prepared with reference to embodiment 13.

MS m/z (ESI): 641.6 [M+H]+,

### Embodiment 34

### Preparation of (S)-4-(4-acryloyl-2-methylpiperazin-1-yl)-6-chloro-1-(2-isopropyl-4-(methylthio)pyridin-3-yl)-7-(o-benzyl)pyrido [2,3-d] pyrimidin-2(1H)-one

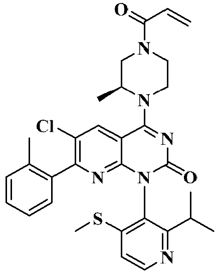

(*S*)-4-(4-Acryloyl-2-methylpiperazin-1-yl)-6-chloro-1-(2-isopropyl-4-(methylthio)pyridin-3-yl)-7-(*o*-benzyl)pyrido[2,3-*d*]pyrimidin-2(1*H*)-one was prepared with reference to embodiment 13.

MS m/z (ESI): 589.1 [M+H]⁺, 591.1 [M+H+2]⁺.

### Embodiment 35

### Preparation of (S)-4-(4-acryloyl-2-methylpiperazin-1-yl)-6-chloro-7-(2-chlorophenyl)-1-(2-isopropyl-4-(methylthio)pyridin-3-yl)pyrido[2,3-d]pyrimidin-2(1H)-one

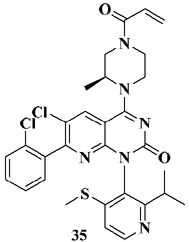

(*S*)-4-(4-Acryloyl-2-methylpiperazin-1-yl)-6-chloro-7-(2-chlorophenyl)-1-(2-isopropyl-4-(methylthio)pyridin-3-yl)pyrido[2,3-*d*]pyrimidin-2(1*H*)-onewasprepared with reference to embodiment 13.

MS m/z (ESI): 609.6 [M+H]+,

### Embodiment 36

### Preparation of (S)-4-(4-acryloyl-2-methylpiperazin-1-yl)-6-chloro-7-(2-fluoro-6-(trifluoromethyl)phenyl)-1-(2-isopropyl-4-(methylthio)pyridin-3-yl)pyrido[2,3-d]pyrimidin-2(1H)-one

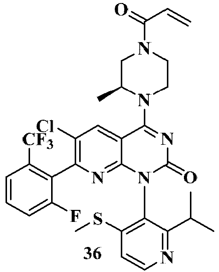

(*S*)-4-(4-Acryloyl-2-methylpiperazin-1-yl)-6-chloro-7-(2-fluoro-6-(trifluoromethyl)phenyl)-1-(2-isopropyl-4-(methylthio)pyridin-3-yl)pyrido[2,3-*d*]pyrimidin-2(1H)-one was prepared with reference to embodiment 13.

MS m/z (ESI): 661.1 [M+H]+,

### Embodiment 37

### Preparation of 4-((2S,5R)-4-acryloyl-2,5-dimethylpiperazin-1-yl)-6-chloro-1-(2-isopropyl-4-(methylthio)pyridin-3-yl)-7-(o-benzyl)pyrido [2,3-d] pyrimidin-2(1H)-one

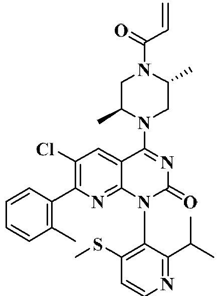

4-((2*S*,5*R*)-4-Acryloyl-2,5-dimethylpiperazin-1-yl)-6-chloro-1-(2-isopropyl-4-(methylthio)pyridin-3-yl)-7-(o-benzyl)pyrido[2,3-*d*]pyrimidin-2(1*H*)-one was prepared with reference to embodiment 13.

MS m/z (ESI): 603.1 [M+H]⁺, 605.1 [M+H+2]⁺.

### Embodiment 38

### Preparation of 4-((2S,5R)-4-acryloyl-2,5-dimethylpiperazin-1-yl)-6-chloro-7-(2-chlorophenyl)-1-(2-isopropyl-4-(methylthio)pyridin-3-yl)pyrido[2,3-d]pyrimidin-2(1H)-one

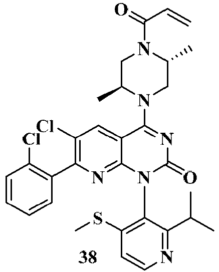

4-((2*S*,5*R*)-4-Acryloyl-2,5-dimethylpiperazin-1-yl)-6-chloro-7-(2-chlorophenyl)-1-(2-isopropyl-4-(methylthio)pyridin-3-yl)pyrido[2,3-d]pyrimidin-2(1*H*)-one was prepared with reference to embodiment 13.

MS m/z (ESI): 623.6 [M+H]+,

### Embodiment 39

### Preparation of 4-((2S,5R)-(4-acryloyl-2,5-dimethylpiperazin-1-yl)-6-chloro-7-(2-fluoro-6-(trifluoromethyl)phenyl)-1-(2-isopropyl-4-(methylthio)pyridin-3-yl)pyrido[2,3-d]pyrimidin-2(1H)-one

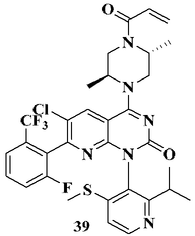

4-((2*S*,5*R*)-(4-Acryloyl-2,5-dimethylpiperazin-1-yl)-6-chloro-7-(2-fluoro-6-(trifluoromethyl)phenyl)-1-(2-isopropyl-4-(methylthio)pyridin-3-yl)pyrido[2,3-*d*]pyrimidin-2(1H)-one was prepared with reference to embodiment 13.

MS m/z (ESI): 675.1 [M+H]+,

### Embodiment 40

### Preparation of 4-((S)-4-acryloyl-2-methylpiperazin-1-yl)-7-(2-amino-3,5-dichloro-6-fluorophenyl)-6-chloro-1-(2-isopropyl-4-(methylthio)pyridin-3-yl)pyrido[2,3-d]pyrimidin-2(1H)-one

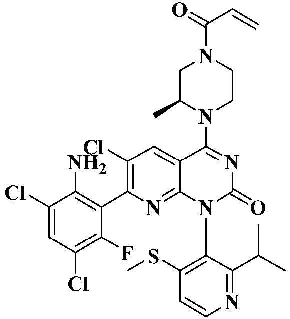

4-((*S*)-4-Acryloyl-2-methylpiperazin-1-yl)-7-(2-amino-3,5-dichloro-6-fluorophenyl)-6-chloro-1-(2-isopropyl-4-(methylthio)pyridin-3-yl)pyrido[2,3-*d*]pyrimidin-2(1*H*)-one was prepared with reference to embodiment 13.

MS m/z (ESI): 676.1 [M+H]⁺, 678.1 [M+H+2]⁺.

¹H NMR (400 MHz, Methanol-*d*₄) δ 8.40 - 8.32 (m, 2H), 7.51 (t, *J* = 7.6 Hz, 1H), 7.22 (d, *J =* 5.4 Hz, 1H), 7.05 (t, *J =* 8.4 Hz, 2H), 6.86 - 6.79 (m, 1H), 6.37 - 6.26 (m, 1H), 5.84 (d, *J* = 10.6 Hz, 1H), 5.08 (m, 2H), 4.56-4.46 (m, 2H), 4.21-4.08 (m, 1H), 3.85-3.62 (m, 2H), 2.86-2.82 (m, 1H), 2.40 (s, 3H), 1.47 (d, *J* = 6.6 Hz, 3H), 1.21 - 1.19 (d, *J =* 6.8 Hz, 3H), 1.04 (d, *J* = 6.8 Hz, 3H).

### 2. Biological test evaluation of compounds

The present disclosure is further described below in conjunction with test embodiments to explain the present disclosure, but these embodiments are not meant to limit the scope of the present disclosure.

### Test embodiment 1. Determination of the inhibitory effect on NCI-H358/Mia PaCa-2 cell proliferation activity

### 1.1 Experimental purpose:

To determine the inhibitory effect of the compounds of the embodiments on the proliferation activity of KRAS G12C mutant cell lines NCI-H358 and Mia PaCa-2 cells.

### 1.2. Experimental instruments and reagents:

### 1.2.1 Instrument:

Microplate reader (BioTek Synergy H1)
Pipette (Eppendorf & Rainin)

### 1.2.2 Reagents:

NCI-H358 was purchased from Nanjing Cobioer Biotechnology Co., Ltd.;
Mia PaCa-2 was purchased from ATCC;
Cell Titer-Glo cells were purchased from Promega Company, and the article number was G7573;
RPMI 1640 was purchased from Gibco, the article number was 22400089;
DMEM was purchased from Gibco, the article number is 11995065;
FBS was purchased from Gibco, the article number was 10091148;
PBS was purchased from Gibco, the article number was 10010023;
Trypsin was purchased from GIBCO, the article was 25200056;
The cell culture plate was purchased from Corning Company, the article number was 3610.

### 1.3. Experimental methods:

When NCI-H358 or Mia PaCa-2 cells were cultured to the appropriate fusion level, the NCI-H358 or Mia PaCa-2 cells were collected, and the cells were adjusted to the appropriate cell concentration using a complete medium, and the cell suspension was spread in a 96-well plate, 90 µL per well, and placed in a 37°C, 5% CO₂ incubator overnight; and compound solutions of different concentrations were prepared using DMSO and culture medium; and a solvent control was set, the compound solution was added to a 96-well plate, 10 µL per well, at 37°C in a 5% CO₂ incubator for 72 hours; CellTiter-Glo solution was added thereto and the mixture was mixed well by shaking, incubated for 10 min in the dark, and read by BioTek Synergy H1 microplate reader.

### 1.4. Experimental data processing methods:

The luminescence signal values were used to calculate the inhibition rate, the concentration and the inhibition rate were fitted to a nonlinear regression curve using Graphpad Prism software, then the IC₅₀ value was obtained.

### 1.5. Experimental results:

The experimental results are shown in Table 8, IC₅₀ values of the inhibitory activity of the compounds of the embodiments on the proliferation of NCI-H358 and Mia PaCa-2 cells.

**Table 8**

| Embodiment number | NCI-H358 IC₅₀ (nM) | Mia PaCa-2 IC₅₀ (nM) |
|---|---|---|
| Embodiment 1 | 40 | 60 |
| Embodiment 1-1 | 28 | 55 |
| Embodiment 2 | 50 | 43 |
| Embodiment 2-1 | 35 | 29 |
| Embodiment 3 | 14 | 41 |
| Embodiment 4 | 48 | 47 |
| Embodiment 5 | 23 | 30 |
| Embodiment 9 | 5.4 | 8.6 |
| Embodiment 9-1 | 6.6 | 3.5 |
| Embodiment 10 | 39 | 59 |
| Embodiment 11 | 68 | 76 |
| Embodiment 12 | 79 | 68 |
| Embodiment 13 | 5.9 | 7.6 |
| Embodiment 13-1 | 6.6 | 3.3 |
| Embodiment 14 | 16 | 23 |
| Embodiment 14-1 | 17 | 11 |
| Embodiment 15 | 79 | 68 |
| Embodiment 17 | NT | 18 |
| Embodiment 18 | NT | 7 |
| Embodiment 19 | NT | 5.2 |
| Embodiment 20 | NT | 5.3 |
| Embodiment 22 | NT | 6.0 |
| Embodiment 25 | NT | 4.9 |
| Embodiment 30 | NT | 14 |
| Embodiment 32 | NT | 59 |
| Embodiment 40 | 32 | 16 |

| | | |
|---|---|---|
| Note: "NT" means not tested. | | |

### 1.6. Experimental conclusion:

According to the data, the compounds of the embodiments of the present disclosure have a good inhibitory effect on the proliferation of NCI-H358 and Mia PaCa-2 cells.

### Test Embodiment 2. Determination of the ability of the compound of the present disclosure to improve the binding stability (melting temperature) of KRAS G12C protein

### 2.1. Experimental purpose:

To determine the ability of the compound to improve the stability of KRAS G12C protein (the degree of increase in protein melting temperature can be used to characterize the compound's ability to bind to KRAS G12C protein).

### 2.2. Experimental reagents and instruments:

### 2.2.1 Experimental instruments:

Quantitative PCR instrument (Quantstudio6 Flex) was purchased from Life Company;
Pipettes were purchased from Eppendorf or Rainin Company.

### 2.2.2 Experimental reagents:

Protein Thermal Shift^{™} Dye Kit was purchased from Thermofisher Company, the article number was 4461146;
KRAS G12C protein was purchased from Beijing SinoBiological Co., Ltd., the article number was 12259-H07E2;
HEPES, 1M Buffer Solution was purchased from Thermofisher Company, the article number was 15630080;
DTT was purchased from Sigma Company, the article number was 43816-50mL;
NaCl was purchased from Sinopharm Chemical Reagent Co., Ltd., the article number was 10019318.

### 2.3 Experimental methods:

In this experiment, the thermal shift method was used to test the degree of change in the melting temperature (Tm) of the KRAS G12C protein before and after the binding of the compound, in order to characterize the ability of the compound to improve the stability of the KRAS G12C protein.

The specific experiment operation was as follows:
A solution containing 20 µM HEPES (pH 7.5), 1 mM DTT, 5X SYPRO Orange and 150 mM NaCl was prepared as the experimental buffer, and a final concentration of 5.37 µM human KRAS G12C protein was added thereto. The reaction mixture was divided into 8 rows of PCR tubes, each 19.5 µL, and 0.5 µL of the test compound or DMSO were added respectively, so that the total reaction system was 20 µL, the final concentration of the compound was 10 µM, and 2.5% DMSO was set as the solvent control. After incubating at room temperature in the dark for 1 hour, the PCR tube was put into the PCR instrument, QuantStudio Software v1.3 was opened, and the melting temperature of KRAS G12C protein in different treatment groups was detected by melt curve function (heating from 25°C to 95°C, 0.03°C/s).

### 2.4. Experimental data processing methods:

The experimental data file of PCR instrument was imported into thermal shift software, and the melting temperature (Tm) of each treatment group was obtained, and the change value of melting temperature (ΔTm) was obtained by subtracting the Tm of DMSO solvent control group.

### 2.5. Experimental results:

According to the above scheme, the compounds of the present disclosure show the ability to increase the melting temperature of the protein as shown in Table 9 in the experiment of improving the binding stability of KRAS G12C protein.

**Table 9**

| Embodiment number | Tm (°C) DMSO | Tm (°C) | ΔTm (°C ) |
|---|---|---|---|
| Embodiment 1 | 48.6 | 60.2 | 11.6 |
| Embodiment 2 | 48.7 | 57.2 | 8.5 |
| Embodiment 3 | 50.6 | 61.5 | 10.9 |
| Embodiment 4 | 49.5 | 61.2 | 11.7 |
| Embodiment 5 | 48.6 | 64.4 | 15.8 |
| Embodiment 9 | 46.8 | 60.2 | 13.4 |
| Embodiment 13 | 47.0 | 58.0 | 11.0 |

### 2.6 Experimental conclusion:

The above data show that the compounds of the embodiments of the present disclosure have good binding ability to KRAS G12C protein.

### Test Embodiment 3. The inhibitory activity of the compounds of the present disclosure on Miapaca-2 cell P-ERK

### 3.1. Experimental purpose:

To determine the inhibitory activity of the compounds of the embodiments on the level of phosphorylated ERK in KRAS G12C mutant cells Mia PaCa-2.

### 3.2. Experimental instruments:

### 3.2.1 Instrument:

Microplate reader (BioTek Synergy H1);
Pipette (Eppendorf & Rainin).

### 3.2.2 Reagents:

Phosphorylated ERK1/2 (T202-Y204) LANCE Ultra Cellular Detection Kit was purchased from PerkinElmer Company, the article number was TRF4000M;
The cell culture plate was purchased from Corning, the article number was 3610;
White opaque OptiPlate^{™}-384 plate was purchased from PerkinElmer, the article number was 6007290.

### 3.3. Experimental methods:

When Mia PaCa-2 cells were cultured to the appropriate fusion level, Mia PaCa-2 cells were collected, and the cell density was adjusted to 1 × 10⁶/mL using complete culture medium, the cell suspension was spread on a 96-well plate, 50 µL per well, and placed adherent to the wall in a 37°C, 5% CO₂ incubator overnight, compound solutions with different concentrations were prepared using DMSO and complete culture medium, a solvent control was set, the compound solution was added to a 96-well plate, 25 µL per well, and placed in a 37°C, 5% CO₂ incubator for 2 hours of continuous culture, the supernatant was discarded from the cell culture plate, 50 µL of lysis solution was added to each well, and lysing was performed for 30 minutes by shaking at room temperature, then the mixture was centrifuged at 1000 rpm for 1 minute, 15 µL of supernatant was transferred to 384 well plate, 5 µL of detection mixture (Eu-labeled anti-ERK 1/2 (T202-Y204) antibody with final concentration of 0.5 nM and ULight labeled anti-ERK1/2 antibody with final concentration of 5 nM) was added to each well, centrifuged at 1000 rpm for 1 minute and mixed uniformly, the reaction was carried out overnight at room temperature, the plate was read with BioTek Synergy H1, and the signal values was detected at 620 nm and 665 nm emission wavelengths by time-resolved fluorescence program.

### 3.4. Experimental data processing methods:

The ratio of the signal values at 665 nm and 620 nm emission wavelength were calculated, and the ratio was used to calculate the inhibition rate, the concentration and the inhibition rate were fitted to a nonlinear regression curve using Graphpad Prism software, then the IC₅₀ value was obtained.

### 3.5. Experimental results:

**Table 10 IC₅₀ values of pERK inhibition on Mia PaCa-2 cells**

| Embodiment number | Mia PaCa-2 pERK IC₅₀ (nM) |
|---|---|
| Embodiment 2-1 | 38 |
| Embodiment 5 | 30 |
| Embodiment 9-1 | 5.0 |
| Embodiment 13-1 | 4.2 |
| Embodiment 14-1 | 20 |

### 3.6. Experimental conclusion:

The above data show that the compounds of the embodiments of the present disclosure have a good inhibitory effect on pERK in Mia PaCa-2 cells.

### Test Embodiment 4. Determination of pharmacokinetics in mice

### 4.1. Research purpose:

To study the pharmacokinetic behavior of the compounds in mice (plasma) after oral administration using Balb/c mice as test animals.

### 4.2. Test scheme:

### 4.2.1 Test drugs:

The compound of the embodiment of the present disclosure was self-made;

### 4.2.2 Test animals:

Balb/c Mice, male, purchased from Shanghai Jiesijie Laboratory Animal Co., Ltd, Animal Production License No. (SCXK (Shanghai) 2013-0006 N0. 311620400001794).

### 4.2.3 Drug preparation:

5 g of Hydroxyethyl cellulose (HEC, CMC-Na, viscosity: 800-1200 Cps) was weighed, dissolved in 1000 mL of purified water, and 10 g of Tween 80 was added. The mixture was mixed well to form a clear solution.

The compounds of the embodiments were weighed and added into 4-mL glass bottles, respectively, 2.4 mL of the solution was added, and ultrasound was performed for 10 minutes to obtain a colorless clear solution with a concentration of 1 mg/mL.

### 4.2.4 Administration:

Balb/C mice, males; PO, after overnight fasting, respectively, at a dose of 10 mg/kg, administered in a volume of 10 mL/kg.

### 4.2.5 Sample collection:

Blood samples were collected before administration and 0.083 h, 0.25 h, 0.5 h, 1 h, 2 h, 4 h, 6 h and 8 h after administration, the blood was placed in EDTA-2K tube, centrifuged at 4°C 6000 rpm for 6 min to separate plasma, and stored at -80°C; food was consumed 4 hours after drug administration.

### 4.3 Experimental results:

The final determination results obtained by applying LCMS/MS method are shown in Table 11.

**Table 11: Pharmacokinetic parameters of the compounds in mice**

| Embodiment number | Tmax (hr) | Cmax (ng/mL) | AUC_{0-∞} (ng/mL*hr) | T_{1/2} (hr) | MRT (hr) |
|---|---|---|---|---|---|
| Embodiment 2-1 | 0.25 | 1823 | 1373 | 0.6 | 0.7 |
| Embodiment 13-1 | 0.25 | 264 | 347 | 1.0 | 1.5 |

### 4.4 Experimental conclusion:

The above data show that the compounds of the embodiments of the present disclosure have good pharmacokinetic parameters in mice.

### Test Embodiment 5. Tumor inhibition experiment on MiaPaca 2 transplanted tumor model

### 5.1 Experimental purpose:

BALB/c nude mice were used as the test animals, and the human pancreatic cancer cell MiaPaca 2 xenograft (CDX) model was used for *in vivo* pharmacodynamic experiments to evaluate the antitumor effects of the test compounds.

5.2 Experimental instruments and reagents:

5.2.1 Instrument:
Ultra Clean Bench (BSC-1300II A2, Shanghai Boxun Industrial Co., Ltd. Medical Equipment Factory);
CO₂ incubator (Thermo-311, Thermo);
Centrifuge (Centrifuge 5720R, Eppendorf);
Fully automatic cell counter (Countess II, Life Technologies);
Pipette (10-20 µL, Eppendorf);
Microscope (Ts 2, Nikon);
Vernier caliper (CD-6" AX, Mitutoyo Japan);
Cell culture flask (T25/T75/T225, Corning);
Constant temperature water tank (HWS12, Shanghai Yiheng Science).

### 5.2.2 Reagents:

DMEM (11995-065, Gibco);
Fetal bovine serum (FBS) (10091-148, Gibco);
0.25% trypsin (25200-056, GIBCO);
Penicillin double antibody (P/S) (SV30010, GE);
Phosphate buffer (PBS) (10010-023, Gibco);
Matrigel (356234, Corning);
Gln (25030-081, Gibco).

### 5.3 Experimental operation:

MiaPaca 2 cells were removed from the cell bank, revived and added to DMEM medium (containing 10% FBS, 1% Glu, 1% P/S) and incubated in a CO₂ incubator (incubator temperature was 37°C, CO₂ concentration was 5%). After the cells were spread to 80-90% of the bottom of the culture flask, the cells were continued to be cultured in the CO₂ incubator. The process was repeated until the number of cells met the *in vivo* pharmacological inoculation requirement, and the cells in logarithmic growth period were collected and counted with an automatic cell counter, resuspended with PBS and Matrigel (volume ratio 1: 1) according to the count results, made into a cell suspension (the density was 8× 10 ⁷/mL), and placed in an ice box for use.

BALB/c nude mice, female, 6-8 weeks old, weighing about 18-22 g. The mice were kept in an environment free of special pathogens and in a single ventilated cage with 5 mice in each cage. All cages, bedding and water were sterilized before use, and all animals had free access to standard certified commercial laboratory diets. Nude mice were labeled with disposable universal ear tags for mice and rats before the start of the experiment, and the skin of the inoculation site was disinfected with 75% medical alcohol before inoculation, 0.1 mL (containing 8* 10⁶ cells) of MiaPaca 2 tumor cells were inoculated subcutaneously on the right back of each mouse. When the tumor volume reached 100- 200 mm³, the group administration was started. The tested compounds were administered daily by oral intragastric administration, dosage/frequency (6 mg/kg QD × 3w), and the efficacy of each group at the end of the experiment was shown in Table 5.

### 5.4 Data processing:

The tumor volume (mm³) was measured with vernier caliper twice a week, the calculation formula was V=0.5*D*D*D, wherein D and d were the long and short diameter of the tumor, respectively. The anti-tumor efficacy was determined by dividing the average tumor increased volume of the compound-treated animals by the average tumor increased volume of the untreated animals. The formula of tumor inhibition rate is: TGI (%) = 1-[(Vt-V0) administration group/(Vt-V0) solvent control group] * 100%. After the experiment, all animals were euthanized.

### 5.5 Experimental results:

**Table 12: Pharmacodynamic parameters of the compounds in transplanted tumor mice**

| **Grouping** | **Tumor volume (mm³, Mean ± SD)** | | Δ**T**/Δ**C (%)** | **TGI (%)** |
|---|---|---|---|---|
| | **Day 0** | **Day 21** | **Day 21** | **Day 21** |
| Vehicle QD × 3w | 178±30 | 868±234 | - | - |
| Embodiment 2-1 | 177±38 | 67±34 | -62.36 | 162.36 |
| Embodiment 9-1 | 178±40 | 72±18 | -59.56 | 159.56 |
| Embodiment 13-1 | 178±34 | 41±19 | -76.76 | 176.76 |

### 5.6 Experimental conclusion:

The above data show that after oral administration for 21 days, the compounds of the embodiments of the present disclosure can significantly inhibit the growth of transplanted tumor in MiaPaca 2 nude mice under the condition of oral administration of 6 mg/kg per day.

### Test Embodiment 6. In vivo pharmacodynamic study on human lung cancer NCI-H358 cell xenograft tumor model

### 6.1 Experimental purpose:

To evaluate the efficacy of the compound *in vivo* on xenograft tumor model of human lung cancer NCI-H358 cells.

6.2 Experimental instruments and reagents:

6.2.1 Instruments:
1) Biological safety cabinet (BSC-1300II A2, Shanghai Boxun Industrial Co., Ltd., Medical Equipment Factory);
2) Ultra-clean bench (CJ-2F, Suzhou Fengshi Laboratory Animal Equipment Co.)
3) CO₂ incubator (Thermo-311, Thermo);
4) Centrifuge (Centrifuge 5720R, Eppendorf);
5) Fully automatic cell counter (Countess II, Life Technologies);
6) Vernier caliper (CD-6" AX, Mitutoyo Japan);
7) Cell culture flask (T75/T225, Corning);
8) Electronic balance (CPA2202S, Sartorius);
9) Electronic balance (BSA2202S-CW, Sartorius);
10) Electronic balance (BS124S, Sartorius).

### 6.2.2 Reagents:

1) RPMI-1640 medium (22400-089, Gibco);
2) DMEM medium (11995-065, Gibco);
3) Fetal bovine serum (FBS) (10099-141C, Gibco);
4) Phosphate buffer (PBS) (10010-023, Gibco);
5) Tween 80 (30189828, Sinopharm reagent);
6) Sodium carboxymethyl cellulose (30036365, Sinopharm reagent.)

### 6.3 Experimental operation and data processing:

### 6.3.1 Test animals:

BALB/c nude mice, 6-8 weeks old, female, purchased from Shanghai Xipuer-Bikai Experimental Animal Co., Ltd.

### 6.3.2 Cell culture and cell suspension preparation

1) MiaPaca-2 cells were taken out from the cell bank and resuscitated with DMEM medium (DMEM + 10% FBS), the resuscitated cells were placed in a cell culture flask (labeled with cell type, date, name of cultured person, etc.) and cultured in a CO₂ incubator (incubator temperature was 37°C, CO₂ concentration was 5%) (the method of resuscitating NCI-H358 cells was the same as MiaPaca-2 cells in test embodiment 5, and the culture medium was changed to RPMI-1640 medium).
2) Passage was conducted every three to five days, and the cells was continued to be cultured in CO₂ incubator after passage. The process was repeated until the cell count meets the *in vivo* pharmacodynamic requirements.
3) MiaPaca-2 cells were collected and counted by automatic cell counter, according to the counting results, the cells were re-suspended with PBS and Matrigel (ratio was 1:1) to make cell suspension (cell density was 5 × 10⁷/mL), then placed in a ice box for use (NCI-H358 cells were re-suspended with PBS without adding Matrigel, cell density was 1 × 10⁸/mL).

### 6.3.3 Sample preparation:

1) Solvent: solvent (0.5% CMC-Na+1% Tween 80), storage condition: 4°C.
   0.5 g of CMC-Na was weighed, dissolved in ddH₂O, then 1.0 mL of Tween 80 was added and the mixture was stirred to mix well, and the volume was finally set to 100 mL.
2) Compound to be tested (10 mg/kg) was prepared:
   8.42 mg of AMG510 compound was weighed, 8.260 mL of solvent was added, a uniform solution was obtained by ultrasound, vortexing and stirring.
   7.81 mg of embodiment compound 13-1 was weighed, 7.654 mL of solvent was added, a uniform solution was obtained by ultrasound, vortexing and stirring.

### 6.3.3 Cell inoculation

1) Before inoculation, nude mice were labeled with disposable universal ear tags of rats and mice;
2) when inoculating, the cell suspension was mixed well, 0.1-1 mL cell suspension was extracted with a 1 mL syringe, bubbles were removed, and then the syringe was put on an ice bag for later use;
3) the nude mice was held with left hand, the right back of nude mice near the right shoulder (inoculation site) was disinfected with 75% alcohol, and inoculation was started after 30 seconds;
4) the experimental nude mice were inoculated in turn (each mouse was inoculated with 0.1 mL of cell suspension);

### 6.3.4 Tumor-bearing mouse were measured, grouped, and administered:

1) According to the tumor growth, the tumor was measured on the 18th day after inoculation, and the tumor size was calculated.
   Tumor volume calculation: tumor volume (mm³) = length (mm) × width (mm) × width (mm)/2
2) The tumor-bearing mice were grouped according to their body weight and tumor size using a randomized grouping method.
3) According to the grouping results, the administration of the test drug was started (administration method: oral administration; administration dose: 10 mg/kg; administration volume: 10 mL/kg; administration frequency: once a day; administration period: 21 days; solvents: 0.5% CMC/1% Tween 80).
4) The tumor was measured and weighed twice a week after the test drug was started to be given.
5) After the experiment, all animals were euthanized.
6) Data was processed with software such as Excel.

### 6.4 Data processing:

Calculation of TGI (%) of compound tumor inhibition rate: when there was no tumor regression, TGI (%) = [(1-(mean tumor volume at the end of the administration in a treatment group - mean tumor volume at the start of administration in the treatment group))/(mean tumor volume at the end of treatment in the solvent control group - mean tumor volume at the start of treatment in the solvent control group)] × 100%. When there was tumor regression, TGI (%) = [1-(mean tumor volume at the end of dosing in a treatment group - mean tumor volume at the beginning of dosing in the treatment group)/mean tumor volume at the beginning of dosing in the treatment group] × 100%.

### 6.5 Experimental results:

**Table 13: Pharmacodynamic parameters of the compounds in transplanted tumor mice**

| **Grouping** | **Tumor volume (mm³, Mean ± SD)** | | Δ**T**/Δ**C (%)** | **TGI (%)** |
|---|---|---|---|---|
| | **Day 0** | **Day 15** | **Day 15** | **Day 15** |
| Vehicle QD × 3w | 202±58 | 400±111 | - | - |
| Embodiment 13-1 10 mpk | 203±74 | 267±155 | 32.59 | 67.41 |
| AMG-510 10 mpk | 202±72 | 324±204 | 61.98 | 38.02 |

### 6.6 Experimental conclusion:

The above data show that after 15 days of continuous oral administration, the compounds of the embodiments of the present disclosure significantly inhibited the growth of the tumors of nude mouse transplanted with human lung cancer NCI-H358 cells under the condition of oral administration of 10 mg/kg per day, which was significantly better than the reference data.

### Test Embodiment 7. hERG potassium channel inhibitory activity test

### 7.1 Cell preparation

7.1.1 CHO-hERG cells were cultured in a 175 cm² flask, when the cell density reached 60-80%, the culture medium was removed, the cells were washed with 7 mL PBS, and then digested with 3 mL Detachin.

7.1.2 After complete digestion, 7 mL culture medium was added to neutralize, then the mixture was centrifuged, the supernatant was aspirated, and then 5 mL culture medium was added to re-suspend, ensuring 2-5 × 10⁶/mL of cell density.

### 7.2 Solution preparation

**Table 14: Composition of intracellular fluid and extracellular fluid**

| **Reagent** | **Extracellular fluid (mM)** | **Intracellular fluid (mM)** |
|---|---|---|
| CaCl₂ | 2 | 5.374 |
| MgCl₂ | 1 | 1.75 |
| KCI | 4 | 120 |
| NaCl | 145 | - |
| Glucose | 10 | - |
| HEPES | 10 | 10 |
| EGTA | - | 5 |
| Na-ATP | - | 4 |
| pH | 7.40 (adjusted with NaOH), Osmolarity~305 mOsm | 7.25 (adjusted with KOH), Osmolarity-290 mOsm |

### 7.3 Electrophysiological recording process

The process of single cell high impedance sealing and whole cell mode formation were all automatically completed by Qpatch instrument, after obtaining the whole cell recording mode, the cells were clamped at -80 mV, before giving a 5-second +40 mV depolarization stimulus, a 50 millisecond -50 mV prevoltage was given first, and then repolarized to -50 mV for 5 seconds, then returned to -80 mV. This voltage stimulation was applied every 15 seconds and recorded for 2 minutes before giving extracellular fluid recordings for 5 minutes, and then the administration process was started, the compound concentration was given from the lowest test concentration, each test concentration was given for 2.5 minutes, and the positive control compound 3 µM of Cisapride was given after all concentrations were continuously given. At least 3 cells (n ≥ 3) were tested at each concentration.

### 7.4 Test compound:

7.4.1 20 mM of compound mother liquor was diluted with extracellular fluid, 5 µL of 20 mM compound mother liquor was added into 2495 µL of extracellular fluid and diluted 500-fold to 40 µM, and then the final concentration to be tested was obtained by sequential 3-fold serial dilutions in extracellular solution containing 0.2% DMSO.

7.4.2 The highest test concentration was 40 µM, in a total of 6 concentrations of 40, 13.33, 4.44, 1.48, 0.49 and 0.16 µM respectively.

7.4.3 The content of DMSO in the final test concentration was not more than 0.2%, and this concentration of DMSO had no effect on hERG potassium channel.

### 7.5 Data analysis:

The experimental data were analyzed by XLFit software.

### 7.6 Quality Control

Environment: humidity 20-50%, temperature 22 to 25°C
Reagent: The experimental reagent used was purchased from Sigma Company, and the purity was > 98%
The experimental data in the report must meet the following criteria:
Whole cell sealing impedance > 100 M Ω
Tail current amplitude > 400 pA
Pharmacological parameters:
The inhibitory effect of multiple concentrations of Cisapride on hERG channel was set as positive control.

### 7.7 Experimental results:

**Table 15: Inhibition results of the embodiments of the present disclosure at multiple concentrations on hERG current**

| Embodiment number | hERG (µM) |
|---|---|
| Embodiment 2-1 | >30 |
| Embodiment 9-1 | >30 |
| Embodiment 13-1 | >30 |
| Embodiment 14-1 | >30 |

### 7.8 Experimental conclusions:

The inhibition of drugs on the cardiac hERG potassium channel was the main cause of QT prolonged syndrome caused by drugs. It can be seen from the experimental results that the embodiment compound of the present disclosure had no obvious inhibitory effect on the cardiac hERG potassium ion channel, and can avoid the toxic and side effects to the heart at a high dose.

### Test Embodiment 8. Plasma Stability Test Scheme

### 8.1 Experimental purpose:

The purpose of this experiment was to examine the stability of the compounds of the embodiments in mouse, rat, dog and human plasma.

### 8.2 Experimental steps:

### 8.2.1 Solution preparation

### 1) Plasma preparation

Animal or human whole blood was collected, then the blood was put into a test tube containing anticoagulant, centrifuged at 3500 rpm for 10 min, and the upper layer of pale yellow plasma was collected.

### 2) 10 µM of tested compound (m/M/V=C)

The compound was weighed, the stock solution was prepared with DMSO and the working solution was prepared with 100 mM phosphate buffer.

### 3) 10 µM of positive control

### (1) Propantheline (Propantheline Mr=449.4 Da)

2.36 mg of Propantheline was weighed and diluted to 10 mM stock solution with 1 mL of DMSO; 10 µL of 10 mM stock solution was pipetted into 1 mL of 100 mM phosphate buffer to a final concentration of 100 µM.

### (2) Mevinolin (Lovastatin Mr=404.5 Da)

4.05 mg of lovastatin was weighed and diluted to 10 mM stock solution with 1 mL of DMSO; 10 µL of 10 mM stock solution was pipetted into 1 mL of 100 mM phosphate buffer to a final concentration of 100 µM.

### 8.2.2 Experimental process:

1) 285 µL of plasma and 15 µL of 10 µM compound (tested compound) were added in turn in a 96-well plate, and incubated at 37°C.
2) 40 µL was taken out at 0, 15, 30, 60, 90, 120 min, respectively, and 160 µL of acetonitrile termination solution containing internal standard was added.
3) After centrifugation (3500 rpm, 10 min), 50 µL of supernatant was taken out, and then diluted with 50 µL DDH2O and injected to LC-MS/MS.

### 8.3 Chromatographic conditions

instrument: Shimadzu LC-20 AD
chromatographic column: phenomenex gemiu ^{®} C18 (50*4.6 mm, 5 µM particle size);
mobile phase: A: acetonitrile, B: 0.1% formic acid solution 0-8 min: 5% A → 95% A, 2.0-2.1 min: 90% A → 5% A; flow rate: 0.8 mL/min; running time: 5.0 min; injection volume: 5 µL.

### 8.4 Mass spectrum conditions:

instrument: API4000 Liquid Chromatography-Mass Spectrometry, AB, USA;
the ion source was electrospray ionization source (ESI);
the temperature of dry gas (N₂) was 500 °C;
electrospray voltage was 5500V;
the detection method was positive ion detection;
the scanning mode was selective response monitoring (MRM);
the scanning time was 0.1 s.
8.5 Experimental results:

**Table 16: Plasma stability results of compounds of the embodiments**

| **Species and genus** | **Number** | **Residual rate (%)** | | | | | ***t*_{1/2} (min)** |
|---|---|---|---|---|---|---|---|
| | | **0 min** | **15 min** | **30 min** | **60 min** | **120 min** | |
| Human | Propantheline | 100.00 | 79.98 | 50.52 | 14.65 | 0.72 | 21.03 |
| | Embodiment 2-1 | 100.00 | 97.39 | 98.79 | 94.50 | 87.91 | 660.77 |
| | Embodiment 9-1 | 100.00 | 97.95 | 100.46 | 97.37 | 93.81 | 1383.71 |
| | Embodiment 14-1 | 100.00 | 99.15 | 94.74 | 89.14 | 78.85 | 338.58 |
| | Embodiment 13-1 | 100.00 | 102.34 | 100.28 | 90.27 | 87.22 | 500.87 |
| | AMG 510 | 100.00 | 93.77 | 85.72 | 82.51 | 64.44 | 198.80 |
| Rat | Lovastatin | 100.00 | 21.15 | 1.77 | 0.36 | 0.30 | 6.69 |
| | Embodiment 2-1 | 100.00 | 99.04 | 101.03 | 97.16 | 84.63 | 489.40 |
| | Embodiment 9-1 | 100.00 | 97.08 | 94.54 | 90.84 | 82.91 | 453.73 |
| | Embodiment 14-1 | 100.00 | 93.20 | 98.32 | 99.08 | 96.46 | 6505.51 |
| | Embodiment 13-1 | 100.00 | 95.57 | 96.73 | 93.97 | 91.93 | 1159.17 |
| | AMG 510 | 100.00 | 100.85 | 93.03 | 77.54 | 56.77 | 137.24 |
| Mouse | Propantheline | 100.00 | 79.79 | 48.66 | 23.39 | 6.66 | 27.74 |
| | Embodiment 2-1 | 100.00 | 102.67 | 98.51 | 95.49 | 84.58 | 453.87 |
| | Embodiment 9-1 | 100.00 | 103.68 | 104.44 | 97.95 | 88.36 | 551.41 |
| | Embodiment 14-1 | 100.00 | 105.42 | 100.02 | 99.12 | 85.55 | 465.04 |
| | Embodiment 13-1 | 100.00 | 100.58 | 99.52 | 99.64 | 92.32 | 1015.92 |
| | AMG 510 | 100.00 | 97.50 | 82.58 | 81.82 | 63.23 | 184.89 |
| Dog | Lovastatin | 100.00 | 95.25 | 95.45 | 75.66 | 36.25 | 80.22 |
| | Embodiment 2-1 | 100.00 | 97.87 | 96.23 | 92.42 | 85.48 | 534.11 |
| | Embodiment 9-1 | 100.00 | 96.78 | 94.25 | 92.45 | 89.54 | 830.19 |
| | Embodiment 14-1 | 100.00 | 99.98 | 98.79 | 93.69 | 86.16 | 532.60 |
| | Embodiment 13-1 | 100.00 | 97.71 | 96.32 | 94.86 | 92.49 | 1173.33 |
| | AMG 510 | 100.00 | 98.88 | 101.14 | 93.43 | 91.92 | 884.42 |

### 8.6 Experimental conclusions:

The above data show that the plasma stability of the compounds of the embodiments in the present disclosure is high with little species difference.

### Test Embodiment 9. CYP enzyme single point inhibition test

### 9.1 Experimental purpose:

Using human liver microsomal incubation system, the inhibition of CYP450 enzyme subtypes by compounds was rapidly predicted by single point method.

### 9.2 Experimental steps:

### 9.2.1 Solution preparation

2.5 mM NADPH: 100 mM phosphate buffer was added to 4.165 mg of NADPH (reduced nicotinamide adenine dinucleotide phosphate) to 2 mL. 0.25 mg/mL microsome: 4 mL of 100 mM phosphate buffer was added to 50 µL of 20 mg/mL microsome and mixed well.

### Preparation of reaction mixture for compounds to be tested

The embodiment compound to be tested was weighed and diluted to 10 mM with DMSO and then diluted to 100 µM with 100 mM phosphate buffer.

### 9.2.2 Experimental process:

1. In a 96-well plate, 40 µL of liver microsomes, 10 µL of substrate, 10 µL of compound to be tested were pre-incubated for 3 min.
2. 40 µL of NADPH was added.
3. 300 µL of acetonitrile termination solution containing internal standard was added at 20 min.
4. Centrifugal injection.

### 9.3 Experimental results:

**Table 17: Single point inhibition results of CYP enzyme of compounds of the embodiments**

| No. | IC₅₀ (µM) | | | | | |
|---|---|---|---|---|---|---|
| | **1A2** | **2C9** | **2C19** | **2D6** | **3A4-M** | **3A4-T** |
| Control | 0.064 | 0.459 | 0.293 | 0.099 | 0.089 | 0.117 |
| Embodiment 2-1 | >100 | 84.6 | >100 | >100 | 19.0 | >100 |
| Embodiment 9-1 | 48.9 | 59.3 | 43.2 | 44.5 | 4.6 | 18.0 |
| Embodiment 13-1 | 66.7 | 58.8 | 28.8 | 21.2 | 4.7 | 9.1 |

| | | | | | | |
|---|---|---|---|---|---|---|
| Note: Strong inhibition: IC₅₀ < 1 µM; moderate inhibition: 1 µM < IC₅₀ < 10 µM; weak inhibition: IC₅₀ > 10 µM | | | | | | |

### 9.4 Experimental conclusions:

The above data show that the embodiment compound of the present disclosure has no strong inhibition on each CYP enzyme subtype, and the risk of DDI is small.

### Test Embodiment 10. Plasma Protein Binding Rate Test

### 10.1 Experimental purpose:

The purpose of this experimental method was to detect the plasma protein binding of the compounds of the embodiments in plasma.

### 10.2 Experimental instruments and materials:

Liquid-phase mass spectrometer, centrifuge, vortexer, pipette, continuous liquid dispenser, 96-well plate, tissue homogenizer (for tissue sample analysis), 50% methanol aqueous solution, acetonitrile solution with internal standard, blank matrix (plasma, urine or tissue homogenate, etc.)

### 10.3 Experimental steps:

### 10.3.1 Preparation of the stock solution A for the test substance

The embodiment compound was prepared into a 1 mM solution A with DMSO.

### 10.3.2 Preparation of plasma solution B

Solution A was added to the plasma solution and prepared into a 5 µM solution B.

### 10.3.3 Treatment process

1) 200 µL of solution B was added into the membrane.
2) 350 µL of PBS was added to the outside of the membrane.
3) Incubating in a 37°C water bath for 6 hours.
4) The samples were processed for dilution and detected by mass spectrometry.

### 10.4 Chromatographic conditions.

instrument: Shimadzu LC-20 AD;
chromatographic column: phenomenex gemiu ^{®} C18 (50*4.6 mm, 5 µM particle size);
mobile phase: A: Acetonitrile, B: 0.1% formic acid solution 0-0.5 min: 5% A → 90% A, 2.0-2.1 min: 90% A → 5% A; flow rate: 0.8 mL/min; running time: 5.0 min; injection volume: 5 µL.
10.5 Mass spectrum conditions:
instrument: API4000 Liquid Chromatography-Mass Spectrometry, AB, USA;
the ion source was electrospray ionization source (ESI);
the temperature of dry gas (N₂) was 500°C;
electrospray voltage was 5500V;
the detection method was positive ion detection;
the scanning mode was selective response monitoring (MRM); the scanning time was 0.1 s.

### 10.6 Experimental results:

**Table 18: Plasma protein binding rate of compounds of the embodiments**

| No. | Human | Rat | Mouse | Dog |
|---|---|---|---|---|
| Embodiment 2-1 | 98.0 | 90.5 | 88.4 | 82.6 |
| Embodiment 9-1 | 99.8 | 94.9 | 90.1 | 98.7 |
| Embodiment 13-1 | 99.7 | 97.9 | 93.9 | 98.7 |
| Embodiment 14-1 | 96.8 | 95.4 | 96.3 | 92.5 |

### 10.7 Experimental conclusions:

The above data show that the compounds of the embodiments of the present disclosure exhibit high plasma protein binding rate with little species difference.

### Test Embodiment 11. Determination of pharmacokinetics in tumor-bearing mice

### 11.1. Research purpose:

The pharmacokinetic behavior of the compound of the embodiment 13-1 and AMG-510 compound, administered orally at a dose of 6 mg/kg, in mice (plasma, tumor tissue and intestine) was studied using MiaPaca 2 tumor-bearing mice as test animals.

### 11.2. Test scheme:

### 11.2.1 Test drugs:

Embodiment 13-1 of the present disclosure, AMG-510 compound, self-made.

### 11.2.2 Test animals:

24 MiaPaca 2 tumor-bearing mice, females. 3 for each time point (0 h, 1 h, 2 h, 4 h, 6 h, 8 h, 16 h, 24 h). Shanghai xipuer-bikai Laboratory Animal Co., Ltd, Animal Production License No. (SCXK (Shanghai) 2018-0006.

### 11.2.3 Drug formulation:

5 g of Hydroxymethyl cellulose was weighed, dissolved in 1000 mL of purified water, and 10 g of Tween 80 was added. The mixture was mixed well to form a clear solution.

Embodiment compound 13-1 and compound AMG-510 were weighed and dissolved in the solution, the mixture was shaken well, and ultrasound was performed for 15 minutes to obtain a uniform suspension with a concentration of 0.6 mg/mL.

### 11.2.4 Administration:

MiaPaca 2 tumor-bearing mice were administered at a dose of 6 mg/kg in a volume of 10 mL/kg, respectively, based on body weight p.o. after fasting (animals were not administered at point 0 h).

### 11.2.5 Sample collection:

Before and after administration, mice were sacrificed with CO₂, 0.5 mL blood was collected from the heart and placed in EDTA-2K tube, centrifuged at 4°C 6000 rpm for 6 min to separate plasma, and stored at -80°C; after the tumor tissues were weighing, placed in a 2 mL centrifuge tube and stored at-80°C. The duodenum, ileum and colon tissues were cut with scissors, the contents were removed and cleaned twice with PBS, after absorbing water with absorbent paper, they were weighed, placed in a 2 mL centrifuge tube and stored at -80°C.

11.3 Experimental results: the final determination results obtained by applying LCMS/MS method are shown in Table 11:

**Table 19: Pharmacokinetic parameters of the compounds of the present disclosure in mice**

| Number of the compound | | Ratio (T/P) | T_{1/2} (hr) | MRT (hr) |
|---|---|---|---|---|
| AMG-510 | Plasma | 0.42 | 0.3 | 0.8 |
| | Tumor | | 0.3 | 0.7 |
| Embodiment 13-1 | Plasma | 0.57 | 0.5 | 0.9 |
| | Tumor | | 0.9 | 1.5 |

### 11.4 Experimental conclusions:

At a dose of 6 mg/kg, the ratio of exposure of the compound of the embodiment of the present disclosure in the tumor of the mouse to the exposure in the blood was higher than that of AMG-510, with longer T_{1/2} and MRT.

### 3. Studies on salts and crystal forms of compounds

It is well known to those skilled in the art that when the above compounds of the embodiments are shown to have a good inhibitory effect on the proliferation of NCI-H358 and Mia PaCa-2 cells, the pharmaceutically acceptable salts may often have the same pharmacological and pharmacodynamic activities. On this basis, the inventors further study the physical and chemical properties of the salt forms and crystal forms of the corresponding compounds, but the preparation and characterization of the following specific salt forms or crystal forms described below do not limit the scope of protection of the present disclosure, and more salt forms and crystal forms of the compounds of the present disclosure can be obtained by conventional salt-forming or crystallization methods based on the present disclosure by those skilled in the art, and these salt forms and crystal forms are the schemes protected by the present disclosure. Details are as follows:

### 1. Experimental instruments

### 1.1 Parameters of physical chemistry testing instruments

| | | |
|---|---|---|
| X-ray powder diffraction (XRPD) | Instrument model | BRUKER D8 ADVANCE |
| | Diffracted ray | CuK (40 kV, 40 mA) |
| | Scan rate | 10°/min (2θ value) |

| | Scan range | 4° to 40° (2θ value) |
|---|---|---|
| Differential scanning calorimetry (DSC) | Instrument Model | NETZSCH DSC 214 polyma |
| | Purge gas | Nitrogen |
| | Purge speed | 40 mL/min |
| | Heating rate | 10°C /min |
| | Temperature range | 25 to 300°C |
| | Plate type | Aluminium plate |
| Thermogravimetric analysis (TGA) | Instrument model | NETZSCH DSC TG 209 F3 |
| | Purge gas | Nitrogen |
| | Purge speed | 40 mL/min |
| | Heating rate | 10°C /min |
| | Temperature range | Room temperature to 300°C |
| | Plate type | Al₂O₃ |
| Dynamic vapor sorption (DVS) | Instrument model | SMS Intrinsic |
| | Experimental temperature | 25°C |
| | Dying time | 0%RH 120 min |
| | Balance dm/dt | 0.02%/min (minimum 10 min, maximum 180 min) |
| | RH(%) measurement step length | 10% |
| | Measurement | 0-95-0% |
| | gradient | |
| | Cycle | 2 |

### 1.2 Instrument and liquid phase analysis conditions

### 1.2.1 Instruments and equipment

| Instrument name | Model |
|---|---|
| Analytical balance | METTLER TOLEDO XA105 |
| Water purifier | Milli-Q Plus, Millipore |
| High performance liquid chromatography | Thermo Ultimate 3000 |

### 1.2.2 Chromatographic conditions

| Instrument | Thermo Ultimate 3000 | |
|---|---|---|
| Mobile phase A | 25 mM Phosphate buffer (NH₄H₂PO₄, pH2.0) | |
| Mobile phase B | MeOH | |
| Flow rate | 1.0 mL/min | |
| Injection volume | 5.0 µL | |
| Chromatographic column | Waters x-bridge (150 * 4.6 mm, 3.5 µm) | |
| Column temperature | 35°C | |
| Detection wavelength | 235, 238, 326 nm | |

| Elution gradient (min) | A% | B% |
|---|---|---|
| 0.00 | 65 | 35 |
| 10.00 | 20 | 80 |
| 12.00 | 20 | 80 |
| 12.01 | 65 | 35 |
| 15.00 | 65 | 35 |

### 2. Study on salt forms of compounds

### 2.1 Screening salt forms of compound of embodiment 13-1

### 2.1.1 Experimental purpose:

To screen the salt forms of compound.

### 2.1.2 Experimental steps:

### 1) Instruments and equipment

| Name | Model | Source |
|---|---|---|
| Analytical balance | XA105 | METTLER TOLEDO |

| Ultrasonic cleaning machine | SK5200LHC | Shanghai KUDOS Ultrasonic Instrument Co., Ltd |
|---|---|---|
| Pipette | Eppendorf (50 mL, 100 µL) | Eppendorf |

### 2) Operation process

### (i) Solventing out or suspension to form salt

10 mg of compound was weighed, 200 µL of solvent was added thereto, the mixture was stirred at room temperature. Different acids were added respectively thereto, the mixture was stirred overnight, dried by centrifugation or volatilization to obtain a salt of the compound.

| No. | Acid | Solvent | Phenomenon after formic acid | Result |
|---|---|---|---|---|
| 1 | 1.0 M sulfuric acid (methanol solution) | ethanol | change from dissolved clarification to suspension | salt formation |
| 2 | | ethyl acetate | suspension | salt formation |
| 3 | | isopropanol | precipitation after dissolved clarification | salt formation |
| 4 | 1.0 M hydroxyethyl sulfonic acid (methanol solution) | n-butanol | change from dissolved clarification to suspension | salt formation |
| 5 | | ethanol | precipitation after | salt formation |
| | | | dissolved clarification | |
| 6 | | acetone | precipitation after dissolved clarification | salt formation |
| 7 | | 2-butanone | precipitation after dissolved clarification | salt formation |
| 8 | | ethyl acetate | precipitation after dissolved clarification | salt formation |
| 9 | | 1,4-dioxane | precipitation after dissolved clarification | salt formation |
| 10 | | n-butanol | precipitation after dissolved clarification | salt formation |
| 11 | | isopropanol | precipitation after dissolved clarification | salt formation |
| 12 | 1.0 M hydroxyethyl sulfonic acid (methanol solution) | tetrahydrofuran | change from dissolved clarification | salt formation |
| | | | to suspension | |
| 13 | 0.125 M 1,5-naphthalene disulfonic acid (ethanol solution) | acetone | change from dissolved clarification to suspension | salt formation |

### (ii) Salt formation by anti-solvent method

A good solvent was selected, the acid was weighed, the good solvent was added thereto to prepare a stock solution containing the compound in the concentration of 100 mg/mL. An anti-solvent was added thereto, 100 mg of compound was weighed respectively. 1 mL of the good solvent was added, completely dissolved and then filtered. 0.2 mL of filtrate was taken, the anti-solvent was added dropwise thereto respectively (stop adding if there is a precipitate, and adding 1.8 mL of anti-solvent at most), the mixture was stirred for a period of time, and the filtrate was removed by quick centrifugation to obtain the salt of the compound.

| Acid | Good solvent | Anti-solvent | Phenomenon | Result |
|---|---|---|---|---|
| hydroxyethyl sulfonic acid | methanol | H₂O | precipitate out gradually | salt formation |
| | | methyl *tert-*butyl ether | precipitate out gradually | salt formation |
| | | heptane | precipitate out gradually | salt formation |

### 2.1.3 Experimental results:

Through the salt form screening experiment, sulfuric acid, hydroxyethyl sulfonic acid and 1,5-naphthalene disulfonic acid can form salt with the free base of the compound.

As mentioned above, more pharmaceutically acceptable salts can be obtained by those skilled in the art using conventional methods based on the present disclosure.

### 2.2 Quantitative analysis of the hydroxyethyl sulfonate of the compound of embodiment 13-1

### 2.2.1 Quantitative analysis of the hydroxyethyl sulfonate by HPLC

### 2.2.1.1 Experimental purpose:

To determine the number of hydroxyethyl sulfonic acid in the hydroxyethyl sulfonate of the compound of embodiment 13-1

### Experimental steps:

### 1) Chromatographic conditions

| Instrument | HPLC Thermo Ultimate 3000 | |
|---|---|---|
| Mobile phase | A: 25 mM ammonium dihydrogen phosphate aqueous solution, B: methanol | |
| Flow rate | 1.0 mL/min | |
| Injection volume | 10.0 µL | |
| Chromatographic column | Waters XBridge C18 4.6 × 150 mm, 3.5 µm | |
| Column temperature | 35°C | |
| Detection wavelength | 220, 254, 352 nm | |
| Run time | 20 min | |

| Time (min) | A | B |
|---|---|---|
| 0 | 70 | 30 |
| 10 | 15 | 85 |
| 14 | 15 | 85 |
| 15 | 70 | 30 |
| 20 | 70 | 30 |

### 2) Operations

An appropriate amount of the free base of the compound of embodiment 13-1 was weighed, methanol was added thereto to prepare a series of linear solutions with the concentration of 0.05-0.30 mg/mL.

An appropriate amount of the hydroxyethyl sulfonate of the compound of embodiment 13-1 was weighed, methanol was added thereto to prepare a solution containing the hydroxyethyl sulfonate of the compound of embodiment 13-1 with the concentration of 0.25 mg/mL. The above linear solution and a sample solution were taken for injection respectively.

### 2.2.1.3 Experimental results:

| Sample | | Area | mg/mL | | |
|---|---|---|---|---|---|
| STD-1 | | 23.0804 | 0.04808 | | |
| STD-2 | | 45.5656 | 0.09616 | | |
| STD-3 | | 89.1342 | 0.19232 | | |
| STD-4 | | 134.5074 | 0.28848 | | |
| Y= | 0.0022 | X | -0.0018 | | |
| R²= | | 0.9999 | | | |

| Sample | | Area | mg/mL | Average | Cal. |
|---|---|---|---|---|---|
| Sample 1 | | 92.7721 | 0.2022986 | 0.2039 | 81.55% |
| Sample 2 | | 94.1943 | 0.2054275 | | |
| Acid | MW | N (Acid) | Cal. | | |
| 126.13 | 656.60 | 1 | 83.89% | | |

The results of the external standard method show that hydroxyethyl sulfonic acid and free base form a salt in the molar ratio of 1:1.

### 2.2.2 Quantification of the hydroxyethyl sulfonate of the compound of embodiment 13-1 by ELSD

### 2.2.2.1 Experimental purpose:

To determine the number of hydroxyethyl sulfonic acid in the hydroxyethyl sulfonate of the compound of embodiment 13-1

### 2.2.2.2 Experimental steps:

### 1) Chromatographic conditions

| | |
|---|---|
| Dilutent | MeOH |
| Column | ZIC-HILIC (150 * 4.6 mm,5 µm) |
| Mobile phase | 75 mM ammonium acetate solution (pH4.80)/acetonitrile=30:70 |
| Injection volume | 5 µL |
| Flow rate | 1.0 mL/min |
| Column Temperature | 35°C |
| ELSD Temperature | 40°C |

### 2) Operations

An appropriate amount of the hydroxyethyl sulfonic acid was weighed, methanol was added thereto to prepare a series of linear solutions containing the hydroxyethyl sulfonic acid in the concentration of 0.5-1 mg/mL.

An appropriate amount of the hydroxyethyl sulfonate of compound of embodiment 13-1 was weighed, methanol was added to prepare a solution containing the hydroxyethyl sulfonate of compound of embodiment 13-1 with the concentration of 5.0 mg/mL. The above linear solution and a sample solution were taken for injection respectively.

### Experimental results:

| Sample | | Area | mg/mL | | |
|---|---|---|---|---|---|
| STD-1 | | 31.7 | 0.4292 | | |
| STD-2 | | 52.3 | 0.6438 | | |
| STD-3 | | 77.1 | 0.8584 | | |
| Y= | 0.0094 | X | +0.1376 | | |
| R²= | | 0.9972 | | | |

| Sample | | Area | mg/mL | Average | Cal. |
|---|---|---|---|---|---|
| Sample 1 | | 58.7 | 0.6894 | 0.7011 | 14.02% |
| Sample 2 | | 61.2 | 0.7129 | | |
| Acid | MW | N (Acid) | Cal. | | |
| 126.13 | 656.60 | 1 | 16.11% | | |

The number of the hydroxyethyl sulfonic acid in the hydroxyethyl sulfonate of the compound of embodiment 13-1 is calculated to be 1.

### 3. Study on crystal forms of compounds

### 3.1 Study on crystal forms of the compound of embodiment 13-1

### 3.1.1 Experimental purpose:

To screen the salt for crystal form of compound.

### 3.1.2 Experimental steps:

### 1) Instruments and equipment

| Name | Model | Source |
|---|---|---|
| Analytical balance | XA105 | METTLER TOLEDO |
| Ultrasonic cleaning machine | SK5200LHC | Shanghai KUDOS Ultrasonic Instrument Co., Ltd |
| Pipette | Eppendorf (50 mL, 100 µL) | Eppendorf |

### 2) Operation process

### (i) Solventing out in different solvents or suspension to form salt crystal form

10 mg of the compound of embodiment 13-1 was weighed, different reaction solvents were added thereto respectively, and then the final volume of the mixture was 200 µL. The mixture was stirred, added with acid, and stirred for 12 hours. After centrifugation and drying, the XRPD of the mixture was measured.

| No. | Acid | Acid addition amount (µL) | Solvent | Phenomenon after adding acid | XRPD detection result |
|---|---|---|---|---|---|
| 1 | 1.0 hydroxyethyl sulfonic acid (methanol solution) | 18.3 | ethanol | precipitation after dissolved clarification | crystal form |
| 2 | | | acetone | precipitation after dissolved clarification | crystal form |
| 3 | | | 2-butanone | precipitation after dissolved clarification | crystal form |
| 4 | | | ethyl acetate | precipitation after dissolved clarification | crystal form |
| 5 | | | 1,4-dioxane | precipitation after dissolved clarification | crystal form |
| 6 | | | *n*-butanol | precipitation after dissolved clarification | crystal form |
| 7 | | | isopropanol | precipitation after dissolved clarification | crystal form |
| 8 | | | tetrahydrofuran | precipitation after dissolved clarification | crystal form |
| 9 | 1.0 M sulfuric acid (methanol solution) | 18.3 | ethanol | precipitation after dissolved clarification | crystal form |
| 10 | | | ethyl acetate | suspension | crystal form |
| 11 | | | isopropanol | precipitation after dissolved clarification | crystal form |
| 12 | 0.125 M 1,5-naphthalene disulfonic acid (ethanol solution) | 146.4 µL | acetone | change from dissolved clarification to suspension | crystal form |
| 13 | 0.125 M 1,5-naphthalene disulfonic acid (acetone solution) | | acetone | change from dissolved clarification to suspension | crystal form |

### (ii) Salt formation and crystallization by anti-solvent method

The good solvent was selected, the acid was weighed, the good solvent was added thereto to prepare the stock solution containing the compound in the concentration of 100 mg/mL. The anti-solvent was added thereto, 100 mg of compound was weighed respectively, 1 mL of the good solvent was added, completely dissolved and then filtered. 0.2 mL of filtrate was taken, the anti-solvent was added dropwise thereto respectively (stop adding if there is a precipitate, and adding 1.8 mL of anti-solvent at most). The mixture was stirred for a period of time, the filtrate was removed by quick centrifugation, the XRPD of a solid was measured after dying.

| Acid | Good solvent | Anti-solvent | Phenomenon | Result |
|---|---|---|---|---|
| Hydroxyethyl sulfonic acid | Methanol | H₂O | Precipitate out gradually | Crystal form |
| | | Methyl *tert-*butyl ether | Precipitate out gradually | Crystal form |
| | | Heptane | Precipitate out gradually | Crystal form |

### 3.1.3 Experimental results:

Through experiments on the crystal form of the salt of the compound, the resulting salt forms with crystal forms were hydroxyethyl sulfonate, sulfate, and 1,5-naphthalenedisulfonate.

### 3.2 Preparation of crystal forms of the compound of embodiment 13-1

### 3.2.1 Experimental purpose:

To prepare the crystal forms of the compound of embodiment 13-1

### 3.2.2 Experimental steps:

### 1) Instruments and equipment

| Name | Model | Source |
|---|---|---|
| Analytical balance | XA105 | METTLER TOLEDO |
| Ultrasonic cleaning machine | SK5200LHC | Shanghai KUDOS Ultrasonic Instrument Co., Ltd |
| Pipette | Eppendorf (50 mL, 1000 µL) | Eppendorf |

### 2) Operation processes

### I. Preparation of a crystal form I of hydroxyethyl sulfonate

500 mg of the compound of embodiment 13-1 was weighed, 9.08 mL of isopropanol was added, and the mixture was heated to 50°C and stirred. 0.914 mL of hydroxyethyl sulfonic acid (1.0 M in MeOH) was added thereto, precipitated after dissolved clarification, stirred at room temperature for 2 hours. The solid was dried under vacuum at 50°C after filtration to obtain the crystal form I of hydroxyethyl sulfonate, which has an XRPD pattern as shown in FIG. 1, a DSC pattern as shown in FIG. 2, and a TGA pattern as shown in FIG. 3 by detection and analysis.

Alternatively, the compound of embodiment 13-1 (100 g), isopropanol (1200 mL) were added to a 3 L three-necked flask, heated to 40 to 45°C, stirred to dissolved clarification; and the 2- hydroxyethyl sulfonic acid (28.84 g) was dispersed in 800 mL of ethanol, the ethanol solution was added dropwise to the reaction system at a controlled temperature of 39 to 42°C for about 10 minutes. 500 mg of seed crystal was added to the above reaction mixture and a solid was precipitated rapidly. The heating was removed, the reaction mixture was cooled to 25°C and stirred for 12 hours. The reaction mixture was filtered, and the filter cake was washed with 400 mL of isopropanol, drained to dryness and dried under vacuum at 45°C for 16 hours to obtain 92.57 g of a pale yellow solid with a purity of 97.9%, a chiral purity of 92%, and a mass yield of 92%. The pale yellow solid has an XRPD pattern as shown in FIG. 1, a DSC pattern as shown in FIG. 2, and a TGA pattern as shown in FIG. 3 by detection and analysis.

### II. Preparation of a crystal form II of hydroxyethyl sulfonate

10 mg of the compound of embodiment 13-1 was weighed, 0.2 mL of tetrahydrofuran was added, and the mixture was heated to 50°C and stirred. 18.3 µL of hydroxyethyl sulfonic acid (1.0 M in MeOH) was added thereto, precipitated after dissolved clarification, stirred at room temperature for 2 hours. The solid was dried under vacuum at 50°C after filtration to obtain the crystal form II of hydroxyethyl sulfonate, which has an XRPD pattern as shown in FIG. 4, a DSC pattern as shown in FIG. 5, and a TGA pattern as shown in FIG. 6 by detection and analysis.

### III. Preparation of a crystal form III of hydroxyethyl sulfonate

20 mg of the crystal form I of hydroxyethyl sulfonate was weighed, 0.2 mL of methanol and 0.45 mL of methyl tert-butyl ether were added, and the mixture was heated to 50°C and stirred overnight. The solid was dried under vacuum at 50°C after filtration to obtain the crystal form III of hydroxyethyl sulfonate, which has an XRPD pattern as shown in FIG. 7, a DSC pattern as shown in FIG. 8, and a TGA pattern as shown in FIG. 9 by detection and analysis.

### IV. Preparation of a crystal form I of sulfate

10 mg of the compound of embodiment 13-1 was weighed, 0.2 mL of ethanol was added, and the mixture was heated to 50°C and stirred. 18.3 µL of sulfuric acid (1.0 M in MeOH) was added thereto, precipitated after dissolved clarification, stirred at room temperature overnight. The solid was dried under vacuum at 50°C after filtration to obtain the crystal form I of sulfate, which has an XRPD pattern as shown in FIG. 10 by detection and analysis.

### V. Preparation of a crystal form II of sulfate

100 mg of the compound of embodiment 13-1 was weighed, 1.82 mL of isopropanol was added, and the mixture was heated to 50°C and stirred. 183 µL of sulfuric acid (1.0 M in MeOH) was added thereto, precipitated a solid after dissolved clarification, stirred at room temperature overnight. The solid was dried under vacuum at 50°C after filtration to obtain the crystal form II of sulfate, which has an XRPD pattern as shown in FIG. 11 by detection and analysis.

### VI. Preparation of a crystal form III of sulfate

10 mg of the crystal form I of sulfate was weighed, 0.2 mL of isopropanol was added, and the mixture was heated to 50°C and stirred for 5 days. The solid was dried under vacuum at 50°C after filtration to obtain the crystal form III of sulfate, which has an XRPD pattern as shown in FIG. 12 by detection and analysis.

### VII. Preparation of a crystal form IV of sulfate

10 mg of the crystal form I of sulfate was weighed, 0.2 mL of ethyl acetate was added, and the mixture was heated to 50°C and stirred for 5 days. The solid was dried under vacuum at 50°C after filtration to obtain the crystal form III of sulfate, which has an XRPD pattern as shown in FIG. 13 by detection and analysis.

### 4. Solid stability experiment

### 4.1 Solid stability experiment of crystal form I of hydroxyethyl sulfonate of the compound of embodiment 13-1

### 4.1.1 Experimental purpose:

To investigate the physical and chemical stability of crystal form of the compound under high temperature, high humidity, high temperature and high humidity, and light conditions, so as to provide a basis for screening and storage of crystal form.

### 4.1.2 Instrument and liquid chromatographic analysis conditions

| Instrument | HPLC Thermo Ultimate 3000 | |
|---|---|---|
| Mobile phase | A: MeOH, B: H₂O | |
| Flow rate | 1.0 mL/min | |
| Injection volume | 10.0 µL | |
| Chromatographic column | Waters XBridge C18 4.6×150 mm, 3.5 µm | |
| Column temperature | 35°C | |
| Detection wavelength | 220, 254, 352 nm | |
| Run time | 25 min | |

| Time | A | B |
|---|---|---|
| 0 | 40 | 60 |
| 2 | 40 | 60 |
| 12 | 65 | 35 |
| 19 | 65 | 35 |
| 20 | 40 | 60 |
| 25 | 40 | 60 |

### 4.1.3 Experimental scheme

4.1.3.1 An appropriate amount of crystal form I of hydroxyethyl sulfonate of the compound of embodiment 13-1 was weighed and treated under light (≥1.2 × 10⁶ lux-h, 10 days), high humidity (25°C, 75%, 10 days), high humidity (25°C, 90%, 10 days), high temperature (40°C, 30 days), high temperature (60°C, 30 days) and micropowder conditions for a period of time, respectively, then the XRPD of the crystal form I of hydroxyethyl sulfonate was measured.

### 4.1.4.1 Experimental result:

| **No.** | **Condition** | **Crystal form of hydroxyethyl sulfonate** |
|---|---|---|
| - | Initial crystal form | Crystal form I |
| 1 | Light (≥ 1.2 × 10⁶ lux-h, 10 days) | Crystal form I |
| 2 | High humidity (25°C, 75%, 10 days) | Crystal form I |
| 3 | High humidity (25°C, 90%, 10 days) | Crystal form I |
| 4 | High temperature (40°C, 30 days) | Crystal form I |
| 5 | High temperature (60°C, 30 days) | Crystal form I |
| 6 | Micropowder | Crystal form I |

### 4.1.3.2 Experimental scheme

An appropriate amount of crystal form I of hydroxyethyl sulfonate of the compound of embodiment 13-1 was weighed and placed under light (5000 ± 500 lux), high temperature (60°C), high humidity (92.5% RH), and high temperature and high humidity (50°C &75% RH) conditions for 10 days, respectively, and a solution containing free base of the embodiment 13-1 at a concentration of 0.25 mg/mL was prepared by adding diluent methanol, analysed by HPLC, and the change of related substances was calculated according to the peak area normalization method.

### 4.1.4.2 Experimental result:

| Stability of solid (10 days) (impurity increase%) | |
|---|---|
| Condition | Crystal form I of hydroxyethyl sulfonate |
| Light | 0.27 |
| 60°C | 0.26 |
| 92.5% RH | 0.01 |
| 50°C & 75% RH | 0.30 |

The above experimental results show that the crystal form I of hydroxyethyl sulfonate of compound of embodiment 13-1 is relatively stable under light, high humidity, high temperature, and micropowder conditions.

### 4.2 Solid stability experiment of crystal form II of sulfate of the compound of embodiment 13-1

### 4.2.1 Experimental purpose:

To investigate the physical and chemical stability of crystal form of the compound under high temperature, high humidity, high temperature and high humidity, and light conditions, so as to provide a basis for screening and storage of crystal form.

### 4.2.2 Instrument and liquid chromatographic analysis conditions

| Instrument | HPLC Thermo Ultimate 3000 | |
|---|---|---|
| Mobile phase | A: 25 mM ammonium dihydrogen phosphate aqueous solution, B: MeOH | |
| Flow rate | 1.0 mL/min | |
| Injection volume | 10.0 µL | |
| Chromatographic column | Waters Xbridge C18 4.6 × 150 mm, 3.5 µm | |
| Column temperature | 35°C | |
| Detection wavelength | 220, 254, 352 nm | |
| Run time | 20 min | |

| Time (min) | A | B |
|---|---|---|
| 0 | 70 | 30 |
| 10 | 15 | 85 |
| 14 | 15 | 85 |
| 15 | 70 | 30 |
| 20 | 70 | 30 |

### 4.2.3 Experimental scheme

An appropriate amount of crystal form II of sulfate of the compound of embodiment 13-1 was weighed and placed under light (5000 ± 500 lux), high temperature (60°C), high humidity (92.5% RH), and high temperature and high humidity (50°C &75% RH) conditions for 10 days, respectively, and a solution containing free base of the embodiment 13-1 at a concentration of 0.25 mg/mL was prepared by adding diluent methanol, analysed by HPLC, and the change of related substances was calculated according to the peak area normalization method.

### 4.2.4 Experimental result:

| Stability of solid (10 days) (impurity increase %) | |
|---|---|
| Condition | Crystal form II of sulfate |
| Light | 1.12 |
| 60°C | 0.91 |
| 92.5% RH | 0.56 |
| 50°C & 75% RH | 0.15 |

The crystal form II of sulfate is relatively stable under light, high humidity, high temperature and high humidity conditions.

### 5. Solubility experiments in different media

### 5.1 Solubility experiments of the compound of embodiment 13-1 in different media

### 5.1.1 Experimental purpose:

To investigate the solubility of crystal form I of hydroxyethyl sulfonate and crystal form II of sulfate in different pH media, water, artificial simulated gastric fluid (FaSSGF), fasting artificial simulated intestinal fluid (FaSSIF) and non-fasting artificial simulated intestinal fluid (FeSSIF), so as to provide a basis for the assessment of salt druggablitity.

### 5.1.2 Experimental scheme

Approximately 1 mg of different salt forms of the compound was weighed and suspended into 1 mL of artificial simulated gastric fluid (FaSSGF), fasting artificial simulated intestinal fluid (FaSSIF), non-fasting artificial simulated intestinal fluid (FeSSIF), and pure water for 24 hours, respectively, the thermodynamic solubility of the compound at 37°C was measured by HPLC with external standard method.

### 5.1.3 Experimental result: as shown in the following table:

| **Media** | **Solubility (mg/mL)** | |
|---|---|---|
| | **Crystal form I of hydroxyethyl sulfonate** | **Crystal form II of sulfate** |
| FaSSGF | 0.3146 | 0.3865 |
| FaSSIF | 0.1588 | 0.1503 |
| FeSSIF | 0.3473 | 0.3835 |
| H₂O | 0.0074 | 0.0132 |

### 6. Thermodynamic stability experiment

### 6.1 Screening of polycrystal forms of hydroxyethyl sulfonate of the compound of embodiment 13-1

### 6.1.1 Experimental purpose:

To obtain the thermodynamically stable crystal form of hydroxyethyl sulfonate by screening of polycrystal forms.

### 6.1.2 Experimental scheme

10 mg of crystal form I of hydroxyethyl sulfonate was weighed, 200 µL of organic solvent was added respectively, and the mixture was slurried at room temperature and 50°C for 5 days, centrifuged. The supernatant was discarded, and the solid was dried and the XRPD of the solid was measured.

### 6.1.3 Experimental results: as shown in the following table:

| **No.** | **Solvent** | **Hydroxyethyl sulfonate** | |
|---|---|---|---|
| | | **Room temperature** | **50°C** |
| - | Initial crystal form | Crystal form I | |
| 1 | Ethanol | Crystal form I | Crystal form I |
| 2 | Dichloromathane | Crystal form I | Crystal form I |
| 3 | Acetone | Crystal form I | Crystal form I |
| 4 | Ethyl acetate | Crystal form I | Crystal form I |

The above results show that the crystal form I of hydroxyethyl sulfonate is a stable crystal form of hydroxyethyl sulfonate.

### 6.2 Screening experiment of polycrystal forms of sulfate of the compound of embodiment 13-1

### 6.2.1 Experimental purpose:

To obtain the thermodynamically stable crystal form of sulfate by screening of polycrystal forms.

### 6.2.2 Experimental scheme

10 mg of crystal form II of sulfate was weighed, 200 µL of organic solvent was added respectively, the mixture was slurried at 50°C for 5 days, centrifuged. The supernatant was discarded, and the solid was dried, and the XRPD of the solid was measured.

6.2.3 Experimental results: as shown in the following table:

| **No.** | **Solvent** | **Sulfate** |
|---|---|---|
| - | Initial crystal form | Crystal form II |
| 1 | Ethanol | Crystal form II |
| 2 | 2-Methyltetrahydrofuran | Crystal form II |
| 3 | 2-Butanone | Crystal form II |
| 4 | Ethyl acetate | Crystal form II |
| 5 | Toluene | Crystal form II |
| 6 | Isopropyl acetate | Crystal form II |
| 7 | tert-Butanol | Crystal form II |

The above results show that the crystal form II of sulfate is a stable crystal form of sulfate.

## Claims

1. An acid salt of a compound represented by general formula (II), 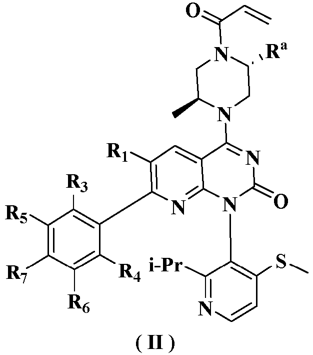 wherein:
R^{a} is selected from hydrogen or methyl;
R₁ is selected from hydrogen, fluorine, chlorine, bromine or methyl;
R₃ is selected from hydrogen, amino, hydroxyl, fluorine, chlorine, methyl, -S(CH₃) or trifluoromethyl;
R₄ is selected from hydrogen, amino, hydroxyl, fluorine, chlorine, -N(CH₃)₂, -NH(CH₃) or fluorine;
R₅ is selected from hydrogen, fluorine, chlorine, bromine, methyl, ethyl, propyl or isopropyl;
R₆ is selected from hydrogen, fluorine, chlorine, bromine, methyl, ethyl, propyl or isopropyl;
R₇ is selected from hydrogen, fluorine, chlorine, bromine or methyl;
acid in the acid salt is selected from hydrochloric acid, sulfuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, ethanesulfonic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, capric acid, caproic acid, octanoic acid, cinnamic acid, citric acid, cyclamic acid, camphor sulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecyl sulfuric acid, dibenzoyl tartaric acid, 1,2-ethanedisulfonic acid, ethanesulfonic acid, formic acid, fumaric acid, galactonic acid, gentisic acid, glutaric acid, 2-ketoglutaric acid, glycolic acid, hippuric acid, hydroxyethyl sulfonic acid, lactobionic acid, ascorbic acid, aspartic acid, lauric acid, camphoric acid, maleate acid, malonic acid, methanesulfonic acid, 1,5-naphthalene disulfonic acid, naphthalene-2-sulfonic acid, nicotinic acid, oleic acid, orotic acid, oxalic acid, palmitic acid, pamoic acid, propionic acid, salicylic acid, 4-aminosalicylic acid, sebacic acid, stearic acid, succinic acid, thiocyanic acid, undecylenic acid, trifluoroacetic acid, benzenesulfonic acid, p-toluenesulfonic acid or L-malic acid; preferably hydrochloric acid, phosphoric acid, ethanesulfonic acid, benzenesulfonic acid, methanesulfonic acid, fumaric acid, hydroxyethyl sulfonic acid, oxalic acid or hydrobromic acid.

2. The acid salt of the compound as claimed in claim 1, wherein, the compound is further represented by general formula (II-A) or (II-B):

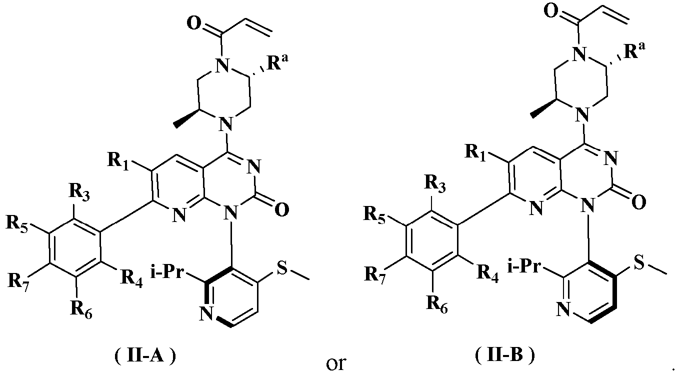

3. The acid salt of the compound as claimed in claim 1 or 2, wherein, the compound is selected from:

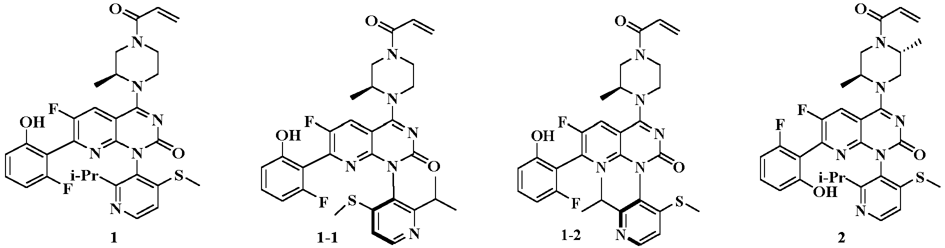

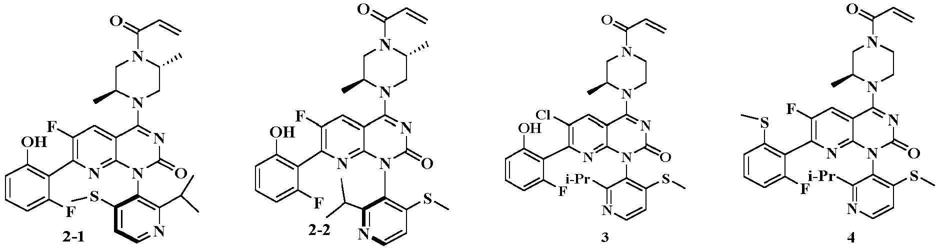

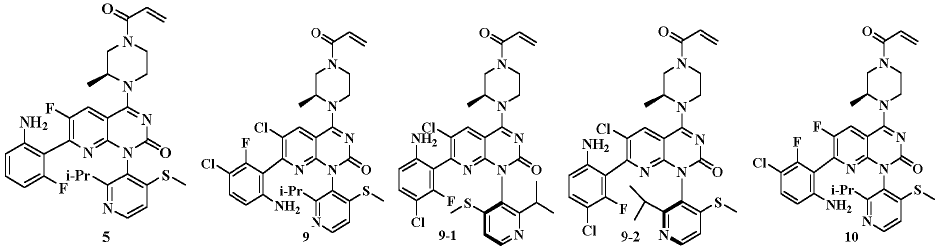

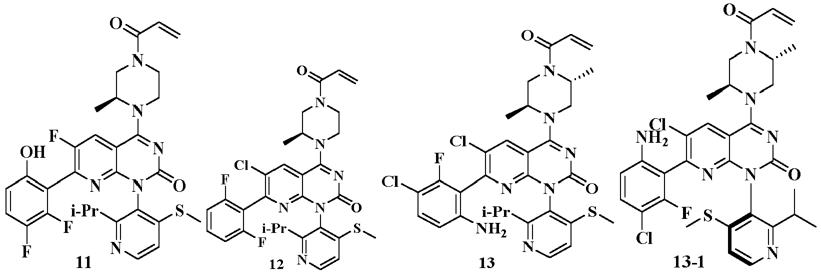

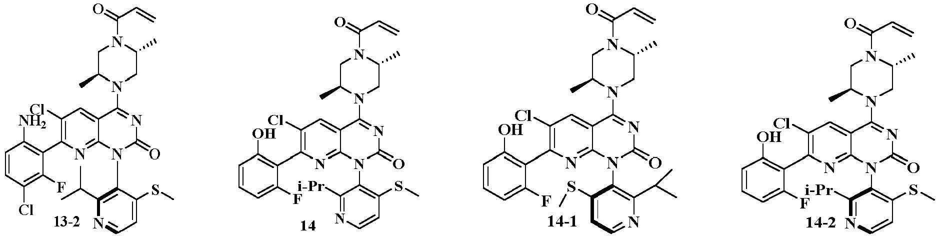

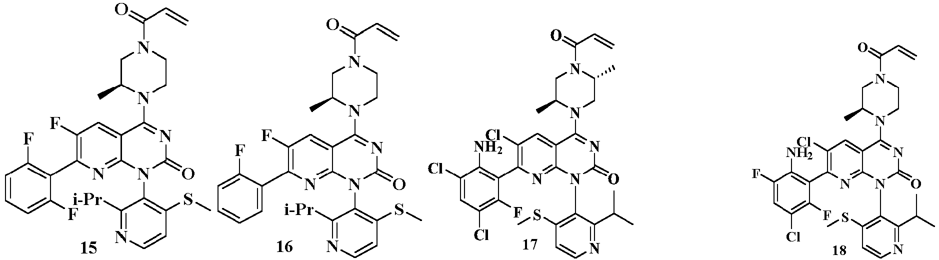

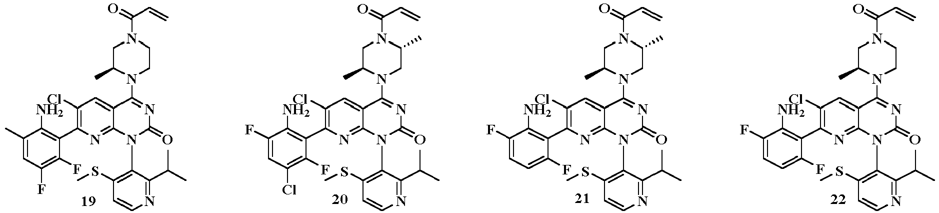

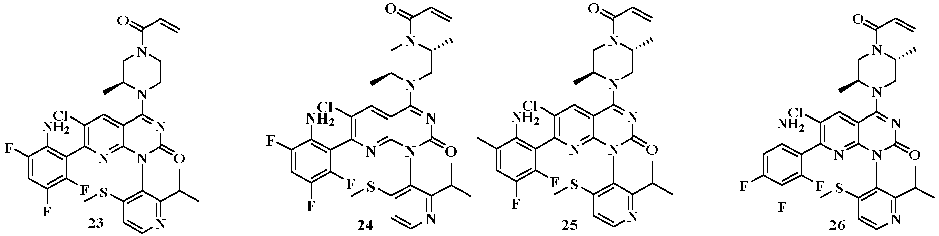

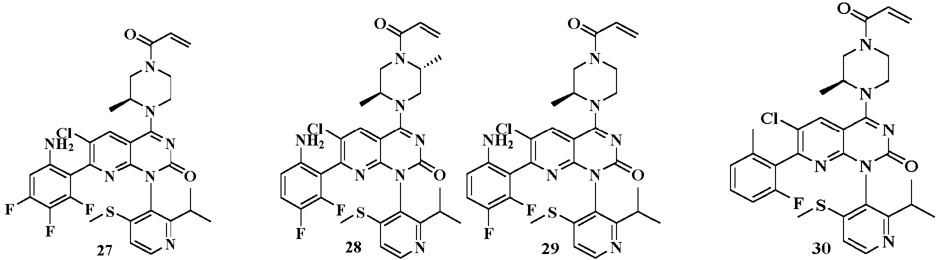

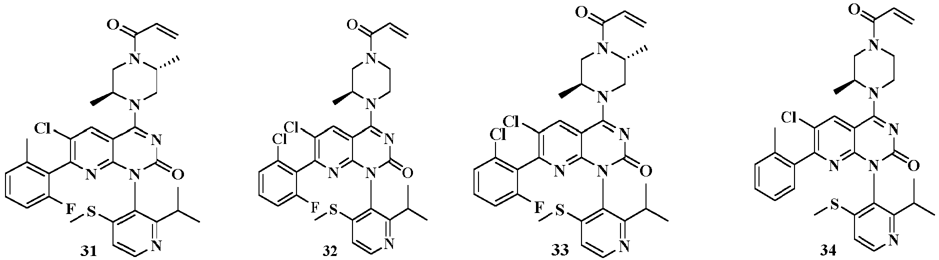

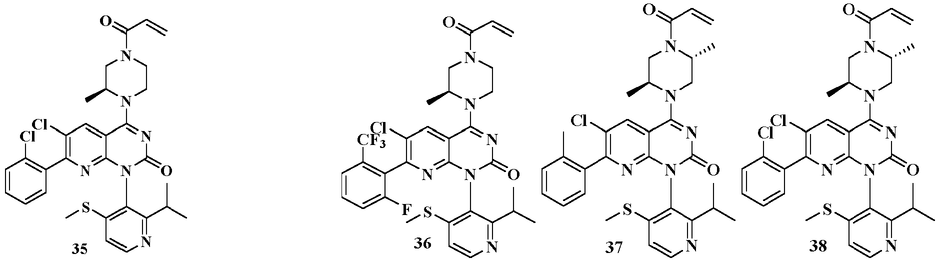

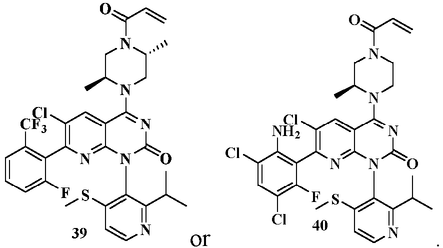

4. The acid salt of the compound as claimed in any one of claims 1 to 3, wherein, the compound is
P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1H)-one;
P-4-((*S*)-4-acryloyl-2-methylpiperazin-1-yl)-6-fluoro-7-(2-fluoro-6-hydroxyphenyl)1-(2-isopropyl-4-(methylthio)pyridin-3-yl)pyrido[2,3-*d*]pyrimidin-2(1*H*)-one;
P-4-((S)-4-acryloyl-2-methylpiperazin-1-yl)-7 -(2-amino-6-fluorophenyl)-6-fluoro-1-(2-isopropyl-4-(methylthio)pyridin-3-yl)pyrido[2,3-*d*]pyrimidin-2(1*H*)-one;
P-4-((2*S*,5*R*)-4-acryloyl-2,5-dimethylpiperazin-1-yl)-6-chloro-7-(2-fluoro-6-hydroxyphenyl)-1-(2-isopropyl-4-(methylthio)pyridin-3-yl)pyrido[2,3-*d*]pyrimidin-2(1*H*)-one;
P-4-((2*S*,5*R*)-4-acryloyl-2,5-dimethylpiperazin-1-yl)-6-fluoro-7-(2-fluoro-6-hydroxyphenyl)-1-(2-isopropyl-4-(methylthio)pyridin-3-yl)pyrido[2,3-d]pyrimidin-2(1H)-one;
acid in the acid salt is selected from hydroxyethyl sulfonic acid, sulfuric acid, 1,5-naphthalene disulfonic acid, methanesulfonic acid, hydrobromic acid, phosphoric acid, benzenesulfonic acid, oxalic acid, maleate acid, adipic acid, hydrochloric acid, citric acid, malonic acid, L-malic acid, pamoic acid, p-toluenesulfonic acid or fumaric acid, preferably hydroxyethyl sulfonic acid or sulfuric acid; wherein, the number of the acid is, for example, 0.2-3; preferably 0.2, 0.5, 1, 1.5, 2, 2.5 or 3; more preferably 0.5, 1, 2 or 3, further preferably 1; and/or, the acid salt is, for example, a hydrate or an anhydrate; and when the acid salt is the hydrate, the number of water is 0.2-3; preferably 0.2, 0.5, 1, 1.5, 2, 2.5 or 3; more preferably 0.5, 1, 2 or 3.

5. The acid salt of the compound as claimed in any one of claims 1 to 4, wherein, the acid salt is in a crystal form;
preferably a crystal form of the acid salt of compound P-4-((2S,5R)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-*d*]pyrimidin-2(1*H*)-one;
a crystal form of the acid salt of P-4-((,S)-4-acryloyl-2-methylpiperazin-1-yl)-6-fluoro-7-(2-fluoro-6-hydroxyphenyl)-1-(2-isopropyl-4-(methylthio)pyridin-3-yl)pyrido[2,3-d]pyrimidin-2(1H)-one;
a crystal form of the acid salt of P-4-((S)-4-acryloyl-2-methylpiperazin-1-yl)-7-(2-amino-6-fluorophenyl)-6-fluoro-1-(2-isopropyl-4-(methylthio)pyridin-3-yl)pyrido[2,3-*d*]pyrimidin-2(1H)-one;
a crystal form of the acid salt of P-4-((2*S*,5*R*)-4-acryloyl-2,5-dimethylpiperazin-1-yl)-6-chloro-7-(2-fluoro-6-hydroxyphenyl)-1-(2-isopropyl-4-(methylthio)pyridin-3-yl)pyrido[2,3-d]pyrimidin-2(1*H*)-one;
a crystal form of the acid salt of P-4-((2*S*,5*R*)-4-acryloyl-2,5-dimethylpiperazin-1-yl)-6-fluoro-7-(2-fluoro-6-hydroxyphenyl)-1-(2-isopropyl-4-(methylthio)pyridin-3-yl)pyrido[2,3-d]pyrimidin-2(1*H*)-one;
more preferably a crystal form of hydroxyethyl sulfonate, a crystal form of sulfate, a crystal form of 1,5-naphthalene disulfonate, a crystal form of methanesulfonate, a crystal form of hydrobromate, a crystal form of phosphate, a crystal form of benzenesulfonate, a crystal form of oxalate, a crystal form of maleate, a crystal form of adipate, a crystal form of hydrochloride, a crystal form of citrate, a crystal form of malonate, a crystal form of L-malate, a crystal form of pamoate, a crystal form of *p*-toluenesulfonate or a crystal form of fumarate.

6. The acid salt of the compound as claimed in claim 5, wherein,
the crystal form of the acid salt of P-4-((2*S*,5*R*)-4-acryloyl-2,5-dimethylpiperazin-1-yl)-7-(6-amino-3-chloro-2-fluorophenyl)-6-chloro-1-(2-isopropyl-4-(methylthio)pyridin-3-yl)pyrido[2,3-d]pyrimidin-2(1*H*)-one is:
a crystal form I of hydroxyethyl sulfonate, the crystal form I of hydroxyethyl sulfonate has an X-ray powder diffraction pattern having a diffraction peak at 2θ angle of 21.7±0.2°; or having a diffraction peak at 8.8±0.2°; or having a diffraction peak at 19.3±0.2°; or having a diffraction peak at 27.6±0.2°; or having a diffraction peak at 10.9±0.2°; or having a diffraction peak at 15.4±0.2°; or having a diffraction peak at 16.7±0.2°; or having a diffraction peak at 15.8±0.2°; or having a diffraction peak at 17.5±0.2°; or having a diffraction peak at 23.8±0.2°; or having a diffraction peak at 10.2±0.2°; or having a diffraction peak at 11.8±0.2°; preferably comprising any 2-5, or 3-5, or 3-6, or 3-8, or 5-8, or 6-8 of the above diffraction peaks, more preferably comprising any 6, 7, or 8 of the diffraction peaks;
a crystal form II of hydroxyethyl sulfonate, the crystal form II of hydroxyethyl sulfonate has an X-ray powder diffraction pattern having a diffraction peak at 2θ angle of 21.7±0.2°; or having a diffraction peak at 8.8±0.2°; or having a diffraction peak at 19.3±0.2°; or having a diffraction peak at 27.6±0.2°; or having a diffraction peak at 10.9±0.2°; or having a diffraction peak at 23.8±0.2°; or having a diffraction peak at 16.7±0.2°; or having a diffraction peak at 15.4±0.2°; or having a diffraction peak at 15.8±0.2°; or having a diffraction peak at 10.0±0.2°; preferably comprising any 2-5, or 3-5, or 3-6, or 3-8, or 5-8, or 6-8 of the above diffraction peaks, more preferably comprising any 6, 7, or 8 of the diffraction peaks;
a crystal form III of hydroxyethyl sulfonate, the crystal form III of hydroxyethyl sulfonate has an X-ray powder diffraction pattern having a diffraction peak at 2θ angle of 19.4±0.2°; or having a diffraction peak at 16.9±0.2°; or having a diffraction peak at 26.6±0.2°; or having a diffraction peak at 14.6±0.2°; or having a diffraction peak at 28.0±0.2°; or having a diffraction peak at 25.6±0.2°; or having a diffraction peak at 20.7±0.2°; or having a diffraction peak at 12.8±0.2°; or having a diffraction peak at 19.1±0.2°; or having a diffraction peak at 27.2±0.2°; preferably comprising any 2-5, or 3-5, or 3-6, or 3-8, or 5-8, or 6-8 of the above diffraction peaks, more preferably comprising any 6, 7, or 8 of the diffraction peaks;
a crystal form I of sulfate, the crystal form I of sulfate has an X-ray powder diffraction pattern having a diffraction peak at 2θ angle of 19.0±0.2°; or having a diffraction peak at 19.4±0.2°; or having a diffraction peak at 12.4±0.2°; or having a diffraction peak at 26.2±0.2°; or having a diffraction peak at 17.6±0.2°; or having a diffraction peak at 18.1±0.2°; or having a diffraction peak at 25.3±0.2°; or having a diffraction peak at 8.8±0.2°; or having a diffraction peak at 21.9±0.2°; or having a diffraction peak at 11.5±0.2°; preferably comprising any 2-5, or 3-5, or 3-6, or 3-8, or 5-8, or 6-8 of the above diffraction peaks, more preferably comprising any 6, 7, or 8 of the diffraction peaks;
a crystal form II of sulfate, the crystal form II of sulfate has an X-ray powder diffraction pattern having a diffraction peak at 2θ angle of 15.5±0.2°; or having a diffraction peak at 11.1±0.2°; or having a diffraction peak at 8.9±0.2°; or having a diffraction peak at 19.3±0.2°; or having a diffraction peak at 22.3±0.2°; or having a diffraction peak at 23.6±0.2°; or having a diffraction peak at 17.4±0.2°; or having a diffraction peak at 27.3±0.2°; or having a diffraction peak at 17.0±0.2°; or having a diffraction peak at 27.9±0.2°; preferably comprising any 2-5, or 3-5, or 3-6, or 3-8, or 5-8, or 6-8 of the above diffraction peaks, more preferably comprising any 6, 7, or 8 of the diffraction peaks;
a crystal form III of sulfate, the crystal form III of sulfate has an X-ray powder diffraction pattern having a diffraction peak at 2θ angle of 19.6±0.2°; or having a diffraction peak at 18.0±0.2°; or having a diffraction peak at 18.4±0.2°; or having a diffraction peak at 16.8±0.2°; or having a diffraction peak at 14.3±0.2°; or having a diffraction peak at 11.8±0.2°; or having a diffraction peak at 14.9±0.2°; or having a diffraction peak at 25.7±0.2°; or having a diffraction peak at 15.4±0.2°; or having a diffraction peak at 23.5±0.2°; preferably comprising any 2-5, or 3-5, or 3-6, or 3-8, or 5-8, or 6-8 of the above diffraction peaks, more preferably comprising any 6, 7, or 8 of the diffraction peaks;
a crystal form IV of sulfate, the crystal form IV of sulfate has an X-ray powder diffraction pattern having a diffraction peak at 2θ angle of 19.4±0.2°; or having a diffraction peak at 18.9±0.2°; or having a diffraction peak at 15.5±0.2°; or having a diffraction peak at 8.8±0.2°; or having a diffraction peak at 18.1±0.2°; or having a diffraction peak at 24.9±0.2°; or having a diffraction peak at 17.4±0.2°; or having a diffraction peak at 12.3±0.2°; or having a diffraction peak at 26.1±0.2°; or having a diffraction peak at 14.5±0.2°; preferably comprising any 2-5, or 3-5, or 3-6, or 3-8, or 5-8, or 6-8 of the above diffraction peaks, more preferably comprising any 6, 7, or 8 of the diffraction peaks.

7. The acid salt of the compound as claimed in claim 6, wherein:
the X-ray powder diffraction pattern of the crystal form I of hydroxyethyl sulfonate comprises at least one or more diffraction peaks at 2θ angles of 21.7±0.2°, 8.8±0.2°, 19.3±0.2°, preferably comprises two of the above diffraction peaks, more preferably comprises three of the diffraction peaks; optionally, further comprises at least one diffraction peak at 2θ angles of 27.6±0.2°, 10.9±0.2°, 15.4±0.2°, 16.7±0.2°, 15.8±0.2°, 10.2±0.2°, 11.8±0.2°, preferably comprises 2, 3, 4 or 5 of the above diffraction peaks; for example,
8.8±0.2°, 27.6±0.2°;
21.7±0.2°, 8.8±0.2°, 10.9±0.2°;
21.7±0.2°, 8.8±0.2°, 27.6±0.2°, 10.9±0.2°;
15.8±0.2°, 8.8±0.2°, 27.6±0.2°, 10.9±0.2°; 21.7±0.2°, 8.8±0.2°, 19.3±0.2°, 15.8±0.2°, 10.9±0.2°, 15.4±0.2°;
10.9±0.2°, 8.8±0.2°, 10.2±0.2°, 27.6±0.2°, 10.9±0.2°, 15.8±0.2°;
the X-ray powder diffraction pattern of the crystal form II of hydroxyethyl sulfonate comprises at least one or more diffraction peaks at 2θ angles of 21.7±0.2°, 10.0±0.2°, 8.8±0.2°, preferably comprises two of the above diffraction peaks, more preferably comprises three of the diffraction peaks; optionally, further comprises at least one diffraction peak at 2θ angles of 19.3±0.2°, 27.6±0.2°, 10.9±0.2°, 23.8±0.2°, 16.7±0.2°, preferably comprises 2, 3, 4 or 5 of the above diffraction peaks; for example,
21.7±0.2°, 10.0±0.2°;
21.7±0.2°, 10.0±0.2°, 19.3±0.2°;
21.7±0.2°, 10.0±0.2°, 8.8±0.2°, 19.3±0.2°;
21.7±0.2°, 10.0±0.2°, 8.8±0.2°, 16.7±0.2°, 27.6±0.2°, 10.9±0.2°;
the X-ray powder diffraction pattern of the crystal form III of hydroxyethyl sulfonate comprises at least one or more diffraction peaks at 2θ angles of 19.4±0.2°, 16.9±0.2°, 26.6±0.2°, preferably comprises two of the above diffraction peaks, more preferably comprises three of the diffraction peaks; optionally, further comprises at least one diffraction peak at 2θ angles of 14.6±0.2°, 28.0±0.2°, 25.6±0.2°, 20.7±0.2°, 12.8±0.2°, preferably comprises 2, 3, 4 or 5 of the above diffraction peaks; for example,
19.4±0.2°, 16.9±0.2°, 26.6±0.2°, 14.6±0.2°, 28.0±0.2°, 25.6±0.2°;
the X-ray powder diffraction pattern of the crystal form I of sulfate comprises at least one or more diffraction peaks at 2θ angles of 19.0±0.2°, 19.4±0.2°, 12.4±0.2°, preferably comprises two of the above diffraction peaks, more preferably comprises three of the diffraction peaks; optionally, further comprises at least one diffraction peak at 2θ angles of 26.2±0.2°, 17.6±0.2°, 18.1±0.2°, 25.3±0.2°, 8.8±0.2°, preferably comprises 2, 3, 4 or 5 of the above diffraction peaks; for example,
19.0±0.2°, 19.4±0.2°, 12.4±0.2°, 26.2±0.2°, 17.6±0.2°, 18.1±0.2°;
the X-ray powder diffraction pattern of the crystal form II of sulfate comprises at least one or more diffraction peaks at 2θ angles of 15.5±0.2°, 11.1±0.2°, 8.9±0.2°, preferably comprises two of the above diffraction peaks, more preferably comprises three of the diffraction peaks; optionally, further comprises at least one diffraction peak at 2θ angles of 19.3±0.2°, 22.3±0.2°, 23.6±0.2°, 17.4±0.2°, 27.3±0.2°, preferably comprises 2, 3, 4 or 5 of the above diffraction peaks; for example,
15.5±0.2°, 11.1±0.2°;
15.5±0.2°, 11.1±0.2°, 8.9±0.2°;
15.5±0.2°, 11.1±0.2°, 8.9±0.2°, 19.3±0.2°;
15.5±0.2°, 11.1±0.2°, 8.9±0.2°, 19.3±0.2°, 22.3±0.2°, 27.3±0.2°;
the X-ray powder diffraction pattern of the crystal form III of sulfate comprises at least one or more diffraction peaks at 2θ angles of 19.6±0.2°, 18.0±0.2°, 18.4±0.2°, preferably comprises two of the above diffraction peaks, more preferably comprises three of the diffraction peaks; optionally, further comprises at least one diffraction peak at 2θ angles of 16.8±0.2°, 14.3±0.2°, 11.8±0.2°, 14.9±0.2°, 25.7±0.2°, preferably comprises 2, 3, 4 or 5 of the above diffraction peaks; for example,
19.6±0.2°, 18.0±0.2°, 18.4±0.2°, 16.8±0.2°, 14.3±0.2°, 11.8±0.2°;
the X-ray powder diffraction pattern of the crystal form IV of sulfate comprises at least one or more diffraction peaks at 2θ angles of 19.4±0.2°, 18.9±0.2°, 15.5±0.2°, preferably comprises two of the above diffraction peaks, more preferably comprises three of the diffraction peaks; optionally, further comprises at least one diffraction peak at 2θ angles of 8.8±0.2°, 18.1±0.2°, 24.9±0.2°, 17.4±0.2°, 12.3±0.2°, preferably comprises 2, 3, 4 or 5 of the above diffraction peaks; for example,
19.4±0.2°, 18.9±0.2°, 15.5±0.2°, 8.8±0.2°, 18.1±0.2°, 24.9±0.2°.

8. The acid salt of the compound as claimed in claims 6 or 7, wherein,
the X-ray powder diffraction pattern of the crystal form I of hydroxyethyl sulfonate optionally also comprises one or more diffraction peaks at 2θ angles of 21.7±0.2°, 8.8±0.2°, 10.2±0.2°, 11.8±0.2°, 13.3±0.2°, 19.3±0.2°, 27.6±0.2°, 10.9±0.2°, 15.4±0.2°, 16.7±0.2°, 15.8±0.2°, 17.5±0.2°, 23.8±0.2°, preferably comprises at least any 2-3, or 4-5, or 6-8 of the above diffraction peaks; further preferably, comprises any 2, 3, 4, 5, 6, 7 or 8 of the diffraction peaks; for example,
8.8±0.2°, 10.2±0.2°, 11.8±0.2°, 13.3±0.2°, 27.6±0.2°, 10.9±0.2°, 15.8±0.2°, 17.5±0.2°;
21.7±0.2°, 8.8±0.2°, 19.3±0.2°, 27.6±0.2°, 10.9±0.2°, 17.5±0.2°, 16.7±0.2°, 15.8±0.2°;
the X-ray powder diffraction pattern of the crystal form II of hydroxyethyl sulfonate optionally also comprises one or more diffraction peaks at 2θ angles of 10.0±0.2°, 21.7±0.2°, 8.8±0.2°, 19.3±0.2°, 27.6±0.2°, 10.9+±0.2°, 23.8±0.2°, 16.7±0.2°, 15.4±0.2°, 15.8±0.2°, 10.0±0.2°; preferably comprises at least any 2-3, or 4-5, or 6-8 of the above diffraction peaks; further preferably, comprises any 2, 3, 4, 5, 6, 7 or 8 of the diffraction peaks; for example,
21.7±0.2°, 8.8±0.2°, 19.3±0.2°, 27.6±0.2°, 10.9+±0.2°, 23.8±0.2°, 16.7±0.2°, 15.4±0.2°;
the X-ray powder diffraction pattern of the crystal form III of hydroxyethyl sulfonate optionally also comprises one or more diffraction peaks at 2θ angles of 19.4±0.2°, 16.9±0.2°, 26.6±0.2°, 14.6±0.2°, 28.0±0.2°, 25.6±0.2°, 20.7±0.2°, 12.8±0.2°, 19.1±0.2°, 27.2±0.2°; preferably comprises at least any 2-3, or 4-5, or 6-8 of the above diffraction peaks; further preferably, comprises any 2, 3, 4, 5, 6, 7 or 8 of the diffraction peaks; for example,
19.4±0.2°, 16.9±0.2°, 26.6±0.2°, 14.6±0.2°, 28.0±0.2°, 25.6±0.2°, 20.7±0.2°, 27.2±0.2°;
the X-ray powder diffraction pattern of the crystal form I of sulfate optionally also comprises one or more diffraction peaks at 2θ angles of 19.0±0.2°, 19.4±0.2°, 12.4±0.2°, 26.2±0.2°, 17.6±0.2°, 18.1±0.2°, 25.3±0.2°, 8.8±0.2°, 21.9±0.2°, 11.5±0.2°; preferably comprises at least any 2-3, or 4-5, or 6-8 of the above diffraction peaks; further preferably, comprises any 2, 3, 4, 5, 6, 7 or 8 of the diffraction peaks; for example,
19.0±0.2°, 19.4±0.2°, 12.4±0.2°, 26.2±0.2°, 25.3±0.2°, 8.8±0.2°, 21.9±0.2°, 11.5±0.2°;
the X-ray powder diffraction pattern of the crystal form II of sulfate optionally also comprises one or more diffraction peaks at 2θ angles of 15.5±0.2°, 11.1±0.2°, 8.9±0.2°, 19.3±0.2°, 22.3±0.2°, 23.6±0.2°, 17.4±0.2°, 27.3±0.2°, 17.0±0.2°, 27.9±0.2°; preferably comprises at least any 2-3, or 4-5, or 6-8 of the above diffraction peaks; further preferably, comprises any 2, 3, 4, 5, 6, 7 or 8 of the diffraction peaks; for example,
15.5±0.2°, 8.9±0.2°, 19.3±0.2°, 22.3±0.2°, 23.6±0.2°, 17.4±0.2°, 27.3±0.2°, 17.0±0.2°;
the X-ray powder diffraction pattern of the crystal form III of sulfate optionally also comprises one or more diffraction peaks at 2θ angles of 19.6±0.2°, 18.0±0.2°, 18.4±0.2°, 16.8±0.2°, 14.3±0.2°, 11.8±0.2°, 14.9±0.2°, 25.7±0.2°, 15.4±0.2°, 23.5±0.2°; preferably comprises at least any 2-3, or 4-5, or 6-8 of the above diffraction peaks; further preferably, comprises any 2, 3, 4, 5, 6, 7 or 8 of the diffraction peaks; for example,
19.6±0.2°, 18.0±0.2°, 18.4±0.2°, 16.8±0.2°, 14.3±0.2°, 11.8±0.2°, 14.9±0.2°, 23.5±0.2°;
the X-ray powder diffraction pattern of the crystal form IV of sulfate optionally also comprises one or more diffraction peaks at 2θ angles of 19.4±0.2°, 18.9±0.2°, 15.5±0.2°, 8.8±0.2°, 18.1±0.2°, 24.9±0.2°, 17.4±0.2°, 12.3±0.2°, 26.1±0.2°, 14.5±0.2°; preferably comprises at least any 2-3, or 4-5, or 6-8 of the above diffraction peaks; further preferably, comprises any 2, 3, 4, 5, 6, 7 or 8 of the diffraction peaks; for example,
19.4±0.2°, 18.9±0.2°, 15.5±0.2°, 8.8±0.2°, 18.1±0.2°, 24.9±0.2°, 17.4±0.2°, 12.3±0.2°.

9. The acid salt of the compound as claimed in claim 6, wherein,
the X-ray powder diffraction pattern of the crystal form I of hydroxyethyl sulfonate comprises one or more diffraction peaks at 2θ angles of 21.7±0.2°, 8.8±0.2°, 10.2±0.2°, 11.8±0.2°, 13.1±0.2°, 19.3±0.2°, 27.6±0.2°, 10.9±0.2°, 13.3±0.2°, 15.4±0.2°, 16.7±0.2°, 15.8±0.2°, 17.5±0.2°, 23.8±0.2°, 14.7±0.2°, 24.3±0.2°, 27.3±0.2°, 23.4±0.2°, 20.6±0.2°, 21.2±0.2°, preferably, comprises any 4, 5, 6, 8 or 10 of the above diffraction peaks; for example,
8.8±0.2°, 19.3±0.2°, 27.6±0.2°, 20.6±0.2°;
8.8±0.2°, 19.3±0.2°, 27.6±0.2°, 10.9±0.2°, 15.4±0.2°, 20.6±0.2°;
21.7±0.2°, 8.8±0.2°, 19.3±0.2°, 27.6±0.2°, 10.9±0.2°, 15.4±0.2°, 16.7±0.2°, 20.6±0.2°;
21.7±0.2°, 8.8±0.2°, 19.3±0.2°, 27.6±0.2°, 10.9±0.2°, 15.4±0.2°, 16.7±0.2°, 15.8±0.2°, 24.3±0.2°, 23.8±0.2°;
8.8±0.2°, 10.2±0.2°, 11.8±0.2°, 13.1±0.2°, 27.6±0.2°, 10.9±0.2°, 13.3±0.2°, 21.2±0.2°, 15.8±0.2°, 17.5±0.2°;
the X-ray powder diffraction pattern of the crystal form II of hydroxyethyl sulfonate comprises one or more diffraction peaks at 2θ angles of 21.7±0.2°, 10.0±0.2°, 8.8±0.2°, 19.3±0.2°, 27.6±0.2°, 10.9±0.2°, 23.8±0.2°, 16.7±0.2°, 15.4±0.2°, 15.8±0.2°, 17.5±0.2°, 14.7±0.2°, 24.4±0.2°, 27.3±0.2°, 29.2±0.2°, preferably, comprises any 4, 5, 6, 8 or 10 of the above diffraction peaks; for example,
10.0±0.2°, 8.8±0.2°, 19.3±0.2°, 29.2±0.2°;
21.7±0.2°, 10.0±0.2°, 8.8±0.2°, 19.3±0.2°, 27.6±0.2°, 29.2±0.2°;
21.7±0.2°, 8.8±0.2°, 19.3±0.2°, 27.6±0.2°, 10.9±0.2°, 23.8±0.2°, 27.3±0.2°, 17.5±0.2°;
10.0±0.2°, 8.8±0.2°, 19.3±0.2°, 27.6±0.2°, 10.9±0.2°, 23.8±0.2°, 16.7±0.2°, 15.4±0.2°, 15.8±0.2°, 17.5±0.2°;
the X-ray powder diffraction pattern of the crystal form III of hydroxyethyl sulfonate comprises one or more diffraction peaks at 2θ angles of 19.4±0.2°, 16.9±0.2°, 26.6±0.2°, 14.6±0.2°, 28.0±0.2°, 25.6±0.2°, 20.7±0.2°, 12.8±0.2°, 19.1±0.2°, 27.2±0.2°, 24.4±0.2°, 15.3±0.2°, 26.2±0.2°, 30.2±0.2°, 27.4±0.2°, preferably, comprises any 4, 5, 6, 8 or 10 of the above diffraction peaks; for example,
19.4±0.2°, 16.9±0.2°, 26.6±0.2°, 14.6±0.2°;
19.4±0.2°, 16.9±0.2°, 26.6±0.2°, 14.6±0.2°, 28.0±0.2°, 27.4±0.2°;
19.4±0.2°, 16.9±0.2°, 26.6±0.2°, 14.6±0.2°, 28.0±0.2°, 25.6±0.2°, 20.7±0.2°, 27.4±0.2°;
19.4±0.2°, 16.9±0.2°, 26.6±0.2°, 14.6±0.2°, 28.0±0.2°, 25.6±0.2°, 20.7±0.2°, 12.8±0.2°, 19.1±0.2°, 27.2±0.2°;
the X-ray powder diffraction pattern of the crystal form I of sulfate comprises one or more diffraction peaks at 2θ angles of 19.0±0.2°, 19.4±0.2°, 12.4±0.2°, 26.2±0.2°, 17.6±0.2°, 18.1±0.2°, 25.3±0.2°, 8.8±0.2°, 21.9±0.2°, 11.5±0.2°, preferably, comprises any 4, 5, 6, 8 or 10 of the above diffraction peaks; for example,
19.0±0.2°, 19.4±0.2°, 12.4±0.2°, 26.2±0.2°;
19.0±0.2°, 19.4±0.2°, 12.4±0.2°, 26.2±0.2°, 17.6±0.2°, 11.5±0.2°;
19.0±0.2°, 19.4±0.2°, 12.4±0.2°, 26.2±0.2°, 17.6±0.2°, 18.1±0.2°, 25.3±0.2°, 8.8±0.2°;
19.0±0.2°, 19.4±0.2°, 12.4±0.2°, 26.2±0.2°, 17.6±0.2°, 18.1±0.2°, 25.3±0.2°, 8.8±0.2°, 21.9±0.2°, 11.5±0.2°;
the X-ray powder diffraction pattern of the crystal form II of sulfate comprises one or more diffraction peaks at 2θ angles of 15.5±0.2°, 11.1±0.2°, 8.9±0.2°, 19.3±0.2°, 22.3±0.2°, 23.6±0.2°, 17.4±0.2°, 27.3±0.2°, 17.0±0.2°, 27.9±0.2°, 15.8±0.2°, 24.2±0.2°, 21.8±0.2°, 10.3±0.2°, 20.6±0.2°, preferably, comprises any 4, 5, 6, 8 or 10 of the above diffraction peaks; for example,
11.1±0.2°, 8.9±0.2°, 19.3±0.2°, 21.8±0.2°;
11.1±0.2°, 8.9±0.2°, 19.3±0.2°, 22.3±0.2°, 20.6±0.2°, 27.9±0.2°;
15.5±0.2°, 11.1±0.2°, 8.9±0.2°, 22.3±0.2°, 23.6±0.2°, 17.4±0.2°, 20.6±0.2°, 27.9±0.2°;
11.1±0.2°, 8.9±0.2°, 19.3±0.2°, 22.3±0.2°, 23.6±0.2°, 17.4±0.2°, 27.3±0.2°, 17.0±0.2°, 27.9±0.2°, 20.6±0.2°;
the X-ray powder diffraction pattern of the crystal form III of sulfate comprises one or more diffraction peaks at 2θ angles of 19.6±0.2°, 18.0±0.2°, 18.4±0.2°, 16.8±0.2°, 14.3±0.2°, 11.8±0.2°, 14.9±0.2°, 25.7±0.2°, 15.4±0.2°, 23.5±0.2°, 18.8±0.2°, 24.7±0.2°, 9.5±0.2°, 8.8±0.2°, 11.1±0.2°, preferably, comprises any 4, 5, 6, 8 or 10 of the above diffraction peaks; for example,
19.6±0.2°, 18.0±0.2°, 18.4±0.2°, 16.8±0.2°;
19.6±0.2°, 18.0±0.2°, 18.4±0.2°, 16.8±0.2°, 14.3±0.2°, 11.1±0.2°;
19.6±0.2°, 18.0±0.2°, 18.4±0.2°, 16.8±0.2°, 14.3±0.2°, 11.8±0.2°, 14.9±0.2°, 11.1±0.2°;
19.6±0.2°, 18.0±0.2°, 18.4±0.2°, 16.8±0.2°, 14.3±0.2°, 11.8±0.2°, 14.9±0.2°, 25.7±0.2°, 15.4±0.2°, 23.5±0.2°;
the X-ray powder diffraction pattern of the crystal form IV of sulfate comprises one or more diffraction peaks at 2θ angles of 19.4±0.2°, 18.9±0.2°, 15.5±0.2°, 8.8±0.2°, 18.1±0.2°, 24.9±0.2°, 17.4±0.2°, 12.3±0.2°, 26.1±0.2°, 14.5±0.2°, 22.2±0.2°, 24.3±0.2°, 21.7±0.2°, 23.6±0.2°, preferably, comprises any 4, 5, 6, 8 or 10 of the above diffraction peaks; for example,
19.4±0.2°, 18.9±0.2°, 15.5±0.2°, 8.8±0.2°;
19.4±0.2°, 18.9±0.2°, 15.5±0.2°, 8.8±0.2°, 18.1±0.2°, 23.6±0.2°;
19.4±0.2°, 18.9±0.2°, 15.5±0.2°, 8.8±0.2°, 18.1±0.2°, 24.9±0.2°, 17.4±0.2°, 23.6±0.2°;
19.4±0.2°, 18.9±0.2°, 15.5±0.2°, 8.8±0.2°, 18.1±0.2°, 24.9±0.2°, 17.4±0.2°, 12.3±0.2°, 26.1±0.2°, 14.5±0.2°.

10. The acid salt of the compound as claimed in any one of claims 6 to 9, wherein,
positions of diffraction peaks with relative peak intensity of top ten in the X-ray powder diffraction pattern of the crystal form I of hydroxyethyl sulfonate and diffraction peaks at corresponding positions in FIG. 1 have a 2θ error of ±0.2° to ±0.5°, preferably ±0.2° to ±0.3°, most preferably ±0.2°;
positions of diffraction peaks with relative peak intensity of top ten in the X-ray powder diffraction pattern of the crystal form II of hydroxyethyl sulfonate and diffraction peaks at corresponding positions in FIG. 4 have a 2θ error of ±0.2° to ±0.5°, preferably ±0.2° to ±0.3°, most preferably ±0.2°;
positions of diffraction peaks with relative peak intensity of top ten in the X-ray powder diffraction pattern of the crystal form III of hydroxyethyl sulfonate and diffraction peaks at corresponding positions in FIG. 7 have a 2θ error of ±0.2° to ±0.5°, preferably ±0.2° to ±0.3°, most preferably ±0.2°;
positions of diffraction peaks with relative peak intensity of top ten in the X-ray powder diffraction pattern of the crystal form I of sulfate and diffraction peaks at corresponding positions in FIG. 10 have a 2θ error of ±0.2° to ±0.5°, preferably ±0.2° to ±0.3°, most preferably ±0.2°;
positions of diffraction peaks with relative peak intensity of top ten in the X-ray powder diffraction pattern of the crystal form II of sulfate and diffraction peaks at corresponding positions in FIG. 11 have a 2θ error of ±0.2° to ±0.5°, preferably ±0.2° to ±0.3°, most preferably ±0.2°;
positions of diffraction peaks with relative peak intensity of top ten in the X-ray powder diffraction pattern of the crystal form III of sulfate and diffraction peaks at corresponding positions in FIG. 12 have a 2θ error of ±0.2° to ±0.5°, preferably ±0.2° to ±0.3°, most preferably ±0.2°;
positions of diffraction peaks with relative peak intensity of top ten in the X-ray powder diffraction pattern of the crystal form IV of sulfate and diffraction peaks at corresponding positions in FIG. 13 have a 2θ error of ±0.2° to ±0.5°, preferably ±0.2° to ±0.3°, most preferably ±0.2°.^{.}

11. The acid salt of the compound as claimed in any one of claims 1 to 10, wherein, the crystal form of the acid salt is a hydrate or an anhydrate, when the crystal form of the acid salt is the hydrate, the number of water is 0.2 to 3, preferably 0.2, 0.5, 1, 1.5, 2, 2.5 or 3, more preferably 0.5, 1, 2 or 3; further, the water in the hydrate is pipeline water, or crystal water, or a combination of both.

12. A method for preparing the acid salt of the compound as claimed in any one of claims 1 to 11, comprising the following steps:
1) weighing an appropriate amount of a free base, and adding a reaction solvent to dissolve;
2) adding an appropriate amount of acid and stirring; wherein an amount of the acid is preferably 1.2 equivalents;
3) centrifuging and drying to obtain the acid salt of the compound or a crystal form thereof;
the reaction solvent is an organic solvent, preferably at least one of ethanol, 2-methyltetrahydrofuran, n-heptane, methyl tert-butyl ether, toluene, isopropyl acetate, *tert-*butanol, *n*-butanol, tetrahydrofuran, acetone, 2-butanone, ethyl acetate or 1,4-dioxane;
the acid is selected from hydrochloric acid, sulfuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, ethanesulfonic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, capric acid, caproic acid, octanoic acid, cinnamic acid, citric acid, cyclamic acid, camphor sulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecyl sulfuric acid, dibenzoyl tartaric acid, 1,2-ethanedisulfonic acid, ethanesulfonic acid, formic acid, fumaric acid, galactonic acid, gentisic acid, glutaric acid, 2-ketoglutaric acid, glycolic acid, hippuric acid, hydroxyethyl sulfonic acid, lactobionic acid, ascorbic acid, aspartic acid, lauric acid, camphoric acid, maleate acid, malonic acid, methanesulfonic acid, 1,5-naphthalene disulfonic acid, naphthalene-2-sulfonic acid, nicotinic acid, oleic acid, orotic acid, oxalic acid, palmitic acid, pamoic acid, propionic acid, salicylic acid, 4-aminosalicylic acid, sebacic acid, stearic acid, succinic acid, thiocyanic acid, undecylenic acid, trifluoroacetic acid, benzenesulfonic acid, p-toluenesulfonic acid or L-malic acid; preferably hydrochloric acid, phosphoric acid, ethanesulfonic acid, benzenesulfonic acid, methanesulfonic acid, fumaric acid, hydroxyethyl sulfonic acid, oxalic acid or hydrobromic acid;or, the method comprises the following steps:
1) weighing an appropriate amount of a free base, and adding a reaction solvent to dissolve;
2) adding an appropriate amount of acid and an organic solvent, stirring and dissolving to clear;
3) adding, optionally, a seed crystal;
4) cooling, filtering to precipitate a solid, and washing with a solvent, drying;
the reaction solvent used in step 1) is an organic solvent, preferably at least one of ethanol, propanol, isopropanol, 2-methyltetrahydrofuran, *n*-heptane, methyl *tert*-butyl ether, toluene, isopropyl acetate, tert-butanol, n-butanol, tetrahydrofuran, acetone, 2-butanone, ethyl acetate or 1,4-dioxane;
the acid in step 2) is selected from hydrochloric acid, sulfuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, ethanesulfonic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, capric acid, caproic acid, octanoic acid, cinnamic acid, citric acid, cyclamic acid, camphor sulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecyl sulfuric acid, dibenzoyl tartaric acid, 1,2-ethanedisulfonic acid, ethanesulfonic acid, formic acid, fumaric acid, galactonic acid, gentisic acid, glutaric acid, 2-ketoglutaric acid, glycolic acid, hippuric acid, hydroxyethyl sulfonic acid, lactobionic acid, ascorbic acid, aspartic acid, lauric acid, camphoric acid, maleate acid, malonic acid, methanesulfonic acid, 1,5-naphthalene disulfonic acid, naphthalene-2-sulfonic acid, nicotinic acid, oleic acid, orotic acid, oxalic acid, palmitic acid, pamoic acid, propionic acid, salicylic acid, 4-aminosalicylic acid, sebacic acid, stearic acid, succinic acid, thiocyanic acid, undecylenic acid, trifluoroacetic acid, benzenesulfonic acid, p-toluenesulfonic acid or L-malic acid; preferably hydrochloric acid, phosphoric acid, ethanesulfonic acid, benzenesulfonic acid, methanesulfonic acid, fumaric acid, hydroxyethyl sulfonic acid, oxalic acid or hydrobromic acid;
the organic solvent in step 2) is selected from one or more of alcohol, ether, ketone or ester solvents, preferably at least one of ethanol, propanol, isopropanol, 2-methyltetrahydrofuran, n-heptane, methyl *tert*-butyl ether, toluene, isopropyl acetate, *tert-*butanol, n-butanol, tetrahydrofuran, acetone, 2-butanone, ethyl acetate or 1,4-dioxane;
the solvent in step 4) is selected from one or more of alcohol, ether, ketone or ester solvents, preferably at least one of ethanol, propanol, isopropanol, 2-methyltetrahydrofuran, n-heptane, methyl *tert*-butyl ether, toluene, isopropyl acetate, tert-butanol, n-butanol, tetrahydrofuran, acetone, 2-butanone, ethyl acetate or 1,4-dioxane.

13. A pharmaceutical composition comprising a therapeutically effective amount of the acid salt of the compound as claimed in any one of claims 1 to 11, and one or more pharmaceutically acceptable carriers, diluents or excipients.

14. The acid salt of the compound as claimed in any one of claims 1 to 11, or the pharmaceutical composition as claimed in claim 13 for use as a medicament for inhibiting KRAS, preferably for use as a medicament for inhibiting KRAS with G12C mutation.

15. The acid salt of the compound as claimed in any one of claims 1 to 11, or the pharmaceutical composition as claimed in claim 13 for use in treating diseases or conditions such as Noonan syndrome, leopard syndrome, leukemia, neuroblastoma, melanoma, esophagus cancer, head and neck tumor, breast cancer, lung cancer and colon cancer; preferably non-small cell lung cancer, colon cancer, esophagus cancer, and head and neck tumor.

## Patentansprüche

1. Saures Salz ("acid salt") einer Verbindung, dargestellt durch die allgemeine Formel (II), 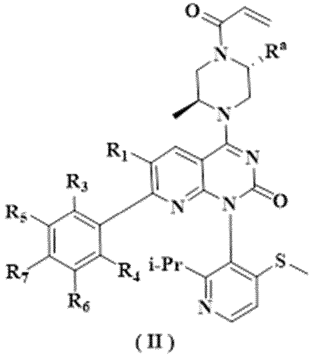 wobei:
R^{a} ausgewählt ist aus Wasserstoff oder Methyl;
R₁ ausgewählt ist aus Wasserstoff, Fluor, Chlor, Brom oder Methyl;
R₃ ausgewählt ist aus Wasserstoff, Amino, Hydroxyl, Fluor, Chlor, Methyl, -S(CH3) oder Trifluormethyl;
R₄ ausgewählt ist aus Wasserstoff, Amino, Hydroxyl, Fluor, Chlor, -N(CH3)2, -NH(CH3) oder Fluor;
R₅ ausgewählt ist aus Wasserstoff, Fluor, Chlor, Brom, Methyl, Ethyl, Propyl oder Isopropyl;
R₆ ausgewählt ist aus Wasserstoff, Fluor, Chlor, Brom, Methyl, Ethyl, Propyl oder Isopropyl;
R₇ ausgewählt ist aus Wasserstoff, Fluor, Chlor, Brom oder Methyl;
Säure im sauren Salz ausgewählt ist aus Salzsäure, Schwefelsäure, Salpetersäure, Bromwasserstoffsäure, Flusssäure, Iodwasserstoffsäure, Phosphorsäure, 2,5-Dihydroxybenzoesäure, 1-Hydroxy-2-naphthoesäure, Essigsäure, Ethansulfonsäure, Dichloressigsäure, Trichloressigsäure, Acetohydroxamsäure, Adipinsäure, Benzolsulfonsäure, 4-Chlorbenzolsulfonsäure, Benzoesäure, 4-Acetylaminobenzoesäure, 4-Aminobenzoesäure, Caprinsäure, Capronsäure, Octansäure, Zimtsäure, Zitronensäure, Cyclaminsäure ("cyclamic acid"), Camphersulfonsäure, Asparaginsäure, Camphersäure, Gluconsäure, Glucuronsäure, Glutaminsäure, Isoascorbinsäure, Milchsäure, Apfelsäure, Mandelsäure, Pyroglutaminsäure, Weinsäure, Dodecylsulfonsäure, Dibenzoylweinsäure, 1,2-Ethandisulfonsäure, Ethansulfonsäure, Ameisensäure, Fumarsäure, Galactonsäure, Gentisinsäure, Glutarsäure, 2-Ketoglutarsäure, Glykolsäure, Hippursäure, Hydroxyethylsulfonsäure, Lactobionsäure, Ascorbinsäure, Asparaginsäure, Laurinsäure, Camphersäure, Maleinsäure, Malonsäure, Methansulfonsäure, 1,5-Naphthalendisulfonsäure, Naphthalin-2-sulfonsäure, Nikotinsäure, Ölsäure, Orotsäure, Oxalsäure, Palmitinsäure, Pamoinsäure, Propionsäure, Salicylsäure, 4-Aminosalicylsäure, Sebacinsäure, Stearinsäure, Bernsteinsäure, Thiocyansäure, Undecylensäure, Trifluoressigsäure, Benzolsulfonsäure, p-Toluolsulfonsäure oder L-Apfelsäure; vorzugsweise Salzsäure, Phosphorsäure, Ethansulfonsäure, Benzolsulfonsäure, Methansulfonsäure, Fumarsäure, Hydroxyethylsulfonsäure, Oxalsäure oder Bromwasserstoffsäure.

2. Saures Salz der Verbindung gemäß Anspruch 1, wobei, die Verbindung ferner durch die allgemeine Formel (II-A) oder (II-B) dargestellt wird:

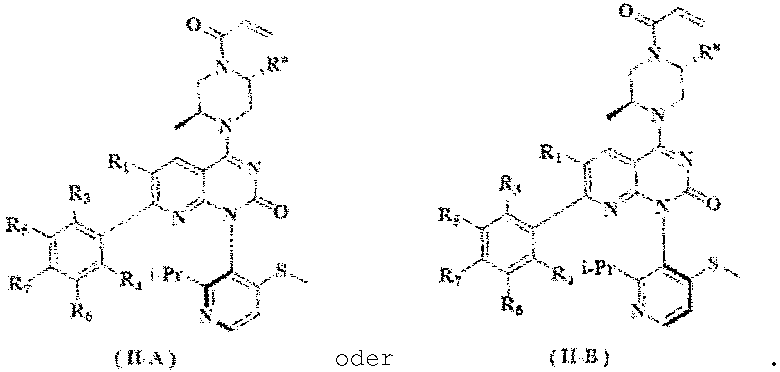

3. Saures Salz der Verbindung gemäß Anspruch 1 oder 2, wobei die Verbindung ausgewählt ist aus:

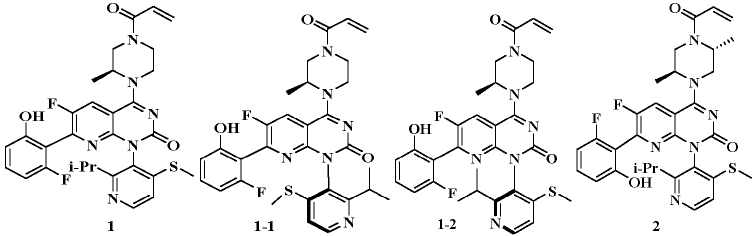

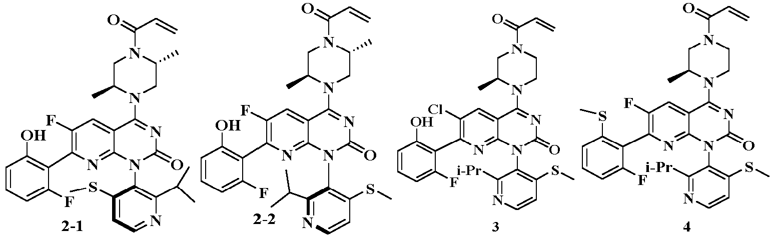

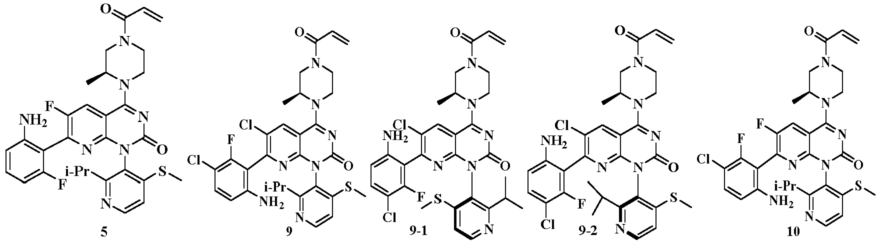

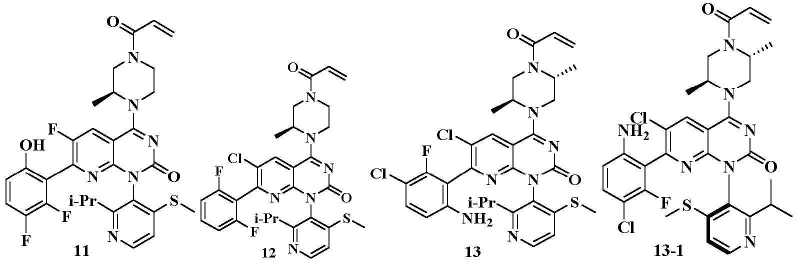

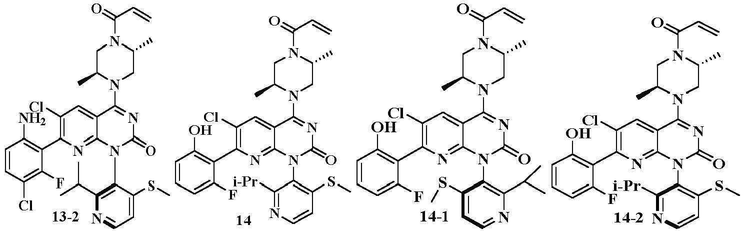

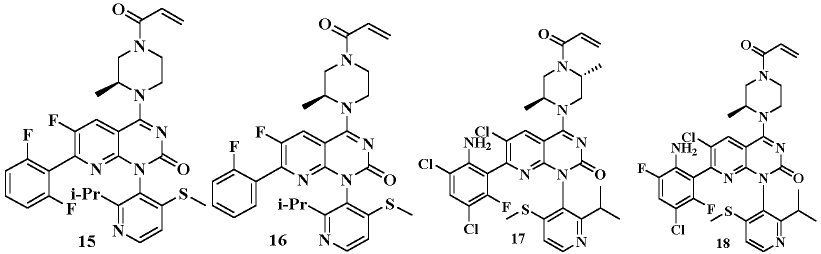

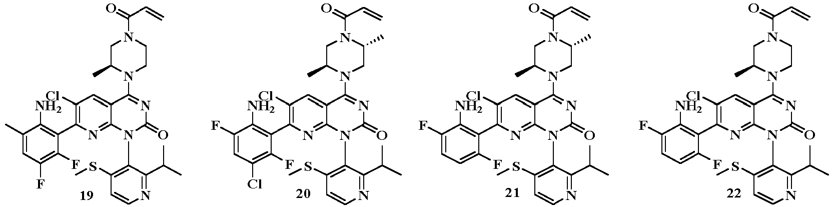

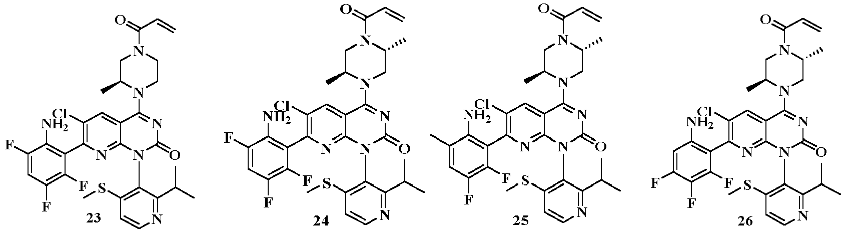

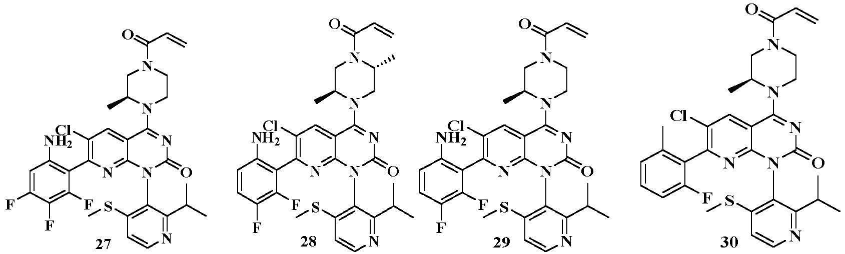

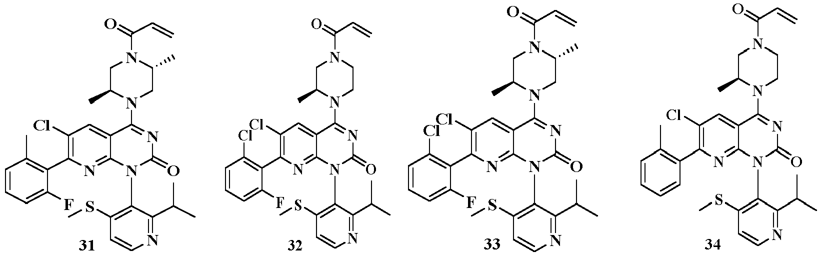

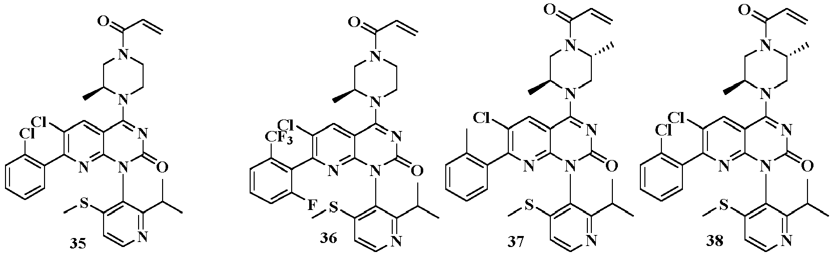

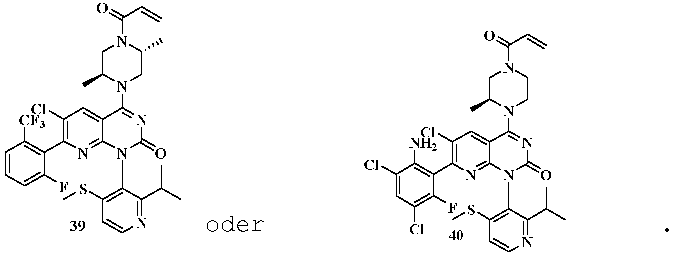

4. Saures Salz der Verbindung gemäß irgendeinem der Ansprüche 1 bis 3, wobei die Verbindung ist
P-4-((2*S*,5*R*)-4-Acryloyl-2,5-dimethylpiperazin-1-yl)-7-(6-amino-3-chlor-2-fluorphenyl)-6-chlor-1-(2-isopropyl-4-(methylthio)pyridin-3-yl)pyrido[2,3-d]pyrimidin-2(1H)-on;
P-4-((*S*)-4-Acryloyl-2-methylpiperazin-1-yl)-6-fluor-7-(2-fluor-6-hydroxyphenyl)-1-(2-isopropyl-4-(methylthio)pyridin-3-yl)pyrido[2,3-d]pyrimidin-2(1H)-on;
P-4-((*S*)-4-Acryloyl-2-methylpiperazin-1-yl)-7-(2-amino-6-fluorphenyl)-6-fluor-1-(2-isopropyl-4-(methylthio)pyridin-3-yl)pyrido[2,3-d]pyrimidin-2(1H)-on;
P-4-((2*S*,5*R*)-4-Acryloyl-2,5-dimethylpiperazin-1-yl)-6-chlor-7-(2-fluor-6-hydroxyphenyl)-1-(2-isopropyl-4-(methylthio)pyridin-3-yl)pyrido[2,3-d]pyrimidin-2(1H)-on;
P-4-((2*S*,5*R*)-4-Acryloyl-2,5-dimethylpiperazin-1-yl)-6-fluor-7-(2-fluor-6-hydroxyphenyl)-1-(2-isopropyl-4-(methylthio)pyridin-3-yl)pyrido[2,3-d]pyrimidin-2(1H)-on;
Säure im sauren Salz ausgewählt ist aus Hydroxyethylsulfonsäure, Schwefelsäure, 1,5-Naphthalindisulfonsäure, Methansulfonsäure, Bromwasserstoffsäure, Phosphorsäure, Benzolsulfonsäure, Oxalsäure, Maleinsäure, Adipinsäure, Salzsäure, Zitronensäure, Malonsäure, L-Apfelsäure, Pamoinsäure, p-Toluolsulfonsäure oder Fumarsäure, vorzugsweise Hydroxyethylsulfonsäure oder Schwefelsäure; wobei die Anzahl der Säuren, zum Beispiel, 0,2-3; vorzugsweise 0,2, 0,5, 1, 1,5, 2, 25 oder 3, noch bevorzugter 0,5, 1, 2 oder 3, weiter bevorzugt 1 ist; und/oder das saure Salz, zum Beispiel, ein Hydrat oder ein Anhydrat ist; und wenn das saure Salz das Hydrat ist, die Anzahl der Wasser 0,2-3; vorzugsweise 0,2, 0,5, 1, 1,5, 2, 2,5 oder 3; noch bevorzugter 0,5, 1,2 oder 3 ist.

5. Saures Salz der Verbindung gemäß irgendeinem der Ansprüche 1 bis 4, wobei das saure Salz in Kristallform ist;
vorzugsweise eine Kristallform des sauren Salzes der Verbindung P-4-((2S,5R)-4-acryloyl-2,5-dimethylpiperazin-1-yl)-7-(6-amino-3-chlor-2-fluorphenyl)-6-chlor-1-(2-isopropyl-4-(methylthio)pyridin-3-yl)pyrido[2,3-d]pyrimidin-2(1H)-on;
eine Kristallform des sauren Salzes von P-4-((S)-4-Acryloyl-2-methylpiperazin-1-yl)-6-fluor-7-(2-fluor-6-hydroxyphenyl)-1-(2-isopropyl-4-(methylthio)pyridin-3-yl)pyrido[2,3-d]pyrimidin-2(1*H*)-on;
eine Kristallform des sauren Salzes von P-4-((S)-4-Acryloyl-2-methylpiperazin-1-yl)-7-(2-Amino-6-fluorphenyl)-6-fluor-1-(2-isopropyl-4-(methylthio)pyridin-3-yl)pyrido[2,3-d]pyrimidin-2(1H)-on;
eine Kristallform des sauren Salzes von P-4-((2S,5R)-4-Acryloyl-2,5-dimethylpiperazin-1-yl)-6-chlor-7-(2-fluor-6-hydroxyphenyl)-1-(2-isopropyl-4-(methylthio)pyridin-3-yl)pyrido[2,3-d]pyrimidin-2(1*H*)-on;
eine Kristallform des sauren Salzes von P-4-((2S,5R)-4-Acryloyl-2,5-dimethylpiperazin-1-yl)-6-fluor-7-(2-fluor-6-hydroxyphenyl)-1-(2-isopropyl-4-(methylthio)pyridin-3-yl)pyrido[2,3-d]pyrimidin-2(1*H*)-on;
noch bevorzugter eine Kristallform von Hydroxyethylsulfonat, eine Kristallform von Sulfat, eine Kristallform von 1,5-Naphthalendisulfonat, eine Kristallform von Methansulfonat, eine Kristallform von Hydrobromat, eine Kristallform von Phosphat, eine Kristallform von Benzolsulfonat, eine Kristallform von Oxalat, eine Kristallform von Maleat, eine Kristallform von Adipat, eine Kristallform von Hydrochlorid, eine Kristallform von Citrat, eine Kristallform von Malonat, eine Kristallform von L-Malat, eine Kristallform von Pamoat, eine Kristallform von p-Toluolsulfonat oder eine Kristallform von Fumarat.

6. Saures Salz der Verbindung gemäß Anspruch 5, wobei die Kristallform des sauren Salzes von P-4-((2S,5R)-4-Acryloyl-2,5-dimethylpiperazin-1-yl)-7-(6-amino-3-chlor-2-fluorphenyl)-6-chlor-1-(2-isopropyl-4-(methylthio)pyridin-3-yl)pyrido[2,3-d]pyrimidin-2(1H)-on:
eine Kristallform I von Hydroxyethylsulfonat ist, die Kristallform I von Hydroxyethylsulfonat hat ein Röntgenpulverdiffraktionsmuster ("X-ray powder diffraction pattern"), das einen Diffraktionspeak ("diffraction peak") bei einem 2θ-Winkel von 21,7 ± 0,2° hat; oder einen Diffraktionspeak bei 8,8 ± 0,2° hat;
oder einen Diffraktionspeak bei 19,3 ± 0,2° hat; oder einen Diffraktionspeak bei 27,6 ± 0,2° hat; oder einen Diffraktionspeak bei 10,9 ± 0,2° hat; oder einen Diffraktionspeak bei 15,4 ± 0,2° hat; oder einen Diffraktionspeak bei 16,7±0,2° hat;
oder einen Diffraktionspeak bei 15,8 ± 0,2° hat; oder einen Diffraktionspeak bei 17,5 ±0,2° hat;
oder einen Diffraktionspeak bei 23,8 ± 0,2° hat; oder einen Diffraktionspeak bei 10,2±0,2° hat; oder einen Diffraktionspeak bei 11,8±0,2° hat; vorzugsweise umfassend irgendwelche 2-5, oder 3-5, oder 3-6, oder 3-8, oder 5-8, oder 6-8 der oben genannten Diffraktionspeaks, noch bevorzugter umfassend irgendwelche 6, 7 oder 8 der Diffraktionspeaks;
eine Kristallform II von Hydroxyethylsulfonat ist, die Kristallform II von Hydroxyethylsulfonat hat ein Röntgenpulverdiffraktionsmuster, das einen Diffraktionspeak bei einem 2θ-Winkel von 21,7 ± 0,2° hat; oder einen Diffraktionspeak bei 8,8 ± 0,2° hat;
oder einen Diffraktionspeak bei 19,3 ± 0,2° hat; oder einen Diffraktionspeak bei 27,6 ± 0,2°hat; oder einen Diffraktionspeak bei 10,9 ± 0,2° hat; oder einen Diffraktionspeak bei 23,8 ± 0,2° hat; oder einen Diffraktionspeak bei 16,7 ± 0,2° hat; oder einen Diffraktionspeak bei 15,4 ± 0,2° hat; oder einen Diffraktionspeak bei 15,8 ± 0,2° hat; oder einen Diffraktionspeak bei 10,0 ± 0,2° hat; vorzugsweise irgendwelche 2-5 oder 3-5 oder 3-6 oder 3-8 oder 5-8 oder 6-8 der oben genannten Diffraktionspeaks umfasst, noch bevorzugter irgendwelche 6, 7 oder 8 der Diffraktionspeaks umfasst;
eine Kristallform III von Hydroxyethylsulfonat ist, die Kristallform III von Hydroxyethylsulfonat hat ein Röntgenpulverdiffraktionsmuster, das einen Diffraktionspeak bei einem 2θ-Winkel von 19,4 ± 0,2° hat; oder einen Diffraktionspeak bei 16,9 ± 0,2° hat;
oder einen Diffraktionspeak bei 26,6 ± 0,2° hat; oder einen Diffraktionspeak bei 14,6 ± 0,2° hat; oder einen Diffraktionspeak bei 28,0 ± 0,2° hat; oder einen Diffraktionspeak bei 25,6 ± 0,2° hat; oder einen Diffraktionspeak bei 20,7 ± 0,2° hat; oder einen Diffraktionspeak bei 12,8 ± 0,2° hat; oder einen Diffraktionspeak bei 19,1 ± 0,2° hat; oder einen Diffraktionspeak bei 27,2 ± 0,2° hat; vorzugsweise irgendwelche 2-5 oder 3-5 oder 3-6 oder 3-8 oder 5-8 oder 6-8 der oben genannten Diffraktionspeaks umfasst, noch bevorzugter irgendwelche 6, 7 oder 8 der Diffraktionspeaks umfasst;
eine Kristallform I von Sulfat ist, die Kristallform I von Sulfat hat ein Röntgenpulverdiffraktionsmuster, das einen Diffraktionspeak bei einem 2θ-Winkel von 19,0 ± 0,2° hat; oder einen Diffraktionspeak bei 19,4 ± 0,2° hat; oder einen Diffraktionspeak bei 12,4 ± 0,2° hat;
oder einen Diffraktionspeak bei 26,2 ± 0,2° hat; oder einen Diffraktionspeak bei 17,6 ± 0,2° hat; oder einen Diffraktionspeak bei 18,1 ± 0,2° hat; oder einen Diffraktionspeak bei 25,3 ± 0,2° hat; oder einen Diffraktionspeak bei 8,8 ± 0,2° hat; oder einen Diffraktionspeak bei 21,9 ± 0,2° hat; oder einen Diffraktionspeak bei 11,5 ± 0,2° hat; vorzugsweise irgendwelche 2-5 oder 3-5 oder 3-6 oder 3-8 oder 5-8 oder 6-8 der oben genannten Diffraktionspeaks umfasst, noch bevorzugter irgendwelche 6, 7 oder 8 der Diffraktionspeaks umfasst;
eine Kristallform II von Sulfat ist, die Kristallform II von Sulfat hat ein Röntgenpulverdiffraktionsmuster, das einen Diffraktionspeak bei einem 2θ-Winkel von 15,5 ± 0,2° hat; oder einen Diffraktionspeak bei 11,1 ± 0,2° hat; oder einen Diffraktionspeak bei 8,9 ± 0,2° hat;
oder einen Diffraktionspeak bei 19,3 ± 0,2° hat; oder einen Diffraktionspeak bei 22,3 ± 0,2° hat; oder einen Diffraktionspeak bei 23,6 ± 0,2° hat; oder einen Diffraktionspeak bei 17,4 ± 0,2° hat; oder einen Diffraktionspeak bei 27,3 ± 0,2° hat; oder einen Diffraktionspeak bei 17,0 ± 0,2° hat; oder einen Diffraktionspeak bei 27,9 ± 0,2° hat; vorzugsweise irgendwelche 2-5 oder 3-5 oder 3-6 oder 3-8 oder 5-8 oder 6-8 der oben genannten Diffraktionspeaks umfasst, noch bevorzugter irgendwelche 6, 7 oder 8 der Diffraktionspeaks umfasst;
eine Kristallform III von Sulfat ist, die Kristallform III von Sulfat hat ein Röntgenpulverdiffraktionsmuster, das einen Diffraktionspeak bei einem 2θ-Winkel von 19,6 ± 0,2° hat; oder einen Diffraktionspeak bei 18,0 ± 0,2° hat; oder einen Diffraktionspeak bei 18,4 ± 0,2° hat;
oder einen Diffraktionspeak bei 16,8 ± 0,2° hat; oder einen Diffraktionspeak bei 14,3 ± 0,2° hat; oder einen Diffraktionspeak bei 11,8 ± 0,2° hat; oder einen Diffraktionspeak bei 14,9 ± 0,2° hat; oder einen Diffraktionspeak bei 25,7 ± 0,2° hat; oder einen Diffraktionspeak bei 15,4±0,2° hat; oder einen Diffraktionspeak bei 23,5±0,2° hat; vorzugsweise irgendwelche 2-5 oder 3-5 oder 3-6 oder 3-8 oder 5-8 oder 6-8 der oben genannten Diffraktionspeaks umfasst, noch bevorzugter irgendwelche 6, 7 oder 8 der Diffraktionspeaks umfasst;
eine Kristallform IV von Sulfat ist, die Kristallform IV von Sulfat hat ein Röntgenpulverdiffraktionsmuster, das einen Diffraktionspeak bei einem 2θ-Winkel von 19,4 ± 0,2° hat; oder einen Diffraktionspeak bei 18,9 ± 0,2° hat; oder einen Diffraktionspeak bei 15,5 ± 0,2° hat;
oder einen Diffraktionspeak bei 8,8 ± 0,2° hat; oder einen Diffraktionspeak bei 18,1 ± 0,2° hat; oder einen Diffraktionspeak bei 24,9 ± 0,2° hat; oder einen Diffraktionspeak bei 17,4 ± 0,2° hat; oder einen Diffraktionspeak bei 12,3 ± 0,2° hat; oder einen Diffraktionspeak bei 26,1±0,2° hat; oder einen Diffraktionspeak bei 14,5±0,2° hat; vorzugsweise irgendwelche 2-5 oder 3-5 oder 3-6 oder 3-8 oder 5-8 oder 6-8 der oben genannten Diffraktionspeaks umfasst, noch bevorzugter irgendwelche 6, 7 oder 8 der Diffraktionspeaks umfasst.

7. Saures Salz der Verbindung gemäß Anspruch 6, wobei:
das Röntgenpulverdiffraktionsmuster der Kristallform I von Hydroxyethylsulfonat mindestens einen oder mehrere Diffraktionspeaks bei 2θ-Winkeln von 21,7 ± 0,2°, 8,8 ± 0,2°, 19,3 ± 0,2° umfasst, vorzugsweise zwei der oben genannten Diffraktionspeaks umfasst, noch bevorzugter drei der Diffraktionspeaks umfasst; optional ferner mindestens einen Diffraktionspeak bei 2θ-Winkeln von 27,6 ± 0,2°, 10,9 ± 0,2°, 15,4 ± 0,2°, 16,7 ± 0,2°, 15,8 ± 0,2°, 10,2 ± 0,2°, 11,8 ± 0,2° umfasst, vorzugsweise 2, 3, 4 oder 5 der oben genannten Diffraktionspeaks umfasst; beispielsweise
8,8±0,2°, 27,6±0,2°;
21,7±0,2°, 8,8±0,2°, 10,9±0,2°;
21,7±0,2°, 8,8±0,2°, 27,6±0,2°, 10,9±0,2°;
15,8±0,2°, 8,8±0,2°, 27,6±0,2°, 10,9±0,2°; 21,7±0,2°, 8,8±0,2°, 19,3±0,2°, 15,8±0,2°, 10,9±0,2°, 15,4±0,2°;
10,9±0,2°, 8,8±0,2°, 10,2±0,2°, 27,6±0,2°, 10,9±0,2°, 15,8±0,2°;
das Röntgenpulverdiffraktionsmuster der Kristallform II von Hydroxyethylsulfonat mindestens einen oder mehrere Diffraktionspeaks bei 2θ-Winkeln von 21,7 ± 0,2°, 10,0 ± 0,2°, 8,8 ± 0,2° umfasst, vorzugsweise zwei der oben genannten Diffraktionspeaks umfasst, noch bevorzugter drei der Diffraktionspeaks umfasst; optional ferner mindestens einen Diffraktionspeak bei 2θ-Winkeln von 19,3 ± 0,2°, 27,6 ± 0,2°, 10,9±0,2°, 23,8±0,2° umfasst, 16,7±0,2°, vorzugsweise 2, 3, 4 oder 5 der oben genannten Diffraktionspeaks umfasst; beispielsweise 21,7±0,2°, 10,0±0,2°;
21,7±0,2°, 10,0±0,2°, 19,3±0,2°;
21,7±0,2°, 10,0±0,2°, 8,8±0,2°, 19,3±0,2°;
21,7±0,2°, 10,0±0,2°, 8,8±0,2°, 16,7±0,2°, 27,6±0,2°, 10,9±0,2°;
das Röntgenpulverdiffraktionsmuster der Kristallform III von Hydroxyethylsulfonat mindestens einen oder mehrere Diffraktionspeaks bei 2θ-Winkeln von 19,4 ± 0,2°, 16,9 ± 0,2°, 26,6 ± 0,2° umfasst, vorzugsweise zwei der oben genannten Diffraktionspeaks umfasst, noch bevorzugter drei der Diffraktionspeaks umfasst; optional ferner mindestens einen Diffraktionspeak bei 2θ-Winkeln von 14,6 ± 0,2°, 28,0 ± 0,2°, 25,6 ± 0,2°, 20,7±0,2°, 12,8±0,2° umfasst, vorzugsweise 2, 3, 4 oder 5 der oben genannten Diffraktionspeaks umfasst; beispielsweise 19,4±0,2°, 16,9±0,2°, 26,6±0,2°, 14,6±0,2°, 28,0±0,2°, 25,6±0,2°;
das Röntgenpulverdiffraktionsmuster der Kristallform I von Sulfat mindestens einen oder mehrere Diffraktionspeaks bei 2θ-Winkeln von 19,0 ± 0,2°, 19,4 ± 0,2°, 12,4 ± 0,2° umfasst, vorzugsweise zwei der oben genannten Diffraktionspeaks umfasst, noch bevorzugter drei der Diffraktionspeaks umfasst; optional ferner mindestens einen Diffraktionspeak bei 2θ-Winkeln von 26,2 ± 0,2°, 17,6 ± 0,2°, 18,1 ± 0,2°, 25,3 ± 0,2°, 8,8 ± 0,2° umfasst, vorzugsweise 2, 3, 4 oder 5 der oben genannten Diffraktionspeak umfasst; beispielsweise 19,0±0,2°, 19,4±0,2°, 12,4±0,2°, 26,2±0,2°, 17,6±0,2°, 18,1±0,2°;
das Röntgenpulverdiffraktionsmuster der Kristallform II von Sulfat mindestens einen oder mehrere Diffraktionspeaks bei 2θ-Winkeln von 15,5 ± 0,2°, 11,1 ± 0,2°, 8,9 ± 0,2° umfasst, vorzugsweise zwei der oben genannten Diffraktionspeaks umfasst, noch bevorzugter drei der Diffraktionspeaks umfasst; optional ferner mindestens einen Diffraktionspeak bei 2θ-Winkeln von 19,3 ± 0,2°, 22,3±0,2°, 23,6±0,2°, 17,4±0,2°, 27,3±0,2° umfasst, vorzugsweise 2, 3, 4 oder 5 der oben genannten Diffraktionspeak umfasst; beispielsweise 15,5±0,2°, 11,1±0,2°;
15,5±0,2°, 11,1±0,2°, 8,9±0,2°;
15,5±0,2°, 11,1±0,2°, 8,9±0,2°, 19,3±0,2°;
15,5±0,2°, 11,1±0,2°, 8,9±0,2°, 19,3±0,2°, 22,3±0,2°, 27,3±0,2°;
das Röntgenpulverdiffraktionsmuster der Kristallform III von Sulfat mindestens einen oder mehrere Diffraktionspeaks bei 2θ-Winkeln von 19,6 ± 0,2°, 18,0 ± 0,2°, 18,4 ± 0,2° umfasst, vorzugsweise zwei der oben genannten Diffraktionspeaks umfasst, noch bevorzugter drei der Diffraktionspeaks umfasst; optional ferner mindestens einen weiteren Diffraktionspeak bei 2θ-Winkeln von 16,8 ± 0,2°, 14,3 ± 0,2°, 11,8 ± 0,2°, 14,9±0,2°, 25,7±0,2 umfasst, vorzugsweise 2, 3, 4 oder 5 der oben genannten Diffraktionspeaks umfasst;
beispielsweise
19,6±0,2°, 18,0±0,2°, 18,4±0,2°, 16,8±0,2°, 14,3±0,2°, 11,8±0,2°;
das Röntgenpulverdiffraktionsmuster der Kristallform IV von Sulfat mindestens einen oder mehrere Diffraktionspeaks bei 2θ-Winkeln von 19,4 ± 0,2°, 18,9 ± 0,2°, 15,5 ± 0,2° umfasst, vorzugsweise zwei der oben genannten Diffraktionspeaks umfasst, noch bevorzugter drei der Diffraktionspeaks umfasst; optional ferner mindestens einen Diffraktionspeak bei 2θ-Winkeln von 8,8 ± 0,2°, 18,1 ± 0,2°, 24,9±0,2°, 17,4±0,2°, 12,3±0,2° umfasst, vorzugsweise 2, 3, 4 oder 5 der oben genannten Diffraktionspeak umfasst; beispielsweise 19,4±0,2°, 18,9±0,2°, 15,5±0,2°, 8,8±0,2°, 18,1±0,2°, 24,910,2°.

8. Saures Salz der Verbindung gemäß Anspruch 6 oder 7, wobei,
das Röntgenpulverdiffraktionsmuster der Kristallform I von Hydroxyethylsulfonat optional auch einen oder mehrere Diffraktionspeaks bei 2θ-Winkeln von 21,7±0,2°, 8,8±0,2°, 10,210,2°, 11,810,2°, 13,3±0,2°, 19,3±0,2°, 27,6±0,2°, 10,9±0,2°, 15,4±0,2°, 16,7±0,2°, 15,8±0,2°, 17,5±0,2°, 23,8±0,2° umfasst, vorzugsweise mindestens irgendwelche 2-3 oder 4-5 oder 6-8 der oben genannten Diffraktionspeaks umfasst; noch bevorzugter irgendwelche 2, 3, 4, 5, 6, 7 oder 8 der Diffraktionspeaks umfasst; beispielsweise
8,8±0,2°, 10,2±0,2°, 11,810,2°, 13,3±0,2°, 27,6±0,2°, 10,9±0,2°, 15,8±0,2°, 17,5±0,2°;
21,7±0,2°, 8,8±0,2°, 19,3±0,2°, 27,6±0,2°, 10,9±0,2°, 17,5±0,2°, 16,7±0,2°, 15,8±0,2°;
das Röntgenpulverdiffraktionsmuster der Kristallform II von Hydroxyethylsulfonat optional auch einen oder mehrere Diffraktionspeaks bei 2θ-Winkeln von 10,0 ± 0,2°, 21,7 ± 0,2°, 8,8 ± 0,2°, 19,3 ± 0,2°, 27,6 ± 0,2°, 10,9+±0,2°, 23,8±0,2°, 16,7±0,2°, 15,4±0,2°, 15,8±0,2°, 100±0,2° umfasst; vorzugsweise mindestens irgendwelche 2-3 oder 4-5 oder 6-8 der oben genannten Diffraktionspeaks umfasst; weiter bevorzugt irgendwelche 2, 3, 4, 5, 6, 7 oder 8 der Diffraktionspeaks umfasst; beispielsweise
21,7±0,2°, 8,8±0,2°, 19,3±0,2°, 27,6±0,2°, 10,9+10,2°, 23,8±0,2°, 16,7±0,2°, 15,4±0,2°;
das Röntgenpulverdiffraktionsmuster der Kristallform III von Hydroxyethylsulfonat optional auch einen oder mehrere Diffraktionspeaks bei 2θ-Winkeln von 19,4 ± 0,2°, 16,9 ± 0,2°, 26,6 ± 0,2°, 14,6 ± 0,2°, 28,0 ± 0,2°, 25,6 ± 0,2°, 20,7 ± 0,2°, 12,8±0,2°, 19,110,2°, 27,2±0,2° umfasst; vorzugsweise mindestens irgendwelche 2-3 oder 4-5 oder 6-8 der oben genannten Diffraktionspeaks umfasst; weiter bevorzugt irgendwelche 2, 3, 4, 5, 6, 7 oder 8 der Diffraktionspeaks umfasst; beispielsweise
19,4±0,2°, 16,9±0,2°, 26,6±0,2°, 14,6±0,2°, 28,0±0,2°, 25,6±0,2°, 20,7±0,2°, 27,2±0,2°;
das Röntgenpulverdiffraktionsmuster der Kristallform I von Sulfat optional auch einen oder mehrere Diffraktionspeaks bei 2θ-Winkeln von 19,0 ± 0,2°, 19,4 ± 0,2°, 12,4 ± 0,2°, 26,2±0,2°, 17,6±0,2°, 18,1±0,2°, 25,3±0,2°, 8,8±0,2°, 21,9±0,2°, 11,5±0,2° umfasst; vorzugsweise mindestens irgendwelche 2-3 oder 4-5 oder 6-8 der oben genannten Diffraktionspeaks umfasst; weiter bevorzugt irgendwelche 2, 3, 4, 5, 6, 7 oder 8 der Diffraktionspeaks umfasst; beispielsweise 19,0±0,2°, 19,4±0,2°, 12,4±0,2°, 26,2±0,2°, 25,3±0,2°, 8,8±0,2°, 21,9±0,2°, 15,4±0,2°;
das Röntgenpulverdiffraktionsmuster der Kristallform II von Sulfat optional auch einen oder mehrere Diffraktionspeaks bei 2θ-Winkeln von 15,5±0,2°, 11,1±0,2°, 8,9±0,2°, 19,3±0,2°, 22,3±0,2°, 23,6±0,2°, 17,4±0,2°, 27,3±0,2°, 17,0±0,2°, 27,9 ± 0,2° umfasst; vorzugsweise mindestens irgendwelche 2-3 oder 4-5 oder 6-8 der oben genannten Diffraktionspeaks umfasst; weiter bevorzugt irgendwelche 2, 3, 4, 5, 6, 7 oder 8 der Diffraktionspeaks umfasst; beispielsweise 15,5±0,2°, 8,9±0,2°, 19,3±0,2°, 22,3±0,2°, 23,6±0,2°, 17,4±0,2°, 27,3±0,2°, 17,0±0,2°;
das Röntgenpulverdiffraktionsmuster der Kristallform III von Sulfat optional auch einen oder mehrere Diffraktionspeaks bei 2θ-Winkeln von 19,6 ± 0,2°, 18,0 ± 0,2°, 18,4 ± 0,2°, 16,8 ± 0,2°, 14,3 ± 0,2°, 11,8 ± 0,2°, 14,9 ± 0,2°, 25,7±0,2°, 15,4±0,2°, 23,5±0,2° umfasst; vorzugsweise mindestens irgendwelche 2-3 oder 4-5 oder 6-8 der oben genannten Diffraktionspeaks umfasst; weiter bevorzugt irgendwelche 2, 3, 4, 5, 6, 7 oder 8 der Diffraktionspeaks umfasst; beispielsweise 19,6 ± 0,2°, 18,0 ± 0,2°, 18,4 ± 0,2°, 16,8 ± 0,2°, 14,3 ± 0,2°, 11,8 ± 0,2°, 14,9 ± 0,2°, 23,5 ± 0,2°;
das Röntgenpulverdiffraktionsmuster der Kristallform IV von Sulfat optional auch einen oder mehrere Diffraktionspeaks bei 2θ-Winkeln von 19,4 ± 0,2°, 18,9 ± 0,2°, 15,5 ± 0,2°, 8,8 ± 0,2°, 18,1 ± 0,2°, 24,9 ± 0,2°, 17,4 ± 0,2°, 12,3 ± 0,2°, 26,1±0,2°, 14,5±0,2° umfasst; vorzugsweise mindestens irgendwelche 2-3 oder 4-5 oder 6-8 der oben genannten Diffraktionspeaks umfasst; weiter bevorzugt irgendwelche 2, 3, 4, 5, 6, 7 oder 8 der Diffraktionspeaks umfasst; beispielsweise 19,4 ± 0,2°, 18,9 ± 0,2°, 15,5 ± 0,2°, 8,8 ± 0,2°, 18,1 ± 0,2°, 24,9 ± 0,2°, 17,4 ± 0,2°, 12,3 ± 0,2°.

9. Saures Salz der Verbindung gemäß Anspruch 6, wobei, das Röntgenpulverdiffraktionsmuster der Kristallform I von Hydroxyethylsulfonat einen oder mehrere Diffraktionspeaks bei 2θ-Winkeln von 21,7 ± 0,2°, 8,8 ± 0,2°, 10,2±0,2°, 11,8±0,2°, 13,1±0,2°, 19,3±0,2°, 27,6±0,2°, 10,9±0,2°, 13,3±0,2°, 15,4±0,2°, 16,7±0,2°, 15,8±0,2°, 17,5±0,2°, 23,8±0,2°, 14,7±0,2°, 24,3±0,2°, 27,3±0,2°, 23,4±0,2°, 20,6±0,2°, 21,2±0,2° umfasst, vorzugsweise irgendwelche 4, 5, 6, 8 oder 10 der oben genannten Diffraktionspeaks umfasst; beispielsweise 8,8±0,2°, 19,3±0,2°, 27,6±0,2°, 20,6 ± 0,2 °C;
8,8 ± 0,2 °C, 19,3 ± 0,2 °C, 27,6 ± 0,2 °C, 10,9 ± 0,2 °C, 15,4 ± 0,2 °C, 20,6 ± 0,2 °C;
21,7 ± 0,2 °C, 8,8 ± 0,2 °C, 19,3±0,2°, 27,6±0,2°, 10,9±0,2°, 15,4±0,2°, 16,7±0,2°, 20,6±0,2°;
21,7±0,2°, 8,8±0,2°, 19,3±0,2°, 27,6±0,2°, 10,9±0,2°, 15,4±0,2°, 16,7±0,2°, 15,8±0,2°, 24,3±0,2°, 23,8±0,2°;
8,8±0,2°, 10,2±0,2°, 11,810,2°, 13,110,2°, 27,6±0,2°, 10,9±0,2°, 13,3±0,2°, 21,2±0,2°, 15,8±0,2°, 17,5±0,2°;
das Röntgenpulverdiffraktionsmuster der Kristallform II von Hydroxyethylsulfonat einen oder mehrere Diffraktionspeaks bei 2θ-Winkeln von 21,7 ± 0,2°, 10,0 ± 0,2°, 8,8 ± 0,2°, 19,3 ± 0,2°, 27,6 ± 0,2°, 10,9 ± 0,2°, 23,8 ± 0,2°, 16,7 ± 0,2°, 15,4 ± 0,2°, 15,8 ± 0,2°, 17,5 ± 0,2°, 14,7 ± 0,2°, 24,4 ± 0,2°, 27,3 ± 0,2°, 29,2±0,2° umfasst, vorzugsweise irgendwelche 4, 5, 6, 8 oder 10 der obigen Diffraktionspeaks umfasst; beispielsweise 10,0±0,2°, 8,8±0,2°, 19,3±0,2°, 29,2±0,2°;
21,7 ± 0,2°, 10,0 ± 0,2°, 8,8 ± 0,2°, 19,3 ± 0,2°, 27,6 ± 0,2°, 29,2 ± 0,2°;
21,7 ± 0,2°, 8,8 ± 0,2°, 19,3 ± 0,2°, 27,6 ± 0,2°, 10,9 ± 0,2°, 23,8 ± 0,2°, 27,3 ± 0,2°, 17,5 ± 0,2°;
10,0 ± 0,2°, 8,8 ± 0,2°, 19,3 ± 0,2°, 27,6 ± 0,2°, 10,9±0,2°, 23,8±0,2°, 16,7±0,2°, 15,4±0,2°, 15,8±0,2°, 17,5±0,2°;
das Röntgenpulverdiffraktionsmuster der Kristallform III von Hydroxyethylsulfonat einen oder mehrere Diffraktionspeaks bei 2θ-Winkeln von 19,4 ± 0,2°, 16,9 ± 0,2°, 26,6 ± 0,2°, 14,6 ± 0,2°, 28,0 ± 0,2°, 25,6 ± 0,2°, 20,7 ± 0,2°, 12,8 ± 0,2°, 19,1 ± 0,2°, 27,2 ± 0,2°, 24,4 ± 0,2°, 15,3 ± 0,2°, 26,2 ± 0,2°, 30,2±0,2°, 27,4±0,2° umfasst, vorzugsweise irgendwelche 4, 5, 6, 8 oder 10 der oben genannten Diffraktionspeaks umfasst;
beispielsweise
19,4±0,2°, 16,9±0,2°, 26,6±0,2°, 14,6±0,2°;
19,4±0,2°, 16,9±0,2°, 26,6±0,2°, 14,6±0,2°, 28,0±0,2°, 27,4±0,2°;
19,4±0,2°, 16,9±0,2°, 26,6±0,2°, 14,6±0,2°, 28,0±0,2°, 25,6±0,2°, 20,7±0,2°, 27,4±0,2°;
19,4±0,2°, 16,9±0,2°, 26,6±0,2°, 14,6±0,2°, 28,0±0,2°, 25,6±0,2°, 20,7±0,2°, 12,8±0,2°, 19,1±0,2°, 27,2±0,2°;
das Röntgenpulverdiffraktionsmuster der Kristallform I von Sulfat einen oder mehrere Diffraktionspeaks bei 2θ-Winkeln von 19,0 ± 0,2°, 19,4 ± 0,2°, 12,4 ± 0,2°, 26,2 ± 0,2°, 17,6 ± 0,2°, 18,1±0,2°, 25,3±0,2°, 8,8±0,2°, 21,9±0,2°, 11,5±0,2° umfasst, vorzugsweise irgendwelche 4, 5, 6, 8 oder 10 der oben genannten Diffraktionspeaks umfasst; beispielsweise
19,0±0,2°, 19,4±0,2°, 12,4±0,2°, 26,2±0,2°;
19,0±0,2°, 19,4±0,2°, 12,4±0,2°, 26,2±0,2°, 17,6±0,2°, 11,5±0,2°;
19,0±0,2°, 19,4±0,2°, 12,4±0,2°, 26,2±0,2°, 17,6±0,2°, 18,1±0,2°, 25,3±0,2°, 8,8±0,2°;
19,0±0,2°, 19,4±0,2°, 12,4±0,2°, 26,2±0,2°, 17,6±0,2°, 18,1±0,2°, 25,3±0,2°, 8,8±0,2°, 21,9±0,2°, 11,5±0,2°;
das Röntgenpulverdiffraktionsmuster der Kristallform II von Sulfat einen oder mehrere Diffraktionspeaks bei 2θ-Winkeln von 15,5 ± 0,2°, 11,1 ± 0,2°, 8,9 ± 0,2°, 19,3 ± 0,2°, 22,3 ± 0,2°, 23,6 ± 0,2°, 17,4 ± 0,2°, 27,3 ± 0,2°, 17,0 ± 0,2°, 27,9 ± 0,2°, 15,8 ± 0,2°, 24,2±0,2°, 21,8±0,2°, 10,3±0,2°, 20,6±0,2° umfasst, vorzugsweise irgendwelche 4, 5, 6, 8 oder 10 der oben genannten Diffraktionspeaks umfasst; beispielsweise 11,1±0,2°, 8,9±0,2°, 19,3±0,2°, 21,8±0,2°;
11,1±0,2°, 8,9±0,2°, 19,3±0,2°, 22,3±0,2°, 20,6±0,2°, 27,9±0,2°;
15,5±0,2°, 11,1±0,2°, 8,9±0,2°, 22,3±0,2°, 23,6±0,2°, 17,4±0,2°, 20,6±0,2°, 27,9±0,2°;
11,1±0,2°, 8,9±0,2°, 19,3±0,2°, 22,3±0,2°, 23,6±0,2°, 17,4±0,2°, 27,3±0,2°, 17,0±0,2°, 27,9±0,2°, 20,6±0,2°;
das Röntgenpulverdiffraktionsmuster der Kristallform III von Sulfat einen oder mehrere Diffraktionspeaks bei 2θ-Winkeln von 19,6 ± 0,2°, 18,0 ± 0,2°, 18,4 ± 0,2°, 16,8 ± 0,2°, 14,3 ± 0,2°, 11,8 ± 0,2°, 14,9 ± 0,2°, 25,7 ± 0,2°, 15,4 ± 0,2°, 23,5 ± 0,2°, 18,8 ± 0,2°, 24,7±0,2°, 9,5±0,2°, 8,8±0,2°, 11,1±0,2°umfasst, vorzugsweise irgendwelche 4, 5, 6, 8 oder 10 der oben genannten Diffraktionspeaks umfasst; beispielsweise 19,6±0,2°, 18,0±0,2°, 18,4±0,2°, 16,8±0,2°;
19,6±0,2°, 18,0±0,2°, 18,4±0,2°, 16,8±0,2°, 14,3±0,2°, 11,1±0,2°;
19,6±0,2°, 18,0±0,2°, 18,4±0,2°, 16,8±0,2°, 14,3±0,2°, 11,8±0,2°, 14,9±0,2°, 11,1±0,2°;
19,6±0,2°, 18,0±0,2°, 18,4±0,2°, 16,8±0,2°, 14,3±0,2°, 11,8±0,2°, 14,9±0,2°, 25,7±0,2°, 15,4±0,2°, 23,5±0,2°;
das Röntgenpulverdiffraktionsmuster der Kristallform IV von Sulfat einen oder mehrere Diffraktionspeaks bei 2θ-Winkeln von 19,4 ± 0,2°, 18,9 ± 0,2°, 15,5 ± 0,2°, 8,8 ± 0,2°, 18,1 ± 0,2°, 24,9 ± 0,2°, 17,4 ± 0,2°, 12,3 ± 0,2°, 26,1 ± 0,2°, 14,5 ± 0,2°, 22,2 ± 0,2°, 24,3±0,2°, 21,7±0,2°, 23,6±0,2° umfasst, vorzugsweise irgendwelche 4, 5, 6, 8 oder 10 der oben genannten Diffraktionspeaks umfasst; zum Beispiel
19,4±0,2°, 18,9±0,2°, 15,5±0,2°, 8,8±0,2°;
19,4±0,2°, 18,9±0,2°, 15,5±0,2°, 8,8±0,2°, 18,1±0,2°, 23,6±0,2°;
19,4±0,2°, 18,9±0,2°, 15,5±0,2°, 8,8±0,2°, 18,1±0,2°, 24,9±0,2°, 17,4±0,2°, 23,6±0,2°;
19,4±0,2°, 18,9±0,2°, 15,5±0,2°, 8,8±0,2°, 18,1±0,2°, 24,9±0,2°, 17,4±0,2°, 12,3±0,2°, 26,1±0,2°, 14,5±0,2°.

10. Saures Salz der Verbindung gemäß irgendeinem der Ansprüche 6 bis 9, wobei,
Positionen der Diffraktionspeaks mit der relativen Peakintensität der zehn höchsten Peaks im Röntgenpulverdiffraktionsmuster der Kristallform I von Hydroxyethylsulfonat und Diffraktionspeaks an den entsprechenden Positionen in FIG. 1 einen 2θ-Fehler von ±0,2° bis ±0,5°, vorzugsweise ±0,2° bis ±0,3°, am meisten bevorzugt ±0,2° haben;
Positionen der Diffraktionspeaks mit der relativen Peakintensität der zehn höchsten Peaks im Röntgenpulverdiffraktionsmuster der Kristallform II von Hydroxyethylsulfonat und Diffraktionspeaks an den entsprechenden Positionen in FIG. 4 einen 2θ-Fehler von ±0,2° bis ±0,5°, vorzugsweise ±0,2° bis ±0,3°, am meisten bevorzugt ±0,2° haben;
Positionen der Diffraktionspeaks mit der relativen Peakintensität der zehn höchsten Peaks im Röntgenpulverdiffraktionsmuster der Kristallform III von Hydroxyethylsulfonat und Diffraktionspeaks an den entsprechenden Positionen in Fig. 7 einen 2θ-Fehler von ±0,2° bis ±0,5°, vorzugsweise ±0,2° bis ±0,3°, am meisten bevorzugt ±0,2° haben;
Positionen der Diffraktionspeaks mit der relativen Peakintensität der zehn höchsten Peaks im Röntgenpulverdiffraktionsmuster der Kristallform I von Sulfat und Diffraktionspeaks an den entsprechenden Positionen in Fig. 10 einen 2θ-Fehler von ±0,2° bis ±0,5°, vorzugsweise ±0,2° bis ±0,3°, am meisten bevorzugt ±0,2° haben;
Positionen der Beugungspeaks mit der relativen Peakintensität der zehn höchsten Peaks im Röntgenpulverdiffraktionsmuster der Kristallform II von Sulfat und Diffraktionspeaks an den entsprechenden Positionen in FIG. 11 einen 2θ-Fehler von ±0,2° bis ±0,5°, vorzugsweise ±0,2° bis ±0,3°, am meisten bevorzugt ±0,2° haben;
Positionen der Diffraktionspeaks mit der relativen Peakintensität der zehn höchsten Peaks im Röntgenpulverdiffraktionsmuster der Kristallform III von Sulfat und Diffraktionspeaks an den entsprechenden Positionen in FIG. 12 einen 2θ-Fehler von ±0,2° bis ±0,5°, vorzugsweise ±0,2° bis ±0,3°, am meisten bevorzugt ±0,2° haben;
Positionen der Beugungspeaks mit relativer Peakintensität der zehn höchsten Peaks im Röntgenpulverdiffraktionsmuster der Kristallform IV von Sulfat und Diffraktionspeaks an den entsprechenden Positionen in FIG. 13 einen 2θ-Fehler von ±0,2° bis ±0,5°, vorzugsweise ±0,2° bis ±0,3°, am meisten bevorzugt ±0,2° haben.

11. Saures Salz der Verbindung gemäß irgendeinem der Ansprüche 1 bis 10, wobei die Kristallform des sauren Salzes ein Hydrat oder ein Anhydrat ist, wenn die Kristallform des sauren Salzes das Hydrat ist, die Anzahl der Wasser 0,2 bis 3, vorzugsweise 0,2, 0,5, 1, 1,5, 2, 2.5 oder 3, noch bevorzugter 0,5, 1, 2 oder 3, ist; ferner das Wasser im Hydrat Leitungswasser oder Kristallwasser oder eine Kombination aus beiden ist.

12. Verfahren zum Herstellen des sauren Salzes der Verbindung gemäß irgendeinem der Ansprüche 1 bis 11, umfassend die folgenden Schritte:
1) Abwiegen einer geeigneten Menge einer freien Base und Zugeben eines Reaktionslösungsmittels zum Auflösen;
2) Zugeben einer geeigneten Menge einer Säure und Rühren; wobei die Menge der Säure vorzugsweise 1,2 Äquivalente ist;
3) Zentrifugieren und Trocknen, um das saure Salz der Verbindung oder eine Kristallform davon zu erhalten;
das Reaktionslösungsmittel ist ein organisches Lösungsmittel, vorzugsweise mindestens eines aus Ethanol, 2-Methyltetrahydrofuran, n-Heptan, Methyl-tert-butylether, Toluol, Isopropylacetat, tert-Butanol, n-Butanol, Tetrahydrofuran, Aceton, 2-Butanon, Ethylacetat oder 1,4-Dioxan;
die Säure ist ausgewählt aus Salzsäure, Schwefelsäure, Salpetersäure, Bromwasserstoffsäure, Flusssäure, Iodwasserstoffsäure, Phosphorsäure, 2,5-Dihydroxybenzoesäure, 1-Hydroxy-2-naphthoesäure, Essigsäure, Ethansulfonsäure, Dichloressigsäure, Trichloressigsäure, Acetohydroxamsäure, Adipinsäure, Benzolsulfonsäure, 4-Chlorbenzolsulfonsäure, Benzoesäure, 4-Acetylaminobenzoesäure, 4-Aminobenzoesäure, Caprinsäure, Capronsäure, Octansäure, Zimtsäure, Zitronensäure, Cyclaminsäure, Kampfersulfonsäure, Asparaginsäure, Camphorsäure, Gluconsäure, Glucuronsäure, Glutaminsäure, Isoascorbinsäure, Milchsäure, Apfelsäure, Mandelsäure, Pyroglutaminsäure, Weinsäure, Dodecylsulfonsäure, Dibenzoylweinsäure, 1,2-Ethandisulfonsäure, Ethansulfonsäure, Ameisensäure, Fumarsäure, Galactonsäure, Gentisinsäure, Glutarsäure, 2-Ketoglutarsäure, Glykolsäure, Hippursäure, Hydroxyethylsulfonsäure, Lactobionsäure, Ascorbinsäure, Asparaginsäure, Laurinsäure, Camphersäure, Maleinsäure, Malonsäure, Methansulfonsäure, 1,5-Naphthalendisulfonsäure, Naphthalin-2-sulfonsäure, Nikotinsäure, Ölsäure, Orotsäure, Oxalsäure, Palmitinsäure, Pamoinsäure, Propionsäure, Salicylsäure, 4-Aminosalicylsäure, Sebacinsäure, Stearinsäure, Bernsteinsäure, Thiocyansäure, Undecylensäure, Trifluoressigsäure, Benzolsulfonsäure, p-Toluolsulfonsäure oder L-Apfelsäure; vorzugsweise Salzsäure, Phosphorsäure, Ethansulfonsäure, Benzolsulfonsäure, Methansulfonsäure, Fumarsäure, Hydroxyethylsulfonsäure, Oxalsäure oder Bromwasserstoffsäure; oder das Verfahren umfasst die folgenden Schritte:
1) Abwiegen einer geeigneten Menge einer freien Base und Zugeben eines Reaktionslösungsmittels zum Auflösen;
2) Zugeben einer geeigneten Menge an Säure und einem organischen Lösungsmittel, Rühren und Auflösen bis zur Klärung;
3) optional Zugeben eines Impfkristalls;
4) Abkühlen, Filtrieren zum Ausfällen eines Feststoffs und Waschen mit einem Lösungsmittel, Trocknen;
das in Schritt 1) verwendete Reaktionslösungsmittel ist ein organisches Lösungsmittel, vorzugsweise mindestens eines aus Ethanol, Propanol, Isopropanol, 2-Methyltetrahydrofuran, n-Heptan, Methyl-tert-butylether, Toluol, Isopropylacetat, tert-Butanol, n-Butanol, Tetrahydrofuran, Aceton, 2-Butanon, Ethylacetat oder 1,4-Dioxan;
die Säure in Schritt 2) ist ausgewählt aus Salzsäure, Schwefelsäure, Salpetersäure, Bromwasserstoffsäure, Flusssäure, Iodwasserstoffsäure, Phosphorsäure, 2,5-Dihydroxybenzoesäure, 1-Hydroxy-2-naphthoesäure, Essigsäure, Ethansulfonsäure, Dichloressigsäure, Trichloressigsäure, Acetohydroxamsäure, Adipinsäure, Benzolsulfonsäure, 4-Chlorbenzolsulfonsäure, Benzoesäure, 4-Acetylaminobenzoesäure, 4-Aminobenzoesäure, Caprinsäure, Capronsäure, Octansäure, Zimtsäure, Zitronensäure, Cyclaminsäure, Kampfersulfonsäure, Asparaginsäure, Camphorsäure, Gluconsäure, Glucuronsäure, Glutaminsäure, Isoascorbinsäure, Milchsäure, Apfelsäure, Mandelsäure, Pyroglutaminsäure, Weinsäure, Dodecylsulfonsäure, Dibenzoylweinsäure, 1,2-Ethandisulfonsäure, Ethansulfonsäure, Ameisensäure, Fumarsäure, Galactonsäure, Gentisinsäure, Glutarsäure, 2-Ketoglutarsäure, Glykolsäure, Hippursäure, Hydroxyethylsulfonsäure, Lactobionsäure, Ascorbinsäure, Asparaginsäure, Laurinsäure, Kampfer, Maleinsäure, Malonsäure, Methansulfonsäure, 1,5-Naphthalendisulfonsäure, Naphthalin-2-sulfonsäure, Nikotinsäure, Ölsäure, Orotsäure, Oxalsäure, Palmitinsäure, Pamoinsäure, Propionsäure, Salicylsäure, 4-Aminosalicylsäure, Sebacinsäure, Stearinsäure, Bernsteinsäure, Thiocyansäure, Undecylensäure, Trifluoressigsäure, Benzolsulfonsäure, p-Toluolsulfonsäure oder L-Apfelsäure; vorzugsweise Salzsäure, Phosphorsäure, Ethansulfonsäure, Benzolsulfonsäure, Methansulfonsäure, Fumarsäure, Hydroxyethylsulfonsäure, Oxalsäure oder Bromwasserstoffsäure;
das organische Lösungsmittel in Schritt 2) ist ausgewählt aus einem oder mehreren aus Alkohol-, Ether-, Keton- oder Esterlösungsmitteln, vorzugsweise mindestens einem aus Ethanol, Propanol, Isopropanol, 2-Methyltetrahydrofuran, n-Heptan, Methyl-tert-butylether, Toluol, Isopropylacetat, tert-Butanol, n-Butanol, Tetrahydrofuran, Aceton, 2-Butanon, Ethylacetat oder 1,4-Dioxan;
das Lösungsmittel in Schritt 4) ist ausgewählt aus einem oder mehreren aus Alkohol-, Ether-, Keton- oder Esterlösungsmitteln, vorzugsweise mindestens einem aus Ethanol, Propanol, Isopropanol, 2-Methyltetrahydrofuran, n-Heptan, Methyl-tert-butylether, Toluol, Isopropylacetat, tert-Butanol, n-Butanol, Tetrahydrofuran, Aceton, 2-Butanon, Ethylacetat oder 1,4-Dioxan.

13. Pharmazeutische Zusammensetzung, umfassend eine therapeutisch wirksame Menge des sauren Salzes der Verbindung gemäß irgendeinem der Ansprüche 1 bis 11 und einen oder mehrere pharmazeutisch verträgliche Träger, Verdünnungsmittel oder Hilfsstoffe.

14. Saures Salz der Verbindung gemäß irgendeinem der Ansprüche 1 bis 11 oder die pharmazeutische Zusammensetzung gemäß Anspruch 13 zur Verwendung als Medikament zur Inhibierung von KRAS, vorzugsweise zur Verwendung als Medikament zur Inhibierung von KRAS mit G12C-Mutation.

15. Saures Salz der Verbindung gemäß irgendeinem der Ansprüche 1 bis 11 oder die pharmazeutische Zusammensetzung gemäß Anspruch 13 zur Verwendung bei der Behandlung von Krankheiten oder Zuständen wie Noonan-Syndrom, Leopard-Syndrom, Leukämie, Neuroblastom, Melanom, Speiseröhrenkrebs, Kopf- und Halstumor, Brustkrebs, Lungenkrebs und Darmkrebs, vorzugsweise nicht-kleinzelligem Lungenkrebs, Darmkrebs, Speiseröhrenkrebs und Kopf- und Halstumor.

## Revendications

1. Sel acide d'un composé représenté par la formule générale (II), 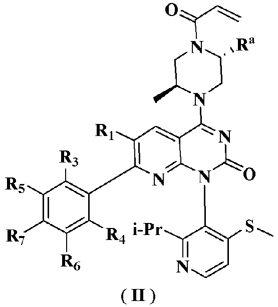 dans lequel :
R^{a} est choisi parmi hydrogène ou méthyle ;
R₁ est choisi parmi hydrogène, fluor, chlore, brome ou méthyle ;
R₃ est choisi parmi hydrogène, amino, hydroxyle, fluor, chlore, méthyle, -S(CH₃) ou trifluorométhyle ;
R₄ est choisi parmi hydrogène, amino, hydroxyle, fluor, chlore, -N(CH₃)_{2,} - NH(CH₃) ou fluor ;
R₅ est choisi parmi hydrogène, fluor, chlore, brome, méthyle, éthyle, propyle ou isopropyle ;
R₆ est choisi parmi hydrogène, fluor, chlore, brome, méthyle, éthyle, propyle ou isopropyle ;
R₇ est choisi parmi hydrogène, fluor, chlore, brome ou méthyle ;
l'acide dans le sel acide est choisi parmi l'acide chlorhydrique, l'acide sulfurique, l'acide nitrique, l'acide bromhydrique, l'acide fluorhydrique, l'acide iodhydrique, l'acide phosphorique, l'acide 2,5-dihydroxybenzoïque, l'acide 1-hydroxy-2-naphthoïque, l'acide acétique, l'acide éthanesulfonique, l'acide dichloroacétique, l'acide trichloroacétique, l'acide acétohydroxamique, l'acide adipique, l'acide benzènesulfonique, l'acide 4-chlorobenzènesulfonique, l'acide benzoïque, l'acide 4-acétylaminobenzoïque, l'acide 4-aminobenzoïque, l'acide caprique, l'acide caproïque, l'acide octanoïque, l'acide cinnamique, l'acide citrique, l'acide cyclamique, l'acide sulfonique de camphre, l'acide aspartique, l'acide camphorique, l'acide gluconique, l'acide glucuronique, l'acide glutamique, l'acide isoascorbique, l'acide lactique, l'acide malique, l'acide mandélique, l'acide pyroglutamique, l'acide tartrique, l'acide dodécyl sulfurique, l'acide dibenzoyl tartrique, l'acide 1,2-éthanedisulfonique, l'acide éthanesulfonique, l'acide formique, l'acide fumarique, l'acide galactonique, l'acide gentisique, l'acide glutarique, l'acide 2-cétoglutarique, l'acide glycolique, l'acide hippurique, l'acide hydroxyéthylsulfonique, l'acide lactobionique, l'acide ascorbique, l'acide aspartique, l'acide laurique, l'acide camphorique, l'acide maléate, l'acide malonique, l'acide méthanesulfonique, l'acide 1,5-naphtalène disulfonique, l'acide naphtalène-2-sulfonique, l'acide nicotinique, l'acide oléique, l'acide orotique, l'acide oxalique, l'acide palmitique, l'acide pamoïque, l'acide propionique, l'acide salicylique, l'acide 4-aminosalicylique, l'acide sébacique, l'acide stéarique, l'acide succinique, l'acide thiocyanique, l'acide undécylénique, l'acide trifluoroacétique, l'acide benzènesulfonique, l'acide p-toluènesulfonique ou l'acide L-malique ; de préférence l'acide chlorhydrique, l'acide phosphorique, l'acide éthanesulfonique, l'acide benzènesulfonique, l'acide méthanesulfonique, l'acide fumarique, l'acide hydroxyéthylsulfonique, l'acide oxalique ou l'acide bromhydrique.

2. Sel acide du composé selon la revendication 1, dans lequel le composé est représenté en outre par la formule générale (II-A) ou (II-B) :

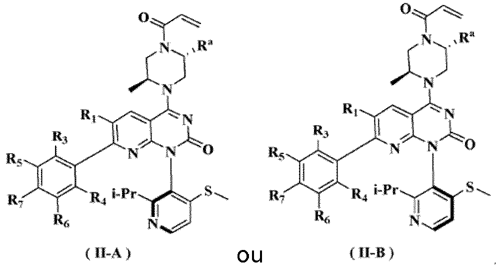

3. Sel acide du composé selon la revendication 1 ou la revendication 2, dans lequel le composé est choisi parmi :

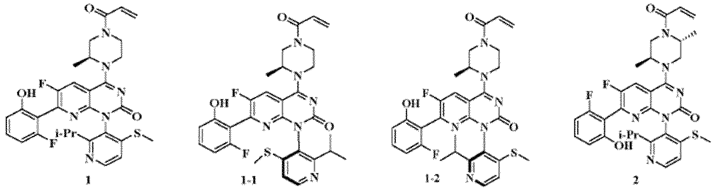

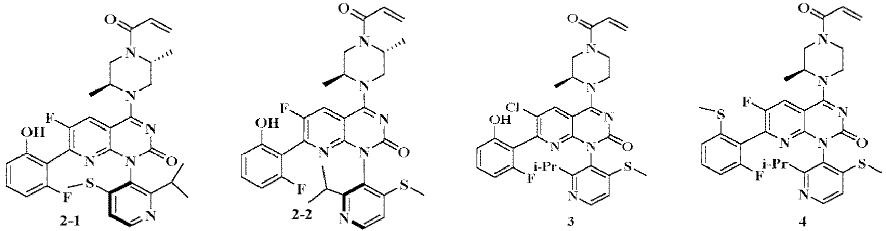

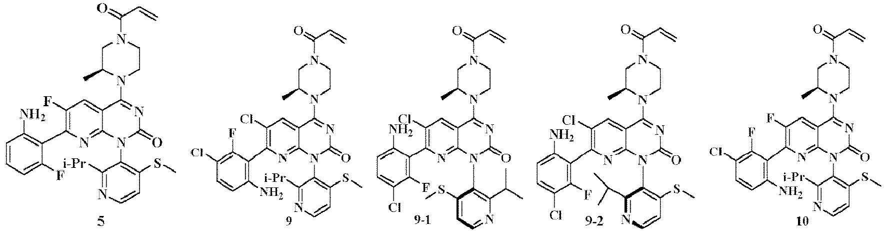

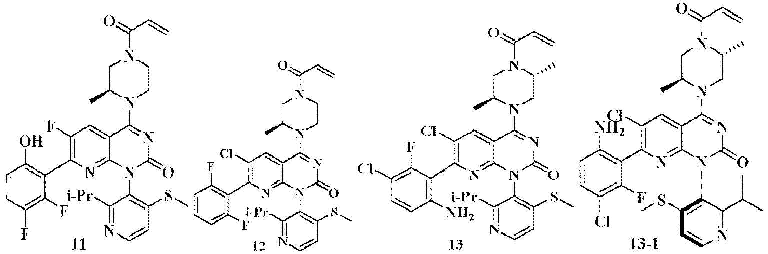

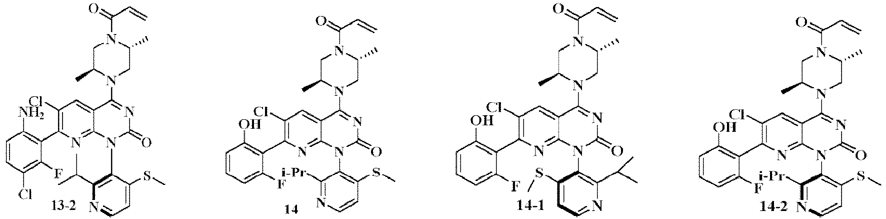

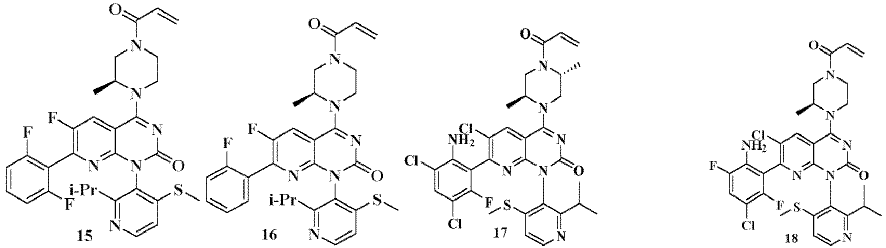

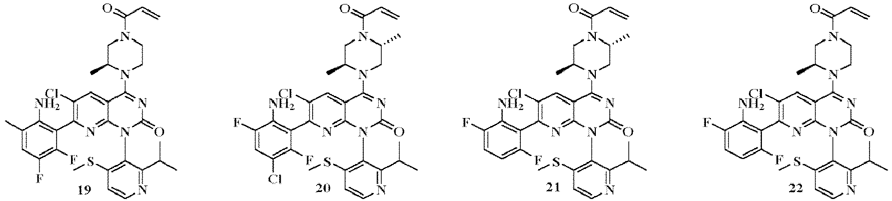

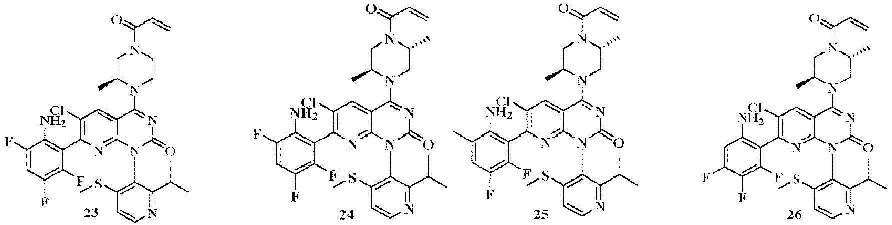

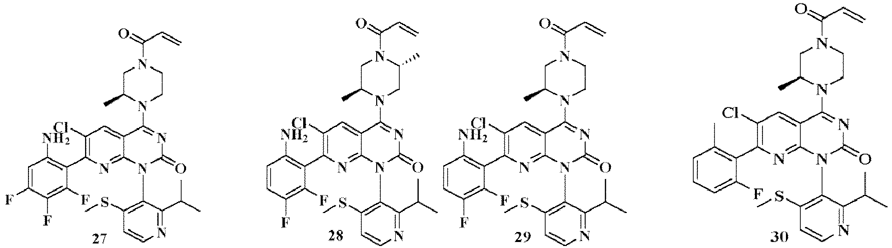

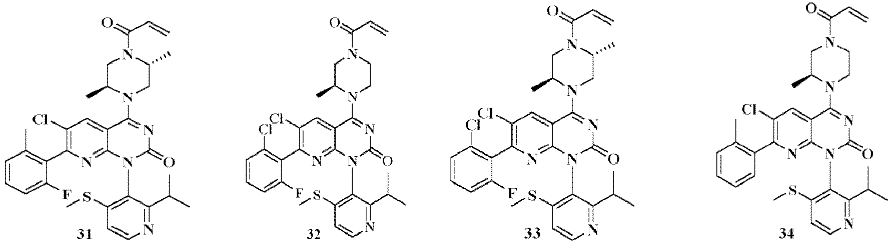

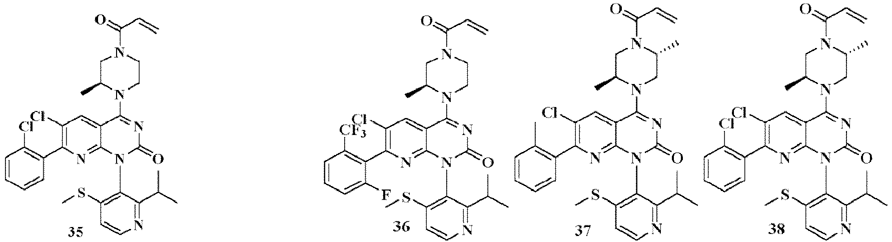

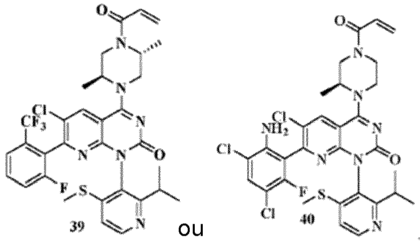

4. Sel acide du composé selon l'une quelconque des revendications 1 à 3, dans lequel le composé est
P-4-((2*S*,5*R*)-4-acryloyl-2,5-diméthylpipérazin-1-yl)-7-(6-amino-3-chloro-2-fluorophényl)-6-chloro-1-(2-isopropyl-4-(méthylthio)pyridin-3-yl)pyrido[2,3-d]pyrimidin-2(1H)-one ;
P-4-((*S*)-4-acryloyl-2-méthylpipérazin-1-yl)-6-fluoro-7-(2-fluoro-6-hydroxy phényl)-1-(2-isopropyl-4-(méthylthio)pyridin-3-yl)pyrido[2,3-*d*]pyrimidin-2(1*H*)-one ;
P-4-((*S*)-4-acryloyl-2-méthylpipérazin-1-yl)-7-(2-amino-6-fluorophényl)-6-fluoro-1-(2-isopropyl-4-(méthylthio)pyridin-3-yl)pyrido[2,3-*d*]pyrimidin-2(1*H*)-one ;
P-4-((2*S*,5*R*)-4-acryloyl-2,5-diméthylpipérazin-1-yl)-6-chloro-7-(2-fluoro-6-hydroxyphényl)-1-(2-isopropyl-4-(méthylthio)pyridin-3-yl)pyrido[2,3-d]pyrimidin-2(1*H*)- one;
P-4-((2*S*,5*R*)-4-acryloyl-2,5-diméthylpipérazin-1-yl)-6-fluoro-7-(2-fluoro-6-hydroxyphényl)-1-(2-isopropyl-4-(méthylthio)pyridin-3-yl)pyrido[2,3-d]pyrimidin-2(1*H*)-one ;
l'acide dans le sel acide est choisi parmi l'acide hydroxyéthylsulfonique, l'acide sulfurique, l'acide 1,5-naphtalène disulfonique, l'acide méthanesulfonique, l'acide bromhydrique, l'acide phosphorique, l'acide benzènesulfonique, l'acide oxalique, l'acide maléate, l'acide adipique, l'acide chlorhydrique, l'acide citrique, l'acide malonique, l'acide L-malique, l'acide pamoïque, l'acide p-toluènesulfonique ou l'acide fumarique, de préférence l'acide hydroxyéthylsulfonique ou l'acide sulfurique ; dans lequel le nombre de l'acide est, par exemple, 0,2-3 ; de préférence 0,2, 0,5, 1, 1,5, 2, 2,5 ou 3 ; plus préférentiellement 0,5, 1, 2 ou 3, de préférence encore 1 ; et/ou le sel acide est, par exemple, un hydrate ou un anhydrate ; et lorsque le sel acide est l'hydrate, le nombre d'eau est 0,2-3 ; de préférence 0,2, 0,5, 1, 1,5, 2, 2,5 ou 3 ; plus préférentiellement 0,5, 1, 2 ou 3.

5. Sel acide du composé selon l'une quelconque des revendications 1 à 4, dans lequel le sel acide est sous forme cristalline ;
de préférence une forme cristalline du sel acide du composé P-4-((2S,5R)-4-acryloyl-2,5-diméthylpipérazin-1-yl)-7-(6-amino-3-chloro-2-fluorophényl)-6-chloro-1-(2-isopropyl-4-(méthylthio)pyridin-3-yl)pyrido[2,3-*d*]pyrimidin-2(1*H*)-one ;
une forme cristalline du sel acide de P-4-((*S*)-4-acryloyl-2-méthylpipérazin-1-yl)-6-fluoro-7-(2-fluoro-6-hydroxyphényl)-1-(2-isopropyl-4-(méthylthio)pyridin-3-yl)pyrido[2,3-*d*]pyrimidin-2(1*H*)-one ;
une forme cristalline du sel acide de P-4-((*S*)-4-acryloyl-2-méthylpipérazin-1-yl)-7-(2-amino-6-fluorophényl)-6-fluoro-1-(2-isopropyl-4-(méthylthio)pyridin-3-yl)pyrido[2,3-*d*]pyrimidin-2(1*H*)-one ;
une forme cristalline du sel acide de P-4-((2S,5R)-4-acryloyl-2,5-diméthylpipérazin-1-yl)-6-chloro-7-(2-fluoro-6-hydroxyphényl)-1-(2-isopropyl-4-(méthylthio)pyridin-3-yl)pyrido[2,3-*d*]pyrimidin-2(1*H*)-one ;
une forme cristalline du sel acide de P-4-((2S,5R)-4-acryloyl-2,5-diméthylpipérazin-1-yl)-6-fluoro-7-(2-fluoro-6-hydroxyphényl)-1-(2-isopropyl-4-(méthylthio)pyridin-3-yl)pyrido[2,3-*d*]pyrimidin-2(1*H*)-one ;
plus préférentiellement une forme cristalline d'hydroxyéthylsulfonate, une forme cristalline de sulfate, une forme cristalline de 1,5-naphtalène disulfonate, une forme cristalline de méthanesulfonate, une forme cristalline de bromhydrate, une forme cristalline de phosphate, une forme cristalline de benzènesulfonate, une forme cristalline d'oxalate, une forme cristalline de maléate, une forme cristalline d'adipate, une forme cristalline d'hydrochlorure, une forme cristalline de citrate, une forme cristalline de malonate, une forme cristalline de L-malate, une forme cristalline de pamoate, une forme cristalline de p-toluènesulfonate ou une forme cristalline de fumarate.

6. Sel acide du composé selon la revendication 5, dans lequel
la forme cristalline du sel acide de P-4-((2S,5R)-4-acryloyl-2,5-diméthylpipérazin-1-yl)-7-(6-amino-3-chloro-2-fluorophényl)-6-chloro-1-(2-isopropyl-4-(méthylthio)pyridin-3-yl)pyrido[2,3-*d*]pyrimidin-2(1*H*)-one est :
une forme cristalline I d'hydroxyéthylsulfonate, la forme cristalline I d'hydroxyéthylsulfonate présente un diagramme de diffraction de rayons X sur poudre présentant un pic de diffraction à un angle 2θ de 21,7±0,2° ; ou présentant un pic de diffraction à 8,8±0,2° ; ou présentant un pic de diffraction à 19,3±0,2° ; ou présentant un pic de diffraction à 27,6±0,2° ; ou présentant un pic de diffraction à 10,9±0,2° ; ou présentant un pic de diffraction à 15,4±0,2° ; ou présentant un pic de diffraction à 16,7±0,2° ; ou présentant un pic de diffraction à 15,8±0,2° ; ou présentant un pic de diffraction à 17,5±0,2° ; ou présentant un pic de diffraction à 23,8±0,2° ; ou présentant un pic de diffraction à 10,2±0,2° ; ou présentant un pic de diffraction à 11,8±0,2° ; comprenant de préférence 2-5, ou 3-5, ou 3-6, ou 3-8, ou 5-8, ou 6-8 des pics de diffraction ci-dessus, comprenant plus préférentiellement 6, 7, ou 8 des pics de diffraction ;
une forme cristalline II d'hydroxyéthylsulfonate, la forme cristalline Il d'hydroxyéthylsulfonate présente un diagramme de diffraction de rayons X sur poudre présentant un pic de diffraction à un angle 2θ de 21,7±0,2° ; ou présentant un pic de diffraction à 8,8±0,2° ; ou présentant un pic de diffraction à 19,3±0,2° ; ou présentant un pic de diffraction à 27,6±0,2° ; ou présentant un pic de diffraction à 10,9±0,2° ; ou présentant un pic de diffraction à 23,8±0,2° ; ou présentant un pic de diffraction à 16,7±0,2° ; ou présentant un pic de diffraction à 15,4±0,2° ; ou présentant un pic de diffraction à 15,8±0,2° ; ou présentant un pic de diffraction à 10,0±0,2° ; comprenant de préférence 2-5, ou 3-5, ou 3-6, ou 3-8, ou 5-8, ou 6-8 des pics de diffraction ci-dessus, comprenant plus préférentiellement 6, 7, ou 8 des pics de diffraction ;
une forme cristalline III d'hydroxyéthylsulfonate, la forme cristalline III d'hydroxyéthylsulfonate présente un diagramme de diffraction de rayons X sur poudre présentant un pic de diffraction à un angle 2θ de 19,4±0,2° ; ou présentant un pic de diffraction à 16,9±0,2° ; ou présentant un pic de diffraction à 26,6±0,2° ; ou présentant un pic de diffraction à 14,6±0,2° ; ou présentant un pic de diffraction à 28,0±0,2° ; ou présentant un pic de diffraction à 25,6±0,2° ; ou présentant un pic de diffraction à 20,7±0,2° ; ou présentant un pic de diffraction à 12,8±0,2° ; ou présentant un pic de diffraction à 19,1±0,2° ; ou présentant un pic de diffraction à 27,2±0,2° ; comprenant de préférence 2-5, ou 3-5, ou 3-6, ou 3-8, ou 5-8, ou 6-8 des pics de diffraction ci-dessus, comprenant plus préférentiellement 6, 7, ou 8 des pics de diffraction ;
une forme cristalline I de sulfate, la forme cristalline I de sulfate présente un diagramme de diffraction de rayons X sur poudre présentant un pic de diffraction à un angle 2θ de 19,0±0,2° ; ou présentant un pic de diffraction à 19,4±0,2° ; ou présentant un pic de diffraction à 12,4±0,2° ; ou présentant un pic de diffraction à 26,2±0,2° ; ou présentant un pic de diffraction à 17,6±0,2° ; ou présentant un pic de diffraction à 18,1±0,2° ; ou présentant un pic de diffraction à 25,3±0,2° ; ou présentant un pic de diffraction à 8,8±0,2° ; ou présentant un pic de diffraction à 21,9±0,2° ; ou présentant un pic de diffraction à 11,5±0,2° ; comprenant de préférence 2-5, ou 3-5, ou 3-6, ou 3-8, ou 5-8, ou 6-8 des pics de diffraction ci-dessus, comprenant plus préférentiellement 6, 7, ou 8 des pics de diffraction ;
une forme cristalline II de sulfate, la forme cristalline II de sulfate présente un diagramme de diffraction de rayons X sur poudre présentant un pic de diffraction à un angle 2θ de 15,5±0,2° ; ou présentant un pic de diffraction à 11,1±0,2° ; ou présentant un pic de diffraction à 8,9±0,2° ; ou présentant un pic de diffraction à 19,3±0,2° ; ou présentant un pic de diffraction à 22,3±0,2° ; ou présentant un pic de diffraction à 23,6±0,2° ; ou présentant un pic de diffraction à 17,4±0,2° ; ou présentant un pic de diffraction à 27,3±0,2° ; ou présentant un pic de diffraction à 17,0±0,2° ; ou présentant un pic de diffraction à 27,9±0,2° ; comprenant de préférence 2-5, ou 3-5, ou 3-6, ou 3-8, ou 5-8, ou 6-8 des pics de diffraction ci-dessus, comprenant plus préférentiellement 6, 7, ou 8 des pics de diffraction ;
une forme cristalline III de sulfate, la forme cristalline III de sulfate présente un diagramme de diffraction de rayons X sur poudre présentant un pic de diffraction à un angle 2θ de 19,6±0,2° ; ou présentant un pic de diffraction à 18,0±0,2° ; ou présentant un pic de diffraction à 18,4±0,2° ; ou présentant un pic de diffraction à 16,8±0,2° ; ou présentant un pic de diffraction à 14,3±0,2° ; ou présentant un pic de diffraction à 11,8±0,2° ; ou présentant un pic de diffraction à 14,9±0,2° ; ou présentant un pic de diffraction à 25,7±0,2° ; ou présentant un pic de diffraction à 15,4±0,2° ; ou présentant un pic de diffraction à 23,5±0,2° ; comprenant de préférence 2-5, ou 3-5, ou 3-6, ou 3-8, ou 5-8, ou 6-8 des pics de diffraction ci-dessus, comprenant plus préférentiellement 6, 7, ou 8 des pics de diffraction ;
une forme cristalline IV de sulfate, la forme cristalline IV de sulfate présente un diagramme de diffraction de rayons X sur poudre présentant un pic de diffraction à un angle 2θ de 19,4±0,2° ; ou présentant un pic de diffraction à 18,9±0,2° ; ou présentant un pic de diffraction à 15,5±0,2° ; ou présentant un pic de diffraction à 8,8±0,2° ; ou présentant un pic de diffraction à 18,1±0,2° ; ou présentant un pic de diffraction à 24,9±0,2° ; ou présentant un pic de diffraction à 17,4±0,2° ; ou présentant un pic de diffraction à 12,3±0,2° ; ou présentant un pic de diffraction à 26,1±0,2° ; ou présentant un pic de diffraction à 14,5±0,2° ; comprenant de préférence 2-5, ou 3-5, ou 3-6, ou 3-8, ou 5-8, ou 6-8 des pics de diffraction ci-dessus, comprenant plus préférentiellement 6, 7, ou 8 des pics de diffraction.

7. Sel acide du composé selon la revendication 6, dans lequel :
le diagramme de diffraction de rayons X sur poudre de la forme cristalline I d'hydroxyéthylsulfonate comprend au moins un ou plusieurs pics de diffraction aux angles 2θ de 21,7±0,2°, 8,8±0,2°, 19,3±0,2°, comprend de préférence deux des pics de diffraction ci-dessus, comprend plus préférentiellement trois des pics de diffraction ; facultativement, comprend en outre au moins un pic de diffraction aux angles 2θ de 27,6±0,2°, 10,9±0,2°, 15,4±0,2°, 16,7±0,2°, 15,8±0,2°, 10,2±0,2°, 11,8±0,2°, comprend de préférence 2, 3, 4 ou 5 des pics de diffraction ci-dessus ; par exemple,
8,8±0,2°, 27,6±0,2° ;
21,7±0,2°, 8,8±0,2°, 10,9±0,2° ;
21,7±0,2°, 8,8±0,2°, 27,6±0,2°, 10,9±0,2° ;
15,8±0,2°, 8,8±0,2°, 27,6±0,2°, 10,9±0,2° ; 21,7±0,2°, 8,8±0,2°, 19,3±0,2°,
15,8±0,2°, 10,9±0,2°, 15,4±0,2° ;
10,9±0,2°, 8,8±0,2°, 10,2±0,2°, 27,6±0,2°, 10,9±0,2°, 15,8±0,2° ;
le diagramme de diffraction de rayons X sur poudre de la forme cristalline Il d'hydroxyéthylsulfonate comprend au moins un ou plusieurs pics de diffraction aux angles 2θ de 21,7±0,2°, 10,0±0,2°, 8,8±0,2°, comprend de préférence deux des pics de diffraction ci-dessus, comprend plus préférentiellement trois des pics de diffraction ; facultativement, comprend en outre au moins un pic de diffraction aux angles 2θ de 19,3±0,2°, 27,6±0,2°, 10,9±0,2°, 23,8±0,2°, 16,7±0,2°, comprend de préférence 2, 3, 4 ou 5 des pics de diffraction ci-dessus ; par exemple,
21,7±0,2°, 10,0±0,2° ;
21,7±0,2°, 10,0±0,2°, 19,3±0,2° ;
21,7±0,2°, 10,0±0,2°, 8,8±0,2°, 19,3±0,2° ;
21,7±0,2°, 10,0±0,2°, 8,8±0,2°, 16,7±0,2°, 27,6±0,2°, 10,9±0,2° ;
le diagramme de diffraction de rayons X sur poudre de la forme cristalline III d'hydroxyéthylsulfonate comprend au moins un ou plusieurs pics de diffraction aux angles 2θ de 19,4±0,2°, 16,9±0,2°, 26,6±0,2°, comprend de préférence deux des pics de diffraction ci-dessus, comprend plus préférentiellement trois des pics de diffraction ; facultativement, comprend en outre au moins un pic de diffraction aux angles 2θ de 14,6±0,2°, 28,0±0,2°, 25,6±0,2°, 20,7±0,2°, 12,8±0,2°, comprend de préférence 2, 3, 4 ou 5 des pics de diffraction ci-dessus ; par exemple,
19,4±0,2°, 16,9±0,2°, 26,6±0,2°, 14,6±0,2°, 28,0±0,2°, 25,6±0,2° ;
le diagramme de diffraction de rayons X sur poudre de la forme cristalline I de sulfate comprend au moins un ou plusieurs pics de diffraction aux angles 2θ de 19,0±0,2°, 19,4±0,2°, 12,4±0,2°, comprend de préférence deux des pics de diffraction ci-dessus, comprend plus préférentiellement trois des pics de diffraction ; facultativement, comprend en outre au moins un pic de diffraction aux angles 2θ de 26,2±0,2°, 17,6±0,2°, 18,1±0,2°, 25,3±0,2°, 8,8±0,2°, comprend de préférence 2, 3, 4 ou 5 des pics de diffraction ci-dessus ; par exemple,
19,0±0,2°, 19,4±0,2°, 12,4±0,2°, 26,2±0,2°, 17,6±0,2°, 18,1±0,2° ;
le diagramme de diffraction de rayons X sur poudre de la forme cristalline II de sulfate comprend au moins un ou plusieurs pics de diffraction aux angles 2θ de 15,5±0,2°, 11,1±0,2°, 8,9±0,2°, comprend de préférence deux des pics de diffraction ci-dessus, comprend plus préférentiellement trois des pics de diffraction ; facultativement, comprend en outre au moins un pic de diffraction aux angles 2θ de 19,3±0,2°, 22,3±0,2°, 23,6±0,2°, 17,4±0,2°, 27,3±0,2°, comprend de préférence 2, 3, 4 ou 5 des pics de diffraction ci-dessus ; par exemple,
15,5±0,2°, 11,1±0,2° ;
15,5±0,2°, 11,1±0,2°, 8,9±0,2° ;
15,5±0,2°, 11,1±0,2°, 8,9±0,2°, 19,3±0,2° ;
15,5±0,2°, 11,1±0,2°, 8,9±0,2°, 19,3±0,2°, 22,3±0,2°, 27,3±0,2° ;
le diagramme de diffraction de rayons X sur poudre de la forme cristalline III de sulfate comprend au moins un ou plusieurs pics de diffraction aux angles 2θ de 19,6±0,2°, 18,0±0,2°, 18,4±0,2°, comprend de préférence deux des pics de diffraction ci-dessus, comprend plus préférentiellement trois des pics de diffraction ; facultativement, comprend en outre au moins un pic de diffraction aux angles 2θ de 16,8±0,2°, 14,3±0,2°, 11,8±0,2°, 14,9±0,2°, 25,7±0,2°, comprend de préférence 2, 3, 4 ou 5 des pics de diffraction ci-dessus ; par exemple,
19,6±0,2°, 18,0±0,2°, 18,4±0,2°, 16,8±0,2°, 14,3±0,2°, 11,8±0,2° ;
le diagramme de diffraction de rayons X sur poudre de la forme cristalline IV de sulfate comprend au moins un ou plusieurs pics de diffraction aux angles 2θ de 19,4±0,2°, 18,9±0,2°, 15,5±0,2°, comprend de préférence deux des pics de diffraction ci-dessus, comprend plus préférentiellement trois des pics de diffraction ; facultativement, comprend en outre au moins un pic de diffraction aux angles 2θ de 8,8±0,2°, 18,1±0,2°, 24,9±0,2°, 17,4±0,2°, 12,3±0,2°, comprend de préférence 2, 3, 4 ou 5 des pics de diffraction ci-dessus ; par exemple,
19,4±0,2°, 18,9±0,2°, 15,5±0,2°, 8,8±0,2°, 18,1±0,2°, 24,9±0,2°.

8. Sel acide du composé selon les revendications 6 ou 7, dans lequel
le diagramme de diffraction de rayons X sur poudre de la forme cristalline I d'hydroxyéthylsulfonate comprend facultativement également un ou plusieurs pics de diffraction aux angles 2θ de 21,7±0,2°, 8,8±0,2°, 10,2±0,2°, 11,8±0,2°, 13,3±0,2°, 19,3±0,2°, 27,6±0,2°, 10,9±0,2°, 15,4±0,2°, 16,7±0,2°, 15,8±0,2°, 17,5±0,2°, 23,8±0,2°, comprend de préférence au moins 2-3, ou 4-5, ou 6-8 des pics de diffraction ci-dessus ; comprend de préférence encore 2, 3, 4, 5, 6, 7 ou 8 des pics de diffraction ; par exemple,
8,8±0,2°, 10,2±0,2°, 11,8±0,2°, 13,3±0,2°, 27,6±0,2°, 10,9±0,2°, 15,8±0,2°, 17,5±0,2° ;
21,7±0,2°, 8,8±0,2°, 19,3±0,2°, 27,6±0,2°, 10,9±0,2°, 17,5±0,2°, 16,7±0,2°, 15,8±0,2° ;
le diagramme de diffraction de rayons X sur poudre de la forme cristalline Il d'hydroxyéthylsulfonate comprend facultativement également un ou plusieurs pics de diffraction aux angles 2θ de 10,0±0,2°, 21,7±0,2°, 8,8±0,2°, 19,3±0,2°, 27,6±0,2°, 10,9+±0,2°, 23,8±0,2°, 16,7±0,2°, 15,4±0,2°, 15,8±0,2°, 10,0±0,2° ; comprend de préférence au moins 2-3, ou 4-5, ou 6-8 des pics de diffraction ci-dessus ; comprend de préférence encore 2, 3, 4, 5, 6, 7 ou 8 des pics de diffraction ; par exemple, 21,7±0,2°, 8,8±0,2°, 19,3±0,2°, 27,6±0,2°, 10,9+±0,2°, 23,8±0,2°, 16,7±0,2°, 15,4±0,2° ;
le diagramme de diffraction de rayons X sur poudre de la forme cristalline III d'hydroxyéthylsulfonate comprend facultativement également un ou plusieurs pics de diffraction aux angles 2θ de 19,4±0,2°, 16,9±0,2°, 26,6±0,2°, 14,6±0,2°, 28,0±0,2°, 25,6±0,2°, 20,7±0,2°, 12,8±0,2°, 19,1±0,2°, 27,2±0,2° ; comprend de préférence au moins 2-3, ou 4-5, ou 6-8 des pics de diffraction ci-dessus ; comprend de préférence encore 2, 3, 4, 5, 6, 7 ou 8 des pics de diffraction ; par exemple,
19,4±0,2°, 16,9±0,2°, 26,6±0,2°, 14,6±0,2°, 28,0±0,2°, 25,6±0,2°, 20,7±0,2°, 27,2±0,2° ;
le diagramme de diffraction de rayons X sur poudre de la forme cristalline I de sulfate comprend facultativement également un ou plusieurs pics de diffraction aux angles 2θ de 19,0±0,2°, 19,4±0,2°, 12,4±0,2°, 26,2±0,2°, 17,6±0,2°, 18,1±0,2°, 25,3±0,2°, 8,8±0,2°, 21,9±0,2°, 11,5±0,2° ; comprend de préférence au moins 2-3, ou 4-5, ou 6-8 des pics de diffraction ci-dessus ; comprend de préférence encore 2, 3, 4, 5, 6, 7 ou 8 des pics de diffraction ; par exemple,
19,0±0,2°, 19,4±0,2°, 12,4±0,2°, 26,2±0,2°, 25,3±0,2°, 8,8±0,2°, 21,9±0,2°, 11,5±0,2° ;
le diagramme de diffraction de rayons X sur poudre de la forme cristalline II de sulfate comprend facultativement également un ou plusieurs pics de diffraction aux angles 2θ de 15,5±0,2°, 11,1±0,2°, 8,9±0,2°, 19,3±0,2°, 22,3±0,2°, 23,6±0,2°, 17,4±0,2°, 27,3±0,2°, 17,0±0,2°, 27,9±0,2° ; comprend de préférence au moins 2-3, ou 4-5, ou 6-8 des pics de diffraction ci-dessus ; comprend de préférence encore 2, 3, 4, 5, 6, 7 ou 8 des pics de diffraction ; par exemple,
15,5±0,2°, 8,9±0,2°, 19,3±0,2°, 22,3±0,2°, 23,6±0,2°, 17,4±0,2°, 27,3±0,2°, 17,0±0,2° ;
le diagramme de diffraction de rayons X sur poudre de la forme cristalline III de sulfate comprend facultativement également un ou plusieurs pics de diffraction aux angles 2θ de 19,6±0,2°, 18,0±0,2°, 18,4±0,2°, 16,8±0,2°, 14,3±0,2°, 11,8±0,2°, 14,9±0,2°, 25,7±0,2°, 15,4±0,2°, 23,5±0,2° ; comprend de préférence au moins 2-3, ou 4-5, ou 6-8 des pics de diffraction ci-dessus ; comprend de préférence encore 2, 3, 4, 5, 6, 7 ou 8 des pics de diffraction ; par exemple,
19,6±0,2°, 18,0±0,2°, 18,4±0,2°, 16,8±0,2°, 14,3±0,2°, 11,8±0,2°, 14,9±0,2°, 23,5±0,2° ;
le diagramme de diffraction de rayons X sur poudre de la forme cristalline IV de sulfate comprend facultativement également un ou plusieurs pics de diffraction aux angles 2θ de 19,4±0,2°, 18,9±0,2°, 15,5±0,2°, 8,8±0,2°, 18,1±0,2°, 24,9±0,2°, 17,4±0,2°, 12,3±0,2°, 26,1±0,2°, 14,5±0,2° ; comprend de préférence au moins 2-3, ou 4-5, ou 6-8 des pics de diffraction ci-dessus ; comprend de préférence encore 2, 3, 4, 5, 6, 7 ou 8 des pics de diffraction ; par exemple,
19,4±0,2°, 18,9±0,2°, 15,5±0,2°, 8,8±0,2°, 18,1±0,2°, 24,9±0,2°, 17,4±0,2°, 12,3±0,2°.

9. Sel acide du composé selon la revendication 6, dans lequel
le diagramme de diffraction de rayons X sur poudre de la forme cristalline I d'hydroxyéthylsulfonate comprend un ou plusieurs pics de diffraction aux angles 2θ de 21,7±0,2°, 8,8±0,2°, 10,2±0,2°, 11,8±0,2°, 13,1±0,2°, 19,3±0,2°, 27,6±0,2°, 10,9±0,2°, 13,3±0,2°, 15,4±0,2°, 16,7±0,2°, 15,8±0,2°, 17,5±0,2°, 23,8±0,2°, 14,7±0,2°, 24,3±0,2°, 27,3±0,2°, 23,4±0,2°, 20,6±0,2°, 21,2±0,2°, comprend de préférence 4, 5, 6, 8 ou 10 des pics de diffraction ci-dessus ; par exemple,
8,8±0,2°, 19,3±0,2°, 27,6±0,2°, 20,6±0,2° ;
8,8±0,2°, 19,3±0,2°, 27,6±0,2°, 10,9±0,2°, 15,4±0,2°, 20,6±0,2° ;
21,7±0,2°, 8,8±0,2°, 19,3±0,2°, 27,6±0,2°, 10,9±0,2°, 15,4±0,2°, 16,7±0,2°, 20,6±0,2° ;
21,7±0,2°, 8,8±0,2°, 19,3±0,2°, 27,6±0,2°, 10,9±0,2°, 15,4±0,2°, 16,7±0,2°, 15,8±0,2°, 24,3±0,2°, 23,8±0,2° ;
8,8±0,2°, 10,2±0,2°, 11,8±0,2°, 13,1±0,2°, 27,6±0,2°, 10,9±0,2°, 13,3±0,2°, 21,2±0,2°, 15,8±0,2°, 17,5±0,2° ;
le diagramme de diffraction de rayons X sur poudre de la forme cristalline Il d'hydroxyéthylsulfonate comprend un ou plusieurs pics de diffraction aux angles 2θ de 21,7±0,2°, 10,0±0,2°, 8,8±0,2°, 19,3±0,2°, 27,6±0,2°, 10,9±0,2°, 23,8±0,2°, 16,7±0,2°, 15,4±0,2°, 15,8±0,2°, 17,5±0,2°, 14,7±0,2°, 24,4±0,2°, 27,3±0,2°, 29,2±0,2°, comprend de préférence 4, 5, 6, 8 ou 10 des pics de diffraction ci-dessus ; par exemple,
10,0±0,2°, 8,8±0,2°, 19,3±0,2°, 29,2±0,2° ;
21,7±0,2°, 10,0±0,2°, 8,8±0,2°, 19,3±0,2°, 27,6±0,2°, 29,2±0,2° ;
21,7±0,2°, 8,8±0,2°, 19,3±0,2°, 27,6±0,2°, 10,9±0,2°, 23,8±0,2°, 27,3±0,2°, 17,5±0,2° ;
10,0±0,2°, 8,8±0,2°, 19,3±0,2°, 27,6±0,2°, 10,9±0,2°, 23,8±0,2°, 16,7±0,2°, 15,4±0,2°, 15,8±0,2°, 17,5±0,2° ;
le diagramme de diffraction de rayons X sur poudre de la forme cristalline III d'hydroxyéthylsulfonate comprend un ou plusieurs pics de diffraction aux angles 2θ de 19,4±0,2°, 16,9±0,2°, 26,6±0,2°, 14,6±0,2°, 28,0±0,2°, 25,6±0,2°, 20,7±0,2°, 12,8±0,2°, 19,1±0,2°, 27,2±0,2°, 24,4±0,2°, 15,3±0,2°, 26,2±0,2°, 30,2±0,2°, 27,4±0,2°, comprend de préférence 4, 5, 6, 8 ou 10 des pics de diffraction ci-dessus ; par exemple,
19,4±0,2°, 16,9±0,2°, 26,6±0,2°, 14,6±0,2° ;
19,4±0,2°, 16,9±0,2°, 26,6±0,2°, 14,6±0,2°, 28,0±0,2°, 27,4±0,2° ;
19,4±0,2°, 16,9±0,2°, 26,6±0,2°, 14,6±0,2°, 28,0±0,2°, 25,6±0,2°, 20,7±0,2°, 27,4±0,2° ;
19,4±0,2°, 16,9±0,2°, 26,6±0,2°, 14,6±0,2°, 28,0±0,2°, 25,6±0,2°, 20,7±0,2°, 12,8±0,2°, 19,1±0,2°, 27,2±0,2° ;
le diagramme de diffraction de rayons X sur poudre de la forme cristalline I de sulfate comprend un ou plusieurs pics de diffraction aux angles 2θ de 19,0±0,2°, 19,4±0,2°, 12,4±0,2°, 26,2±0,2°, 17,6±0,2°, 18,1±0,2°, 25,3±0,2°, 8,8±0,2°, 21,9±0,2°, 11,5±0,2°, comprend de préférence 4, 5, 6, 8 ou 10 des pics de diffraction ci-dessus ; par exemple,
19,0±0,2°, 19,4±0,2°, 12,4±0,2°, 26,2±0,2° ;
19,0±0,2°, 19,4±0,2°, 12,4±0,2°, 26,2±0,2°, 17,6±0,2°, 11,5±0,2° ;
19,0±0,2°, 19,4±0,2°, 12,4±0,2°, 26,2±0,2°, 17,6±0,2°, 18,1±0,2°, 25,3±0,2°, 8,8±0,2° ;
19,0±0,2°, 19,4±0,2°, 12,4±0,2°, 26,2±0,2°, 17,6±0,2°, 18,1±0,2°, 25,3±0,2°, 8,8±0,2°, 21,9±0,2°, 11,5±0,2° ;
le diagramme de diffraction de rayons X sur poudre de la forme cristalline II de sulfate comprend un ou plusieurs pics de diffraction aux angles 2θ de 15,5±0,2°, 11,1±0,2°, 8,9±0,2°, 19,3±0,2°, 22,3±0,2°, 23,6±0,2°, 17,4±0,2°, 27,3±0,2°, 17,0±0,2°, 27,9±0,2°, 15,8±0,2°, 24,2±0,2°, 21,8±0,2°, 10,3±0,2°, 20,6±0,2°, comprend de préférence 4, 5, 6, 8 ou 10 des pics de diffraction ci-dessus ; par exemple,
11,1±0,2°, 8,9±0,2°, 19,3±0,2°, 21,8±0,2° ;
11,1±0,2°, 8,9±0,2°, 19,3±0,2°, 22,3±0,2°, 20,6±0,2°, 27,9±0,2° ;
15,5±0,2°, 11,1±0,2°, 8,9±0,2°, 22,3±0,2°, 23,6±0,2°, 17,4±0,2°, 20,6±0,2°, 27,9±0,2° ;
11,1±0,2°, 8,9±0,2°, 19,3±0,2°, 22,3±0,2°, 23,6±0,2°, 17,4±0,2°, 27,3±0,2°, 17,0±0,2°, 27,9±0,2°, 20,6±0,2° ;
le diagramme de diffraction de rayons X sur poudre de la forme cristalline III de sulfate comprend un ou plusieurs pics de diffraction aux angles 2θ de 19,6±0,2°, 18,0±0,2°, 18,4±0,2°, 16,8±0,2°, 14,3±0,2°, 11,8±0,2°, 14,9±0,2°, 25,7±0,2°, 15,4±0,2°, 23,5±0,2°, 18,8±0,2°, 24,7±0,2°, 9,5 ± 0,2°, 8,8 ± 0,2°, 11,1 ± 0,2°, comprend de préférence 4, 5, 6, 8 ou 10 des pics de diffraction ci-dessus ; par exemple,
19,6±0,2°, 18,0±0,2°, 18,4±0,2°, 16,8±0,2° ;
19,6±0,2°, 18,0±0,2°, 18,4±0,2°, 16,8±0,2°, 14,3±0,2°, 11,1±0,2° ;
19,6±0,2°, 18,0±0,2°, 18,4±0,2°, 16,8±0,2°, 14,3±0,2°, 11,8±0,2°, 14,9±0,2°, 11,1±0,2° ;
19,6±0,2°, 18,0±0,2°, 18,4±0,2°, 16,8±0,2°, 14,3±0,2°, 11,8±0,2°, 14,9±0,2°, 25,7±0,2°, 15,4±0,2°, 23,5±0,2° ;
le diagramme de diffraction de rayons X sur poudre de la forme cristalline IV de sulfate comprend un ou plusieurs pics de diffraction aux angles 2θ de 19,4±0,2°, 18,9±0,2°, 15,5±0,2°, 8,8±0,2°, 18,1±0,2°, 24,9±0,2°, 17,4±0,2°, 12,3±0,2°, 26,1±0,2°, 14,5±0,2°, 22,2±0,2°, 24,3±0,2°, 21,7±0,2°, 23,6±0,2°, comprend de préférence 4, 5, 6, 8 ou 10 des pics de diffraction ci-dessus ; par exemple,
19,4±0,2°, 18,9±0,2°, 15,5±0,2°, 8,8±0,2° ;
19,4±0,2°, 18,9±0,2°, 15,5±0,2°, 8,8±0,2°, 18,1±0,2°, 23,6±0,2° ;
19,4±0,2°, 18,9±0,2°, 15,5±0,2°, 8,8±0,2°, 18,1±0,2°, 24,9±0,2°, 17,4±0,2°, 23,6±0,2° ;
19,4±0,2°, 18,9±0,2°, 15,5±0,2°, 8,8±0,2°, 18,1±0,2°, 24,9±0,2°, 17,4±0,2°, 12,3±0,2°, 26,1±0,2°, 14,5±0,2°.

10. Sel acide du composé selon l'une quelconque des revendications 6 à 9, dans lequel
les positions des pics de diffraction avec une intensité de pic relative des dix les plus élevés dans le diagramme de diffraction de rayons X sur poudre de la forme cristalline I d'hydroxyéthylsulfonate et des pics de diffraction aux positions correspondantes sur la Figure 1 ont une erreur 2θ de ±0,2° à ±0,5°, de préférence de ±0,2° à ±0,3°, le plus préférentiellement de ±0,2° ;
les positions des pics de diffraction avec une intensité de pic relative des dix les plus élevés dans le diagramme de diffraction de rayons X sur poudre de la forme cristalline II d'hydroxyéthylsulfonate et des pics de diffraction aux positions correspondantes sur la Figure 4 ont une erreur 2θ de ±0,2° à ±0,5°, de préférence de ±0,2° à ±0,3°, le plus préférentiellement de ±0,2° ;
les positions des pics de diffraction avec une intensité de pic relative des dix les plus élevés dans le diagramme de diffraction de rayons X sur poudre de la forme cristalline III d'hydroxyéthylsulfonate et des pics de diffraction aux positions correspondantes sur la Figure 7 ont une erreur 2θ de ±0,2° à ±0,5°, de préférence de ±0,2° à ±0,3°, le plus préférentiellement de ±0,2° ;
les positions des pics de diffraction avec une intensité de pic relative des dix les plus élevés dans le diagramme de diffraction de rayons X sur poudre de la forme cristalline I de sulfate et des pics de diffraction aux positions correspondantes sur la Figure 10 ont une erreur 2θ de ±0,2° à ±0,5°, de préférence de ±0,2° à ±0,3°, le plus préférentiellement de ±0,2° ;
les positions des pics de diffraction avec une intensité de pic relative des dix les plus élevés dans le diagramme de diffraction de rayons X sur poudre de la forme cristalline Il de sulfate et des pics de diffraction aux positions correspondantes sur la Figure 11 ont une erreur 2θ de ±0,2° à ±0,5°, de préférence de ±0,2° à ±0,3°, le plus préférentiellement de ±0,2° ;
les positions des pics de diffraction avec une intensité de pic relative des dix les plus élevés dans le diagramme de diffraction de rayons X sur poudre de la forme cristalline III de sulfate et des pics de diffraction aux positions correspondantes sur la Figure 12 ont une erreur 2θ de ±0,2° à ±0,5°, de préférence de ±0,2° à ±0,3°, le plus préférentiellement de ±0,2° ;
les positions des pics de diffraction avec une intensité de pic relative des dix les plus élevés dans le diagramme de diffraction de rayons X sur poudre de la forme cristalline IV de sulfate et des pics de diffraction aux positions correspondantes sur la Figure 13 ont une erreur 2θ de ±0,2° à ±0,5°, de préférence de ±0,2° à ±0,3°, le plus préférentiellement de ±0,2°.

11. Sel acide du composé selon l'une quelconque des revendications 1 à 10, dans lequel la forme cristalline du sel acide est un hydrate ou un anhydrate, lorsque la forme cristalline du sel acide est l'hydrate, le nombre d'eau est de 0,2 à 3, de préférence 0,2, 0,5, 1, 1,5, 2, 2,5 ou 3, plus préférentiellement 0,5, 1, 2 ou 3 ; en outre, l'eau dans l'hydrate est de l'eau de canalisation, ou de l'eau cristalline, ou une combinaison des deux.

12. Procédé pour préparer le sel acide du composé selon l'une quelconque des revendications 1 à 11, comprenant les étapes suivantes :
1) le pesage d'une quantité appropriée d'une base libre, et l'ajout d'un solvant réactionnel pour dissoudre ;
2) l'ajout d'une quantité appropriée d'acide et l'agitation ; dans lequel une quantité de l'acide est de préférence de 1,2 équivalents ;
3) la centrifugation et le séchage pour obtenir le sel acide du composé ou une forme cristalline de celui-ci ; le solvant réactionnel est un solvant organique, de préférence au moins l'un parmi l'éthanol, le 2-méthyltétrahydrofurane, le n-heptane, le méthyl *tert*-butyl éther, le toluène, l'acétate d'isopropyle, le tert-butanol, le n-butanol, le tétrahydrofurane, l'acétone, le 2-butanone, l'acétate d'éthyle ou le 1,4-dioxane ;
l'acide est choisi parmi l'acide chlorhydrique, l'acide sulfurique, l'acide nitrique, l'acide bromhydrique, l'acide fluorhydrique, l'acide iodhydrique, l'acide phosphorique, l'acide 2,5-dihydroxybenzoïque, l'acide 1-hydroxy-2-naphthoïque, l'acide acétique, l'acide éthanesulfonique, l'acide dichloroacétique, l'acide trichloroacétique, l'acide acétohydroxamique, l'acide adipique, l'acide benzènesulfonique, l'acide 4-chlorobenzènesulfonique, l'acide benzoïque, l'acide 4-acétylaminobenzoïque, l'acide 4-aminobenzoïque, l'acide caprique, l'acide caproïque, l'acide octanoïque, l'acide cinnamique, l'acide citrique, l'acide cyclamique, l'acide sulfonique de camphre, l'acide aspartique, l'acide camphorique, l'acide gluconique, l'acide glucuronique, l'acide glutamique, l'acide isoascorbique, l'acide lactique, l'acide malique, l'acide mandélique, l'acide pyroglutamique, l'acide tartrique, l'acide dodécyl sulfurique, l'acide dibenzoyl tartrique, l'acide 1,2-éthanedisulfonique, l'acide éthanesulfonique, l'acide formique, l'acide fumarique, l'acide galactonique, l'acide gentisique, l'acide glutarique, l'acide 2-cétoglutarique, l'acide glycolique, l'acide hippurique, l'acide hydroxyéthylsulfonique, l'acide lactobionique, l'acide ascorbique, l'acide aspartique, l'acide laurique, l'acide camphorique, l'acide maléate, l'acide malonique, l'acide méthanesulfonique, l'acide 1,5-naphtalène disulfonique, l'acide naphtalène-2-sulfonique, l'acide nicotinique, l'acide oléique, l'acide orotique, l'acide oxalique, l'acide palmitique, l'acide pamoïque, l'acide propionique, l'acide salicylique, l'acide 4-aminosalicylique, l'acide sébacique, l'acide stéarique, l'acide succinique, l'acide thiocyanique, l'acide undécylénique, l'acide trifluoroacétique, l'acide benzènesulfonique, l'acide p-toluènesulfonique ou l'acide L-malique ; de préférence l'acide chlorhydrique, l'acide phosphorique, l'acide éthanesulfonique, l'acide benzènesulfonique, l'acide méthanesulfonique, l'acide fumarique, l'acide hydroxyéthylsulfonique, l'acide oxalique ou l'acide bromhydrique ; ou le procédé comprend les étapes suivantes :
1) le pesage d'une quantité appropriée d'une base libre, et l'ajout d'un solvant réactionnel pour dissoudre ;
2) l'ajout d'une quantité appropriée d'acide et d'un solvant organique, l'agitation et la dissolution pour clarifier ;
3) l'ajout, facultativement, d'un germe cristallin ;
4) le refroidissement, le filtrage pour précipiter un solide, et le lavage avec un solvant, le séchage ;
le solvant réactionnel utilisé à l'étape 1) est un solvant organique, de préférence au moins l'un parmi l'éthanol, le propanol, l'isopropanol, le 2-méthyltétrahydrofurane, le *n*-heptane, le méthyl *tert*-butyléther, le toluène, l'acétate d'isopropyle, le *tert-*butanol, le *n*-butanol, le tétrahydrofurane, l'acétone, le 2-butanone, l'acétate d'éthyle ou le 1,4-dioxane ;
l'acide de l'étape 2) est choisi parmi l'acide chlorhydrique, l'acide sulfurique, l'acide nitrique, l'acide bromhydrique, l'acide fluorhydrique, l'acide iodhydrique, l'acide phosphorique, l'acide 2,5-dihydroxybenzoïque, l'acide I-hydroxy-2-naphthoïque, l'acide acétique, l'acide éthanesulfonique, l'acide dichloroacétique, l'acide trichloroacétique, l'acide acétohydroxamique, l'acide adipique, l'acide benzènesulfonique, l'acide 4-chlorobenzènesulfonique, l'acide benzoïque, l'acide 4-acétylaminobenzoïque, l'acide 4-aminobenzoïque, l'acide caprique, l'acide caproïque, l'acide octanoïque, l'acide cinnamique, l'acide citrique, l'acide cyclamique, l'acide sulfonique de camphre, l'acide aspartique, l'acide camphorique, l'acide gluconique, l'acide glucuronique, l'acide glutamique, l'acide isoascorbique, l'acide lactique, l'acide malique, l'acide mandélique, l'acide pyroglutamique, l'acide tartrique, l'acide dodécyl sulfurique, l'acide dibenzoyl tartrique, l'acide 1,2-éthanedisulfonique, l'acide éthanesulfonique, l'acide formique, l'acide fumarique, l'acide galactonique, l'acide gentisique, l'acide glutarique, l'acide 2-cétoglutarique, l'acide glycolique, l'acide hippurique, l'acide hydroxyéthylsulfonique, l'acide lactobionique, l'acide ascorbique, l'acide aspartique, l'acide laurique, l'acide camphorique, l'acide maléate, l'acide malonique, l'acide méthanesulfonique, l'acide 1,5-naphtalène disulfonique, l'acide naphtalène-2-sulfonique, l'acide nicotinique, l'acide oléique, l'acide orotique, l'acide oxalique, l'acide palmitique, l'acide pamoïque, l'acide propionique, l'acide salicylique, l'acide 4-aminosalicylique, l'acide sébacique, l'acide stéarique, l'acide succinique, l'acide thiocyanique, l'acide undécylénique, l'acide trifluoroacétique, l'acide benzènesulfonique, l'acide p-toluènesulfonique ou l'acide L-malique ; de préférence l'acide chlorhydrique, l'acide phosphorique, l'acide éthanesulfonique, l'acide benzènesulfonique, l'acide méthanesulfonique, l'acide fumarique, l'acide hydroxyéthylsulfonique, l'acide oxalique ou l'acide bromhydrique ;
le solvant organique à l'étape 2) est choisi parmi un ou plusieurs parmi les solvants alcool, éther, cétone ou ester, de préférence au moins l'un parmi l'éthanol, le propanol, l'isopropanol, le 2-méthyltétrahydrofurane, le *n*-heptane, le méthyl *tert*-butyl éther, le toluène, l'acétate d'isopropyle, le *tert*-butanol, le *n*-butanol, le tétrahydrofurane, l'acétone, le 2-butanone, l'acétate d'éthyle ou le 1,4-dioxane ;
le solvant à l'étape 4) est choisi parmi un ou plusieurs parmi les solvants alcool, éther, cétone ou ester, de préférence au moins l'un parmi l'éthanol, le propanol, l'isopropanol, le 2-méthyltétrahydrofurane, le *n*-heptane, le méthyl *tert*-butyl éther, le toluène, l'acétate d'isopropyle, le *tert*-butanol, le *n*-butanol, le tétrahydrofurane, l'acétone, le 2-butanone, l'acétate d'éthyle ou le 1,4-dioxane.

13. Composition pharmaceutique comprenant une quantité thérapeutiquement efficace du sel acide du composé selon l'une quelconque des revendications 1 à 11, et un ou plusieurs supports, diluants ou excipients pharmaceutiquement acceptables.

14. Sel acide du composé selon l'une quelconque des revendications 1 à 11, ou composition pharmaceutique selon la revendication 13 pour son utilisation en tant que médicament pour inhiber le gène KRAS, de préférence pour son utilisation en tant que médicament pour inhiber le gène KRAS avec mutation G12C.

15. Sel acide du composé selon l'une quelconque des revendications 1 à 11, ou composition pharmaceutique selon la revendication 13 pour son utilisation dans le traitement de maladies ou d'affections telles que le syndrome de Noonan, le syndrome léopard, la leucémie, le neuroblastome, le mélanome, le cancer de l'œsophage, la tumeur de la tête et du cou, le cancer du sein, le cancer du poumon et le cancer du côlon ; de préférence le cancer du poumon non à petites cellules, le cancer du côlon, le cancer de l'œsophage et la tumeur de la tête et du cou.
